(19) Europäisches Patentamt
European Patent Office
Office européen des brevets

(11) EP 2 189 534 B1

(12) EUROPEAN PATENT SPECIFICATION

(45) Date of publication and mention of the grant of the patent:
**04.11.2015 Bulletin 2015/45**

(51) Int Cl.:
*C12N 15/82* (2006.01) *A01H 5/00* (2006.01)

(21) Application number: **09178866.1**

(22) Date of filing: **02.08.2007**

(54) **Plants transformed with SYT-polypeptide having increased yield under abiotic stress and a method for making the same**

Pflanzen mit SYT-Polypeptid transformiert, die unter abiotischem Stress einen erhöhten Ertrag aufweisen und Methoden zur Herstellung dieser Pflanzen

Plantes transformées avec un polypeptide-SYT qui sous un stress abiotique, présentant un rendement amelioré, et leur procédé de production

(84) Designated Contracting States:
**AT BE BG CH CY CZ DE DK EE ES FI FR GB GR HU IE IS IT LI LT LU LV MC MT NL PL PT RO SE SI SK TR**

(30) Priority:
**12.10.2006 US 851258 P**
**01.12.2006 US 868095 P**
**12.10.2006 US 851250 P**
**12.10.2006 US 851265 P**
**17.11.2006 US 859717 P**
**10.08.2006 US 836804 P**
**06.10.2006 EP 06121928**
**27.10.2006 EP 06123066**
**31.10.2006 EP 06123237**
**05.10.2006 EP 06121856**
**02.08.2006 EP 06118347**
**20.09.2006 EP 06120994**

(43) Date of publication of application:
**26.05.2010 Bulletin 2010/21**

(62) Document number(s) of the earlier application(s) in accordance with Art. 76 EPC:
**07788181.1 / 2 054 516**

(73) Proprietor: **CropDesign N.V.**
**9052 Zwijnaarde (BE)**

(72) Inventors:
- **Sanz Molinero, Ana Isabel**
  **9050 Gentbrugge (BE)**
- **Reuzeau, Christophe**
  **24350 Tocan Saint Apre (FR)**
- **Frankard, Valerie**
  **1410 Waterloo (BE)**
- **Mironov, Vladimir**
  **9000 Gent (BE)**

(56) References cited:
**WO-A2-2006/079655 US-A1- 2004 034 888**

- **HORIGUCHI GOROU ET AL: "The transcription factor AtGRF5 and the transcription coactivator AN3 regulate cell proliferation in leaf primordia of Arabidopsis thaliana" PLANT JOURNAL, vol. 43, no. 1, July 2005 (2005-07), pages 68-78, XP002410132 ISSN: 0960-7412**
- **JEONG HOE KIM ET AL: "THE ATGRF FAMILY OF PUTATIVE TRANSCRIPTION FACTORS US INVOLVED IN LEAF AND COTYLEDON GROWTH IN ARABIDOPSIS" PLANT JOURNAL, BLACKWELL SCIENTIFIC PUBLICATIONS, OXFORD, GB, vol. 36, no. 1, October 2003 (2003-10), pages 94-104, XP008058318 ISSN: 0960-7412**
- **KIM JEONG HOE ET AL: "A transcriptional coactivator, AtGIF1, is involved in regulating leaf growth and morphology in Arabidopsis" PROCEEDINGS OF THE NATIONAL ACADEMY OF SCIENCES OF USA, NATIONAL ACADEMY OF SCIENCE, WASHINGTON, DC, US, vol. 101, no. 36, 7 September 2004 (2004-09-07), pages 13374-13379, XP002362467 ISSN: 0027-8424**
- **PATENT ABSTRACTS OF JAPAN vol. 2003, no. 12, 5 December 2003 (2003-12-05) & JP 2004 350553 A (JAPAN SCIENCE & TECHNOLOGY AGENCY), 16 December 2004 (2004-12-16)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KIM J-H ET AL: "STUDIES ON THE FUNCTION OF OSGRF1-LIKE (ATGRL) GENES IN ARABIDOPSIS" PLANT BIOLOGY, NEW YORK, NY, US, August 2002 (2002-08), page 56, XP008058260
- GOROU HORIGUCHI ET AL: "Coordination of cell proliferation and cell expansion in the control of leaf size in Arabidopsis thaliana" JOURNAL OF PLANT RESEARCH, SPRINGER-VERLAG, TO LNKD- DOI:10.1007/S10265-005-0232-4, vol. 119, no. 1, 1 January 2006 (2006-01-01), pages 37-42, XP019375380 ISSN: 1618-0860

SEQUENCE LISTING

<110> CropDesign N.V.

<120> Plants having increased yield and method for making the same

<130> PF58401-prio

<160> 153

<170> PatentIn version 3.3

<210> 1
<211> 2151
<212> DNA
<213> Arabidopsis thaliana

<400> 1

```
atgtgctgtg gatcagaccg attaaaccag atcgtgtcat caagatcttc gttgccaatt      60
tctttcgagg aagataacaa tcttgttacc aacacagaca tgaatcactt aacagtcgaa     120
acagaggata cgtttgcgag cttgcttgag cttgcagcta acaacgatgt tgaaggtgta     180
aggctatcta tcgagagaga cccttcttgt gtagacgaag ctggtctctg gtacggtcgt     240
caaaaaggtt ctaaagctat ggtcaacgat tacaggactc cgttgatggt tgctgctact     300
tacggaagca ttgatgtgat caagcttatt gtttctttga ctgatgctga cgtgaaccgt     360
gcttgcggga atgatcagac cactgcgtta cactgcgctg cttctggagg agctgtgaat     420
gctatccaag ttgttaagct gcttcttgca gctggagctg atttgaatct gttggatgct     480
gaaggtcaac gagctggtga tgttattgtt gttcctccta agcttgaagg cgtgaagctg     540
atgcttcagg agcttctttc tgctgatgga tcatctactg cggagcggaa tctacgggtt     600
gtgacaaatg ttccgaatag aagctcatct ccgtgtcatt ctcctactgg agagaatggt     660
ggatcagggt ctggttcacc gctcggctct ccttttaagc tgaaatctac tgaattcaag     720
aaagagtatc cggttgatcc gtctttgcca gatatcaaga acagtatcta cgcgactgat     780
gagtttagaa tgtattcctt caaggtccgg ccttgctctc gtgcttattc acatgattgg     840
actgagtgtc cttttgttca cccgggtgaa aacgcgagga ggagagaccc gaggaagttc     900
cattacagct gcgttccttg cccggatttt aggaaaggag cttgtaggag aggagatatg     960
tgtgagtatg cgcacggtgt gtttgaatgc tggcttcatc cggctcagta caggacccgt    1020
ctttgcaaag atggaacagg ctgtgctcgg cgggtttgtt tctttgcgca tacacccgag    1080
gagcttcgac ctttgtacgc atcaactggt tcagcggttc cttcgcctag atcgaatgct    1140
gattatgcag ctgctttgag tctccttcct ggttctccat caggagtctc tgtcatgtcc    1200
ccgctttccc catcagcagc ggggaacgga atgtctcatt cgaatatggc ttggccacaa    1260
ccaaatgtcc ctgcgttgca cttaccagga agcaatctac agtcaagcag gctaaggtct    1320
tctctcaatg caagggatat cccgacggat gagttcaata tgttagcgga ttacgagcag    1380
cagcaactcc tcaacgagta ttccaatgct ctgagccgtt ctggtcggat gaaatcaatg    1440
cctccttcga atcttgaaga tcttttctca gcagaaggct cttcatctcc ccggttcact    1500
gattccgctt tagcttccgc ggtgttctcg cctacacaca agtcagctgt cttcaaccag    1560
ttccaacaac agcaacagca gcagcagagc atgttgtctc caatcaacac aagcttttct    1620
tcaccaaaga gcgttgacca ctcattgttt tcaggtggag gaagaatgtc tcctcggaat    1680
gttgttgaac caatatcacc catgagtgct cgggtttcca tgttggctca gtgcgtgaag    1740
caacaacaac agcaacagca gcagcagcag cagcaacatc agttccgtag ccttagctcc    1800
agagagctca gaacaaactc tagcccaatc gttggttcac cggtaaacaa caacacatgg    1860
tcatcaaaat ggggatcttc aaatggtcaa ccggattggg gaatgagctc agaagcactt    1920
ggtaagttga gatcttcgtc atcgtttgat ggtgatgagc ctgatgtgtc atgggtccag    1980
tcactggtga aggagactcc agcagaagcc aaagagaaag cagcaacatc ttcctcaggg    2040
gaacacgtga tgaagcagcc aaatccggtt gaaccggtaa tggatcatgc tgggctagaa    2100
gcttggattg agcaaatgca gctcgatcag cttgtggctc agcagaattg a             2151
```

<210> 2
<211> 716
<212> PRT
<213> Arabidopsis thaliana

<400> 2

```
Met Cys Cys Gly Ser Asp Arg Leu Asn Gln Ile Val Ser Ser Arg Ser
1               5                   10                  15
Ser Leu Pro Ile Ser Phe Glu Glu Asp Asn Asn Leu Val Thr Asn Thr
            20                  25                  30
Asp Met Asn His Leu Thr Val Glu Thr Glu Asp Thr Phe Ala Ser Leu
        35                  40                  45
Leu Glu Leu Ala Ala Asn Asn Asp Val Glu Gly Val Arg Leu Ser Ile
    50                  55                  60
Glu Arg Asp Pro Ser Cys Val Asp Glu Ala Gly Leu Trp Tyr Gly Arg
65                  70                  75                  80
Gln Lys Gly Ser Lys Ala Met Val Asn Asp Tyr Arg Thr Pro Leu Met
                85                  90                  95
Val Ala Ala Thr Tyr Gly Ser Ile Asp Val Ile Lys Leu Ile Val Ser
            100                 105                 110
Leu Thr Asp Ala Asp Val Asn Arg Ala Cys Gly Asn Asp Gln Thr Thr
        115                 120                 125
Ala Leu His Cys Ala Ala Ser Gly Gly Ala Val Asn Ala Ile Gln Val
    130                 135                 140
Val Lys Leu Leu Leu Ala Ala Gly Ala Asp Leu Asn Leu Leu Asp Ala
145                 150                 155                 160
Glu Gly Gln Arg Ala Gly Asp Val Ile Val Val Pro Pro Lys Leu Glu
                165                 170                 175
Gly Val Lys Leu Met Leu Gln Glu Leu Leu Ser Ala Asp Gly Ser Ser
            180                 185                 190
Thr Ala Glu Arg Asn Leu Arg Val Val Thr Asn Val Pro Asn Arg Ser
        195                 200                 205
Ser Ser Pro Cys His Ser Pro Thr Gly Glu Asn Gly Gly Ser Gly Ser
    210                 215                 220
Gly Ser Pro Leu Gly Ser Pro Phe Lys Leu Lys Ser Thr Glu Phe Lys
225                 230                 235                 240
Lys Glu Tyr Pro Val Asp Pro Ser Leu Pro Asp Ile Lys Asn Ser Ile
                245                 250                 255
Tyr Ala Thr Asp Glu Phe Arg Met Tyr Ser Phe Lys Val Arg Pro Cys
            260                 265                 270
Ser Arg Ala Tyr Ser His Asp Trp Thr Glu Cys Pro Phe Val His Pro
        275                 280                 285
Gly Glu Asn Ala Arg Arg Arg Asp Pro Arg Lys Phe His Tyr Ser Cys
    290                 295                 300
Val Pro Cys Pro Asp Phe Arg Lys Gly Ala Cys Arg Arg Gly Asp Met
305                 310                 315                 320
Cys Glu Tyr Ala His Gly Val Phe Glu Cys Trp Leu His Pro Ala Gln
                325                 330                 335
Tyr Arg Thr Arg Leu Cys Lys Asp Gly Thr Gly Cys Ala Arg Arg Val
            340                 345                 350
Cys Phe Phe Ala His Thr Pro Glu Glu Leu Arg Pro Leu Tyr Ala Ser
        355                 360                 365
Thr Gly Ser Ala Val Pro Ser Pro Arg Ser Asn Ala Asp Tyr Ala Ala
    370                 375                 380
Ala Leu Ser Leu Leu Pro Gly Ser Pro Ser Gly Val Ser Val Met Ser
385                 390                 395                 400
Pro Leu Ser Pro Ser Ala Ala Gly Asn Gly Met Ser His Ser Asn Met
                405                 410                 415
Ala Trp Pro Gln Pro Asn Val Pro Ala Leu His Leu Pro Gly Ser Asn
            420                 425                 430
Leu Gln Ser Ser Arg Leu Arg Ser Ser Leu Asn Ala Arg Asp Ile Pro
        435                 440                 445
Thr Asp Glu Phe Asn Met Leu Ala Asp Tyr Glu Gln Gln Gln Leu Leu
```

```
    450                 455                 460
Asn Glu Tyr Ser Asn Ala Leu Ser Arg Ser Gly Arg Met Lys Ser Met
465                 470                 475                 480
Pro Pro Ser Asn Leu Glu Asp Leu Phe Ser Ala Glu Gly Ser Ser Ser
                485                 490                 495
Pro Arg Phe Thr Asp Ser Ala Leu Ala Ser Ala Val Phe Ser Pro Thr
            500                 505                 510
His Lys Ser Ala Val Phe Asn Gln Phe Gln Gln Gln Gln Gln Gln Gln
        515                 520                 525
Gln Ser Met Leu Ser Pro Ile Asn Thr Ser Phe Ser Ser Pro Lys Ser
    530                 535                 540
Val Asp His Ser Leu Phe Ser Gly Gly Gly Arg Met Ser Pro Arg Asn
545                 550                 555                 560
Val Val Glu Pro Ile Ser Pro Met Ser Ala Arg Val Ser Met Leu Ala
                565                 570                 575
Gln Cys Val Lys Gln Gln Gln Gln Gln Gln Gln Gln Gln Gln Gln Gln
            580                 585                 590
His Gln Phe Arg Ser Leu Ser Ser Arg Glu Leu Arg Thr Asn Ser Ser
        595                 600                 605
Pro Ile Val Gly Ser Pro Val Asn Asn Asn Thr Trp Ser Ser Lys Trp
    610                 615                 620
Gly Ser Ser Asn Gly Gln Pro Asp Trp Gly Met Ser Ser Glu Ala Leu
625                 630                 635                 640
Gly Lys Leu Arg Ser Ser Ser Ser Phe Asp Gly Asp Glu Pro Asp Val
                645                 650                 655
Ser Trp Val Gln Ser Leu Val Lys Glu Thr Pro Ala Glu Ala Lys Glu
            660                 665                 670
Lys Ala Ala Thr Ser Ser Ser Gly Glu His Val Met Lys Gln Pro Asn
        675                 680                 685
Pro Val Glu Pro Val Met Asp His Ala Gly Leu Glu Ala Trp Ile Glu
    690                 695                 700
Gln Met Gln Leu Asp Gln Leu Val Ala Gln Gln Asn
705                 710                 715
```

<210> 3
<211> 13
<212> PRT
<213> Artificial sequence

<220>
<223> motif 1

<220>
<221> VARIANT
<222> (1)..(1)
<223> \replace = "Ala"

<220>
<221> VARIANT
<222> (7)..(7)
<223> \replace = "Thr"

<400> 3

```
Pro Cys Ser Arg Ala Tyr Ser His Asp Trp Thr Glu Cys
1               5                   10
```

<210> 4

<211> 12
<212> PRT
<213> Artificial sequence

<220>
<223> motif 2

<400> 4

```
His Pro Gly Glu Asn Ala Arg Arg Arg Asp Pro Arg
1               5                   10
```

<210> 5
<211> 19
<212> > PRT
<213> Artificial sequence

<220>
<223> motif 3

<220>
<221> VARIANT
<222> (3)..(3)
<223> \replace = "Ile"

<220>
<221> VARIANT
<222> (6)..(6)
<223> \replace = "Ser"

<220>
<221> VARIANT
<222> (11)..(11)
<223> \replace = "Ser"

<220>
<221> VARIANT
<222> (14)..(14)
<223> \replace = "Lys"

<400> 5

```
His Gly Val Phe Glu Cys Trp Leu His Pro Ala Gln Tyr Arg Thr Arg
1               5                   10                  15
Leu Cys Lys
```

<210> 6
<211> 5
<212> PRT
<213> Artificial sequence

<220>
<223> motif 4

<400> 6

Cys Phe Phe Ala His
1 5

<210> 7
<211> 54
<212> DNA
<213> Artificial sequence

<220>
<223> primer: prm06717

<400> 7
ggggacaagt ttgtacaaaa aagcaggctt aaacaatgtg ctgtggatca gacc 54

<210> 8
<211> 50
<212> DNA
<213> Artificial sequence

<220>
<223> primer: prm06718

<400> 8
ggggaccact ttgtacaaga aagctgggtg gttaggtctc tcaattctgc 50

<210> 9
<211> 981
<212> DNA
<213> Oryza sativa

<400> 9

```
aagaggcaag agcatccgta ttaaccagcc ttttgagact tgagagtgtg tgtgactcga      60
tccagcgtag tttcagttcg tgtgttggtg agtgattcca gccaagtttg cgatggcttc     120
tcagcaggaa cgggctagct accacgccgg cgagaccaag gcccgcgccg aggagaagac     180
ggggcgcatg atgggcacgg cgcaggagaa ggcgcgggag gccaaggaca cggcgtccga     240
cgccgcgggg cgcgcgatgg gcaggggaca cggcgccaag gaggcgacca aggagaaggc     300
gtacgagacc aaggacgcga ccaaggagaa ggcgtacgag gcaaaggacg cggcctccga     360
cgccaccggc cgcgccatgg acaagggccg cggcgccgcg ggcgccacga gggacaaggc     420
gtacgatgcc aaggacaggg cggctgacac ggcgcagtcc gccgccgacc gcgcccgcga     480
cggcgccggg cagaccggga gctacattgg acagaccgcc gaggccgcca agcagaaagc     540
ggccggcgcc gcgcagtacg ccaaggagac cgcgatcgcc ggcaaggaca agaccggcgc     600
cgtgctccag caggcagggg agcaggtgaa gagcgtggcg gtgggggcga aggacgcggt     660
gatgtacacg ctcgggatgt caggcgataa caagaacaac gccgctgccg gcaaggacac     720
cagcacctac aagcctggaa ctgggagtga ctaccagtaa tacggtagaa gaagcatgtg     780
tcgtctttgg cactgatgcc aaagtgtacg tgttgtatcc tcttttttaa gtttcagctc     840
gacttcgacg tgttcggtgt cacactttgg tttttcagtt gtgctcaact gttcatgttt     900
ctggttccat ggagggccag tgtggaggtc aatgtttaag ctttcgtttt aaaatctgat     960
aataaagttg gttaagacct g                                               981
```

<210> 10
<211> 3372
<212> DNA
<213> Eucalyptus grandis

<400> 10

```
ggaagacgaa gagcaacaaa ataggtctca ctccctcctc tctcctctct cctctctctt      60
ctttctctct cttctgcttc taacaaagtc tcttccttga gagacagggc tgcgtcgtcg     120
tctttctctc tcctcgctgc gagcttctga gaaagttcaa ttctttttct cttgttctct     180
ctctctaccc ttctgggtac cactgtgaag ctccggtctt ttctattttt tttttttttg     240
ggctatctgg gtctgggcaa atccatcgcg cgctctgctc tggactgaga ggccgtcagt     300
ggctttagat ctgcgacgcc tcttgcttgc tcagtgagct gggctagttc aaatcgacga     360
agaaagcatg cgctagtgat tggtgtgggt aatacactgc attcgatctc tactaagtat     420
ccccaagtat actaagatcc cgttctcagc catgagtcaa ctgaccattc agactgagga     480
cacttttgcc agcttgcttg agcttgctgc taacaacgac acagaatctt tcggacggtg     540
tgtggaacgt gatccttcga gcatagatga aattggatat tggtatggtc gccaaaaggg     600
ttcgaagcag gtggtcaata tgcaaagaac tcctcttatg gtggctgcta catatggtag     660

tgttgatgta atgagactca ttctttgcct atctgatgct gatgtgaatc gaacctgcag     720
cacagacaag agcacagccc ttcactgtgc tgcctctggt ggtgctgtga atgctgtaga     780
tgctgtgagg ctactcctgt cagctggtgc tgacccaagt ttagcagatg ctaacggtca     840
gcggcctgtg gatgttattg ttgttcctcc aaagctcctt tcaataaagt ttgctcttga     900
agagctcttg tcgaccgaag gatctgtaaa tgaacacaat ctgagagtgt ccgtagccac     960
ttccaattca acctctcccc cactttcatc ttccccggat aatggttccc cagcatctgc    1020
taattgttct tcccccaaga actcaaagtt aagtgatgcc cctgttcttt atgcatcaga    1080
aaagaaggaa tacccggtgg atccatctct tccagatatc aagaatagca tttactcaac    1140
agatgaattc cgaatgtatt cttttaaagt gcggccttgt tcacgagcgt actcgcatga    1200
ttggacggag tgccctttg ttcatccagg ggagaatgcc cgtagaaggg atccaaggaa    1260
gttccactac agctgtgtcc cttgccctga tttccggaag ggtgcttgta gacgtggaga    1320
tatgtgtgaa tatgctcatg gtgtttttga gtgctggctc catcctgctc agtatcggac    1380
tcgattatgc aaggatggta caagttgtgc tcggagagtg tgcttctttg cccacacgga    1440
gcaagagctg cgtccattgt acgtctccac tggttctgct gttccgtctc ctcgctcgag    1500
tacctctgga gctgctgcca tggattttgc tgcagccatg agcctcttac ctggttcccc    1560
atcatcagta tccatcatgt ccccttcacc cttcactcct cccatgtctc catctgctaa    1620
tggtatttct cacccatctg ttgcctggcc ccagcaaaat gtaccaactt tgcatcttcc    1680
cggaagcaat cttcagtcca gccgcttgag atcttctctt aatgcaagag atattcctca    1740
ggaggatttt gacttgctgt cagattatga tgtgcaacag cagcagctcc taaatgagtt    1800
ttccatcctt tcacaacaat cgatgggtgc taattccttg aaccgttctg gtcggctgaa    1860
aactttgacc ccctcaaacc ttgatgatct cttctctgct gagagctcat cccctcgcta    1920
cgctgatcaa gccctggctt ctgctgtttt ttcaccaacg cacaaatctg cagtaatcaa    1980
tcaatttcag cagcagcagc agagcatgtt atcacccatc aacacaacct tctctcctaa    2040
gagtgtcgac caccctttgt tgcaagcgtc tttcggtgtt caatctgggc gaatgtcccc    2100
tcgtaacatg gatcccatct ctcctataag ttctcgtgtg tcgatgttgg cccaacgaga    2160
gaaacagcaa cagcaattac gcagcctaag ctctcgtgaa ctcggttcca attcagccgc    2220
cattgtgggt tcccccgtgg gttcttggtc gaaatgggga gctacaaatg ggaaaccaga    2280
ctgggctgtt agtgcagatg aactaggtaa gcttcgcagg tctaattcat ttgagcttgg    2340
gaacaatggt gaggagccag atctttcatg ggttcaatcc ctcgttaaag aatctcctac    2400
cgagatgaaa gaaaagcttt cgtcaactct ctctggtgtt ccagcccccg ctacatccag    2460
tgaggttccg agtatcagct cgcagatgga atcggttgat cacgaagtgc taggagcatg    2520
gctccagcag atgcagctcg atccgctcgt ggctcagcaa aactaggttg tttttttttcc    2580
tacatggcct tgaggaagta gacagcggaa agttttttttt ggtaaatact atgtttttttc    2640
tggaaatttt tgatgctggg ggtggggtct ggaagaagat aacaaggcag gaaaggggtc    2700
agtgaagtca ctggagaaaa ggaattcatt tttaaccatt ttatcattct attacaacag    2760
aaagtaggga aaaaaaagga agaccctctg ggttatgaag agaaattaaa cccaggctag    2820
gcgttctcct ttctaatatt tccaatttta ggtccatatt actgtcattt cctttttgcc    2880
gtcttatcat atttcatcaa aatggaactg gggactaatg tttgttccat tctttcgctc    2940
ttctgattta tttgcaccct tggggtaaga tcaaaagaga aattatgatc attttctttt    3000
gaggatattt tttttcccca atatttgtga gaatgaaagt taagagggga tatgatgtgt    3060
ctggtgttgt agtatgaaaa accaataacc gagttcacct gttgctgctg gtggtagaag    3120
aagtggagaa gaagctatga tcctttgatg taacagtcaa tcaaacattt taatacccttt   3180
attttttgtt tcctcatgta atccatcctt tgtgattgtc ctctctctct ctctctctct    3240
ctctctctcc ctccccgtgt tctttcttca taagcgtctt gcttgtcgat ctgtaaatta    3300
ttgaaaaggg tcatggaaag ccgtgccggt gtggattctc atttttgcaa aaaaaaaaaa    3360
aaaaaaaaaa aa                                                         3372
```

<210> 11
<211> 704
<212> PRT
<213> Eucalyptus grandis

<400> 11

```
Met Ser Gln Leu Thr Ile Gln Thr Glu Asp Thr Phe Ala Ser Leu Leu
1               5                   10                  15
Glu Leu Ala Ala Asn Asn Asp Thr Glu Ser Phe Gly Arg Cys Val Glu
            20                  25                  30
Arg Asp Pro Ser Ser Ile Asp Glu Ile Gly Tyr Trp Tyr Gly Arg Gln
        35                  40                  45
```

Lys Gly Ser Lys Gln Val Val Asn Met Gln Arg Thr Pro Leu Met Val
    50                  55                  60
Ala Ala Thr Tyr Gly Ser Val Asp Val Met Arg Leu Ile Leu Cys Leu
65                  70                  75                  80
Ser Asp Ala Asp Val Asn Arg Thr Cys Ser Thr Asp Lys Ser Thr Ala
                85                  90                  95
Leu His Cys Ala Ala Ser Gly Gly Ala Val Asn Ala Val Asp Ala Val
            100                 105                 110
Arg Leu Leu Leu Ser Ala Gly Ala Asp Pro Ser Leu Ala Asp Ala Asn
        115                 120                 125
Gly Gln Arg Pro Val Asp Val Ile Val Val Pro Pro Lys Leu Leu Ser
    130                 135                 140
Ile Lys Phe Ala Leu Glu Glu Leu Leu Ser Thr Glu Gly Ser Val Asn
145                 150                 155                 160
Glu His Asn Leu Arg Val Ser Val Ala Thr Ser Asn Ser Thr Ser Pro
                165                 170                 175
Pro Leu Ser Ser Ser Pro Asp Asn Gly Ser Pro Ala Ser Ala Asn Cys
            180                 185                 190
Ser Ser Pro Lys Asn Ser Lys Leu Ser Asp Ala Pro Val Leu Tyr Ala
        195                 200                 205
Ser Glu Lys Lys Glu Tyr Pro Val Asp Pro Ser Leu Pro Asp Ile Lys
    210                 215                 220
Asn Ser Ile Tyr Ser Thr Asp Glu Phe Arg Met Tyr Ser Phe Lys Val
225                 230                 235                 240
Arg Pro Cys Ser Arg Ala Tyr Ser His Asp Trp Thr Glu Cys Pro Phe
                245                 250                 255
Val His Pro Gly Glu Asn Ala Arg Arg Arg Asp Pro Arg Lys Phe His
            260                 265                 270
Tyr Ser Cys Val Pro Cys Pro Asp Phe Arg Lys Gly Ala Cys Arg Arg
        275                 280                 285
Gly Asp Met Cys Glu Tyr Ala His Gly Val Phe Glu Cys Trp Leu His
    290                 295                 300
Pro Ala Gln Tyr Arg Thr Arg Leu Cys Lys Asp Gly Thr Ser Cys Ala
305                 310                 315                 320
Arg Arg Val Cys Phe Phe Ala His Thr Glu Gln Glu Leu Arg Pro Leu
                325                 330                 335
Tyr Val Ser Thr Gly Ser Ala Val Pro Ser Pro Arg Ser Ser Thr Ser
            340                 345                 350
Gly Ala Ala Ala Met Asp Phe Ala Ala Ala Met Ser Leu Leu Pro Gly
        355                 360                 365
Ser Pro Ser Ser Val Ser Ile Met Ser Pro Ser Pro Phe Thr Pro Pro
    370                 375                 380
Met Ser Pro Ser Ala Asn Gly Ile Ser His Pro Ser Val Ala Trp Pro
385                 390                 395                 400
Gln Gln Asn Val Pro Thr Leu His Leu Pro Gly Ser Asn Leu Gln Ser
                405                 410                 415
Ser Arg Leu Arg Ser Ser Leu Asn Ala Arg Asp Ile Pro Gln Glu Asp
            420                 425                 430
Phe Asp Leu Leu Ser Asp Tyr Asp Val Gln Gln Gln Gln Leu Leu Asn
        435                 440                 445
Glu Phe Ser Ile Leu Ser Gln Gln Ser Met Gly Ala Asn Ser Leu Asn
    450                 455                 460
Arg Ser Gly Arg Leu Lys Thr Leu Thr Pro Ser Asn Leu Asp Asp Leu
465                 470                 475                 480
Phe Ser Ala Glu Ser Ser Ser Pro Arg Tyr Ala Asp Gln Ala Leu Ala
                485                 490                 495
Ser Ala Val Phe Ser Pro Thr His Lys Ser Ala Val Ile Asn Gln Phe
            500                 505                 510
Gln Gln Gln Gln Gln Ser Met Leu Ser Pro Ile Asn Thr Thr Phe Ser

```
            515                     520                     525
Pro Lys Ser Val Asp His Pro Leu Leu Gln Ala Ser Phe Gly Val Gln
        530                     535                     540
Ser Gly Arg Met Ser Pro Arg Asn Met Asp Pro Ile Ser Pro Ile Ser
545                     550                     555                     560
Ser Arg Val Ser Met Leu Ala Gln Arg Glu Lys Gln Gln Gln Gln Leu
                565                     570                     575
Arg Ser Leu Ser Ser Arg Glu Leu Gly Ser Asn Ser Ala Ala Ile Val
            580                     585                     590
Gly Ser Pro Val Gly Ser Trp Ser Lys Trp Gly Ala Thr Asn Gly Lys
        595                     600                     605
Pro Asp Trp Ala Val Ser Ala Asp Glu Leu Gly Lys Leu Arg Arg Ser
    610                     615                     620
Asn Ser Phe Glu Leu Gly Asn Asn Gly Glu Glu Pro Asp Leu Ser Trp
625                     630                     635                     640
Val Gln Ser Leu Val Lys Glu Ser Pro Thr Glu Met Lys Glu Lys Leu
                645                     650                     655
Ser Ser Thr Leu Ser Gly Val Pro Ala Pro Ala Thr Ser Ser Glu Val
            660                     665                     670
Pro Ser Ile Ser Ser Gln Met Glu Ser Val Asp His Glu Val Leu Gly
        675                     680                     685
Ala Trp Leu Gln Gln Met Gln Leu Asp Pro Leu Val Ala Gln Gln Asn
    690                     695                     700
```

<210> 12
<211> 1860
<212> DNA
<213> Oryza sativa

<400> 12

```
atgggggagc ctgggggcgc cgaggcggcc gtctccgcga ggctgctcga gctggcggcc     60
gacgacaacg cggcggggct cggggagctc ctcgcggcgt ggccctccct cgccgacgag    120
cccgcgccgt ggtacacccc ggcgcggggc gcggagccgc tgaccccgct catggtcgcc    180
gccgtgtacg gctcggtggg ctgcctcgac gcgctcctct cgccgcccta cctcgtggac    240
cccaaccgcg cctcggcgtc gtcgctctcc accccgctcc acctcgccgc cgcgggcggg    300
tccgcctccg cccccgcggc ggtctcccgc ctcctcgccg ccggcgccga cccggccctc    360
ctcgaccacc tccagcgccg ggcgtccgac ctcgtcgcgc tcccgcccaa ctcgctcccg    420
ctcaagaacc acctcctctc cctcctcggc gcccgcaagg agtggcctcc cgacccctcc    480
ctccccgaca tcaagaacgg cgcctacgcc tccgacgact tcaggatgta ctcgttcaag    540
gtgcgcgcgt gctcgcgggc ctactcccat gactggacgg agtgcccctt cgtccacccc    600
ggcgagaacg cgcggcggcg cgacccgagg aagtaccact acagctgcgt gccgtgcccg    660
gagttcaaga aggggccgg gtgcaggaga ggggacatgt gcgagtacgc gcacggggtg     720
ttcgagagct ggctccaccc ggcgcagtac cggacgcgcc tctgcaagga cggcgtcggc    780
tgcgcccgcc gcgtctgctt cttcgcccac acgcccgacg agctccgccc gctctacgtc    840
tccacgggct ccgccgtgcc gtcgccgcgc ggggcgttgg agatggcggc ggcggcggcg    900
gcgatgggga tggggctgtc gtcgccgggg tcgtcgtcgt tcacgccgcc gctatcgccg    960
tcggccggcg ggggcggggg cgggggcggg ggcagcggcg gcggcggcgc gtggccgcag   1020
cagccgagcg tgccggcgct ctgcctgccc gggagcgccg ggaacctcca cctgagccgg   1080
ctgcgcacgt cgctgagcgc gcgcgacatg gccgtcgacg agctgctcgc cgcggcggcg   1140
gcggcggcgg actacgacgg cctcgtcgcc tcccccgcct ccatccggtc cgcgaggggg   1200
aaggcgcttg tgccgtcaaa tctcgacgag ctcttctccg ctgagctcgc cgccgccgcg   1260
gcgtcgcgct cgccgcgcta cgccgaccaa ggcggcgccg cgttctcccc gacccgcaag   1320
gccaccgtgc tcaaccaatt ccagctgcag cagcagcata gcttgctctc gccgcgggcg   1380
gccgcggtga caccagagcc ggtctcccca atgagctccc gcctcctcgc cgcgctggcg   1440
cagcgggaga agatgcagca gcagacgctg cggagcatga gctcacggga cctcggcaac   1500
gccgcgtcgc tgctggtcgg ctcgccggtg agctcgagca tgtccaaatg gggttcccc    1560
tccggcaacc cggactgggg cgccgacgac gaggagctcg gccgcctcaa gcgttgctcc   1620
tcgttcgagc tccggtccgg agccgccaat ggcaaccatg agcctgacct ctcatgggtc   1680
aacaccctag tgaaggagcc gacaccggag aagatgatga cgacgacatc ggcaatggat   1740

tccattggca tcttgggaca gaacacaagc cgtgatcaca tcgtcggagg cgaggatgac   1800
actgccggag tcatcagcag ctggcttgaa cagctccagc tcgatgagat ggttgtctag   1860
```

<210> 13
<211> 619
<212> PRT
<213> Oryza sativa

<400> 13

```
Met Gly Glu Pro Gly Gly Ala Glu Ala Ala Val Ser Ala Arg Leu Leu
1               5                   10                  15
Glu Leu Ala Ala Asp Asp Asn Ala Ala Gly Leu Gly Glu Leu Leu Ala
            20                  25                  30
Ala Trp Pro Ser Leu Ala Asp Glu Pro Ala Pro Trp Tyr Thr Pro Ala
        35                  40                  45
Arg Gly Ala Glu Pro Leu Thr Pro Leu Met Val Ala Ala Val Tyr Gly
    50                  55                  60
Ser Val Gly Cys Leu Asp Ala Leu Leu Ser Pro Pro Tyr Leu Val Asp
65                  70                  75                  80
Pro Asn Arg Ala Ser Ala Ser Ser Leu Ser Thr Pro Leu His Leu Ala
                85                  90                  95
Ala Ala Gly Gly Ser Ala Ser Ala Pro Ala Ala Val Ser Arg Leu Leu
            100                 105                 110
Ala Ala Gly Ala Asp Pro Ala Leu Leu Asp His Leu Gln Arg Arg Ala
        115                 120                 125
Ser Asp Leu Val Ala Leu Pro Pro Asn Ser Leu Pro Leu Lys Asn His
    130                 135                 140
Leu Leu Ser Leu Leu Gly Ala Arg Lys Glu Trp Pro Pro Asp Pro Ser
145                 150                 155                 160
Leu Pro Asp Ile Lys Asn Gly Ala Tyr Ala Ser Asp Asp Phe Arg Met
                165                 170                 175
Tyr Ser Phe Lys Val Arg Ala Cys Ser Arg Ala Tyr Ser His Asp Trp
            180                 185                 190
Thr Glu Cys Pro Phe Val His Pro Gly Glu Asn Ala Arg Arg Arg Asp
        195                 200                 205
Pro Arg Lys Tyr His Tyr Ser Cys Val Pro Cys Pro Glu Phe Lys Lys
    210                 215                 220
Gly Ala Gly Cys Arg Arg Gly Asp Met Cys Glu Tyr Ala His Gly Val
225                 230                 235                 240
Phe Glu Ser Trp Leu His Pro Ala Gln Tyr Arg Thr Arg Leu Cys Lys
                245                 250                 255
Asp Gly Val Gly Cys Ala Arg Arg Val Cys Phe Phe Ala His Thr Pro
            260                 265                 270
Asp Glu Leu Arg Pro Leu Tyr Val Ser Thr Gly Ser Ala Val Pro Ser
        275                 280                 285
Pro Arg Gly Ala Leu Glu Met Ala Ala Ala Ala Ala Ala Met Gly Met
    290                 295                 300
Gly Leu Ser Ser Pro Gly Ser Ser Ser Phe Thr Pro Pro Leu Ser Pro
305                 310                 315                 320
Ser Ala Gly Gly Gly Gly Gly Gly Gly Gly Gly Ser Gly Gly Gly Gly
                325                 330                 335
Ala Trp Pro Gln Gln Pro Ser Val Pro Ala Leu Cys Leu Pro Gly Ser
            340                 345                 350
Ala Gly Asn Leu His Leu Ser Arg Leu Arg Thr Ser Leu Ser Ala Arg
        355                 360                 365
Asp Met Ala Val Asp Glu Leu Leu Ala Ala Ala Ala Ala Ala Ala Asp
    370                 375                 380
Tyr Asp Gly Leu Val Ala Ser Pro Ala Ser Ile Arg Ser Ala Arg Gly
385                 390                 395                 400
```

```
Lys Ala Leu Val Pro Ser Asn Leu Asp Glu Leu Phe Ser Ala Glu Leu
                405                 410                 415
Ala Ala Ala Ala Ala Ser Arg Ser Pro Arg Tyr Ala Asp Gln Gly Gly
            420                 425                 430
Ala Ala Phe Ser Pro Thr Arg Lys Ala Thr Val Leu Asn Gln Phe Gln
        435                 440                 445
Leu Gln Gln Gln His Ser Leu Leu Ser Pro Arg Ala Ala Ala Val Thr
    450                 455                 460
Pro Glu Pro Val Ser Pro Met Ser Ser Arg Leu Leu Ala Ala Leu Ala
465                 470                 475                 480
Gln Arg Glu Lys Met Gln Gln Gln Thr Leu Arg Ser Met Ser Ser Arg
                485                 490                 495
Asp Leu Gly Asn Ala Ala Ser Leu Leu Val Gly Ser Pro Val Ser Ser
            500                 505                 510
Ser Met Ser Lys Trp Gly Phe Pro Ser Gly Asn Pro Asp Trp Gly Ala
        515                 520                 525
Asp Asp Glu Glu Leu Gly Arg Leu Lys Arg Cys Ser Ser Phe Glu Leu
    530                 535                 540
Arg Ser Gly Ala Ala Asn Gly Asn His Glu Pro Asp Leu Ser Trp Val
545                 550                 555                 560
Asn Thr Leu Val Lys Glu Pro Thr Pro Glu Lys Met Met Thr Thr Thr
                565                 570                 575
Ser Ala Met Asp Ser Ile Gly Ile Leu Gly Gln Asn Thr Ser Arg Asp
            580                 585                 590
His Ile Val Gly Gly Glu Asp Asp Thr Ala Gly Val Ile Ser Ser Trp
        595                 600                 605
Leu Glu Gln Leu Gln Leu Asp Glu Met Val Val
    610                 615
```

<210> 14
<211> 2106
<212> DNA
<213> Medicago truncatula

<400> 14

```
atgaaaaatc taactgttcg tactgatgat tctttttcca gcttacttga acatgcttct      60
aacaatgatt ttgaagattt caaggtagct ctagatagtg atgcttcact tattaatgaa     120
gttggcttct ggtatgtccg tcaaaaggga tctaaccaaa ttgttcttga gcaccgaacc     180
cctttaatgg tggctgcttc ctatgggagt attgatattc taaagcttat actctcatat     240
cccgaggctg atgttaattt ctcctgtgga actgataaaa gcactgctct tcactgtgct     300
gcctcaagtg gttcagttaa tgctgttgat gctataaaat tgcttttatc agctggtgct     360
gatatcaatt ctgtggatgc taatgggaaa cgccctgtgg atgttatcgt tgttcctatt     420
gttgttcctc ataagctcga aggtgttaaa acaattcttg aagaacttct ctcagacagt     480
gcttctgaag gatctgtgga tgattgctct cttcccctgt ctcttatttc atcgagtcct     540
ggttcatctg cccctttatc atctgctgaa aatggatctc catcctctcc tgtggctccc     600
aagtttacag atacagctgt taattctaca tcagaaaaga aagagtatcc agttgaccca     660
tctcttcctg acataaaaaa cagcatgtat gccacagatg aattccgcat gtattcattc     720
aaggttcgtc cttgttctcg tgcatactct catgattgga ctgagtgtcc ttttgtgcat     780
cctggagaga atgctcgaag gagagaccct agaaagtttc actacagctg tgtgccatgc     840
cctgatttta ggaaaggggc ttgccgacgt tcggatatgt gtgaatatgc tcatggagta     900
ttcgagtgct ggctacaccc agctcagtat cggacaaggc tgtgcaaaga cggtatgggt     960
tgtaaccgaa gggtgtgctt cttcgctcac tcacctgaag agctgcgtcc gctgtatgtg    1020
tccactggtt ctgctgttcc ttcaccccga tcagctgctt ctactgctaa tgtcatggac    1080
atggctgctg ctatgagcct tttccctggt tcaccatcat caatctcttt gatgtctcaa    1140
tcaccctttg cacagcctcc tctatctcca tctgcaaatg gcaataatgc ttggccacag    1200
cccaatgtgc cagctcttca tttaccagga agcattaatc aaactagtcg tttgagatct    1260
tctcttagtg cccgtgatat gccacacgac gacttcaaca atatgttgca agactttgat    1320
gggcagcagc agatactaaa tgacttgagc tgtttctcac agccccgtcc tggtgctatt    1380
tcagttggtc gatctggccg ccctaaaaca ctaactccct caaatctgga tgatcttttt    1440

tgtgctgaga ttgcttcatc tcctaggtat tccgaccccg ctgcggcttc tgtattttcc    1500
ccaacacaca aatctgctgt cttcaaccag tttcaacagc ttcaaagctc cttatcaccc    1560
atcaacacaa atgtcatgtc tcctacaaac gtagagcatc ccctgttcca ccaggcttca    1620
tatggtctct cttctcctgg aaggatgtca ccaagaagta tggaagccct atctccaatg    1680
agttctcggc tgtcagcttt tgctcagcgt gagaaacaac agcagcagca gcaacagctg    1740
cgtagcctca gctcaagaga actcggtgct aacaatcctc tctcagctgt tgggtcccct    1800
gttaactcct ggtccaagtg gggatcatcc cctattggaa aagctgattg gtcggtaaat    1860
ccaaatgact tcggtcaaac acagagatca acttcttttg agcatggaaa caatggagaa    1920
gagcctgatg taggttgggt ccattccctt gtcaaggatc ccacacctga gaagaaagag    1980
aagcttgcag gttccggccc aattccatcc gttgaaaaga atcccaatcc tcaagcggac    2040
ggcattgatc actctgtttt gggagcttgg ctcgagcaac tgcagctgga tcaacttgta    2100
gtctag                                                               2106
```

<210> 15
<211> 701
<212> PRT
<213> Medicago truncatula

<400> 15

```
Met Lys Asn Leu Thr Val Arg Thr Asp Asp Ser Phe Ser Ser Leu Leu
1               5                   10                  15
Glu His Ala Ser Asn Asn Asp Phe Glu Asp Phe Lys Val Ala Leu Asp
            20                  25                  30
Ser Asp Ala Ser Leu Ile Asn Glu Val Gly Phe Trp Tyr Val Arg Gln
        35                  40                  45
Lys Gly Ser Asn Gln Ile Val Leu Glu His Arg Thr Pro Leu Met Val
    50                  55                  60
Ala Ala Ser Tyr Gly Ser Ile Asp Ile Leu Lys Leu Ile Leu Ser Tyr
65                  70                  75                  80
Pro Glu Ala Asp Val Asn Phe Ser Cys Gly Thr Asp Lys Ser Thr Ala
                85                  90                  95
Leu His Cys Ala Ala Ser Ser Gly Ser Val Asn Ala Val Asp Ala Ile
            100                 105                 110
Lys Leu Leu Leu Ser Ala Gly Ala Asp Ile Asn Ser Val Asp Ala Asn
        115                 120                 125
Gly Lys Arg Pro Val Asp Val Ile Val Val Pro Ile Val Val Pro His
    130                 135                 140
Lys Leu Glu Gly Val Lys Thr Ile Leu Glu Glu Leu Leu Ser Asp Ser
145                 150                 155                 160
Ala Ser Glu Gly Ser Val Asp Asp Cys Ser Leu Pro Leu Ser Leu Ile
                165                 170                 175
Ser Ser Ser Pro Gly Ser Ser Ala Pro Leu Ser Ser Ala Glu Asn Gly
            180                 185                 190
Ser Pro Ser Ser Pro Val Ala Pro Lys Phe Thr Asp Thr Ala Val Asn
        195                 200                 205
Ser Thr Ser Glu Lys Lys Glu Tyr Pro Val Asp Pro Ser Leu Pro Asp
    210                 215                 220
Ile Lys Asn Ser Met Tyr Ala Thr Asp Glu Phe Arg Met Tyr Ser Phe
225                 230                 235                 240
Lys Val Arg Pro Cys Ser Arg Ala Tyr Ser His Asp Trp Thr Glu Cys
                245                 250                 255
Pro Phe Val His Pro Gly Glu Asn Ala Arg Arg Arg Asp Pro Arg Lys
            260                 265                 270
Phe His Tyr Ser Cys Val Pro Cys Pro Asp Phe Arg Lys Gly Ala Cys
        275                 280                 285
Arg Arg Ser Asp Met Cys Glu Tyr Ala His Gly Val Phe Glu Cys Trp
    290                 295                 300
Leu His Pro Ala Gln Tyr Arg Thr Arg Leu Cys Lys Asp Gly Met Gly
305                 310                 315                 320
```

Cys Asn Arg Arg Val Cys Phe Phe Ala His Ser Pro Glu Glu Leu Arg
325 330 335
Pro Leu Tyr Val Ser Thr Gly Ser Ala Val Pro Ser Pro Arg Ser Ala
340 345 350
Ala Ser Thr Ala Asn Val Met Asp Met Ala Ala Ala Met Ser Leu Phe
355 360 365
Pro Gly Ser Pro Ser Ser Ile Ser Leu Met Ser Gln Ser Pro Phe Ala
370 375 380
Gln Pro Pro Leu Ser Pro Ser Ala Asn Gly Asn Asn Ala Trp Pro Gln
385 390 395 400
Pro Asn Val Pro Ala Leu His Leu Pro Gly Ser Ile Asn Gln Thr Ser
405 410 415
Arg Leu Arg Ser Ser Leu Ser Ala Arg Asp Met Pro His Asp Asp Phe
420 425 430
Asn Asn Met Leu Gln Asp Phe Asp Gly Gln Gln Gln Ile Leu Asn Asp
435 440 445
Leu Ser Cys Phe Ser Gln Pro Arg Pro Gly Ala Ile Ser Val Gly Arg
450 455 460
Ser Gly Arg Pro Lys Thr Leu Thr Pro Ser Asn Leu Asp Asp Leu Phe
465 470 475 480
Cys Ala Glu Ile Ala Ser Ser Pro Arg Tyr Ser Asp Pro Ala Ala Ala
485 490 495
Ser Val Phe Ser Pro Thr His Lys Ser Ala Val Phe Asn Gln Phe Gln
500 505 510
Gln Leu Gln Ser Ser Leu Ser Pro Ile Asn Thr Asn Val Met Ser Pro
515 520 525
Thr Asn Val Glu His Pro Leu Phe His Gln Ala Ser Tyr Gly Leu Ser
530 535 540
Ser Pro Gly Arg Met Ser Pro Arg Ser Met Glu Ala Leu Ser Pro Met
545 550 555 560
Ser Ser Arg Leu Ser Ala Phe Ala Gln Arg Glu Lys Gln Gln Gln Gln
565 570 575
Gln Gln Gln Leu Arg Ser Leu Ser Ser Arg Glu Leu Gly Ala Asn Asn
580 585 590
Pro Leu Ser Ala Val Gly Ser Pro Val Asn Ser Trp Ser Lys Trp Gly
595 600 605
Ser Ser Pro Ile Gly Lys Ala Asp Trp Ser Val Asn Pro Asn Asp Phe
610 615 620
Gly Gln Thr Gln Arg Ser Thr Ser Phe Glu His Gly Asn Asn Gly Glu
625 630 635 640
Glu Pro Asp Val Gly Trp Val His Ser Leu Val Lys Asp Pro Thr Pro
645 650 655
Glu Lys Lys Glu Lys Leu Ala Gly Ser Gly Pro Ile Pro Ser Val Glu
660 665 670
Lys Asn Pro Asn Pro Gln Ala Asp Gly Ile Asp His Ser Val Leu Gly
675 680 685
Ala Trp Leu Glu Gln Leu Gln Leu Asp Gln Leu Val Val
690 695 700

<210> 16
<211> 2841
<212> DNA
<213> Oryza sativa

<400> 16

```
atgaacggca cgccgatctc cgcgtccgcc gcggccggcg tcgacggagt cggcgcggcg     60
gtggcgctgg cggccgcgac caagaagagt gccgccgcgg cggccgccgt cgccgagatg    120
gcgaaaaccc tcaccgtcga cacggacgac gccttcgcgg ggctcctcga gctcgccgcg    180
gacgacgacg cggagggcct gcgccgcgcg ctggagcgcg ccccgcccgc cgccgcggac    240

gaggcgggcc tctggtacgg ccgccgcaag gtcctcgagc accgcacgcc gctgatggtc    300
gcggccacct atggcagcct cgcggtgctt cgcctgctgc tgtccctccc gtccgtcgat    360
gtcaatcgcc gctgtggctc cgacggcacc accgccctcc actgtgcggc gtctggtggc    420
tcgccgtctt gtgtggaggc cgtcaagctg ctgcttgctg ctggggctga tgctgatgcc    480
acggatgctt ccggatatcg tccagctgat gtgatctctg ttcctccaaa gatgtttgac    540
gccaagattg ccctccaaga tcttcttgga tgcccaaagg ctgggcatgg cgttctccgg    600
gtggtgacaa gggccgcaaa ctctatgttg tcacctgtat catcccctac agcagaagat    660
gcacgatctc catcagctgc tgtgatgatg acgacaaagt ttgcagatct tccaagggtt    720
gtgacatcgg aaaagaaaga atatccagtg gatccgtccc ttcccgatat caagaacagc    780
atctatgctt ccgatgagtt ccgcatgtac tcatttaaga tcaggccatg ctcgcgggcg    840
tactcacatg attggactga gtgcccgttt gttcacccag ggagaacgc acggcgtcgg    900
gaccctcgca agtatcacta cagctgtgtg ccatgccccg actttagaaa gggagtttgc    960
cggcgtggtg acatgtgtga atatgctcat ggcgtgttcg agtgttggct ccatccagca   1020
cagtaccgta ctcgcctttg caaggatggc acaagctgta atcgccgtgt ctgtttcttt   1080
gcgcatacaa ctgatgagct ccgaccacta tatgtttcca ctggatctgc agtaccatcc   1140
ccaagagcct cggcaacagc tacaatggag atggctgcag caatgggctt gatgcctggt   1200
tctccatcat cagtttcagc agtcatgtcc ccatttacac caccaatgtc cccttcaggc   1260
aatgggatgc ccccttcatt gggctggcag cagccaaatg ttccgacact acaccttcca   1320
ggcagcagcc ttcagtcgag ccggctccgt acctcactta gtgcaaggga tatgcctgct   1380
gatgattact ccctgatgca ggatattgat tcacagctta taaatgattt gtgctattca   1440
cgtattggtt catcaacagg aaaccacacg tctcggacca agtccctaaa tccgtcaaac   1500
ttggatgatc tcttctctgc tgagatggtc tcttccccga ggtatagtaa tgctgatcag   1560
ggtggtatgt tttcaccatc tcacaaggct gctttcctta atcagttcca gcaacagcag   1620
caggcacttc tttcaccaat caacacagtc ttctccccga agtctgtgga caaccagcag   1680
ttgccttcac actcatctct gttgcaagca tcacttggta tatcctcccc tggccgcatg   1740
tctcctcgat gtgttgaatc tgggtcccct atgaactctc atcttgctgc tgctcttgct   1800
cagcgtgaga agcaacagca gacaatgaga agtctcagtt ctcgtgatct tgggccgagt   1860
gctgcaagag catcaggtgt tgttggctcc cctctaagct catcatggtc aaagtgggga   1920
tcaccttcag ggacacctga ctggggtgtt aatggtgaag aattgggcaa gcttcgccgg   1980
tcatcatcgt ttgagctgag atctggtggt gatgatccag atctctcttg ggtacacaca   2040
ctggttaagg aatctccacc agagaagcaa gtcactactg ctgaatccat aaactctgtt   2100
ggaccttcac cactgatgcc tcccagtgta agcaacggtg aaggtcctag tctgaatgcc   2160
ccgctggatg ggcatgacca agctgctgtt attggagcat tgcttgaaca gatgcagctt   2220
gatcagcata ttggtagtct agcaacataa gcgctgaatg agcctggaaa gtgcaaggag   2280
ttattattct tagttaatga atttggagta attttttcc tgttcattaa gatggtcagc   2340
aagcaaaagg atggatagct gatggtggtg attcagagat tggttttctt tactttattg   2400
aggtaaatca tatacattat tgaggttcca gtaggttgaa agattgaagt accttgattg   2460
gggtcgtttc aagaccgacc caggtagaat cgcaccccgg cagcttcaat tcatcggtca   2520
aaaatatttc cctgttttgt taattaaccc cgttaaaaaa gaagactcgt ttggtgtttc   2580
ggaattcttt tctttacctt agcggtgttt attttgttta ttatgatatt gatacttgat   2640
gtactgatgg gtataaggtt ggttaccagg catgctatag tggtatatca agtcccaaag   2700
tattcttttt ctccctttca ccatttgtcg aggatcatac tatggccttg ttttggtcag   2760
atcttgaggc ctgtataatc cttggatttg taataatgta atattgtcat tgaacttaca   2820
ttgctattgt tttgcaatcg c                                             2841
```

<210> 17
<211> 749
<212> PRT
<213> Oryza sativa

<400> 17

```
Met Asn Gly Thr Pro Ile Ser Ala Ser Ala Ala Ala Gly Val Asp Gly
1               5                   10                  15
Val Gly Ala Ala Val Ala Leu Ala Ala Ala Thr Lys Lys Ser Ala Ala
            20                  25                  30
Ala Ala Ala Ala Val Ala Glu Met Ala Lys Thr Leu Thr Val Asp Thr
        35                  40                  45
Asp Asp Ala Phe Ala Gly Leu Leu Glu Leu Ala Ala Asp Asp Asp Ala
    50                  55                  60
```

```
Glu Gly Leu Arg Arg Ala Leu Glu Arg Ala Pro Pro Ala Ala Ala Asp
65                  70                  75                  80
Glu Ala Gly Leu Trp Tyr Gly Arg Arg Lys Val Leu Glu His Arg Thr
                85                  90                  95
Pro Leu Met Val Ala Ala Thr Tyr Gly Ser Leu Ala Val Leu Arg Leu
            100                 105                 110
Leu Leu Ser Leu Pro Ser Val Asp Val Asn Arg Arg Cys Gly Ser Asp
        115                 120                 125
Gly Thr Thr Ala Leu His Cys Ala Ala Ser Gly Gly Ser Pro Ser Cys
    130                 135                 140
Val Glu Ala Val Lys Leu Leu Leu Ala Ala Gly Ala Asp Ala Asp Ala
145                 150                 155                 160
Thr Asp Ala Ser Gly Tyr Arg Pro Ala Asp Val Ile Ser Val Pro Pro
                165                 170                 175
Lys Met Phe Asp Ala Lys Ile Ala Leu Gln Asp Leu Leu Gly Cys Pro
            180                 185                 190
Lys Ala Gly His Gly Val Leu Arg Val Val Thr Arg Ala Ala Asn Ser
        195                 200                 205
Met Leu Ser Pro Val Ser Ser Pro Thr Ala Glu Asp Ala Arg Ser Pro
    210                 215                 220
Ser Ala Ala Val Met Met Thr Thr Lys Phe Ala Asp Leu Pro Arg Val
225                 230                 235                 240
Val Thr Ser Glu Lys Lys Glu Tyr Pro Val Asp Pro Ser Leu Pro Asp
                245                 250                 255
Ile Lys Asn Ser Ile Tyr Ala Ser Asp Glu Phe Arg Met Tyr Ser Phe
            260                 265                 270
Lys Ile Arg Pro Cys Ser Arg Ala Tyr Ser His Asp Trp Thr Glu Cys
        275                 280                 285
Pro Phe Val His Pro Gly Glu Asn Ala Arg Arg Arg Asp Pro Arg Lys
    290                 295                 300
Tyr His Tyr Ser Cys Val Pro Cys Pro Asp Phe Arg Lys Gly Val Cys
305                 310                 315                 320
Arg Arg Gly Asp Met Cys Glu Tyr Ala His Gly Val Phe Glu Cys Trp
                325                 330                 335
Leu His Pro Ala Gln Tyr Arg Thr Arg Leu Cys Lys Asp Gly Thr Ser
            340                 345                 350
Cys Asn Arg Arg Val Cys Phe Phe Ala His Thr Thr Asp Glu Leu Arg
        355                 360                 365
Pro Leu Tyr Val Ser Thr Gly Ser Ala Val Pro Ser Pro Arg Ala Ser
    370                 375                 380
Ala Thr Ala Thr Met Glu Met Ala Ala Ala Met Gly Leu Met Pro Gly
385                 390                 395                 400
Ser Pro Ser Ser Val Ser Ala Val Met Ser Pro Phe Thr Pro Pro Met
                405                 410                 415
Ser Pro Ser Gly Asn Gly Met Pro Pro Ser Leu Gly Trp Gln Gln Pro
            420                 425                 430
Asn Val Pro Thr Leu His Leu Pro Gly Ser Ser Leu Gln Ser Ser Arg
        435                 440                 445
Leu Arg Thr Ser Leu Ser Ala Arg Asp Met Pro Ala Asp Asp Tyr Ser
    450                 455                 460
Leu Met Gln Asp Ile Asp Ser Gln Leu Ile Asn Asp Leu Cys Tyr Ser
465                 470                 475                 480
Arg Ile Gly Ser Ser Thr Gly Asn His Thr Ser Arg Thr Lys Ser Leu
                485                 490                 495
Asn Pro Ser Asn Leu Asp Asp Leu Phe Ser Ala Glu Met Val Ser Ser
            500                 505                 510
Pro Arg Tyr Ser Asn Ala Asp Gln Gly Gly Met Phe Ser Pro Ser His
        515                 520                 525
Lys Ala Ala Phe Leu Asn Gln Phe Gln Gln Gln Gln Gln Ala Leu Leu
```

```
    530                 535                 540
Ser Pro Ile Asn Thr Val Phe Ser Pro Lys Ser Val Asp Asn Gln Gln
545                 550                 555                 560
Leu Pro Ser His Ser Ser Leu Leu Gln Ala Ser Leu Gly Ile Ser Ser
                565                 570                 575
Pro Gly Arg Met Ser Pro Arg Cys Val Glu Ser Gly Ser Pro Met Asn
            580                 585                 590
Ser His Leu Ala Ala Ala Leu Ala Gln Arg Glu Lys Gln Gln Gln Thr
        595                 600                 605
Met Arg Ser Leu Ser Ser Arg Asp Leu Gly Pro Ser Ala Ala Arg Ala
    610                 615                 620
Ser Gly Val Val Gly Ser Pro Leu Ser Ser Ser Trp Ser Lys Trp Gly
625                 630                 635                 640
Ser Pro Ser Gly Thr Pro Asp Trp Gly Val Asn Gly Glu Glu Leu Gly
                645                 650                 655
Lys Leu Arg Arg Ser Ser Ser Phe Glu Leu Arg Ser Gly Gly Asp Asp
            660                 665                 670
Pro Asp Leu Ser Trp Val His Thr Leu Val Lys Glu Ser Pro Pro Glu
        675                 680                 685
Lys Gln Val Thr Thr Ala Glu Ser Ile Asn Ser Val Gly Pro Ser Pro
    690                 695                 700
Leu Met Pro Pro Ser Val Ser Asn Gly Glu Gly Pro Ser Leu Asn Ala
705                 710                 715                 720
Pro Leu Asp Gly His Asp Gln Ala Ala Val Ile Gly Ala Leu Leu Glu
                725                 730                 735
Gln Met Gln Leu Asp Gln His Ile Gly Ser Leu Ala Thr
            740                 745
```

<210> 18
<211> 2769
<212> DNA
<213> Arabidopsis thaliana

<400> 18

```
acaccagtta ccctctcatc cgttttcgtt ttttttttct ctctttcaaa aatctctcag     60
ctgaggttga tcgatcttct tcttcttctt cctcactctt tagatttgtt ccttttgcat    120
tttacacttt tggatctgaa aatgtggttc tctgtttcgc ccgttaccgt ttagattcag    180
ttctgttttt ttcttacccg atcgcttgat tcggactgtg atctttgatc tttttttcttc   240
tccagtgccg tgaaggatgt gtggtcttgc taagaagctg gatatagagg atactttgac    300
atcactgtca gaccaagaga atgaatcttt ggccaaaccc atgaatgatg ctgctgaatg    360
ggaacattcg ttttctgcct tgcttgagtt tgctgcagac aacgatgtgg aggggtttag    420
gcggcaactc tctgatgtgt cttgtatcaa ccagatgggt ctttggtaca gacggcagag    480
gtttgttaga agaatggttc ttgagcaaag aaccccgctg atggttgctt cgttatatgg    540
gagtttagat gttgtgaagt ttattctttc tttcccggaa gcggagttga atctgtcttg    600
tggtcctgat aaaagtactg ctcttcattg cgctgcttct ggtgcttctg tgaattcctt    660
ggatgttgtc aagttgcttt tgagtgtagg agcagatcct aatatccctg atgctcatgg    720
aaatcgtcct gttgatgttc ttgttgtgtc tccacacgct cctggtttga gaaccatcct    780
tgaagagatc ttgaagaaag acgagattat atctgaagat ctgcatgcct cgtcatctag    840
cttgggatca agtttccggt ctctctcatc atcccctgat aatggttcct cgttactctc    900
cttagattca gtatcctctc cgactaagcc acacggtact gatgtaactt tcgcatcaga    960
gaagaaagag tacccaattg atccatcatt gcctgatatc aaaagcggga tttattcaac   1020
cgatgagttt cgtatgttct cgttcaagat ccgcccatgt tctcgagcat attcccatga   1080
ctggactgaa tgtccatttg cacacccagg tgagaatgca aggagaagag acccgaggaa   1140
gtttcactat acgtgtgttc catgcccgga ttttaagaaa ggatcctgta agcaaggtga   1200
tatgtgtgaa tatgctcatg ggtttttga atgctggcta caccctgctc agtacagaac    1260
acgattgtgc aaggacggaa tgggttgcaa ccgaagggtt tgcttctttg ctcacgcaaa   1320
tgaggagttg cgtcccttgt acccttccac aggatctgga ttgccatctc ctcgggcttc   1380
gtctgctgtt tccgcctcta ctatggacat ggcgtcagtt ttgaacatgt taccaggctc   1440
accatctgct gctcaacatt cgttcacccc accaatatct ccttctggaa atggtagtat   1500

gccccattca tcgatgggtt ggcctcagca gaacataccg gcgttgaatc ttcctggaag   1560
caatatccag ttgagtcgtc tgagatcttc tcttaacgct agagatattc cttctgagca   1620
gcttagcatg ctgcatgagt ttgaaatgca acgtcagctt gctggcgata tgcacagtcc   1680
acgctttatg aatcattccg ctcgtcctaa gacactgaac ccttcaaatc tggaggaact   1740
cttctcagct gaggttgcat ctcctcgttt ctctgatcaa cttgctgttt catctgttct   1800
atcgccttcc cacaagtccg cgcttcttaa tcagctgcag aataataagc agagcatgct   1860
ttctcctatc aagacaaatc taatgtcttc tccaaagaat gtggagcaac attctcttct   1920
gcagcaagcc tcgtcacccc gaggcggaga gcctatttcc ccaatgaatg ctcgaatgaa   1980
acagcagcta cattcacgca gcctaagctc ccgtgatttt ggatctagtc tgccccgtga   2040
tttaatgccg actgattctg gttcgccatt aagtccatgg tcaagttggg accagaccca   2100
tggaagcaag gtggattggt cagtccaatc agatgagtta ggtcggttga gaaaatctca   2160
ttccttggct aataacccaa acagggaagc agatgtttca tgggctcagc agatgttaaa   2220
agactcttca tcacctagga acggaaaccg tgttgtgaac atgaatggtg caaggccatt   2280
gactcaaggt ggttcgagtg tgaatcctca caacagtgac actcgtgaga gcgacattct   2340
tgatgcgtgg cttgaacagc tgcacctaga tcgctgagcc tcagctgcga gagagaggtt   2400
cacatttctg tgaagctgtg aaactgatga ttcgtttatt tattattcaa gaaagcaaac   2460
ggaaacaaaa gcaaactccg ggtaagcttt tttcgattct aataacccta aaaggctcag   2520
tttttttcagg cttctttctg aaatttcttt actttcttat ttttatcacc tcattaaatt  2580
aattattgta tcatctctgt tgtaacaatg gccaaagtgc gcctctatta cttcccggat   2640
ttctgattta catttttttgt atcctctcag tttgtcaatt gtttctaata tctccttcat  2700
atttgtcaaa gaacactgta tgagaaataa taacatattg tttcagctaa taagattcat   2760
tcatttcct                                                           2769
```

<210> 19
<211> 706
<212> PRT
<213> Arabidopsis thaliana

<400> 19

```
Met Cys Gly Leu Ala Lys Lys Leu Asp Ile Glu Asp Thr Leu Thr Ser
1               5                   10                  15
Leu Ser Asp Gln Glu Asn Glu Ser Leu Ala Lys Pro Met Asn Asp Ala
            20                  25                  30
Ala Glu Trp Glu His Ser Phe Ser Ala Leu Leu Glu Phe Ala Ala Asp
        35                  40                  45
Asn Asp Val Glu Gly Phe Arg Arg Gln Leu Ser Asp Val Ser Cys Ile
    50                  55                  60
Asn Gln Met Gly Leu Trp Tyr Arg Arg Gln Arg Phe Val Arg Arg Met
65                  70                  75                  80
Val Leu Glu Gln Arg Thr Pro Leu Met Val Ala Ser Leu Tyr Gly Ser
                85                  90                  95
Leu Asp Val Val Lys Phe Ile Leu Ser Phe Pro Glu Ala Glu Leu Asn
            100                 105                 110
Leu Ser Cys Gly Pro Asp Lys Ser Thr Ala Leu His Cys Ala Ala Ser
        115                 120                 125
Gly Ala Ser Val Asn Ser Leu Asp Val Val Lys Leu Leu Leu Ser Val
    130                 135                 140
Gly Ala Asp Pro Asn Ile Pro Asp Ala His Gly Asn Arg Pro Val Asp
145                 150                 155                 160
Val Leu Val Val Ser Pro His Ala Pro Gly Leu Arg Thr Ile Leu Glu
                165                 170                 175
Glu Ile Leu Lys Lys Asp Glu Ile Ile Ser Glu Asp Leu His Ala Ser
            180                 185                 190
Ser Ser Ser Leu Gly Ser Ser Phe Arg Ser Leu Ser Ser Ser Pro Asp
        195                 200                 205
Asn Gly Ser Ser Leu Leu Ser Leu Asp Ser Val Ser Ser Pro Thr Lys
    210                 215                 220
Pro His Gly Thr Asp Val Thr Phe Ala Ser Glu Lys Lys Glu Tyr Pro
225                 230                 235                 240
```

Ile Asp Pro Ser Leu Pro Asp Ile Lys Ser Gly Ile Tyr Ser Thr Asp
245 250 255
Glu Phe Arg Met Phe Ser Phe Lys Ile Arg Pro Cys Ser Arg Ala Tyr
260 265 270
Ser His Asp Trp Thr Glu Cys Pro Phe Ala His Pro Gly Glu Asn Ala
275 280 285
Arg Arg Arg Asp Pro Arg Lys Phe His Tyr Thr Cys Val Pro Cys Pro
290 295 300
Asp Phe Lys Lys Gly Ser Cys Lys Gln Gly Asp Met Cys Glu Tyr Ala
305 310 315 320
His Gly Val Phe Glu Cys Trp Leu His Pro Ala Gln Tyr Arg Thr Arg
325 330 335
Leu Cys Lys Asp Gly Met Gly Cys Asn Arg Arg Val Cys Phe Phe Ala
340 345 350
His Ala Asn Glu Glu Leu Arg Pro Leu Tyr Pro Ser Thr Gly Ser Gly
355 360 365
Leu Pro Ser Pro Arg Ala Ser Ser Ala Val Ser Ala Ser Thr Met Asp
370 375 380
Met Ala Ser Val Leu Asn Met Leu Pro Gly Ser Pro Ser Ala Ala Gln
385 390 395 400
His Ser Phe Thr Pro Pro Ile Ser Pro Ser Gly Asn Gly Ser Met Pro
405 410 415
His Ser Ser Met Gly Trp Pro Gln Gln Asn Ile Pro Ala Leu Asn Leu
420 425 430
Pro Gly Ser Asn Ile Gln Leu Ser Arg Leu Arg Ser Ser Leu Asn Ala
435 440 445
Arg Asp Ile Pro Ser Glu Gln Leu Ser Met Leu His Glu Phe Glu Met
450 455 460
Gln Arg Gln Leu Ala Gly Asp Met His Ser Pro Arg Phe Met Asn His
465 470 475 480
Ser Ala Arg Pro Lys Thr Leu Asn Pro Ser Asn Leu Glu Glu Leu Phe
485 490 495
Ser Ala Glu Val Ala Ser Pro Arg Phe Ser Asp Gln Leu Ala Val Ser
500 505 510
Ser Val Leu Ser Pro Ser His Lys Ser Ala Leu Leu Asn Gln Leu Gln
515 520 525
Asn Asn Lys Gln Ser Met Leu Ser Pro Ile Lys Thr Asn Leu Met Ser
530 535 540
Ser Pro Lys Asn Val Glu Gln His Ser Leu Leu Gln Gln Ala Ser Ser
545 550 555 560
Pro Arg Gly Gly Glu Pro Ile Ser Pro Met Asn Ala Arg Met Lys Gln
565 570 575
Gln Leu His Ser Arg Ser Leu Ser Ser Arg Asp Phe Gly Ser Ser Leu
580 585 590
Pro Arg Asp Leu Met Pro Thr Asp Ser Gly Ser Pro Leu Ser Pro Trp
595 600 605
Ser Ser Trp Asp Gln Thr His Gly Ser Lys Val Asp Trp Ser Val Gln
610 615 620
Ser Asp Glu Leu Gly Arg Leu Arg Lys Ser His Ser Leu Ala Asn Asn
625 630 635 640
Pro Asn Arg Glu Ala Asp Val Ser Trp Ala Gln Gln Met Leu Lys Asp
645 650 655
Ser Ser Ser Pro Arg Asn Gly Asn Arg Val Val Asn Met Asn Gly Ala
660 665 670
Arg Pro Leu Thr Gln Gly Gly Ser Ser Val Asn Pro His Asn Ser Asp
675 680 685
Thr Arg Glu Ser Asp Ile Leu Asp Ala Trp Leu Glu Gln Leu His Leu
690 695 700
Asp Arg

705

<210> 20
<211> 2674
<212> DNA
<213> Oryza sativa

<400> 20

```
tgcgagtcct cctcctcttc tcgtcgccgt gctactctcg ctttctctct ctctctctct      60
cacttgttcc ccaaggcgag aagcagccgc cgccggcgag cgtcgcgggg gaggggaggg     120
aagggaggga ggagcggtgg atccgggctt gattggattg ggtcggattc gattttggat     180
caaccccgga gggcgggagc ggttgctaca gatgcgttga gctttggtta atctatccgg     240
cgagagataa tgggcgagct tgctgatctc gttgtcgtgc cgtcgcagcc gccgctcgcc     300
ggcggccggc gggacaggct ggcggcgctg ctggagctcg cggcggcgga tgatgttgat     360
gggctcaggg gggcgctcgc ggagggaggc gaggaggcgg cggagttggc tgatggggtc     420
gggctgtggt atggtcggag caaggcgtac gaggcgcgca cgccgctgat ggtggcggcg     480
acgtacggca gcgccggggt ggtctcgctg ctggtgggcc tcggcggttg cgtcgacgtc     540
aaccgtcgcc ctggagccga cggcgccacc gcgctccact gcgccgcctc cggtggctcg     600
cgcaacgccg tcgctgttgt caagctgctt ttggccgctg gcgccgatcc ggccaccccc     660
gattccgccg gccgcttccc cgccgacgtc atcctagctc ctccggcttc gccagatgcc     720
cttggcgatc tcgaggtgct cctcggccgc cgccgagcac tcgccgtggc gacctcggtg     780
gcttcaggtt cgtcatcccc tccgctctcg tcctcaccag atgagggcaa caggtcgccc     840
tcgtcgcgtt cgtcgtcgct gtctcccatc actgtggatc gtgggaagaa ggagtatccg     900
gtggatccaa ctctgccgga catcaagagc agcgtgtatg cttcggatga gttccgcatg     960
tttgcgttca aggtccggcc ctgctcccgt gcctactcac acgactggac tgagtgcccg    1020
tttgtgcacc ccggcgagaa cgcccgccgc cgtgatcccc gcaagcaccc atacactgct    1080
gtgccttgcc ccaactttcg ccggcctggt ggctgcccta gcggcgatag ctgtgagttc    1140
tcgcatggcg tgtttgagag ctggctacac ccatcacagt atcgcacaag gctctgcaag    1200
gagggagcag cttgcgcccg tcgcatttgc ttctttgccc atgatgagga tgagctccgc    1260
catgtgcctc acaacagtgg tgccggcctg ctgtctcccc gcgcttcttc atccattgat    1320
atgactgctg cagctgcgct cgggcttctt ccaggttctc ctaccagaca ctttgcaccg    1380
ccgcctgtgt caccatctgc tgggagcaat ggaggagctg ctgctgcgca ttggctccaa    1440
ggcagtaggc tgcgttcttc tttcaatgca agggatgctg ctgttgatga ccttggcatg    1500
ctcctcgaat gggaatcaca ataccttggg gcactctgcc tgccacccag cagccgcccc    1560
caaccacgcc tttcagctgg tctgagtatc aggccaacaa ttgctccatc caatcttgaa    1620
gacatgtatg cttcagacat ggcaatgtct ccgaggttcc ctaatgacca aggtcactca    1680
gtctactcac cagcccacaa atcagccctc ctcaacaagc ttcatcaaca gaagggcctc    1740
ttatcacctg ttaacaccaa cagaatgtac tccccaaggg ctcttgatcc gtcatctttg    1800
gcacattctc catttggtgg catgtctccc cggtcccccc gtaccatgga acctacatca    1860
cccctaagtg ctcgtgtagg agcccctgcc acacagcggc cttctgttgg ttcaccacgg    1920
aattccagtg cttggggcac cgtggggtcc ccgatgggta aggttgactg ggtgtcgat     1980
agcgaggagc tagtccgctt gagacgccct gcacaaccag ggtttggaga agatgagaca    2040
gatgtatcat gggtgcagtc actggtaagc aatgctgagc ttaatggcaa gaggggcgaa    2100
gtacaaggca tgcctggtac ttctgcattg atgaacaggc ctgacctgaa caatcagggt    2160
gacttgttgg accagacggt gatcggtgct tggcttgagc agatgcacct ggatcagaag    2220
tgatttccaa gggaagccat gaagtcccaa agtggatgaa gcctttattt tgccaaggtt    2280
atttaccaaa gaatagttgt tggtcctagt aaataataat ttattctttt taattcttga    2340
aatttttggt gggcaaagtc agagatggtg gtcaagttca acaaaacatt tggtcacaga    2400
ttggtagctg aaatcagttc cagagattgg taaacaacct cattacttgg ggtcctaact    2460
agtattcttt tgattagctc agatgagtct ttattttagt gggttaaaat tcatatgttc    2520
cccatggtta ttatgtccat gatctcttcc taacaaaaga gagattataa ttgtccattt    2580
ttcatttatc aatgaatgat tttgttaaaa caatgtaagt tacattctta attttttctc    2640
tgttcaatgg aattaccttc cttggttagt cctc                                2674
```

<210> 21
<211> 657
<212> PRT

<213> Oryza sativa

<400> 21

```
Met Gly Glu Leu Ala Asp Leu Val Val Val Pro Ser Gln Pro Pro Leu
1               5                   10                  15
Ala Gly Gly Arg Arg Asp Arg Leu Ala Ala Leu Leu Glu Leu Ala Ala
            20                  25                  30
Ala Asp Asp Val Asp Gly Leu Arg Gly Ala Leu Ala Glu Gly Gly Glu
        35                  40                  45
Glu Ala Ala Glu Leu Ala Asp Gly Val Gly Leu Trp Tyr Gly Arg Ser
    50                  55                  60
Lys Ala Tyr Glu Ala Arg Thr Pro Leu Met Val Ala Ala Thr Tyr Gly
65                  70                  75                  80
Ser Ala Gly Val Val Ser Leu Leu Val Gly Leu Gly Gly Cys Val Asp
                85                  90                  95
Val Asn Arg Arg Pro Gly Ala Asp Gly Ala Thr Ala Leu His Cys Ala
            100                 105                 110
Ala Ser Gly Gly Ser Arg Asn Ala Val Ala Val Val Lys Leu Leu Leu
        115                 120                 125
Ala Ala Gly Ala Asp Pro Ala Thr Pro Asp Ser Ala Gly Arg Phe Pro
    130                 135                 140
Ala Asp Val Ile Leu Ala Pro Pro Ala Ser Pro Asp Ala Leu Gly Asp
145                 150                 155                 160
Leu Glu Val Leu Leu Gly Arg Arg Arg Ala Leu Ala Val Ala Thr Ser
                165                 170                 175
Val Ala Ser Gly Ser Ser Ser Pro Pro Leu Ser Ser Ser Pro Asp Glu
            180                 185                 190
Gly Asn Arg Ser Pro Ser Ser Arg Ser Ser Ser Leu Ser Pro Ile Thr
        195                 200                 205
Val Asp Arg Gly Lys Lys Glu Tyr Pro Val Asp Pro Thr Leu Pro Asp
    210                 215                 220
Ile Lys Ser Ser Val Tyr Ala Ser Asp Glu Phe Arg Met Phe Ala Phe
225                 230                 235                 240
Lys Val Arg Pro Cys Ser Arg Ala Tyr Ser His Asp Trp Thr Glu Cys
                245                 250                 255
Pro Phe Val His Pro Gly Glu Asn Ala Arg Arg Arg Asp Pro Arg Lys
            260                 265                 270
His Pro Tyr Thr Ala Val Pro Cys Pro Asn Phe Arg Arg Pro Gly Gly
        275                 280                 285
Cys Pro Ser Gly Asp Ser Cys Glu Phe Ser His Gly Val Phe Glu Ser
    290                 295                 300
Trp Leu His Pro Ser Gln Tyr Arg Thr Arg Leu Cys Lys Glu Gly Ala
305                 310                 315                 320
Ala Cys Ala Arg Arg Ile Cys Phe Phe Ala His Asp Glu Asp Glu Leu
                325                 330                 335
Arg His Val Pro His Asn Ser Gly Ala Gly Leu Leu Ser Pro Arg Ala
            340                 345                 350
Ser Ser Ser Ile Asp Met Thr Ala Ala Ala Ala Leu Gly Leu Leu Pro
        355                 360                 365
Gly Ser Pro Thr Arg His Phe Ala Pro Pro Pro Val Ser Pro Ser Ala
    370                 375                 380
Gly Ser Asn Gly Gly Ala Ala Ala Ala His Trp Leu Gln Gly Ser Arg
385                 390                 395                 400
Leu Arg Ser Ser Phe Asn Ala Arg Asp Ala Ala Val Asp Asp Leu Gly
                405                 410                 415
Met Leu Leu Glu Trp Glu Ser Gln Tyr Leu Gly Ala Leu Cys Leu Pro
            420                 425                 430
Pro Ser Ser Arg Pro Gln Pro Arg Leu Ser Ala Gly Leu Ser Ile Arg
        435                 440                 445
Pro Thr Ile Ala Pro Ser Asn Leu Glu Asp Met Tyr Ala Ser Asp Met
    450                 455                 460
```

```
Ala Met Ser Pro Arg Phe Pro Asn Asp Gln Gly His Ser Val Tyr Ser
465                 470                 475                 480
Pro Ala His Lys Ser Ala Leu Leu Asn Lys Leu His Gln Gln Lys Gly
                485                 490                 495
Leu Leu Ser Pro Val Asn Thr Asn Arg Met Tyr Ser Pro Arg Ala Leu
            500                 505                 510
Asp Pro Ser Ser Leu Ala His Ser Pro Phe Gly Gly Met Ser Pro Arg
        515                 520                 525
Ser Pro Arg Thr Met Glu Pro Thr Ser Pro Leu Ser Ala Arg Val Gly
    530                 535                 540
Ala Pro Ala Thr Gln Arg Pro Ser Val Gly Ser Pro Arg Asn Ser Ser
545                 550                 555                 560
Ala Trp Gly Thr Val Gly Ser Pro Met Gly Lys Val Asp Trp Gly Val
                565                 570                 575
Asp Ser Glu Glu Leu Val Arg Leu Arg Arg Pro Ala Gln Pro Gly Phe
            580                 585                 590
Gly Glu Asp Glu Thr Asp Val Ser Trp Val Gln Ser Leu Val Ser Asn
        595                 600                 605
Ala Glu Leu Asn Gly Lys Arg Gly Glu Val Gln Gly Met Pro Gly Thr
    610                 615                 620
Ser Ala Leu Met Asn Arg Pro Asp Leu Asn Asn Gln Gly Asp Leu Leu
625                 630                 635                 640
Asp Gln Thr Val Ile Gly Ala Trp Leu Glu Gln Met His Leu Asp Gln
                645                 650                 655
Lys
```

<210> 22
<211> 2223
<212> DNA
<213> Arabidopsis thaliana

<400> 22

```
ttcttcaaaa accccaacca cttcttctcc ccaaaaacct ccaaagtttc aatctttact      60
tctctctttt tctccaagtt atcttctttt ctaggaagag atatgtgcgg tgcaaagagc     120
aacctttgct catctaaaac cctaacagaa gtcgaattca tgaggcagaa atcagaagac     180
ggagcttccg ccacgtgtct cctcgaattc gccgcctgtg atgatctttc atcgtttaag     240
agagagatcg aagagaatcc atcggtggag attgatgagt cagggttttg gtattgcaga     300
cgggtcgggt ctaagaagat gggttttgaa gaaagaacac cacttatggt tgctgctatg     360
tatggaagca tggaagtgtt gaattacata attgccacag gaagatccga tgtgaacaga     420
gtttgcagtg acgagaaagt cactgctctt cactgtgcag tttctggctg ttctgtttct     480
atcgttgaga tcatcaagat cttgcttgat gcttctgctt cacctaattg tgttgacgct     540
aatgggaaca aaccggttga tttgttggct aaagattctc ggtttgttcc taaccagagt     600
agaaaggcgg ttgaggtttt actgaccggg attcatggtt cggttatgga agaagaggag     660
gaggaactga agagtgttgt gactaagtat ccagctgatg catcacttcc tgatattaac     720
gaaggtgttt atggaactga tgattttagg atgtttagct ttaaggttaa gccatgttct     780
agggcttatt cacatgattg gactgaatgt ccttttgttc atcctggtga gaatgcaagg     840
aggagagatc ctaggaagta tccttacact tgtgtgcctt gtcccgagtt tcgtaaaggg     900
tcttgtccta aaggagattc gtgtgagtac gcgcacggtg ttttcgagtc ttggcttcac     960
ccggcgcagt ataggacacg gctttgcaaa gatgagactg gttgtgctag gagagtttgt    1020
ttctttgctc atagacggga tgagttaaga ccggttaatg cttctactgg ttctgcaatg    1080
gtttcaccaa ggtcgtctaa tcagtctcct gagatgtctg ttatgtctcc tttgacgctg    1140
ggatcatcgc caatgaactc tcctatggct aatggtgttc ctttgtctcc aagaaatggt    1200
ggtttatggc agaacagagt taatagcctt acaccaccac cgttgcagct taatggtagc    1260
agattgaagt cgactttgag tgctagagat atggatatgg agatggaact taggtttcgc    1320
ggtttggata accggagact tggtgatctc aagccatcca acctcgaaga gactttcgga    1380
tcatatgact cagcttctgt gatgcaactt caatcaccaa gcaggcattc tcagatgaac    1440
cactatccgt cttcacctgt gaggcagcct cctcctcatg gattcgaatc ttcagcagcc    1500
atggcagctg cagtgatgaa tgcaagatcc tcagcgtttg cgaaacgcag cttgagtttc    1560

aaaccagctc cagtagcttc taatgtctcc gattggggat caccaaatgg gaagcttgag    1620
tggggaatgc aaagagatga gctgaacaag ttgaggagaa gtgcctcctt cggcattcat    1680
ggaaacaaca acaacagtgt gtcacgccct gctagagact acagtgacga gccagatgtg    1740
tcgtgggtga actcactggt gaaagagaat gcaccagaga gagtgaatga gagggttggg    1800
aatacggtga atggtgcagc gagtagagac aagtttaagc tgccgtcgtg ggcagagcaa    1860
atgtatatag accatgagca gcagattgtg gcataagaag cagaaagaaa gatgtgggat    1920
ttatattgct tttgtcttct gggcctctct acacagaatc taacaaatct ggcaataatt    1980
ctttgatttg tgtttgaccc atagtttggt tactagtata tgttttttta tgttcttttt    2040
ttctttgtca ttctcttgtc cttcgtgaca ctatgtaatg attaaaagca aataattgat    2100
gcatgagttc aaatgttctt tgaaggatcc atcttattag ctttgtaatt gttgtgatat    2160
cttaatctta ttggttacgt atttcaagtg ctttagaaaa aatgggccta agagattttg    2220
ggg                                                                  2223
```

<210> 23
<211> 597
<212> PRT
<213> Arabidopsis thaliana

<400> 23

Met Cys Gly Ala Lys Ser Asn Leu Cys Ser Ser Lys Thr Leu Thr Glu
1 5 10 15
Val Glu Phe Met Arg Gln Lys Ser Glu Asp Gly Ala Ser Ala Thr Cys
20 25 30
Leu Leu Glu Phe Ala Ala Cys Asp Asp Leu Ser Ser Phe Lys Arg Glu
35 40 45
Ile Glu Glu Asn Pro Ser Val Glu Ile Asp Glu Ser Gly Phe Trp Tyr
50 55 60
Cys Arg Arg Val Gly Ser Lys Lys Met Gly Phe Glu Glu Arg Thr Pro
65 70 75 80
Leu Met Val Ala Ala Met Tyr Gly Ser Met Glu Val Leu Asn Tyr Ile
85 90 95
Ile Ala Thr Gly Arg Ser Asp Val Asn Arg Val Cys Ser Asp Glu Lys
100 105 110
Val Thr Ala Leu His Cys Ala Val Ser Gly Cys Ser Val Ser Ile Val
115 120 125
Glu Ile Ile Lys Ile Leu Leu Asp Ala Ser Ala Ser Pro Asn Cys Val
130 135 140
Asp Ala Asn Gly Asn Lys Pro Val Asp Leu Leu Ala Lys Asp Ser Arg
145 150 155 160
Phe Val Pro Asn Gln Ser Arg Lys Ala Val Glu Val Leu Leu Thr Gly
165 170 175
Ile His Gly Ser Val Met Glu Glu Glu Glu Glu Glu Leu Lys Ser Val
180 185 190
Val Thr Lys Tyr Pro Ala Asp Ala Ser Leu Pro Asp Ile Asn Glu Gly
195 200 205
Val Tyr Gly Thr Asp Asp Phe Arg Met Phe Ser Phe Lys Val Lys Pro
210 215 220
Cys Ser Arg Ala Tyr Ser His Asp Trp Thr Glu Cys Pro Phe Val His
225 230 235 240
Pro Gly Glu Asn Ala Arg Arg Arg Asp Pro Arg Lys Tyr Pro Tyr Thr
245 250 255
Cys Val Pro Cys Pro Glu Phe Arg Lys Gly Ser Cys Pro Lys Gly Asp
260 265 270
Ser Cys Glu Tyr Ala His Gly Val Phe Glu Ser Trp Leu His Pro Ala
275 280 285
Gln Tyr Arg Thr Arg Leu Cys Lys Asp Glu Thr Gly Cys Ala Arg Arg
290 295 300
Val Cys Phe Phe Ala His Arg Arg Asp Glu Leu Arg Pro Val Asn Ala
305 310 315 320

```
Ser Thr Gly Ser Ala Met Val Ser Pro Arg Ser Ser Asn Gln Ser Pro
                325                 330                 335
Glu Met Ser Val Met Ser Pro Leu Thr Leu Gly Ser Ser Pro Met Asn
            340                 345                 350
Ser Pro Met Ala Asn Gly Val Pro Leu Ser Pro Arg Asn Gly Gly Leu
        355                 360                 365
Trp Gln Asn Arg Val Asn Ser Leu Thr Pro Pro Pro Leu Gln Leu Asn
    370                 375                 380
Gly Ser Arg Leu Lys Ser Thr Leu Ser Ala Arg Asp Met Asp Met Glu
385                 390                 395                 400
Met Glu Leu Arg Phe Arg Gly Leu Asp Asn Arg Arg Leu Gly Asp Leu
                405                 410                 415
Lys Pro Ser Asn Leu Glu Glu Thr Phe Gly Ser Tyr Asp Ser Ala Ser
            420                 425                 430
Val Met Gln Leu Gln Ser Pro Ser Arg His Ser Gln Met Asn His Tyr
        435                 440                 445
Pro Ser Ser Pro Val Arg Gln Pro Pro Pro His Gly Phe Glu Ser Ser
    450                 455                 460
Ala Ala Met Ala Ala Ala Val Met Asn Ala Arg Ser Ser Ala Phe Ala
465                 470                 475                 480
Lys Arg Ser Leu Ser Phe Lys Pro Ala Pro Val Ala Ser Asn Val Ser
                485                 490                 495
Asp Trp Gly Ser Pro Asn Gly Lys Leu Glu Trp Gly Met Gln Arg Asp
            500                 505                 510
Glu Leu Asn Lys Leu Arg Arg Ser Ala Ser Phe Gly Ile His Gly Asn
        515                 520                 525
Asn Asn Asn Ser Val Ser Arg Pro Ala Arg Asp Tyr Ser Asp Glu Pro
    530                 535                 540
Asp Val Ser Trp Val Asn Ser Leu Val Lys Glu Asn Ala Pro Glu Arg
545                 550                 555                 560
Val Asn Glu Arg Val Gly Asn Thr Val Asn Gly Ala Ala Ser Arg Asp
                565                 570                 575
Lys Phe Lys Leu Pro Ser Trp Ala Glu Gln Met Tyr Ile Asp His Glu
            580                 585                 590
Gln Gln Ile Val Ala
        595
```

<210> 24
<211> 1761
<212> DNA
<213> Arabidopsis thaliana

<400> 24

```
atggaaaaag atagtattat gtgcagtgga ccaaagagca atctctgctc ttcaagaacc      60
ttaacagaaa tcgaatcaag gcaaaaggaa gaagaaacaa tgcttctcct cgaattcgct     120
gcttgtgatg atcttgactc gttcaagaga gaggttgaag agaaagggct tgatttggat     180
gagtcagggt tatggtattg cagacgtgtc ggttctaaga agatgggtct tgaagaaaga     240
acacctttaa tggttgcagc tatgtatgga agcataaagg ttttgacttt catcgtttcc     300
actggaaaat ctgatgtgaa cagagcttgt ggtgaagaga gagttactcc gcttcactgt     360
gctgttgctg gctgttctgt gaatatgatt gaagtcatca atgtcttgct tgatgcttct     420
gctttggtta actctgttga tgctaatggg aatcaacctt tggatgtgtt tgttcgagtt     480
tcgaggtttg tggctagtcc gaggaggaaa gcggttgagt tgttgctgag aggaggaggt     540
gttggaggat tgatcgatga ggcggttgaa gaagagatca agattgtctc taagtatcca     600
gctgatgctt ctttaccgga tataaacgaa ggggtttatg gaagtgatga gtttaggatg     660
tatagcttta aggttaagcc atgttctagg gcttattctc atgattggac cgagtgtgct     720
tttgttcatc cgggagaaaa tgcgaggagg agagatccga ggaagtatcc ttacacttgt     780
gtcccctgtc ccgagttccg taaaggatca tgcccgaaag gagattcttg cgagtatgct     840
cacggggttt tcgagtcgtg gcttcacccc gcgcagtata aaacccggct ttgtaaagat     900
gaaacgggtt gtgcaaggaa agtttgtttc tttgctcata aacgcgaaga gatgagacct     960

gttaatgctt caactggctc tgccgtggct cagtctccgt ttagcagctt ggagatgatg    1020
ccagggttgt ctcctcttgc ttattcttca ggagtttcga ctcctccggt ttctccaatg    1080
gctaatggtg ttccttcctc tccaagaaac ggcggatcat ggcagaacag agtcaatacc    1140
cttactccac cggctttgca gctcaatggt ggaagcagat tgaagtccac actgagcgct    1200
agagatatcg atatggagat ggagatggaa ttgagactcc gcggttttgg caacaatgtg    1260
gaagagacgt tcgggtctta tgtttcctct ccaagtagga attctcaaat gggtcaaaac    1320
atgaaccaac attatccatc ttccccggtg agacaaccgc catctcaaca cgggttcgaa    1380
tcttcagcag ctgcagcggt tgcagtgatg aaagcgagat caaccgcctt tgcgaaacgt    1440
agcttgagct tcaaaccagc tactcaagca gcaccacagt cgaatctctc ggattgggga    1500
tctccaaacg ggaagctgga atggggaatg aaaggagaag agctgaataa gatgagaaga    1560
agtgtttcct ttggaatcca tggaaacaac aacaataacg cagctagaga ctacagggac    1620
gagccagatg tgtcatgggt taactcttta gttaaagaca gtactgtggt gtctgagaga    1680
agctttggaa tgaatgagag ggttcggata atgtcgtggg ctgagcaaat gtacagagag    1740
aaggagcaga ctgtggtgta a                                               1761
```

<210> 25
<211> 586
<212> PRT
<213> Arabidopsis thaliana

<400> 25

```
Met Glu Lys Asp Ser Ile Met Cys Ser Gly Pro Lys Ser Asn Leu Cys
1               5                   10                  15
Ser Ser Arg Thr Leu Thr Glu Ile Glu Ser Arg Gln Lys Glu Glu Glu
            20                  25                  30
Thr Met Leu Leu Leu Glu Phe Ala Ala Cys Asp Asp Leu Asp Ser Phe
        35                  40                  45
Lys Arg Glu Val Glu Glu Lys Gly Leu Asp Leu Asp Glu Ser Gly Leu
    50                  55                  60
Trp Tyr Cys Arg Arg Val Gly Ser Lys Lys Met Gly Leu Glu Glu Arg
65                  70                  75                  80
Thr Pro Leu Met Val Ala Ala Met Tyr Gly Ser Ile Lys Val Leu Thr
                85                  90                  95
Phe Ile Val Ser Thr Gly Lys Ser Asp Val Asn Arg Ala Cys Gly Glu
            100                 105                 110
Glu Arg Val Thr Pro Leu His Cys Ala Val Ala Gly Cys Ser Val Asn
        115                 120                 125
Met Ile Glu Val Ile Asn Val Leu Leu Asp Ala Ser Ala Leu Val Asn
    130                 135                 140
Ser Val Asp Ala Asn Gly Asn Gln Pro Leu Asp Val Phe Val Arg Val
145                 150                 155                 160
Ser Arg Phe Val Ala Ser Pro Arg Arg Lys Ala Val Glu Leu Leu Leu
                165                 170                 175
Arg Gly Gly Gly Val Gly Gly Leu Ile Asp Glu Ala Val Glu Glu Glu
            180                 185                 190
Ile Lys Ile Val Ser Lys Tyr Pro Ala Asp Ala Ser Leu Pro Asp Ile
        195                 200                 205
Asn Glu Gly Val Tyr Gly Ser Asp Glu Phe Arg Met Tyr Ser Phe Lys
    210                 215                 220
Val Lys Pro Cys Ser Arg Ala Tyr Ser His Asp Trp Thr Glu Cys Ala
225                 230                 235                 240
Phe Val His Pro Gly Glu Asn Ala Arg Arg Arg Asp Pro Arg Lys Tyr
                245                 250                 255
Pro Tyr Thr Cys Val Pro Cys Pro Glu Phe Arg Lys Gly Ser Cys Pro
            260                 265                 270
Lys Gly Asp Ser Cys Glu Tyr Ala His Gly Val Phe Glu Ser Trp Leu
        275                 280                 285
His Pro Ala Gln Tyr Lys Thr Arg Leu Cys Lys Asp Glu Thr Gly Cys
    290                 295                 300
```

```
Ala Arg Lys Val Cys Phe Phe Ala His Lys Arg Glu Glu Met Arg Pro
305                 310                 315                 320
Val Asn Ala Ser Thr Gly Ser Ala Val Ala Gln Ser Pro Phe Ser Ser
                325                 330                 335
Leu Glu Met Met Pro Gly Leu Ser Pro Leu Ala Tyr Ser Ser Gly Val
            340                 345                 350
Ser Thr Pro Pro Val Ser Pro Met Ala Asn Gly Val Pro Ser Ser Pro
        355                 360                 365
Arg Asn Gly Gly Ser Trp Gln Asn Arg Val Asn Thr Leu Thr Pro Pro
    370                 375                 380
Ala Leu Gln Leu Asn Gly Gly Ser Arg Leu Lys Ser Thr Leu Ser Ala
385                 390                 395                 400
Arg Asp Ile Asp Met Glu Met Glu Met Glu Leu Arg Leu Arg Gly Phe
                405                 410                 415
Gly Asn Asn Val Glu Glu Thr Phe Gly Ser Tyr Val Ser Ser Pro Ser
            420                 425                 430
Arg Asn Ser Gln Met Gly Gln Asn Met Asn Gln His Tyr Pro Ser Ser
        435                 440                 445
Pro Val Arg Gln Pro Pro Ser Gln His Gly Phe Glu Ser Ser Ala Ala
    450                 455                 460
Ala Ala Val Ala Val Met Lys Ala Arg Ser Thr Ala Phe Ala Lys Arg
465                 470                 475                 480
Ser Leu Ser Phe Lys Pro Ala Thr Gln Ala Ala Pro Gln Ser Asn Leu
                485                 490                 495
Ser Asp Trp Gly Ser Pro Asn Gly Lys Leu Glu Trp Gly Met Lys Gly
            500                 505                 510
Glu Glu Leu Asn Lys Met Arg Arg Ser Val Ser Phe Gly Ile His Gly
        515                 520                 525
Asn Asn Asn Asn Asn Ala Ala Arg Asp Tyr Arg Asp Glu Pro Asp Val
    530                 535                 540
Ser Trp Val Asn Ser Leu Val Lys Asp Ser Thr Val Val Ser Glu Arg
545                 550                 555                 560
Ser Phe Gly Met Asn Glu Arg Val Arg Ile Met Ser Trp Ala Glu Gln
                565                 570                 575
Met Tyr Arg Glu Lys Glu Gln Thr Val Val
            580                 585
```

<210> 26
<211> 2709
<212> DNA
<213> Eucalyptus grandis

<400> 26

```
cttctgaaag cttttgact taagacgaga gagaaggaga gaaggtcccc ctcctcgtcc      60
tcgtcccccc gtggatttg aagaagaaaa gtcgcacctt ccttctcctt tcccactcct     120
ccctctgctc gaagctttc tcttccgcag aattacataa aaacctcgac tttgcgcatc     180
attccgattc acctcacacc ttcactttcc cactcgaggt ctcccccctc ttttcctagc     240
tctttccctt tccctccctc tctctcgaga atcgccgcat ttggaggagc tccaatctgc     300
tttgctttgc tttgctctct tcttgctcgg ttccccctca taaggagtcg attatgtgca     360
acggttcttc gaagggtaaa cttttcccct cgagtatggg catggagggc gaattccaca     420
acaaggatgg cgaagcaccc cgtaaatgct ctgccttgct tgaattggca gcctcggacg     480
atctctcgtc gttcaaaagt gaagtggaag agaagggctg cgacgttgat gaggccagct     540
tttggtatgg taggagaatc gggtcgaaga agatgggttt tgaagagagg actccattga     600
tgatctctgc tttgtttgga agcaccaagg tcttgaaata cataatcgag accgccagag     660
ctgatgtcaa caggtcttgt gggtccgaca aggtggccgc cctccattgc gcagccgcgg     720
gtgggtccag ttcttcactt gaaattgtga agctcttgat tgaggcctca gcggatatta     780
attctgtaga tggcaatgga aataggccca tcgacgtgct tgccccggca gggaagtctc     840
gctgcaattc cagaaataag tttgttagat cgttgctgaa aggtgaaaac tatgtcgtgg     900
aaggtgacca atcctttgac atagaaggag aggagaagct agtcgctctt ccaaaggagg     960

gaggcgagaa gaaagagtat cctgttgatg tctctctacc tgacataaac aatgggttct    1020
acagtaccga tgagttccgg atgtatgctt tcaaggtgaa gccttgctcg agggcttact    1080
cccacgactg gaccgagtgc ccgtttgtgc accctgggga gaacgcgagg aggagggacc    1140
cacgcaagta cccttacagc tgtgtccctt gtcctgagtt tcgcaagggt tcgtgcgtaa    1200
ggggggatgc ttgtgagtat gctcatggag tctttgagtc gtggcttcac ccagcgcaat    1260
accgaacccg gctgtgcaag gatgaaactg gttgtactcg caaagtttgc ttctttgctc    1320
acaagtccga agaattgcgt cccgtgtatg cttccacagg ttctgctatg ccctcaccca    1380
agtccttttc agctaatgcc ctagacatga caaccctgag ccccttatcc cttaattcac    1440
catctctgcc tttgcctgct acttccacgc cccccatgtc acctttggct gcctcatctt    1500
cacccaaggg catgaacttg tggcataaca aaattaacct gaccccacca agcctgcagc    1560
ttcctggcag ccggctgaag acggctatga gtgcgcggga cttcgatttt gagttggaat    1620
ttcttgggct ggaaaagcaa gcttctcagc ggcagcaact gatagaagag atttctcgtc    1680
tctcatcgcc ctctcatatg tggaactcgg aatttggcag aaccgcagag ctgaagccca    1740
ctaaccttga tgatgcgttt ggatctcttg acacttctct tttgtctccg ttgcaggggt    1800
cgtcgatgaa aacatcgact cctacccagt tgcaatcccc cacagggctt aaaatttcga    1860
atttgaacca actccgtgcg agctacccgt ctagcagctt gtcgtcctct cctgtgagga    1920
agacctcttc ttttgggttc gactcatcca gtgcagttgc tgcagcagtc atgaactcac    1980
ggtctgctgc tatgacgaag cggagccaga gcttcattga ccgtggagca gtgggtcaac    2040
ggtctggact cattggacct gctaattctg ctcctaggat gtccaacctt tcggactggg    2100
gctcgcctga tgggaagttg gattggggtg ttcaagggga cgagctcaac aagcttagga    2160
agtccgcttc cttcggcttt agaaacaaca gtatggcgaa cccaaacaac gtggcgtctc    2220
ccagtgctga tgagccggac gtgtcgtggg ttggttcatt ggtgaaggat gtggctccgc    2280
ccgaagggta tccacagtat ctgtacatag aacaggagca gatggtggca taactaaagc    2340
gaagagcacc acacgaactc tctcctgatg gcttaagatg acttgtttga cattctttat    2400
attcttacaa acagcgcgtt cttaggagtt agctggagga aagaaggaaa cggtattgag    2460
tttgagattc aggctcttag ctggacagcg aaaatttggg gaaggaagag aatttggttt    2520
cttgcccaac ttagataatg atgcttttga aggcttaaaa gaaagatgaa ggcaaacatt    2580
cttttgttag tattgtatta ttgttttaat ttttcatccc ctctgtcggg gtgtggtggg    2640
tgtcgatgtt tctttcatca gtaaaatata taatgaggtt tactcatcta ttttctacta    2700
aaaaaaaaa                                                            2709
```

<210> 27
<211> 659
<212> PRT
<213> Eucalyptus grandis

<400> 27

```
Met Cys Asn Gly Ser Ser Lys Gly Lys Leu Phe Pro Ser Ser Met Gly
1               5                   10                  15
Met Glu Gly Glu Phe His Asn Lys Asp Gly Glu Ala Pro Arg Lys Cys
            20                  25                  30
Ser Ala Leu Leu Glu Leu Ala Ala Ser Asp Asp Leu Ser Ser Phe Lys
        35                  40                  45
Ser Glu Val Glu Glu Lys Gly Cys Asp Val Asp Glu Ala Ser Phe Trp
    50                  55                  60
Tyr Gly Arg Arg Ile Gly Ser Lys Lys Met Gly Phe Glu Glu Arg Thr
65                  70                  75                  80
Pro Leu Met Ile Ser Ala Leu Phe Gly Ser Thr Lys Val Leu Lys Tyr
                85                  90                  95
Ile Ile Glu Thr Ala Arg Ala Asp Val Asn Arg Ser Cys Gly Ser Asp
            100                 105                 110
Lys Val Ala Ala Leu His Cys Ala Ala Ala Gly Gly Ser Ser Ser Ser
        115                 120                 125
Leu Glu Ile Val Lys Leu Leu Ile Glu Ala Ser Ala Asp Ile Asn Ser
    130                 135                 140
Val Asp Gly Asn Gly Asn Arg Pro Ile Asp Val Leu Ala Pro Ala Gly
145                 150                 155                 160
Lys Ser Arg Cys Asn Ser Arg Asn Lys Phe Val Arg Ser Leu Leu Lys
                165                 170                 175
```

Gly Glu Asn Tyr Val Val Glu Gly Asp Gln Ser Phe Asp Ile Glu Gly
180 185 190
Glu Glu Lys Leu Val Ala Leu Pro Lys Glu Gly Gly Glu Lys Lys Glu
195 200 205
Tyr Pro Val Asp Val Ser Leu Pro Asp Ile Asn Asn Gly Phe Tyr Ser
210 215 220
Thr Asp Glu Phe Arg Met Tyr Ala Phe Lys Val Lys Pro Cys Ser Arg
225 230 235 240
Ala Tyr Ser His Asp Trp Thr Glu Cys Pro Phe Val His Pro Gly Glu
245 250 255
Asn Ala Arg Arg Arg Asp Pro Arg Lys Tyr Pro Tyr Ser Cys Val Pro
260 265 270
Cys Pro Glu Phe Arg Lys Gly Ser Cys Val Arg Gly Asp Ala Cys Glu
275 280 285
Tyr Ala His Gly Val Phe Glu Ser Trp Leu His Pro Ala Gln Tyr Arg
290 295 300
Thr Arg Leu Cys Lys Asp Glu Thr Gly Cys Thr Arg Lys Val Cys Phe
305 310 315 320
Phe Ala His Lys Ser Glu Glu Leu Arg Pro Val Tyr Ala Ser Thr Gly
325 330 335
Ser Ala Met Pro Ser Pro Lys Ser Phe Ser Ala Asn Ala Leu Asp Met
340 345 350
Thr Thr Leu Ser Pro Leu Ser Leu Asn Ser Pro Ser Leu Pro Leu Pro
355 360 365
Ala Thr Ser Thr Pro Pro Met Ser Pro Leu Ala Ala Ser Ser Ser Pro
370 375 380
Lys Gly Met Asn Leu Trp His Asn Lys Ile Asn Leu Thr Pro Pro Ser
385 390 395 400
Leu Gln Leu Pro Gly Ser Arg Leu Lys Thr Ala Met Ser Ala Arg Asp
405 410 415
Phe Asp Phe Glu Leu Glu Phe Leu Gly Leu Glu Lys Gln Ala Ser Gln
420 425 430
Arg Gln Gln Leu Ile Glu Glu Ile Ser Arg Leu Ser Ser Pro Ser His
435 440 445
Met Trp Asn Ser Glu Phe Gly Arg Thr Ala Glu Leu Lys Pro Thr Asn
450 455 460
Leu Asp Asp Ala Phe Gly Ser Leu Asp Thr Ser Leu Leu Ser Pro Leu
465 470 475 480
Gln Gly Ser Ser Met Lys Thr Ser Thr Pro Thr Gln Leu Gln Ser Pro
485 490 495
Thr Gly Leu Lys Ile Ser Asn Leu Asn Gln Leu Arg Ala Ser Tyr Pro
500 505 510
Ser Ser Ser Leu Ser Ser Ser Pro Val Arg Lys Thr Ser Ser Phe Gly
515 520 525
Phe Asp Ser Ser Ser Ala Val Ala Ala Ala Val Met Asn Ser Arg Ser
530 535 540
Ala Ala Met Thr Lys Arg Ser Gln Ser Phe Ile Asp Arg Gly Ala Val
545 550 555 560
Gly Gln Arg Ser Gly Leu Ile Gly Pro Ala Asn Ser Ala Pro Arg Met
565 570 575
Ser Asn Leu Ser Asp Trp Gly Ser Pro Asp Gly Lys Leu Asp Trp Gly
580 585 590
Val Gln Gly Asp Glu Leu Asn Lys Leu Arg Lys Ser Ala Ser Phe Gly
595 600 605
Phe Arg Asn Asn Ser Met Ala Asn Pro Asn Asn Val Ala Ser Pro Ser
610 615 620
Ala Asp Glu Pro Asp Val Ser Trp Val Gly Ser Leu Val Lys Asp Val
625 630 635 640
Ala Pro Pro Glu Gly Tyr Pro Gln Tyr Leu Tyr Ile Glu Gln Glu Gln

645 650 655
Met Val Ala

<210> 28
<211> 2518
<212> DNA
<213> Eucalyptus grandis

<400> 28

```
tctccttcga gtttctttct tcactagaat tcgctcccga gtctgttgtt gctcgtgtag      60
ttttgcttac tccgtccttc gtttagctcg ctgaccagcg cggagctagg agcggtcgct     120
aaaggattac tcgtacaaaa cgtaaactca gctctgccaa ttttcccatg gagggggaat     180
cttacttcga gaaagatgaa aaatattcta attgctcaat cttgctcgaa ttatctgctt     240
cggacgatct cccagctttt gaaaggaaag cgaaagagaa gggctgtaac attgatggtg     300
ctagcttctg gtacggtaga agaattggct caaggaagat gggtcttgaa gagaggactc     360
ctctcatggt ggcttccttg tttggaagct ctagggttgt gaagtacatt ctcgaatctg     420
gcaaagtcga tgtaaatagg gcttgtggtt cggacaaggt cactgccctt cactgtgctg     480
ttgccagtgg ctctgcttct gcggtggagg ttgtcaagct cttgcttcac gcatctgccg     540
atgctaattg cattgatggc aatggaaaga agccaattga tgtgatagcc cttccattaa     600
agtcacgcgg cgattcaagg aggaagctga tggagctgtt gctgaaaggc gataattctg     660
atggggaatt tgaatcccac gaggagaagc cgattgccgc accgcaagca tccaaagagg     720
gaagcgaaaa gaaagagtat caatttcctg ttgatatctc tctgcctgac ataaatgttg     780
ggatttacag tactgatgag ttcagaatgt atgctttcaa agtaaagcct tgctcgcggg     840
catactccca tgactggaca gagtgcccat ttgttcatcc tggcgagaat gcgaggaggc     900
gggaccctcg caagtacccc tacagctgcg tcccttgccc tgaatttcgg aagggatctt     960
gccaaaaggg tgactcctgt gagtacgcgc acggcgtatt tgagtcgtgg cttcatcctg    1020
cacagtatag aacaagactg tgcaaggatg agactggatg tgctcgcaaa gtttgtttct    1080
ttgctcacaa gcccgaagaa ttaaggcctg tctatgcttc gacgggatca gctatgcctt    1140
ccccaaaatc ctactcatca agtgggctgg acatgtccac attgagtcct ctctcaatca    1200
gttctccgtc agcatcgttg cctgttactt caacagcacc catgtctcct cttgcagcct    1260
cgtcatctcc gatgtctgtg aacatgtggc agagcaaggc taacaagctc tccccgccaa    1320
tgctgcagct ctcaggtagt aggctgaaga ctgctttgag tgctagggac ttggacctgg    1380
agatggaatt gcgtggtcta gagagtcaga tggccactca acagcatcag ttgatggaag    1440
agatatctcg tctctcctca ccatcatcct gctttagtag taggattggg gaagtgaaac    1500
ccactaacct cgatgacgtt tttgggtctc cggatcctgc tttgctgcct caattgcagg    1560
ggctgtcaag accttcaaca ccaagccagt tgcaatctcc aactgggctt cagatgcgcc    1620
agaatgcaac ccagtttcgt ggggcgtacc agagcaatgc aaatgcattg tcatctccag    1680
caatgaagca ggcaccttct tatgggtttg actcatctag tgcagttgca gcagcggtga    1740
tgaattcgag gtcagccgct tttgcgaagc ggagtcagag ttttatcgac aggggaatgg    1800
cgtgccctgg aattgccaat tcttccccta tgatgtcttc agctatgtcg agctggagct    1860
cacctcatgg gaaattggat tggggcgtcc aaggagatga gttgaatagg ctgaggaaag    1920
ctgcttcctt taagatgaga agcagcaccg gagcaggtgc taatactgtc tcggcagcag    1980
ccatggctga tgagccagat atttcttggg tcagttcatt ggttaaggac gtgccttctg    2040
cggaggacgc gatgttcgct gcagagaaag gacagcgcac ttatgggaaa gacatccgcg    2100
aaaggattac cccatgggtg gagcagctgt acagagaagt gccacggatg gcgatgtaag    2160
attgccactg caagtcggat gccttagtat gctgactaat tgatattctt tgcatttgtt    2220
ttgaggcatt tggtagccat tagatacgag aaaaggccaa gcagcaggtg gtgtcttggc    2280
aaggaatagg atgcacatag tctgttatcg agtagaatag acttgggaac aatggttata    2340
gccaaatgtt aaaagttatg atattctttt ccaattcttt ctcttcctca tagtaggttt    2400
ctcaccaagt cttttagtga gagcctgcgg gatgtactat atgtttccct tatgtaacgt    2460
ctcttcgttg aaagaaatgg ctttataata taaagcatca agttttttaa aaaaaaa       2518
```

<210> 29
<211> 663
<212> PRT
<213> Eucalyptus grandis

<400> 29

```
Met Glu Gly Glu Ser Tyr Phe Glu Lys Asp Glu Lys Tyr Ser Asn Cys
1               5                   10                  15
Ser Ile Leu Leu Glu Leu Ser Ala Ser Asp Asp Leu Pro Ala Phe Glu
            20                  25                  30
Arg Lys Ala Lys Glu Lys Gly Cys Asn Ile Asp Gly Ala Ser Phe Trp
        35                  40                  45
Tyr Gly Arg Arg Ile Gly Ser Arg Lys Met Gly Leu Glu Glu Arg Thr
    50                  55                  60
Pro Leu Met Val Ala Ser Leu Phe Gly Ser Ser Arg Val Val Lys Tyr
65                  70                  75                  80
Ile Leu Glu Ser Gly Lys Val Asp Val Asn Arg Ala Cys Gly Ser Asp
                85                  90                  95
Lys Val Thr Ala Leu His Cys Ala Val Ala Ser Gly Ser Ala Ser Ala
            100                 105                 110
Val Glu Val Val Lys Leu Leu Leu His Ala Ser Ala Asp Ala Asn Cys
        115                 120                 125
Ile Asp Gly Asn Gly Lys Lys Pro Ile Asp Val Ile Ala Leu Pro Leu
    130                 135                 140
Lys Ser Arg Gly Asp Ser Arg Arg Lys Leu Met Glu Leu Leu Leu Lys
145                 150                 155                 160
Gly Asp Asn Ser Asp Gly Glu Phe Glu Ser His Glu Glu Lys Pro Ile
                165                 170                 175
Ala Ala Pro Gln Ala Ser Lys Glu Gly Ser Glu Lys Lys Glu Tyr Gln
            180                 185                 190
Phe Pro Val Asp Ile Ser Leu Pro Asp Ile Asn Val Gly Ile Tyr Ser
        195                 200                 205
Thr Asp Glu Phe Arg Met Tyr Ala Phe Lys Val Lys Pro Cys Ser Arg
    210                 215                 220
Ala Tyr Ser His Asp Trp Thr Glu Cys Pro Phe Val His Pro Gly Glu
225                 230                 235                 240
Asn Ala Arg Arg Arg Asp Pro Arg Lys Tyr Pro Tyr Ser Cys Val Pro
                245                 250                 255
Cys Pro Glu Phe Arg Lys Gly Ser Cys Gln Lys Gly Asp Ser Cys Glu
            260                 265                 270
Tyr Ala His Gly Val Phe Glu Ser Trp Leu His Pro Ala Gln Tyr Arg
        275                 280                 285
Thr Arg Leu Cys Lys Asp Glu Thr Gly Cys Ala Arg Lys Val Cys Phe
    290                 295                 300
Phe Ala His Lys Pro Glu Glu Leu Arg Pro Val Tyr Ala Ser Thr Gly
305                 310                 315                 320
Ser Ala Met Pro Ser Pro Lys Ser Tyr Ser Ser Ser Gly Leu Asp Met
                325                 330                 335
Ser Thr Leu Ser Pro Leu Ser Ile Ser Ser Pro Ser Ala Ser Leu Pro
            340                 345                 350
Val Thr Ser Thr Ala Pro Met Ser Pro Leu Ala Ala Ser Ser Ser Pro
        355                 360                 365
Met Ser Val Asn Met Trp Gln Ser Lys Ala Asn Lys Leu Ser Pro Pro
    370                 375                 380
Met Leu Gln Leu Ser Gly Ser Arg Leu Lys Thr Ala Leu Ser Ala Arg
385                 390                 395                 400
Asp Leu Asp Leu Glu Met Glu Leu Arg Gly Leu Glu Ser Gln Met Ala
                405                 410                 415
Thr Gln Gln His Gln Leu Met Glu Glu Ile Ser Arg Leu Ser Ser Pro
            420                 425                 430
Ser Ser Cys Phe Ser Ser Arg Ile Gly Glu Val Lys Pro Thr Asn Leu
        435                 440                 445
Asp Asp Val Phe Gly Ser Pro Asp Pro Ala Leu Leu Pro Gln Leu Gln
    450                 455                 460
Gly Leu Ser Arg Pro Ser Thr Pro Ser Gln Leu Gln Ser Pro Thr Gly
```

```
465                 470                 475                 480
Leu Gln Met Arg Gln Asn Ala Thr Gln Phe Arg Gly Ala Tyr Gln Ser
                485                 490                 495
Asn Ala Asn Ala Leu Ser Ser Pro Ala Met Lys Gln Ala Pro Ser Tyr
            500                 505                 510
Gly Phe Asp Ser Ser Ser Ala Val Ala Ala Ala Val Met Asn Ser Arg
        515                 520                 525
Ser Ala Ala Phe Ala Lys Arg Ser Gln Ser Phe Ile Asp Arg Gly Met
    530                 535                 540
Ala Cys Pro Gly Ile Ala Asn Ser Ser Pro Met Met Ser Ser Ala Met
545                 550                 555                 560
Ser Ser Trp Ser Ser Pro His Gly Lys Leu Asp Trp Gly Val Gln Gly
                565                 570                 575
Asp Glu Leu Asn Arg Leu Arg Lys Ala Ala Ser Phe Lys Met Arg Ser
            580                 585                 590
Ser Thr Gly Ala Gly Ala Asn Thr Val Ser Ala Ala Ala Met Ala Asp
        595                 600                 605
Glu Pro Asp Ile Ser Trp Val Ser Ser Leu Val Lys Asp Val Pro Ser
    610                 615                 620
Ala Glu Asp Ala Met Phe Ala Ala Glu Lys Gly Gln Arg Thr Tyr Gly
625                 630                 635                 640
Lys Asp Ile Arg Glu Arg Ile Thr Pro Trp Val Glu Gln Leu Tyr Arg
                645                 650                 655
Glu Val Pro Arg Met Ala Met
            660
```

<210> 30
<211> 2001
<212> DNA
<213> Triticum aestivum

<220>
<221> misc_feature
<222> (481)..(530)
<223> n is a, c, g, or t

<400> 30

```
cgaattccgg tcgacgattt ctcgatttcc ttctctataa cacaacgctc tcttctcttg      60
caaccaaagt acttgttcca gtgtctactc tactcaaaaa ggatttggga catcatgtgc     120
agtgattcga aaagtaaact ttcttcccca accctcgtcg tcatggagaa tagtaacatt     180
cagaagcaga atctggatgg tctctacaac tcggttttgc ttgaattgtc tgcatctgat     240
gattatgaag ctttcaaaag agaggtggag gaaaaaggct tagatgtgaa cgaggcaggc     300
ttttggtacg gtagaagaat tgggtcaaag aagatgggat ctgaaacgag gacccctctg     360
atgattgctt ctttgtttgg aagcgccaag gtgctcaatt atattcttct tcagaaagga     420
ggaggtgttg atgtgaacag ggtctgtggt tctgataggg ccactgctct ccattgtgct     480
nnnnnnnnnn nnnnnnnnnn nnnnnnnnnn nnnnnnnnnn nnnnnnnnnn tcccgggtcg     540
cgatttcgta ggagattcac agagagaaaa gaagcaatta gtgataataa gaaagaatac     600
cctgttgata tatcactgcc agacataaac aacggtgtat atggaacaga tgattttagg     660
atgtacaact tcaaggtgaa gccttgctca agggcttact cccatgactg gaccgagtgt     720
ccattcgttc acccagggga gaacgctagg aggagagacc cacggaaata cccttacagc     780
tgtgttcctt gccctgagtt ccgcaaaggg acctgccaga agggtgattc ctgtgagtat     840
gctcatggtg tttttgagtc ctggctgcat cctgcccaat accggacaag gctttgcaag     900
gatgagactg gctgcgctag aaaagtctgc ttctttgccc acaaacctga agagctacgc     960
cctgtgtatg cttccactgg gtcggctatg ccatcaccaa aatcatattc agctagtgga    1020
cttgacatga cagcgatgag tccattggct ctaagttcca catctttgcc taatgccccc    1080
ccgtttccag cctcacccta tcgtgcgccc tcgttcttct ctcagagtga agctgtgcag    1140
aacaaaataa accttactcc accatcgttg cagctccctg gtagccgact gaaggctgct    1200
ttgagtgcca gggatctgga gatggagatg gaactgctcg gtctagaaag ccctgctcgc    1260
caacaacagc agcagcagca acaattgatc gaagagattg ccaggatctc ttccccatct    1320

ttccggagca aggaattcaa taggattgtt gatttgaatc ctactaacct tgatgacctg    1380
ttagcatctg ctgacccttc tgtattttct caactacatg gactttctgt gcaaccttca    1440
acacccacac aaagtgggct tcagatgcgc caaaacatga accacctccg tgcgagttat    1500
ccatccaaca tcccttcctc tcctgtgagg aagccctcag cttttgggtt tgactcatca    1560
gctgctgtgg caactgcagt gatgaattct aggtctgctg ccttcgcaaa gcgaagccaa    1620
agtttcattg atcgtggagc tgcaacccac catcttgggc tgtcttcagc ttccaactct    1680
tcttgcaggg tatcctctac cctttcagat tggagttccc ctaccgggaa actggattgg    1740
ggtgtaaacg gagacaagct gaacaagctg aggaaatcta cttcctttgg attcagaaac    1800
agtggggtaa ctgcatcccc catagcacag cctgaatttg gtgctgagcc ggatgtctca    1860
tgggttcatt cattggttaa agatgttccc tccgagaggt ctgagatatt tggtgctgag    1920
aagcaacaat atgatctcag taaagagatg cttccaccat ggatggagca gctgtatata    1980
gagcaggagc agatggtagc a                                              2001
```

<210> 31
<211> 667
<212> PRT
<213> Triticum aestivum

<220>
<221> UNSURE
<222> (161)..(177)
<223> Xaa can be any naturally occurring amino acid

<400> 31

```
Arg Ile Pro Val Asp Asp Phe Ser Ile Ser Phe Ser Ile Thr Gln Arg
1               5                   10                  15
Ser Leu Leu Leu Gln Pro Lys Tyr Leu Phe Gln Cys Leu Leu Tyr Ser
            20                  25                  30
Lys Arg Ile Trp Asp Ile Met Cys Ser Asp Ser Lys Ser Lys Leu Ser
        35                  40                  45
Ser Pro Thr Leu Val Val Met Glu Asn Ser Asn Ile Gln Lys Gln Asn
    50                  55                  60
Leu Asp Gly Leu Tyr Asn Ser Val Leu Leu Glu Leu Ser Ala Ser Asp
65                  70                  75                  80
Asp Tyr Glu Ala Phe Lys Arg Glu Val Glu Glu Lys Gly Leu Asp Val
                85                  90                  95
Asn Glu Ala Gly Phe Trp Tyr Gly Arg Arg Ile Gly Ser Lys Lys Met
            100                 105                 110
Gly Ser Glu Thr Arg Thr Pro Leu Met Ile Ala Ser Leu Phe Gly Ser
        115                 120                 125
Ala Lys Val Leu Asn Tyr Ile Leu Leu Gln Lys Gly Gly Gly Val Asp
    130                 135                 140
Val Asn Arg Val Cys Gly Ser Asp Arg Ala Thr Ala Leu His Cys Ala
145                 150                 155                 160
Xaa Xaa Xaa Xaa Xaa Xaa Xaa Xaa Xaa Xaa Xaa Xaa Xaa Xaa Xaa Xaa
                165                 170                 175
Xaa Pro Gly Ser Arg Phe Arg Arg Arg Phe Thr Glu Arg Lys Glu Ala
            180                 185                 190
Ile Ser Asp Asn Lys Lys Glu Tyr Pro Val Asp Ile Ser Leu Pro Asp
        195                 200                 205
Ile Asn Asn Gly Val Tyr Gly Thr Asp Asp Phe Arg Met Tyr Asn Phe
    210                 215                 220
Lys Val Lys Pro Cys Ser Arg Ala Tyr Ser His Asp Trp Thr Glu Cys
225                 230                 235                 240
Pro Phe Val His Pro Gly Glu Asn Ala Arg Arg Arg Asp Pro Arg Lys
                245                 250                 255
Tyr Pro Tyr Ser Cys Val Pro Cys Pro Glu Phe Arg Lys Gly Thr Cys
            260                 265                 270
Gln Lys Gly Asp Ser Cys Glu Tyr Ala His Gly Val Phe Glu Ser Trp
```

```
                275                     280                     285
Leu His Pro Ala Gln Tyr Arg Thr Arg Leu Cys Lys Asp Glu Thr Gly
    290                     295                     300
Cys Ala Arg Lys Val Cys Phe Phe Ala His Lys Pro Glu Glu Leu Arg
305                     310                     315                 320
Pro Val Tyr Ala Ser Thr Gly Ser Ala Met Pro Ser Pro Lys Ser Tyr
                    325                     330                 335
Ser Ala Ser Gly Leu Asp Met Thr Ala Met Ser Pro Leu Ala Leu Ser
                340                     345                 350
Ser Thr Ser Leu Pro Asn Ala Pro Pro Phe Pro Ala Ser Pro Tyr Arg
            355                     360                 365
Ala Pro Ser Phe Phe Ser Gln Ser Glu Ala Val Gln Asn Lys Ile Asn
    370                     375                 380
Leu Thr Pro Pro Ser Leu Gln Leu Pro Gly Ser Arg Leu Lys Ala Ala
385                     390                 395                 400
Leu Ser Ala Arg Asp Leu Glu Met Glu Met Glu Leu Leu Gly Leu Glu
                    405                     410                 415
Ser Pro Ala Arg Gln Gln Gln Gln Gln Gln Gln Gln Leu Ile Glu Glu
                420                     425                 430
Ile Ala Arg Ile Ser Ser Pro Ser Phe Arg Ser Lys Glu Phe Asn Arg
            435                     440                 445
Ile Val Asp Leu Asn Pro Thr Asn Leu Asp Asp Leu Leu Ala Ser Ala
    450                     455                 460
Asp Pro Ser Val Phe Ser Gln Leu His Gly Leu Ser Val Gln Pro Ser
465                     470                 475                 480
Thr Pro Thr Gln Ser Gly Leu Gln Met Arg Gln Asn Met Asn His Leu
                    485                     490                 495
Arg Ala Ser Tyr Pro Ser Asn Ile Pro Ser Ser Pro Val Arg Lys Pro
                500                     505                 510
Ser Ala Phe Gly Phe Asp Ser Ser Ala Ala Val Ala Thr Ala Val Met
            515                     520                 525
Asn Ser Arg Ser Ala Ala Phe Ala Lys Arg Ser Gln Ser Phe Ile Asp
    530                     535                 540
Arg Gly Ala Ala Thr His His Leu Gly Leu Ser Ser Ala Ser Asn Ser
545                     550                 555                 560
Ser Cys Arg Val Ser Ser Thr Leu Ser Asp Trp Ser Ser Pro Thr Gly
                    565                     570                 575
Lys Leu Asp Trp Gly Val Asn Gly Asp Lys Leu Asn Lys Leu Arg Lys
                580                     585                 590
Ser Thr Ser Phe Gly Phe Arg Asn Ser Gly Val Thr Ala Ser Pro Ile
            595                     600                 605
Ala Gln Pro Glu Phe Gly Ala Glu Pro Asp Val Ser Trp Val His Ser
    610                     615                 620
Leu Val Lys Asp Val Pro Ser Glu Arg Ser Glu Ile Phe Gly Ala Glu
625                     630                 635                 640
Lys Gln Gln Tyr Asp Leu Ser Lys Glu Met Leu Pro Pro Trp Met Glu
                    645                     650                 655
Gln Leu Tyr Ile Glu Gln Glu Gln Met Val Ala
                660                     665
```

<210> 32
<211> 2683
<212> DNA
<213> Eucalyptus grandis

<400> 32

```
gcaaaggtcg atcacttcct ccctagaaag cgagtgtgga gttgaagctt gataaccaga     60
ggccgcctct cgtctcgtct cgcccgcctg cgcttgctct gctctccgcg tgccaaggga    120
gtgttcctag gtgctgaatc tttccatgtg tagcggttca aaagggaagg gagagtgaat    180

tcgagaagca gaggatgtcg gcccgtcagt tctcgatcct gctcgagtta tctgctgcgg    240
atgatctgac gaactttaag aaagcagttg aggaagacgg ctacgatatt gatgagtcga    300
gcttgtggta tggtaggagg atcgggtcga agaagattgg gcttgaagag agaactcccc    360
tcatgattgc cgcgatgttc ggcagtatgt ccgtgctgga ttatattatc aagtctggcc    420
gggccaatgt aaacaaggcg tgtggttcag atggtgctac cgcgcttcac tgtgctgcgg    480
ctggtggctc ggtacaatct cctgaggtgg tcaagctgtt gcttgattct tcagcgaatg    540
ctaactccat tgatgcgaat gggaaacgag cgggagactt gatttctgag gtctctggtt    600
cgcccttcaa ttcgagaagg aagactttgg atgtcatgtt gactggaggt gggactgttg    660
agtttgttga ggaaacttac aatctgcctg agaatctggg tagtcaaatt gaaggaaacg    720
aacaaagaga gagtccaacg gcccgcgctt ccaaggatgg ttctgaaaag aaagagtatc    780
ctgtcgacct ttctcttccg gacatcaaca atggaatata tagcacagat gagtttagga    840
tgtattcttt caaagtgaag ccttgctcga gagcttactc tcatgactgg actgagtgtc    900
catttgttca ccctggggag aatgcaagac ggcgtgaccc acggaaatat cactacagct    960
gtgtgccttg ccctgagttc cgcaaggggt catgcaggca aggggatggc tgcgagtatg   1020
ctcatggtat atttgagtgc tggcttcacc cagctcaata tcgcacccgt ctctgtaagg   1080
atgagattgg atgcaccaga aaagtctgtt tctttgccca caaacatgaa gagcttcgtc   1140
cattgtatgc atcaactggt tcggcgcttc cttctccaag atcattttcg cccgttgctg   1200
cttctctaga catgggatca ctgagccctc tctctctcgg ttcttcttca gtccggatac   1260
cgccaacttc aacaccacct atgactccat caggggcctc ttctcccctt ggtgggtcga   1320
tgtggaaaag ccaaattaat agcactccgc ctggcttgca gcttccaggt agcaggttga   1380
gaagcgcatt gagtgctaga gacatggatt tagatgttga cttgatcgat ctagaaaata   1440
attatcgttt gcagaagcag ttgctcgaac actttcctga tctgtcctct cctcgtggtt   1500
ggaacaactc ttcatccacc acgtcggctt tccctgagta ttcaggtgac atgactggag   1560
aaataagtag gttaggagta aaaccaaata atctcgagga tagtttcagg tcattggacc   1620
tgaccctctt gtctcagtta caagggctgt cacttgatgg tgcaatatcc cagctgcaat   1680
ctcctactgg aatgaagatt cggcagaaca tgacccagca gctctactca aactatactg   1740
acaagctttc ctcgtcacct agggcaatgc catcatttgg aaccgatcct tccagagctt   1800
cagcagcagc cactctgagt tccaggtcat tggcatttgc aaaaaggagc cacagcttca   1860
ttgagcggag tacagtgaac agtcagtctg gatattcagc aggtgctgct tctccaactg   1920
caaggatgtc ttcccagaat gactggggct cgcccgatgg caaactagac tggggcattc   1980
aaggggagga gctgaacaag ctgaggaaat ctgcatcatt cgggctcagg agcagcagca   2040
accgcttcca tgcgtctgca gattctgcga cagcaactgt aggggaccca gacatgccct   2100
ggattcagtc cttggcaaag gaagccccgt cacaaaaccc tggcaatttt ggagcagagc   2160
atcagcagca gcagcagcag cagcagcagc agcagtatca tcttaattct ggaggtactg   2220
agctgcttcc agcttgggtg gagcagttgt acgcggatca ggagcagatg gtcgcctgag   2280
atcaacattg gcttcttatc taaccactat tagtcatttc gttattgctt taattttttt   2340
tcttctgagt ctagtattaa tgtctaggat tcgaacgaac tggaaaatta aatctagagg   2400
gaagatggga agaaaagagc aggatggaag gtttctgctc ggtccgagat ttctcatagt   2460
ctattataga ctatcgtatt tctcgttctt ttccgtccca atgttcttga tttggttctc   2520
agcatgtttt ctggatgagg cttacaaact atgtaatctt gtcttgctaa aagaatcaga   2580
gctgcacctg caccaaaggt tgtgatacta ccgcttattg atgatgatga taataataat   2640
aattcggaca tttagtacca agtccgatgt ctcaaaaaaa aaa                      2683
```

<210> 33
<211> 694
<212> PRT
<213> Eucalyptus grandis

<400> 33

```
Met Ser Ala Arg Gln Phe Ser Ile Leu Leu Glu Leu Ser Ala Ala Asp
1               5                   10                  15
Asp Leu Thr Asn Phe Lys Lys Ala Val Glu Glu Asp Gly Tyr Asp Ile
            20                  25                  30
Asp Glu Ser Ser Leu Trp Tyr Gly Arg Arg Ile Gly Ser Lys Lys Ile
        35                  40                  45
Gly Leu Glu Glu Arg Thr Pro Leu Met Ile Ala Ala Met Phe Gly Ser
    50                  55                  60
Met Ser Val Leu Asp Tyr Ile Ile Lys Ser Gly Arg Ala Asn Val Asn
65                  70                  75                  80
```

```
Lys Ala Cys Gly Ser Asp Gly Ala Thr Ala Leu His Cys Ala Ala Ala
                85                  90                  95
Gly Gly Ser Val Gln Ser Pro Glu Val Val Lys Leu Leu Leu Asp Ser
            100                 105                 110
Ser Ala Asn Ala Asn Ser Ile Asp Ala Asn Gly Lys Arg Ala Gly Asp
        115                 120                 125
Leu Ile Ser Glu Val Ser Gly Ser Pro Phe Asn Ser Arg Arg Lys Thr
    130                 135                 140
Leu Asp Val Met Leu Thr Gly Gly Gly Thr Val Glu Phe Val Glu Glu
145                 150                 155                 160
Thr Tyr Asn Leu Pro Glu Asn Leu Gly Ser Gln Ile Glu Gly Asn Glu
                165                 170                 175
Gln Arg Glu Ser Pro Thr Ala Arg Ala Ser Lys Asp Gly Ser Glu Lys
            180                 185                 190
Lys Glu Tyr Pro Val Asp Leu Ser Leu Pro Asp Ile Asn Asn Gly Ile
        195                 200                 205
Tyr Ser Thr Asp Glu Phe Arg Met Tyr Ser Phe Lys Val Lys Pro Cys
    210                 215                 220
Ser Arg Ala Tyr Ser His Asp Trp Thr Glu Cys Pro Phe Val His Pro
225                 230                 235                 240
Gly Glu Asn Ala Arg Arg Arg Asp Pro Arg Lys Tyr His Tyr Ser Cys
                245                 250                 255
Val Pro Cys Pro Glu Phe Arg Lys Gly Ser Cys Arg Gln Gly Asp Gly
            260                 265                 270
Cys Glu Tyr Ala His Gly Ile Phe Glu Cys Trp Leu His Pro Ala Gln
        275                 280                 285
Tyr Arg Thr Arg Leu Cys Lys Asp Glu Ile Gly Cys Thr Arg Lys Val
    290                 295                 300
Cys Phe Phe Ala His Lys His Glu Glu Leu Arg Pro Leu Tyr Ala Ser
305                 310                 315                 320
Thr Gly Ser Ala Leu Pro Ser Pro Arg Ser Phe Ser Pro Val Ala Ala
                325                 330                 335
Ser Leu Asp Met Gly Ser Leu Ser Pro Leu Ser Leu Gly Ser Ser Ser
            340                 345                 350
Val Arg Ile Pro Pro Thr Ser Thr Pro Pro Met Thr Pro Ser Gly Ala
        355                 360                 365
Ser Ser Pro Leu Gly Gly Ser Met Trp Lys Ser Gln Ile Asn Ser Thr
    370                 375                 380
Pro Pro Gly Leu Gln Leu Pro Gly Ser Arg Leu Arg Ser Ala Leu Ser
385                 390                 395                 400
Ala Arg Asp Met Asp Leu Asp Val Asp Leu Ile Asp Leu Glu Asn Asn
                405                 410                 415
Tyr Arg Leu Gln Lys Gln Leu Leu Glu His Phe Pro Asp Leu Ser Ser
            420                 425                 430
Pro Arg Gly Trp Asn Asn Ser Ser Ser Thr Thr Ser Ala Phe Pro Glu
        435                 440                 445
Tyr Ser Gly Asp Met Thr Gly Glu Ile Ser Arg Leu Gly Val Lys Pro
    450                 455                 460
Asn Asn Leu Glu Asp Ser Phe Arg Ser Leu Asp Leu Thr Leu Leu Ser
465                 470                 475                 480
Gln Leu Gln Gly Leu Ser Leu Asp Gly Ala Ile Ser Gln Leu Gln Ser
                485                 490                 495
Pro Thr Gly Met Lys Ile Arg Gln Asn Met Thr Gln Gln Leu Tyr Ser
            500                 505                 510
Asn Tyr Thr Asp Lys Leu Ser Ser Ser Pro Arg Ala Met Pro Ser Phe
        515                 520                 525
Gly Thr Asp Pro Ser Arg Ala Ser Ala Ala Ala Thr Leu Ser Ser Arg
    530                 535                 540
Ser Leu Ala Phe Ala Lys Arg Ser His Ser Phe Ile Glu Arg Ser Thr
```

```
545                 550                 555                 560
Val Asn Ser Gln Ser Gly Tyr Ser Ala Gly Ala Ala Ser Pro Thr Ala
                565                 570                 575
Arg Met Ser Ser Gln Asn Asp Trp Gly Ser Pro Asp Gly Lys Leu Asp
            580                 585                 590
Trp Gly Ile Gln Gly Glu Glu Leu Asn Lys Leu Arg Lys Ser Ala Ser
        595                 600                 605
Phe Gly Leu Arg Ser Ser Ser Asn Arg Phe His Ala Ser Ala Asp Ser
    610                 615                 620
Ala Thr Ala Thr Val Gly Asp Pro Asp Met Pro Trp Ile Gln Ser Leu
625                 630                 635                 640
Ala Lys Glu Ala Pro Ser Gln Asn Pro Gly Asn Phe Gly Ala Glu His
                645                 650                 655
Gln Gln Gln Gln Gln Gln Gln Gln Gln Gln Gln Tyr His Leu Asn Ser
            660                 665                 670
Gly Gly Thr Glu Leu Leu Pro Ala Trp Val Glu Gln Leu Tyr Ala Asp
        675                 680                 685
Gln Glu Gln Met Val Ala
    690
```

<210> 34
<211> 2499
<212> DNA
<213> Arabidopsis thaliana

<400> 34

```
atttgacct taagaagaaa gtgacaagga gaggaagaag aagaaaaaaa acataatttg      60
aggaagaaga aaaaaaattc ggatttgttt tttcaataaa ttgactaatt gagtactcgt     120
ttaaaggaag tgaagagcgg tttttggta gtggtggtcg agaaaagaga gagtttgtct     180
ctgtgactca gagtgaaatc aatagagcgg gaaaagattg ttgcttttt ttgccatggg     240
agttgatgag ctgtctcacc tcaaattctc tcttctgcta gaatcatcag cctgcaatga     300
tttgtccggt tttaagtctc tagttgaaga agaaggtctt gagagcattg atggctctgg     360
tttgtggtat gggaggagat taggatcaaa gaagatgggt tttgaggaga ggacgcctct     420
tatgattgct gccttgtttg gaagcaaaga ggttgttgat tacatcatta gtactggtct     480
tgttgacgtg aaccgctctt gtggctctga tggtgccacg gctcttcact gtgcggtctc     540
tggcttgtct gccaatagcc ttgagattgt tactcttctg ctgaagggct ctgcgaatcc     600
ggattcttgt gatgcttatg gtaacaagcc tggagatgtg attttccctt gtttgagtcc     660
ggtttttagc gcgaggatga aggttttgga gcgtttgttg aaaggaaatg atgatttgaa     720
tgaagttaat gggcaagaag aaagcgagcc agaggttgag gttgaggttg aggtttcgcc     780
tcctcggggg tctgagagga aggagtatcc ggttgatcca acgcttcctg atatcaagaa     840
cggtgtatat gggacggatg agttccggat gtatgctttc aagatcaagc cgtgctctag     900
agcatactct cacgactgga cggaatgtcc ctttgttcat ccgggtgaga acgcaaggag     960
gcgtgatccg aggaagtacc attatagttg tgtcccttgt cctgaattcc ggaaggggtc    1020
ttgttccaga ggtgatactt gcgagtatgc tcatggtatc tttgagtgct ggcttcaccc    1080
ggctcagtac cggactcgtc tctgcaagga cgagacgaat tgctcgagaa gagtttgttt    1140
ctttgcccac aaacccgagg agctgcgtcc tttgtaccct tcaactggat caggtgttcc    1200
gtccccgcgg tcttccttct catcttgcaa ttcctcgacc gctttcgaca tgggaccgat    1260
tagtccgctt cctatcggag caacaaccac acctcctttg agtcctaacg gtgtatcctc    1320
tccaataggt ggaggaaaaa cgtggatgaa ctggcctaac ataacccctc ctgcattgca    1380
gcttccaggg agcagattga aatctgcatt gaatgcaaga gaaatcgatt tctctgaaga    1440
gatgcaaagt cttacttctc caactacatg gaacaacacg ccaatgtcat ctccattctc    1500
cggaaagggc atgaacaggc ttgcaggagg agcaatgagc ccggtgaata gtctcagtga    1560
tatgtttggg acagaggata atacatcggg tttgcagatc cgacgcagcg tcattaaccc    1620
gcagctgcat tccaacagtc tttcttcatc acctgtggga gccaattctc tgttttcgat    1680
ggattcctcc gcagtcttgg cttcaagagc ggctgaattt gctaaacagc gaagccaaag    1740
cttcatagaa cgcaacaacg gactgaatca ccatcccgca atctcttcca tgactacaac    1800
ttgtttaaac gattggggct cattggatgg gaagcttgac tggagcgtcc aaggagacga    1860
gctacagaag ctcagaaaat ccacttcttt ccgtctcaga gccggtggca tggaatcaag    1920
actgcctaac gaagggactg ggctcgaaga gccagatgtc tcatgggtgg agccgctggt    1980

gaaagagcca caggagacaa gactagctcc ggtttggatg gagcaatcat acatggagac    2040
agaacagacc gtggcttgaa tcaaaagttt tgaactttca ttaaccgttc cacaagaagc    2100
aaagtcagaa agattccgag aggtcgatgc taatctattt cattttattt gtttaatgct    2160
ttgttatttt tctttagaat aaaaagaaaa aattcttagg ggacaaaaga gagttcgttt    2220
gtctctctct ctgtctccaa agaaaaacag aggtgaaaaa aggtttcaaa acctaagaaa    2280
ccttgaatta cctcacctca cttccttgat tctttactat tcacaatgag taatcgattt    2340
tttttttttct tggtaacact ctcacgctga atatatatgt tttttagtaa taatataatt    2400
ggaatacaga aatgtattta cacttgtgaa gttagggaaa gtgttgtaat tgtttcttct    2460
aagagttgat ctaagatgtt tgagactata tcttcgctt                           2499
```

<210> 35
<211> 607
<212> PRT
<213> Arabidopsis thaliana

<400> 35

```
Met Gly Val Asp Glu Leu Ser His Leu Lys Phe Ser Leu Leu Leu Glu
1               5                   10                  15
Ser Ser Ala Cys Asn Asp Leu Ser Gly Phe Lys Ser Leu Val Glu Glu
            20                  25                  30
Glu Gly Leu Glu Ser Ile Asp Gly Ser Gly Leu Trp Tyr Gly Arg Arg
        35                  40                  45
Leu Gly Ser Lys Lys Met Gly Phe Glu Glu Arg Thr Pro Leu Met Ile
    50                  55                  60
Ala Ala Leu Phe Gly Ser Lys Glu Val Val Asp Tyr Ile Ile Ser Thr
65                  70                  75                  80
Gly Leu Val Asp Val Asn Arg Ser Cys Gly Ser Asp Gly Ala Thr Ala
                85                  90                  95
Leu His Cys Ala Val Ser Gly Leu Ser Ala Asn Ser Leu Glu Ile Val
            100                 105                 110
Thr Leu Leu Leu Lys Gly Ser Ala Asn Pro Asp Ser Cys Asp Ala Tyr
        115                 120                 125
Gly Asn Lys Pro Gly Asp Val Ile Phe Pro Cys Leu Ser Pro Val Phe
    130                 135                 140
Ser Ala Arg Met Lys Val Leu Glu Arg Leu Leu Lys Gly Asn Asp Asp
145                 150                 155                 160
Leu Asn Glu Val Asn Gly Gln Glu Glu Ser Glu Pro Glu Val Glu Val
                165                 170                 175
Glu Val Glu Val Ser Pro Pro Arg Gly Ser Glu Arg Lys Glu Tyr Pro
            180                 185                 190
Val Asp Pro Thr Leu Pro Asp Ile Lys Asn Gly Val Tyr Gly Thr Asp
        195                 200                 205
Glu Phe Arg Met Tyr Ala Phe Lys Ile Lys Pro Cys Ser Arg Ala Tyr
    210                 215                 220
Ser His Asp Trp Thr Glu Cys Pro Phe Val His Pro Gly Glu Asn Ala
225                 230                 235                 240
Arg Arg Arg Asp Pro Arg Lys Tyr His Tyr Ser Cys Val Pro Cys Pro
                245                 250                 255
Glu Phe Arg Lys Gly Ser Cys Ser Arg Gly Asp Thr Cys Glu Tyr Ala
            260                 265                 270
His Gly Ile Phe Glu Cys Trp Leu His Pro Ala Gln Tyr Arg Thr Arg
        275                 280                 285
Leu Cys Lys Asp Glu Thr Asn Cys Ser Arg Arg Val Cys Phe Phe Ala
    290                 295                 300
His Lys Pro Glu Glu Leu Arg Pro Leu Tyr Pro Ser Thr Gly Ser Gly
305                 310                 315                 320
Val Pro Ser Pro Arg Ser Ser Phe Ser Ser Cys Asn Ser Ser Thr Ala
                325                 330                 335
Phe Asp Met Gly Pro Ile Ser Pro Leu Pro Ile Gly Ala Thr Thr Thr
```

```
                340                 345                 350
Pro Pro Leu Ser Pro Asn Gly Val Ser Ser Pro Ile Gly Gly Gly Lys
            355                 360                 365
Thr Trp Met Asn Trp Pro Asn Ile Thr Pro Pro Ala Leu Gln Leu Pro
        370                 375                 380
Gly Ser Arg Leu Lys Ser Ala Leu Asn Ala Arg Glu Ile Asp Phe Ser
385                 390                 395                 400
Glu Glu Met Gln Ser Leu Thr Ser Pro Thr Thr Trp Asn Asn Thr Pro
                405                 410                 415
Met Ser Ser Pro Phe Ser Gly Lys Gly Met Asn Arg Leu Ala Gly Gly
            420                 425                 430
Ala Met Ser Pro Val Asn Ser Leu Ser Asp Met Phe Gly Thr Glu Asp
        435                 440                 445
Asn Thr Ser Gly Leu Gln Ile Arg Arg Ser Val Ile Asn Pro Gln Leu
    450                 455                 460
His Ser Asn Ser Leu Ser Ser Ser Pro Val Gly Ala Asn Ser Leu Phe
465                 470                 475                 480
Ser Met Asp Ser Ser Ala Val Leu Ala Ser Arg Ala Ala Glu Phe Ala
                485                 490                 495
Lys Gln Arg Ser Gln Ser Phe Ile Glu Arg Asn Asn Gly Leu Asn His
            500                 505                 510
His Pro Ala Ile Ser Ser Met Thr Thr Thr Cys Leu Asn Asp Trp Gly
        515                 520                 525
Ser Leu Asp Gly Lys Leu Asp Trp Ser Val Gln Gly Asp Glu Leu Gln
    530                 535                 540
Lys Leu Arg Lys Ser Thr Ser Phe Arg Leu Arg Ala Gly Gly Met Glu
545                 550                 555                 560
Ser Arg Leu Pro Asn Glu Gly Thr Gly Leu Glu Glu Pro Asp Val Ser
                565                 570                 575
Trp Val Glu Pro Leu Val Lys Glu Pro Gln Glu Thr Arg Leu Ala Pro
            580                 585                 590
Val Trp Met Glu Gln Ser Tyr Met Glu Thr Glu Gln Thr Val Ala
        595                 600                 605
```

<210> 36
<211> 1806
<212> DNA
<213> Oryza sativa

<400> 36

```
atgtgctctg ggccgcgcaa gccgtccaca ccgccgctgc cgcagcagca gaaggaggcg      60
acggtgatgg cggcgtcctt gcttcttgag ctggcggcag cggacgacgt ggcggcggtg     120
aggagggtcg tggaggagga gaaggtgtct cttggcgtgg ctgggttgtg gtatgggcct     180
tcggcgagcg gcgtggcgag gctcgggatg gagcggagga cggcggcgat ggtggcggcg     240
ctgtacggga gcacgggggt gcttgggtat gtcgtggcgg cagcgccggc ggaggccgcg     300
cgcgcgtcgg agacggatgg ggccacgccg ctgcacatgg cggctgccgg tggcgcggcg     360
aacgcggtcg cggccacgcg cctgttgctc gccgcggggg cgtcggtcga cgcgctctcg     420
gcttcggggc tccgcgccgg tgacctcctc ccgcgcgcca ccgcggcgga gaaggccatc     480
cggctgctgc tcaagtcgcc ggccgtgtcg ccgtcgtcgt cgccgaagaa gtcggcctcg     540
ccgccgtcgc cgccgccgcc gcaggaggcg aagaaggagt acccgcctga cctgacgctg     600
cccgacctca agagcggact gttcagcacc gacgagttcc gcatgtacag cttcaaggtg     660
aagccgtgct cccgcgccta ctcccatgac tggaccgagt gccccttcgt ccaccccggc     720
gagaacgcgc gccgccgcga ccctcgccgc tactcctaca gctgcgtgcc ttgcccggag     780
ttccgcaagg gcggctcgtg ccgcaagggc gacgcgtgcg agtacgccca tggcgtgttc     840
gagtgctggc tccacccggc gcagtacagg acgcgcctct gcaaggacga ggtcggctgc     900
gcgcgccgca tctgcttctt cgcccacaag cccgacgagc tccgcgccgt caacccctcc     960
gccgtgtccg tcggcatgca gcccaccgta tcgtcgccgc gctcctcgcc gcccaacggg    1020
ctcgacatgg cggcggcggc ggcggcgatg atgagccccg cctggccgtc gtccccagcg    1080
agccgcctca agacggcgct cggcgcgcgg gagctcgact tcgacctcga gatgctcgcg    1140

ctggaccagt accagcagaa gctgttcgac aaggtgtccg gcgcgccgtc gccgagggcg    1200
agctggggcg ccgcggcgaa cggcctcgcc accgcgtcgc cggcgagggc cgtgccggac    1260
tacaccgacc tgctcggctc cgtcgacccg gccatgctgt cccagctcca cgcgctgtcc    1320
ctcaagcagg ccggcgacat gcccgcgtac agctccatgg cggacaccac gcagatgcac    1380
atgccgacct cgccgatggt gggcggcgcg aacaccgcgt tcgggctgga ccactccatg    1440
gcgaaggcga tcatgagctc ccgcgcctcg gcgttcgcca agcgcagcca gagcttcatc    1500
gaccgcggag gccgcgcccc ggcggcgcgt tcgctcatgt cgccggcgac gaccggcgcg    1560
ccgtccattc tctcggactg gggctcgccg gacggcaagc tggactgggg cgtccagggc    1620
gacgagctgc acaagctccg caagtcggcg tcgttcgcgt tccgcggcca atccgccatg    1680
ccggtggcga cgcacgccgc ggcggcggag ccggacgtgt catgggtgaa ctctcttgtc    1740
aaggacggcc acgccgccgg cgacatattc gcgcagtggc cggagcagga gcagatggtg    1800
gcatga                                                               1806
```

<210> 37
<211> 601
<212> PRT
<213> Oryza sativa

<400> 37

```
Met Cys Ser Gly Pro Arg Lys Pro Ser Thr Pro Pro Leu Pro Gln Gln
1               5                   10                  15
Gln Lys Glu Ala Thr Val Met Ala Ala Ser Leu Leu Leu Glu Leu Ala
            20                  25                  30
Ala Ala Asp Asp Val Ala Ala Val Arg Arg Val Val Glu Glu Glu Lys
        35                  40                  45
Val Ser Leu Gly Val Ala Gly Leu Trp Tyr Gly Pro Ser Ala Ser Gly
    50                  55                  60
Val Ala Arg Leu Gly Met Glu Arg Arg Thr Ala Ala Met Val Ala Ala
65                  70                  75                  80
Leu Tyr Gly Ser Thr Gly Val Leu Gly Tyr Val Val Ala Ala Ala Pro
                85                  90                  95
Ala Glu Ala Ala Arg Ala Ser Glu Thr Asp Gly Ala Thr Pro Leu His
            100                 105                 110
Met Ala Ala Ala Gly Gly Ala Ala Asn Ala Val Ala Ala Thr Arg Leu
        115                 120                 125
Leu Leu Ala Ala Gly Ala Ser Val Asp Ala Leu Ser Ala Ser Gly Leu
    130                 135                 140
Arg Ala Gly Asp Leu Leu Pro Arg Ala Thr Ala Ala Glu Lys Ala Ile
145                 150                 155                 160
Arg Leu Leu Leu Lys Ser Pro Ala Val Ser Pro Ser Ser Ser Pro Lys
                165                 170                 175
Lys Ser Ala Ser Pro Pro Ser Pro Pro Pro Pro Gln Glu Ala Lys Lys
            180                 185                 190
Glu Tyr Pro Pro Asp Leu Thr Leu Pro Asp Leu Lys Ser Gly Leu Phe
        195                 200                 205
Ser Thr Asp Glu Phe Arg Met Tyr Ser Phe Lys Val Lys Pro Cys Ser
    210                 215                 220
Arg Ala Tyr Ser His Asp Trp Thr Glu Cys Pro Phe Val His Pro Gly
225                 230                 235                 240
Glu Asn Ala Arg Arg Arg Asp Pro Arg Arg Tyr Ser Tyr Ser Cys Val
                245                 250                 255
Pro Cys Pro Glu Phe Arg Lys Gly Gly Ser Cys Arg Lys Gly Asp Ala
            260                 265                 270
Cys Glu Tyr Ala His Gly Val Phe Glu Cys Trp Leu His Pro Ala Gln
        275                 280                 285
Tyr Arg Thr Arg Leu Cys Lys Asp Glu Val Gly Cys Ala Arg Arg Ile
    290                 295                 300
Cys Phe Phe Ala His Lys Pro Asp Glu Leu Arg Ala Val Asn Pro Ser
305                 310                 315                 320
```

```
Ala Val Ser Val Gly Met Gln Pro Thr Val Ser Ser Pro Arg Ser Ser
                325                 330                 335
Pro Pro Asn Gly Leu Asp Met Ala Ala Ala Ala Ala Met Met Ser
            340                 345                 350
Pro Ala Trp Pro Ser Ser Pro Ala Ser Arg Leu Lys Thr Ala Leu Gly
        355                 360                 365
Ala Arg Glu Leu Asp Phe Asp Leu Glu Met Leu Ala Leu Asp Gln Tyr
    370                 375                 380
Gln Gln Lys Leu Phe Asp Lys Val Ser Gly Ala Pro Ser Pro Arg Ala
385                 390                 395                 400
Ser Trp Gly Ala Ala Ala Asn Gly Leu Ala Thr Ala Ser Pro Ala Arg
                405                 410                 415
Ala Val Pro Asp Tyr Thr Asp Leu Leu Gly Ser Val Asp Pro Ala Met
            420                 425                 430
Leu Ser Gln Leu His Ala Leu Ser Leu Lys Gln Ala Gly Asp Met Pro
        435                 440                 445
Ala Tyr Ser Ser Met Ala Asp Thr Thr Gln Met His Met Pro Thr Ser
    450                 455                 460
Pro Met Val Gly Gly Ala Asn Thr Ala Phe Gly Leu Asp His Ser Met
465                 470                 475                 480
Ala Lys Ala Ile Met Ser Ser Arg Ala Ser Ala Phe Ala Lys Arg Ser
                485                 490                 495
Gln Ser Phe Ile Asp Arg Gly Gly Arg Ala Pro Ala Ala Arg Ser Leu
            500                 505                 510
Met Ser Pro Ala Thr Thr Gly Ala Pro Ser Ile Leu Ser Asp Trp Gly
        515                 520                 525
Ser Pro Asp Gly Lys Leu Asp Trp Gly Val Gln Gly Asp Glu Leu His
    530                 535                 540
Lys Leu Arg Lys Ser Ala Ser Phe Ala Phe Arg Gly Gln Ser Ala Met
545                 550                 555                 560
Pro Val Ala Thr His Ala Ala Ala Ala Glu Pro Asp Val Ser Trp Val
                565                 570                 575
Asn Ser Leu Val Lys Asp Gly His Ala Ala Gly Asp Ile Phe Ala Gln
            580                 585                 590
Trp Pro Glu Gln Glu Gln Met Val Ala
        595                 600
```

<210> 38
<211> 1692
<212> DNA
<213> Hordeum vulgare

<400> 38

```
cggcacgagg cacatccatc atctaacctc acctctcctc tcctcccctc tcctcctacc      60
aaacccaaaa ccaagcagag caagagcaag agcaagagca agagcaagca agcatgtgcc     120
ctggcctgcg caacctcgcc gccgccatgc caccctccgc ccacgaccac ccctcctcct     180
acctgctcga gctcgccgcc gacgacgacc tccccgcctt ccgccgcgcc gtccaggagg     240
acaacctctc cctcgacgcc gcatccccga ggtacgagcc atcccccaaa tcagaccaac     300
aacaacaaca cgccccagct cgcgctccac ctgcgcaccc ccgccatggt cgccgcgctc     360
tacggcagca ccaccgtcct ctcctacgtc ctctccatcg cccccctccga ggccgcccgc     420
gcctccgcat ccgacggcgc caccccgctc ctcctcgccc accagggccg cgcgccatcc     480
gcgccccacg ccgcacgcct cctcctcacc gacggcgcat catcgtcctc cctactcgcg     540
ccccaagctc accctctcaa ccaccaaaac caaaaccaaa acagccccac caagaaagac     600
tcgccgccgg actccaggag gaccaccacc aagaaggact actcctccgc ctccgactcc     660
cagacggagg acatcaacgc gggcgtcttc gccaccgacg acttccggat gtacagcttc     720
aaggtgaacc cgtgctcccg cgcctacacg cacgactgga ccgagtgccc cttcgcccac     780
cccggcgaga acgcgcgccg ccgcgacccg cgccgcgtgc catactcgtg cgtcccatgc     840
ccggacttcc gccgcgaccc ggccgcatgc cgcaagggcg acgcctgcga gtacgcgcac     900
ggcgtcttcg agtcatggct ccaccccgcg cagtaccgca ccaggctctg caaggacgag     960

gtcggatgcc cgcgccgcat ctgcttcttc gcgcacggcg cccgacagct acgcgccgtc    1020
aacccctccg ccgcatccat ggactcgcca tccccaactt cctcttcgcc gccgcgaacc    1080
tccaggccgg ccgcgctcac cgcgtcgctc agctcgcggg acctcgactt ggacgccgac    1140
aaccaggccc agtacgcgcg caggatgatg atggccaggg ccaactcccc gccggactac    1200
tcgcccgacc tcgtcgccgc ctacgtacag gcgctctcct ccctgcaaca gcagcagcat    1260
cagcagaacc agcaacagca gcatcagcag cagaaccagc accagcagca acatcagcag    1320
aaccagcacc agcagcatca gcagcaacat cagcagagca tggggatggg ggggctgagc    1380
gcccgcgccg ccgccttcac caaccgcagc cagaccttcg tgcaccgctc tccgtccccg    1440
gctccggcgc ggtcgttcaa gtctccggcg ccgtcgtcca tgctcgcgga ctgggggtcg    1500
ccggacggga agctggactg gggcgtgcag gccgcggagc tgcgcaagtc cacgtctttc    1560
ggagtcagaa gcagcagcag gccgcatcat gagacgacga gggcggagga caacatgtac    1620
ccgtcgtgga tgaaggacgg cagcgatatg ctgctggcgg cgcggtggtc ggacctggag    1680
cagatggtcg cc                                                        1692
```

<210> 39
<211> 564
<212> PRT
<213> Hordeum vulgare

<400> 39

```
Arg His Glu Ala His Pro Ser Ser Asn Leu Thr Ser Pro Leu Leu Pro
1               5                   10                  15
Ser Pro Pro Thr Lys Pro Lys Thr Lys Gln Ser Lys Ser Lys Ser Lys
            20                  25                  30
Ser Lys Ser Lys Gln Ala Cys Ala Leu Ala Cys Ala Thr Ser Pro Pro
        35                  40                  45
Pro Cys His Pro Pro Pro Thr Thr Thr Pro Pro Pro Thr Cys Ser Ser
    50                  55                  60
Ser Pro Pro Thr Thr Thr Ser Pro Pro Ser Ala Ala Pro Ser Arg Arg
65                  70                  75                  80
Thr Thr Ser Pro Ser Thr Pro His Pro Arg Gly Thr Ser His Pro Pro
                85                  90                  95
Asn Gln Thr Asn Asn Asn Asn Thr Pro Gln Leu Ala Leu His Leu Arg
            100                 105                 110
Thr Pro Ala Met Val Ala Ala Leu Tyr Gly Ser Thr Thr Val Leu Ser
        115                 120                 125
Tyr Val Leu Ser Ile Ala Pro Ser Glu Ala Ala Arg Ala Ser Ala Ser
    130                 135                 140
Asp Gly Ala Thr Pro Leu Leu Leu Ala His Gln Gly Arg Ala Pro Ser
145                 150                 155                 160
Ala Pro His Ala Ala Arg Leu Leu Leu Thr Asp Gly Ala Ser Ser Ser
                165                 170                 175
Ser Leu Leu Ala Pro Gln Ala His Pro Leu Asn His Gln Asn Gln Asn
            180                 185                 190
Gln Asn Ser Pro Thr Lys Lys Asp Ser Pro Pro Asp Ser Arg Arg Thr
        195                 200                 205
Thr Thr Lys Lys Asp Tyr Ser Ser Ala Ser Asp Ser Gln Thr Glu Asp
    210                 215                 220
Ile Asn Ala Gly Val Phe Ala Thr Asp Asp Phe Arg Met Tyr Ser Phe
225                 230                 235                 240
Lys Val Asn Pro Cys Ser Arg Ala Tyr Thr His Asp Trp Thr Glu Cys
                245                 250                 255
Pro Phe Ala His Pro Gly Glu Asn Ala Arg Arg Arg Asp Pro Arg Arg
            260                 265                 270
Val Pro Tyr Ser Cys Val Pro Cys Pro Asp Phe Arg Arg Asp Pro Ala
        275                 280                 285
Ala Cys Arg Lys Gly Asp Ala Cys Glu Tyr Ala His Gly Val Phe Glu
    290                 295                 300
Ser Trp Leu His Pro Ala Gln Tyr Arg Thr Arg Leu Cys Lys Asp Glu
```

```
305                 310                 315                 320
Val Gly Cys Pro Arg Arg Ile Cys Phe Phe Ala His Gly Ala Arg Gln
                325                 330                 335
Leu Arg Ala Val Asn Pro Ser Ala Ala Ser Met Asp Ser Pro Ser Pro
            340                 345                 350
Thr Ser Ser Ser Pro Pro Arg Thr Ser Arg Pro Ala Ala Leu Thr Ala
        355                 360                 365
Ser Leu Ser Ser Arg Asp Leu Asp Leu Asp Ala Asp Asn Gln Ala Gln
    370                 375                 380
Tyr Ala Arg Arg Met Met Met Ala Arg Ala Asn Ser Pro Pro Asp Tyr
385                 390                 395                 400
Ser Pro Asp Leu Val Ala Ala Tyr Val Gln Ala Leu Ser Ser Leu Gln
                405                 410                 415
Gln Gln Gln His Gln Gln Asn Gln Gln Gln Gln His Gln Gln Gln Asn
            420                 425                 430
Gln His Gln Gln Gln His Gln Gln Asn Gln His Gln Gln His Gln Gln
        435                 440                 445
Gln His Gln Gln Ser Met Gly Met Gly Gly Leu Ser Ala Arg Ala Ala
    450                 455                 460
Ala Phe Thr Asn Arg Ser Gln Thr Phe Val His Arg Ser Pro Ser Pro
465                 470                 475                 480
Ala Pro Ala Arg Ser Phe Lys Ser Pro Ala Pro Ser Ser Met Leu Ala
                485                 490                 495
Asp Trp Gly Ser Pro Asp Gly Lys Leu Asp Trp Gly Val Gln Ala Ala
            500                 505                 510
Glu Leu Arg Lys Ser Thr Ser Phe Gly Val Arg Ser Ser Ser Arg Pro
        515                 520                 525
His His Glu Thr Thr Arg Ala Glu Asp Asn Met Tyr Pro Ser Trp Met
    530                 535                 540
Lys Asp Gly Ser Asp Met Leu Leu Ala Ala Arg Trp Ser Asp Leu Glu
545                 550                 555                 560
Gln Met Val Ala
```

<210> 40
<211> 3610
<212> DNA
<213> Pinus radiata

<400> 40

```
tgtttccagg cgggcactaa agcaagggag ggggtaggct ttactttctg ctctgcgcaa     60
agaacgttga aatcaatcgc cctggctggt ctggcgtgac tactagattc aatttcttca    120
tggccgtctt cacataccca ttctttaccg gttcagagct gtgatcttta tttttaacag    180
ccacaatcat ggtttgtgtt tccagtgtta tgatctgagt gaagttcgtt ctttttctcg    240
tgacccaggc ttgatactag gccggacctt tctgaggtgg aagagatcta tacatttgag    300
gcctattttg tgtagccatg tgtggaggcc cagaacattt gaagcctgcc agcccacacg    360
aaggagaaga taaagtcaaa atggccgaga atcagtctat caaagtgaag gaattgtctg    420
aatcttgttc aagtctacat gaactagctg ctaataatga ccttattggc tttaagaaag    480
caatggagga agaagggtca aagatagatg aggttaactt ttggtacggg aggcagaatg    540
gttctaatca gatggtcctg gagcaaagga ctccattgat ggttgctgca ctttatggca    600
gtgtagatgc gctgagttac atcttatcca tttatgtaac ttgtggagca gatgttaacc    660
aagcctgtgg gtcagataac tccactgcct tgcattgtgc ggctgtggga gggtctgcct    720
gtgcagttga aactgtaaaa ttgttacttc atgcaggcag tgatgtgaat cgcttggatg    780
cttatggcag aagaccagca gatgtgatta tggtttctcc taagctaacc gaaatcaagg    840
ccaagctaga agaaatgtta aacgcagctg gttcatgtca aacttctccg gcaaagttgc    900
ctaacatagt ttcagggcca cctgggtttg agtcaaaggg gatggagtcc atgtccccat    960
tgccattgtt gcctctttca ttgtctttag aagcatccaa taatagatca ggttgtgtga   1020
attctccaac atcttcgcca aagtccatgg aagcattaaa gggtttcggt gatgttaatg   1080
agaagaagga atatcctgtg gacccttctt ttccagacat aaagaatagc atctatacta   1140
```

```
cagatgaatt tcggatgttt tccttcaagg tgcggccatg ttcacgggca tattctcatg    1200
attggactga atgcccattt gtgcatcctg gtgaaaatgc cagaaggcgg gatccaagaa    1260
ggtatcatta tagctgtgtt ccttgcccag attttcggaa agggacttgt aggcgcagtg    1320
atgtttgtga atatgcacac ggtgtttttg agtgctggtt acatcctgct caatatagga    1380
cacggttgtg caaagatggg actaattgtt cacgtagagt ttgcttcttt gctcacacat    1440
ctgaggaact acgccctctc attgtctcta ctgggtctgc tgttccatcc ccaagggcat    1500
catcatctct ggacatgaca tctgtcatga gtcctcttgc cctggttct  ccctcttcag    1560
tttcaatgat gtcacccttc ctatcaaatc ctcagcaagg cagtgtgctt actccgccta    1620
tgtctccatc agcgtcctct gtaaatggat atggaggctg gccacagcct aatgtaccaa    1680
ccttacacct tcctggtagc aatgttcaaa ccagccgtct tagagcggaa cttaatgcca    1740
gagacatgcc tgttgaggat tctcctcgaa tttcagacta tgaagggcag caactcctga    1800
atgatttttc tccactgtcc acacaagcca ggctgaatgc tgctgctgct gttatatctg    1860
gtggcgggaa caccacaaca aggtctggaa aatacaagag tcacgggatc aatactgttg    1920
ctccaacgaa tcttgaagac ttgtttgcct ccgaggtaac atctcctaga gtagcagttc    1980
ttgaaccttc catcttttct cagatgagtc cccaaatgca agctcataag actgcccagg    2040
catatatgca gattcaaaac cagatgctgc ctcctataaa tacacaggca ttttcgcagg    2100
gaattacaca gatgcagcag gctgcaatag agcctcagag ccctggacat tctttgatgc    2160
aatcaccttt ccaatcttcc tcgtatgggt tgggatcccc tggtagaatg tcacctcgtt    2220
gtgtggatgt ggaacgtcat aatacatgtg ggtctccctt atcaccggct atggctgcaa    2280
cgataaattc aagaatggct atggctgctt ttgttcagag ggaaaaacgg agccatagtt    2340
cccgtgactt gggagctaat gtgaatccca gttcatggtc tgattggggc tcgcctacag    2400
gtaaagttga ctggggggtt caaggagaag agttgagcaa attaagaaag tcggcttcat    2460
ttggtccccg cagttatgaa gaaccggatt tgtcttgggt tcaaacactg gtaaaggaaa    2520
ctacaccaga gggtaaagat ggaggaaatg taagctgttc tggggaaact ccacacaagg    2580
ggcaaataga aaatgttgat cattcagttt tgggtgcctg gattgaacag atgcagcttg    2640
atcagattgt agcttgagat taggattatt tatttggagt ggtggtaggg ataggctcat    2700
ttaaaattca atttctcatt ttttactatt tcttttataa aaattcccca ttatagttta    2760
ggaaatagtc tggttttcta cctattatca gaattacacc tgcaggaaat tttggaggaa    2820
agcatgcaaa aagtagatag ggatgttatt cctatcagca ggttgacaag ctgaaaatca    2880
cttgggtggt agaccagaga atgacactat tttttgttga catggcaact gaagatgctg    2940
ttttctttac ttatcattaa caaccctata tatatttgtt ttgaaagaac tgagcggaga    3000
aatgttgtca gttggttact ctgcgcaagg ccttggaaga aatccaagat gtggcatctt    3060
ggtgcatttt taatttatca agtgtgaaat ccataacagg tttcagtgag tgacttctga    3120
ggttgtatat ggaaaaacct atgatgttgg ctgtctactg ctatttttct gtgcctaaac    3180
tgtcaactaa agtttgcagg tggcaatttt gtggcagcat atttgcacat tgaagcggat    3240
ggtctgcacc tgctatagaa gttttcgagt ctgtagaatt tgatggtgca agatgatttt    3300
ctagttgata tatttggaag gctttgccaa agtagtggca tgtacatttt gcaaaaattt    3360
aaaggatggc aatccattgt tttgccatgt agcttcactt tattgattag gtggaaagga    3420
attttgagac acttcaattt gtgcatactt ttgttctgaa ctgcaaaatc agtctcttgt    3480
gatgtcctca aggctattat gctcagggat ttgcctaaaa ccataagtgg ccttagataa    3540
ggtaccattg tattaccttt tattgtttgg atattttatt tatgaaagtg aatttatttt    3600
aaaaaaaaaa                                                            3610
```

<210> 41
<211> 779
<212> PRT
<213> Pinus radiata

<400> 41

```
Met Cys Gly Gly Pro Glu His Leu Lys Pro Ala Ser Pro His Glu Gly
1               5                   10                  15
Glu Asp Lys Val Lys Met Ala Glu Asn Gln Ser Ile Lys Val Lys Glu
            20                  25                  30
Leu Ser Glu Ser Cys Ser Ser Leu His Glu Leu Ala Ala Asn Asn Asp
        35                  40                  45
Leu Ile Gly Phe Lys Lys Ala Met Glu Glu Glu Gly Ser Lys Ile Asp
    50                  55                  60
Glu Val Asn Phe Trp Tyr Gly Arg Gln Asn Gly Ser Asn Gln Met Val
65                  70                  75                  80
```

Leu Glu Gln Arg Thr Pro Leu Met Val Ala Ala Leu Tyr Gly Ser Val
85 90 95
Asp Ala Leu Ser Tyr Ile Leu Ser Ile Tyr Val Thr Cys Gly Ala Asp
100 105 110
Val Asn Gln Ala Cys Gly Ser Asp Asn Ser Thr Ala Leu His Cys Ala
115 120 125
Ala Val Gly Gly Ser Ala Cys Ala Val Glu Thr Val Lys Leu Leu Leu
130 135 140
His Ala Gly Ser Asp Val Asn Arg Leu Asp Ala Tyr Gly Arg Arg Pro
145 150 155 160
Ala Asp Val Ile Met Val Ser Pro Lys Leu Thr Glu Ile Lys Ala Lys
165 170 175
Leu Glu Glu Met Leu Asn Ala Ala Gly Ser Cys Gln Thr Ser Pro Ala
180 185 190
Lys Leu Pro Asn Ile Val Ser Gly Pro Pro Gly Phe Glu Ser Lys Gly
195 200 205
Met Glu Ser Met Ser Pro Leu Pro Leu Leu Pro Leu Ser Leu Ser Leu
210 215 220
Glu Ala Ser Asn Asn Arg Ser Gly Cys Val Asn Ser Pro Thr Ser Ser
225 230 235 240
Pro Lys Ser Met Glu Ala Leu Lys Gly Phe Gly Asp Val Asn Glu Lys
245 250 255
Lys Glu Tyr Pro Val Asp Pro Ser Phe Pro Asp Ile Lys Asn Ser Ile
260 265 270
Tyr Thr Thr Asp Glu Phe Arg Met Phe Ser Phe Lys Val Arg Pro Cys
275 280 285
Ser Arg Ala Tyr Ser His Asp Trp Thr Glu Cys Pro Phe Val His Pro
290 295 300
Gly Glu Asn Ala Arg Arg Arg Asp Pro Arg Arg Tyr His Tyr Ser Cys
305 310 315 320
Val Pro Cys Pro Asp Phe Arg Lys Gly Thr Cys Arg Arg Ser Asp Val
325 330 335
Cys Glu Tyr Ala His Gly Val Phe Glu Cys Trp Leu His Pro Ala Gln
340 345 350
Tyr Arg Thr Arg Leu Cys Lys Asp Gly Thr Asn Cys Ser Arg Arg Val
355 360 365
Cys Phe Phe Ala His Thr Ser Glu Glu Leu Arg Pro Leu Ile Val Ser
370 375 380
Thr Gly Ser Ala Val Pro Ser Pro Arg Ala Ser Ser Ser Leu Asp Met
385 390 395 400
Thr Ser Val Met Ser Pro Leu Ala Pro Gly Ser Pro Ser Ser Val Ser
405 410 415
Met Met Ser Pro Phe Leu Ser Asn Pro Gln Gln Gly Ser Val Leu Thr
420 425 430
Pro Pro Met Ser Pro Ser Ala Ser Ser Val Asn Gly Tyr Gly Gly Trp
435 440 445
Pro Gln Pro Asn Val Pro Thr Leu His Leu Pro Gly Ser Asn Val Gln
450 455 460
Thr Ser Arg Leu Arg Ala Glu Leu Asn Ala Arg Asp Met Pro Val Glu
465 470 475 480
Asp Ser Pro Arg Ile Ser Asp Tyr Glu Gly Gln Gln Leu Leu Asn Asp
485 490 495
Phe Ser Pro Leu Ser Thr Gln Ala Arg Leu Asn Ala Ala Ala Ala Val
500 505 510
Ile Ser Gly Gly Gly Asn Thr Thr Thr Arg Ser Gly Lys Tyr Lys Ser
515 520 525
His Gly Ile Asn Thr Val Ala Pro Thr Asn Leu Glu Asp Leu Phe Ala
530 535 540
Ser Glu Val Thr Ser Pro Arg Val Ala Val Leu Glu Pro Ser Ile Phe

```
545                 550                 555                 560
Ser Gln Met Ser Pro Gln Met Gln Ala His Lys Thr Ala Gln Ala Tyr
                565                 570                 575
Met Gln Ile Gln Asn Gln Met Leu Pro Pro Ile Asn Thr Gln Ala Phe
            580                 585                 590
Ser Gln Gly Ile Thr Gln Met Gln Gln Ala Ala Ile Glu Pro Gln Ser
        595                 600                 605
Pro Gly His Ser Leu Met Gln Ser Pro Phe Gln Ser Ser Ser Tyr Gly
    610                 615                 620
Leu Gly Ser Pro Gly Arg Met Ser Pro Arg Cys Val Asp Val Glu Arg
625                 630                 635                 640
His Asn Thr Cys Gly Ser Pro Leu Ser Pro Ala Met Ala Ala Thr Ile
                645                 650                 655
Asn Ser Arg Met Ala Met Ala Ala Phe Val Gln Arg Glu Lys Arg Ser
            660                 665                 670
His Ser Ser Arg Asp Leu Gly Ala Asn Val Asn Pro Ser Ser Trp Ser
        675                 680                 685
Asp Trp Gly Ser Pro Thr Gly Lys Val Asp Trp Gly Val Gln Gly Glu
    690                 695                 700
Glu Leu Ser Lys Leu Arg Lys Ser Ala Ser Phe Gly Pro Arg Ser Tyr
705                 710                 715                 720
Glu Glu Pro Asp Leu Ser Trp Val Gln Thr Leu Val Lys Glu Thr Thr
                725                 730                 735
Pro Glu Gly Lys Asp Gly Gly Asn Val Ser Cys Ser Gly Glu Thr Pro
            740                 745                 750
His Lys Gly Gln Ile Glu Asn Val Asp His Ser Val Leu Gly Ala Trp
        755                 760                 765
Ile Glu Gln Met Gln Leu Asp Gln Ile Val Ala
    770                 775
```

<210> 42
<211> 3610
<212> DNA
<213> Pinus radiata

<400> 42

```
tgtttccagg cgggcactaa agcaagggag ggggtaggct ttactttctg ctctgcgcaa      60
agaacgttga aatcaatcgc cctggctggt ctggcgtgac tactagattc aatttcttca     120
tggccgtctt cacataccca ttctttaccg gttcagagct gtgatcttta tttttaacag     180
ccacaatcat ggtttgtgtt tccagtgtta tgatctgagt gaagttcgtt ctttttctcg     240
tgacccaggc ttgatactag gccggacctt tctgaggtgg aagagatcta tacatttgag     300
gcctattttg tgtagccatg tgtggaggcc cagaacattt gaagcctgcc agcccacacg     360
aaggagaaga taaagtcaaa atggccgaga atcagtctat caaagtgaag gaattgtctg     420
aatcttgttc aagtctacat gaactagctg ctaataatga ccttattggc tttaagaaag     480
caatggagga agaagggtca aagatagatg aggttaactt ttggtacggg aggcagaatg     540
gttctaatca gatggtcctg gagcaaagga ctccattgat ggttgctgca ctttatggca     600
gtgtagatgc gctgagttac atcttatcca tttatgtaac ttgtggagca gatgttaacc     660
aagcctgtgg gtcagataac tccactgcct tgcattgtgc ggctgtggga gggtctgcct     720
gtgcagttga aactgtaaaa ttgttacttc atgcaggcag tgatgtgaat cgcttggatg     780
cttatggcag aagaccagca gatgtgatta tggtttctcc taagctaacc gaaatcaagg     840
ccaagctaga agaaatgtta aacgcagctg gttcatgtca aacttctccg gcaaagttgc     900
ctaacatagt ttcagggcca cctgggtttg agtcaaaggg gatggagtcc atgtccccat     960
tgccattgtt gcctctttca ttgtctttag aagcatccaa taatagatca ggttgtgtga    1020
attctccaac atcttcgcca aagtccatgg aagcattaaa gggtttcggt gatgttaatg    1080
agaagaagga atatcctgtg gacccttctt ttccagacat aaagaatagc atctatacta    1140
cagatgaatt tcggatgttt tccttcaagg tgcggccatg ttcacgggca tattctcatg    1200
attggactga atgcccattt gtgcatcctg gtgaaaatgc cagaaggcgg gatccaagaa    1260
ggtatcatta tagctgtgtt ccttgcccag attttcggaa agggacttgt aggcgcagtg    1320
atgtttgtga atatgcacac ggtgtttttg agtgctggtt acatcctgct caatatagga    1380
```

```
cacggttgtg caaagatggg actaattgtt cacgtagagt ttgcttcttt gctcacacat   1440
ctgaggaact acgccctctc attgtctcta ctgggtctgc tgttccatcc ccaagggcat   1500
catcatctct ggacatgaca tctgtcatga gtcctcttgc cctggttct ccctcttcag    1560
tttcaatgat gtcacccttc ctatcaaatc ctcagcaagg cagtgtgctt actccgccta   1620
tgtctccatc agcgtcctct gtaaatggat atggaggctg gccacagcct aatgtaccaa   1680
ccttacacct tcctggtagc aatgttcaaa ccagccgtct tagagcggaa cttaatgcca   1740
gagacatgcc tgttgaggat tctcctcgaa tttcagacta tgaagggcag caactcctga   1800
atgattttc tccactgtcc acacaagcca ggctgaatgc tgctgctgct gttatatctg   1860
gtggcgggaa caccacaaca aggtctggaa aatacaagag tcacgggatc aatactgttg   1920
ctccaacgaa tcttgaagac ttgtttgcct ccgaggtaac atctcctaga gtagcagttc   1980
ttgaaccttc catcttttct cagatgagtc cccaaatgca agctcataag actgcccagg   2040
catatatgca gattcaaaac cagatgctgc ctcctataaa tacacaggca ttttcgcagg   2100
gaattacaca gatgcagcag gctgcaatag agcctcagag ccctggacat tctttgatgc   2160
aatcaccttt ccaatcttcc tcgtatgggt tgggatcccc tggtagaatg tcacctcgtt   2220
gtgtggatgt ggaacgtcat aatacatgtg ggtctccctt atcaccggct atggctgcaa   2280
cgataaattc aagaatggct atggctgctt ttgttcagag ggaaaaacgg agccatagtt   2340
cccgtgactt gggagctaat gtgaatccca gttcatggtc tgattggggc tcgcctacag   2400
gtaaagttga ctggggggtt caaggagaag agttgagcaa attaagaaag tcggcttcat   2460
ttggtccccg cagttatgaa gaaccggatt tgtcttgggt tcaaacactg gtaaaggaaa   2520
ctacaccaga gggtaaagat ggaggaaatg taagctgttc tggggaaact ccacacaagg   2580
ggcaaataga aaatgttgat cattcagttt tgggtgcctg gattgaacag atgcagcttg   2640
atcagattgt agcttgagat taggattatt tatttggagt ggtggtaggg ataggctcat   2700
ttaaaattca atttctcatt ttttactatt tcttttataa aaattcccca ttatagttta   2760
ggaaatagtc tggttttcta cctattatca gaattacacc tgcaggaaat tttggaggaa   2820
agcatgcaaa aagtagatag ggatgttatt cctatcagca ggttgacaag ctgaaaatca   2880
cttgggtggt agaccagaga atgacactat tttttgttga catggcaact gaagatgctg   2940
ttttctttac ttatcattaa caaccctata tatatttgtt ttgaaagaac tgagcggaga   3000
aatgttgtca gttggttact ctgcgcaagg ccttggaaga aatccaagat gtggcatctt   3060
ggtgcatttt taatttatca agtgtgaaat ccataacagg tttcagtgag tgacttctga   3120
ggttgtatat ggaaaaacct atgatgttgg ctgtctactg ctatttttct gtgcctaaac   3180
tgtcaactaa agtttgcagg tggcaatttt gtggcagcat atttgcacat tgaagcggat   3240
ggtctgcacc tgctatagaa gttttcgagt ctgtagaatt tgatggtgca agatgatttt   3300
ctagttgata tatttggaag gctttgccaa agtagtggca tgtacatttt gcaaaaattt   3360
aaaggatggc aatccattgt tttgccatgt agcttcactt tattgattag gtggaaagga   3420
attttgagac acttcaattt gtgcatactt ttgttctgaa ctgcaaaatc agtctcttgt   3480
gatgtcctca aggctattat gctcagggat ttgcctaaaa ccataagtgg ccttagataa   3540
ggtaccattg tattaccttt tattgtttgg atattttatt tatgaaagtg aatttatttt   3600
aaaaaaaaaa                                                          3610
```

<210> 43
<211> 749
<212> PRT
<213> Pinus radiata

<400> 43

```
Met Lys Glu Met Ala Glu Tyr Cys Ser Pro Ala Leu Leu Glu Leu Ala
1               5                   10                  15
Ala Asn Asn Asp Leu Ser Gly Phe Lys Gln Ala Val Glu Glu Gly Gly
            20                  25                  30
Ser Ser Val Asn Glu Arg Gly Leu Trp Tyr Gly Arg Gln Ile Gly Ser
        35                  40                  45
Gly Gln Lys Met Val Leu Glu Gln Arg Thr Pro Leu Met Val Ala Ala
    50                  55                  60
Leu Tyr Gly Ser Leu Asp Val Leu Ser Tyr Met Leu Ser Gly Gly Arg
65                  70                  75                  80
Val Asp Val Asn Gln Ser Cys Gly Ser Asp Met Ser Thr Ala Leu His
                85                  90                  95
Cys Ala Ala Ala Gly Gly Ser Ile Leu Ala Ile Glu Thr Val Gly Met
            100                 105                 110
```

```
Leu Ile Lys Ala Gly Ala Asp Val Asn Phe Met Asn Ala Gly Gly Arg
        115                 120                 125
Lys Pro Ala Asp Val Ile Met Val Ser Pro Lys Leu Ala His Phe Lys
    130                 135                 140
Asn Val Leu Glu Asp Leu Leu Ile Met Gly Ser Asn Ser Pro Met Lys
145                 150                 155                 160
Ile Pro Cys Arg Val Ser Gly Ser Gly Phe Tyr Leu Pro Glu Gly Gly
                165                 170                 175
Gly Cys Phe Phe Asp Glu His Gly Cys Val Val Ser Val Pro Thr Ser
            180                 185                 190
Ser Pro Leu Phe Ser Ser Pro Asp Ala Thr Ser Pro Ala Thr Val Asn
        195                 200                 205
Ser Pro Leu Ser Ser Pro Pro Thr Ser Leu Asp Thr Pro Lys Asn Leu
    210                 215                 220
Cys Asp Cys Gly Gln Lys Lys Glu Phe Ala Val Asp Ser Ser Leu Pro
225                 230                 235                 240
Asp Ile Lys Asn Ser Ile Tyr Ser Thr Asp Glu Phe Arg Met Tyr Ser
                245                 250                 255
Phe Lys Val Arg Pro Cys Ser Arg Ala Tyr Ser His Asp Trp Thr Glu
            260                 265                 270
Cys Pro Phe Val His Pro Gly Glu Asn Ala Arg Arg Arg Asp Pro Arg
        275                 280                 285
Lys Tyr His Tyr Ser Cys Val Pro Cys Pro Asp Phe Arg Lys Gly Ala
    290                 295                 300
Cys Arg Arg Gly Asp Val Cys Glu Tyr Ala His Gly Val Phe Glu Cys
305                 310                 315                 320
Trp Leu His Pro Ala Gln Tyr Arg Thr Arg Leu Cys Lys Asp Gly Thr
                325                 330                 335
Asn Cys Ser Arg Arg Val Cys Phe Phe Ala His Thr Pro Glu Glu Leu
            340                 345                 350
Arg Pro Leu Tyr Pro Pro Ala Cys Ser Ser Met Leu Ser Gln Arg Thr
        355                 360                 365
Thr Met Thr Ser Ser Asp Lys Met Ala Val Met His Pro Leu Ala Pro
    370                 375                 380
Gly Ser Ala Ser Ser Val Leu Met Met Ser Ser Ser Asn Ser Ser Gln
385                 390                 395                 400
Ser Ser Phe Pro Asn Ser Pro Val Ser Pro Leu Ser Ser Ala Asn Thr
                405                 410                 415
Ser Ser His Ser Ser Phe Gly Gly Gly Ser Trp Ala His Pro Asn Leu
            420                 425                 430
Pro Thr Leu His Leu Ser Asn Gly Ala Leu Gln Ala Ser Arg Leu Arg
        435                 440                 445
Thr Ala Val Asn Ala Arg Asp Met His Pro Asp Cys Ser Ile Glu Ser
    450                 455                 460
Gly Asp Tyr Glu Gly Gln Leu Leu Asn Glu Phe Ala Tyr Leu Ser Thr
465                 470                 475                 480
Gln Ala Arg Gly Asn Gly Pro Met Ala Thr Val Ser Ser Ser Gly Asn
                485                 490                 495
Thr Pro Cys Arg Pro Arg Lys Phe Arg Ala His Asn Val Ala Pro Thr
            500                 505                 510
Asn Leu Glu Asp Leu Phe Ala Ser Glu Val Phe Ser Pro Lys Met Thr
        515                 520                 525
Ala Ser Glu Ser Ala Phe Leu Ser Glu Ile Gln Ser His Lys Ser Ala
    530                 535                 540
Gln Leu Ser Pro Gln Leu Gln Ser Gln Met Leu Ser Ser Phe Asn Thr
545                 550                 555                 560
Gln Val Tyr Pro Gln Gly Ser Thr Gln Gly Gln Met His Met Gln His
                565                 570                 575
Gly Gly Val Asp Cys Gln Ser Pro Ser Val Phe Leu Ser Pro Pro Pro
```

```
            580                 585                 590
Val Gln Leu Ala Ser Tyr Ser Leu Ser Ser Leu Gly Pro Leu Ser Ser
        595                 600                 605
Leu Thr Gly Glu Leu Glu Arg Gln Asn Ser Asn Gly Ser Pro Leu Ser
    610                 615                 620
Pro Ile Met Ser Thr Ala Ala Asp Ser Arg Ala Val Ala Phe Ser Gln
625                 630                 635                 640
Arg Asp Lys Gly Ser Ser Arg Ser Gly Asp Leu Gly Gly Ala Thr Thr
                645                 650                 655
Trp Ser Glu Trp Gly Ser Pro Thr Gly Lys Val Asn Trp Gly Ile Arg
            660                 665                 670
Gly Glu Glu Leu Gln Lys Phe Arg Lys Ser Ala Ser Phe Gly Ile Arg
        675                 680                 685
Ser Ser Asp Glu Pro Asp Leu Ser Trp Val Gln Lys Leu Phe Lys Glu
    690                 695                 700
Ala Pro Met Glu Ser Met Asp Arg Gly Thr Met Gly Arg Ser Met Asp
705                 710                 715                 720
Ile Ala Asn Ser Val Gln Met Glu Ala Thr Asp Leu Gly Gly Trp Ile
                725                 730                 735
Ser Gln Ile Asn Pro Asp Gln Val Ala Pro Leu Thr Leu
            740                 745
```

<210> 44
<211> 711
<212> PRT
<213> Glycine max

<400> 44

```
Lys Ser Ala Asn Asp Lys Glu Met Lys Ser Leu Thr Val Asn Thr Glu
1               5                   10                  15
Asp Ser Phe Ser Ser Leu Leu Glu Leu Ala Ser Asn Asn Asp Ile Glu
            20                  25                  30
Gly Phe Lys Val Leu Leu Glu Lys Asp Ser Ser Ser Ile Asn Glu Val
        35                  40                  45
Gly Leu Trp Tyr Gly Arg Gln Asn Gly Ser Lys Gln Phe Val Leu Glu
    50                  55                  60
His Arg Thr Pro Leu Met Val Ala Ala Thr Tyr Gly Ser Ile Asp Val
65                  70                  75                  80
Met Lys Ile Ile Leu Leu Cys Pro Glu Ala Asp Val Asn Phe Ala Cys
                85                  90                  95
Gly Ala Asn Lys Thr Thr Ala Leu His Cys Ala Ala Ser Gly Gly Ser
            100                 105                 110
Ala Asn Ala Val Asp Ala Val Lys Ile Leu Leu Ser Ala Gly Ala Asp
        115                 120                 125
Val Asn Gly Val Asp Ala Asn Gly Asn Arg Pro Ile Asp Val Ile Ala
    130                 135                 140
Val Pro Pro Lys Leu Gln Gly Ala Lys Ala Val Leu Glu Glu Leu Leu
145                 150                 155                 160
Ser Asp Ser Ala Ser Glu Gly Ser Ile Gly Glu Phe Ser Val Pro Val
                165                 170                 175
Ser Val Asn Thr Ser Ser Leu Gly Ser Pro Gly His Ser Ser Asn Gly
            180                 185                 190
Met Pro Tyr Thr Pro Ser Ser Ser Pro Pro Ser Pro Val Val Ala Lys
        195                 200                 205
Phe Thr Asp Ala Ala Val Cys Ser Leu Ser Glu Lys Lys Glu Tyr Pro
    210                 215                 220
Ile Asp Pro Ser Leu Pro Asp Ile Lys Asn Ser Ile Tyr Ala Thr Asp
225                 230                 235                 240
Glu Phe Arg Met Phe Ser Phe Lys Val Arg Pro Cys Ser Arg Ala Tyr
```

```
                245                 250                 255
Ser His Asp Trp Thr Glu Cys Pro Phe Val His Pro Gly Glu Asn Ala
            260                 265                 270
Arg Arg Arg Asp Pro Arg Lys Phe His Tyr Ser Cys Val Pro Cys Pro
        275                 280                 285
Asp Phe Arg Lys Gly Ala Cys Arg Arg Gly Asp Met Cys Glu Tyr Ala
    290                 295                 300
His Gly Val Phe Glu Cys Trp Leu His Pro Ala Gln Tyr Arg Thr Arg
305                 310                 315                 320
Leu Cys Lys Asp Gly Thr Ser Cys Asn Arg Arg Val Cys Phe Phe Ala
                325                 330                 335
His Thr Ala Glu Glu Leu Arg Pro Leu Tyr Val Ser Thr Gly Ser Ala
            340                 345                 350
Val Pro Ser Pro Arg Ser Ser Ala Ser Ala Pro Asn Val Met Asp Met
        355                 360                 365
Ala Ala Ala Met Ser Leu Leu Pro Gly Ser Pro Ser Ser Val Ser Ser
    370                 375                 380
Met Ser Pro Ser His Phe Gly Gln Pro Met Ser Pro Ser Ala Asn Gly
385                 390                 395                 400
Met Ser Leu Ser Ser Ala Trp Ala Gln Pro Asn Val Ser Ala Leu His
                405                 410                 415
Leu Pro Gly Ser Asn Leu Gln Ser Ser Arg Leu Arg Ser Ser Leu Ser
            420                 425                 430
Ala Arg Asp Met Pro Pro Asp Asp Leu Asn Met Met Ser Asp Leu Asp
        435                 440                 445
Gly Gln Gln Gln His Pro Leu Asn Asp Leu Ser Cys Tyr Leu Gln Pro
    450                 455                 460
Arg Pro Gly Ala Gly Ser Val Ser Arg Ser Gly Arg Ser Lys Ile Leu
465                 470                 475                 480
Thr Pro Ser Asn Leu Glu Asp Leu Phe Ser Ala Glu Ile Ser Ser Ser
                485                 490                 495
Pro Arg Tyr Ser Asp Pro Ala Ala Gly Ser Val Phe Ser Pro Thr His
            500                 505                 510
Lys Ser Ala Val Leu Asn Gln Phe Gln Gln Leu Gln Ser Met Leu Ser
        515                 520                 525
Pro Ile Asn Thr Asn Leu Leu Ser Pro Lys Asn Val Glu His Pro Leu
    530                 535                 540
Leu Gln Ala Ser Phe Gly Val Ser Pro Ser Gly Arg Met Ser Pro Arg
545                 550                 555                 560
Ser Val Glu Pro Ile Ser Pro Met Ser Ser Arg Ile Ser Ala Phe Ala
                565                 570                 575
Gln Arg Glu Lys Gln Gln Gln Gln Gln Gln Gln Leu Arg Ser Leu Ser
            580                 585                 590
Ser Arg Asp Leu Gly Ala Asn Ser Pro Ala Ser Leu Val Gly Ser Pro
        595                 600                 605
Ala Asn Pro Trp Ser Lys Trp Gly Ser Pro Asn Gly Lys Ala Asp Trp
    610                 615                 620
Ser Val Asn Gly Asp Thr Leu Gly Arg Gln Met Arg Arg Ser Ser Ser
625                 630                 635                 640
Phe Glu Leu Lys Asn Asn Gly Glu Glu Pro Asp Leu Ser Trp Val Gln
                645                 650                 655
Ser Leu Val Lys Glu Ser Pro Pro Glu Met Ile Lys Glu Lys Phe Ala
            660                 665                 670
Ser Pro Met Pro Thr Ala Ser Ala Asp Gly Pro Asn Ser Asn Ser Gln
        675                 680                 685
Ile Glu Ser Ile Asp His Ser Val Leu Gly Ala Trp Leu Glu Gln Met
    690                 695                 700
Gln Leu Asp Gln Leu Val Val
705                 710
```

<210> 45
<211> 643
<212> PRT
<213> Glycine max

<400> 45

```
Arg Gly Ser Gly Phe Pro Gly Arg Pro Thr Arg Pro Arg Ser Gly Arg
1               5                   10                  15
Thr Arg Gly Arg Thr Arg Gly Val Asn Phe Ala Cys Gly Ala Asn Lys
            20                  25                  30
Thr Thr Ala Leu His Cys Ala Ala Ser Gly Ala Ser Thr Lys Ala Val
        35                  40                  45
Asp Ala Val Lys Leu Leu Leu Ser Ala Gly Ala Asp Val Asn Cys Val
    50                  55                  60
Asp Ala Asn Gly Asn Arg Pro Ile Asp Val Ile Ala Val Pro Pro Lys
65                  70                  75                  80
Leu Gln Gly Ala Lys Ala Val Leu Glu Glu Leu Leu Ser Asp Asn Ala
                85                  90                  95
Ser Asp Val Ser Val Gly Glu Phe Ser Val Pro Val Ser Val Asn Ser
            100                 105                 110
Ser Ser Pro Gly Ser Pro Ala His Ser Ser Asn Gly Met Pro Tyr Thr
        115                 120                 125
Pro Ser Val Ser Pro Pro Ser Pro Val Ala Ala Lys Phe Thr Asp Ala
    130                 135                 140
Ala Ile Cys Ser Leu Ser Glu Lys Ala Arg Glu Tyr Pro Ile Asp Pro
145                 150                 155                 160
Ser Leu Pro Asp Ile Lys Asn Ser Ile Tyr Ala Thr Asp Glu Phe Arg
                165                 170                 175
Met Phe Ser Phe Lys Val Arg Pro Cys Ser Arg Ala Tyr Ser His Asp
            180                 185                 190
Trp Thr Glu Cys Pro Phe Val His Pro Gly Glu Asn Ala Arg Arg Arg
        195                 200                 205
Asp Pro Arg Lys Phe His Tyr Ser Cys Val Pro Cys Pro Asp Phe Arg
    210                 215                 220
Lys Gly Ala Cys Arg Arg Gly Asp Met Cys Glu Tyr Ala His Gly Val
225                 230                 235                 240
Phe Glu Cys Trp Leu His Pro Ala Gln Tyr Arg Thr Arg Leu Cys Lys
                245                 250                 255
Asp Gly Thr Ser Cys Asn Arg Arg Val Cys Phe Phe Ala His Thr Ala
            260                 265                 270
Glu Glu Leu Arg Pro Leu Tyr Val Ser Thr Gly Ser Ala Ala Pro Ser
        275                 280                 285
Pro Arg Ser Ser Ala Ser Gly Pro Asn Val Met Asp Met Ala Ala Ala
    290                 295                 300
Met Ser Leu Phe Pro Gly Ser Pro Ser Ser Gly Ser Ser Ile Ser Leu
305                 310                 315                 320
Ser Ile Ser Phe Ser Leu Asp Pro Met Ser Pro Ser Ala Asn Gly Met
                325                 330                 335
Pro Leu Ser Ser Ala Trp Ala Gln Pro Asn Val Pro Ala Leu His Leu
            340                 345                 350
Pro Gly Ser Asn Leu Gln Ser Ser Arg Leu Arg Ser Ser Leu Ser Ala
        355                 360                 365
Arg Asp Ile Pro Pro Glu Asp Leu Asn Met Met Ser Asp Leu Asp Gly
    370                 375                 380
Gln Gln Gln His His Leu Asn Asp Leu Ser Cys Tyr Ile Gln Pro Arg
385                 390                 395                 400
Pro Gly Ala Ser Ser Val Ser Arg Ser Gly Arg Ser Lys Thr Leu Thr
                405                 410                 415
```

```
Pro Ser Asn Leu Glu Glu Leu Phe Ser Ala Glu Ile Ser Leu Ser Pro
            420                 425                 430
Arg Tyr Ser Asp Pro Ala Ala Gly Ser Val Phe Ser Pro Thr His Lys
        435                 440                 445
Ser Ala Val Leu Asn Gln Phe Gln Gln Leu Gln Ser Met Leu Ser Pro
    450                 455                 460
Ile Asn Thr Asn Leu Leu Ser Pro Lys Asn Val Glu His Pro Leu Phe
465                 470                 475                 480
Gln Ala Ser Phe Gly Val Ser Pro Ser Gly Arg Met Ser Pro Arg Ser
                485                 490                 495
Val Glu Pro Ile Ser Pro Met Ser Ala Arg Leu Ser Ala Phe Ala Gln
            500                 505                 510
Arg Glu Lys Gln Gln Gln Gln Leu Arg Ser Val Ser Ser Arg Asp Leu
        515                 520                 525
Gly Ala Asn Ser Pro Ala Ser Leu Val Gly Ser Pro Ala Asn Pro Trp
    530                 535                 540
Ser Lys Trp Gly Ser Pro Ile Gly Lys Ala Asp Trp Ser Val Asn Gly
545                 550                 555                 560
Asp Ser Leu Gly Arg Gln Met Arg Arg Ser Ser Ser Phe Glu Arg Lys
                565                 570                 575
Asn Asn Gly Glu Glu Pro Asp Leu Ser Trp Val Gln Ser Leu Val Lys
            580                 585                 590
Glu Ser Pro Pro Glu Met Ile Lys Glu Lys Phe Ala Ser Pro Met Pro
        595                 600                 605
Thr Ala Ser Ala Asp Gly Pro Asn Ser Asn Ser Gln Ile Glu Ser Ile
    610                 615                 620
Asp His Ser Val Leu Gly Ala Trp Leu Glu Gln Met Gln Leu Asp Gln
625                 630                 635                 640
Leu Val Val
```

<210> 46
<211> 669
<212> PRT
<213> Glycine max

<400> 46

```
Ser His Glu Met Asn His Leu Ser Leu Asp Thr Glu Asp Ser Leu Ala
1               5                   10                  15
Ser Leu Leu Leu Glu Leu Ala Ala Asn Asn Asp Val Ser Gly Phe Lys
            20                  25                  30
Arg Leu Ile Glu Cys Glu Pro Ser Ser Ile Asp Glu Val Gly Leu Trp
        35                  40                  45
Tyr Gly Arg His Lys Glu Ser Lys Lys Met Val Asn Glu Gln Arg Thr
    50                  55                  60
Pro Leu Met Val Ala Ala Thr Tyr Gly Ser Ile Asp Val Met Thr Leu
65                  70                  75                  80
Ile Leu Ser Leu Ser Glu Ala Asp Val Asn Arg Ser Ser Gly Leu Asp
                85                  90                  95
Lys Ser Thr Ala Leu His Cys Ala Ala Ser Gly Gly Ser Glu Asn Ala
            100                 105                 110
Val Asp Ala Val Lys Leu Leu Leu Glu Ala Gly Ala Asp Arg Asn Ser
        115                 120                 125
Val Asp Ala Asn Gly Arg Arg Pro Gly Asp Val Ile Val Ser Pro Pro
    130                 135                 140
Lys Leu Asp Tyr Val Lys Lys Ser Leu Glu Glu Leu Leu Gly Ser Asp
145                 150                 155                 160
Asp Trp Ser Leu Leu Arg Val Met Arg Ser Thr Cys Asn Gly Cys Ser
                165                 170                 175
```

```
Ala Glu Asp Leu Lys Met Lys Thr Asn Glu Val Ser Glu Lys Lys Glu
        180                 185                 190
Tyr Pro Val Asp Leu Ser Leu Pro Asp Ile Lys Asn Ser Ile Tyr Ser
    195                 200                 205
Ser Asp Glu Phe Arg Met Tyr Ser Phe Lys Val Arg Pro Cys Ser Arg
    210                 215                 220
Ala Tyr Ser His Asp Trp Thr Glu Cys Pro Phe Val His Pro Gly Glu
225                 230                 235                 240
Asn Ala Arg Arg Arg Asp Pro Arg Lys Phe His Tyr Ser Cys Val Pro
                245                 250                 255
Cys Pro Glu Phe Arg Lys Gly Ala Cys Arg Arg Gly Asp Met Cys Glu
        260                 265                 270
Tyr Ala His Gly Val Phe Glu Cys Trp Leu His Pro Ala Gln Tyr Arg
    275                 280                 285
Thr Arg Leu Cys Lys Asp Gly Thr Asn Cys Ala Arg Arg Val Cys Phe
    290                 295                 300
Phe Ala His Thr Asn Glu Glu Leu Arg Pro Leu Tyr Val Ser Thr Gly
305                 310                 315                 320
Ser Ala Val Pro Ser Pro Arg Ser Ser Ala Ser Ser Ala Met Asp Phe
                325                 330                 335
Val Ala Ala Ile Ser Pro Ser Ser Met Ser Val Met Ser Pro Ser Pro
        340                 345                 350
Phe Thr Pro Pro Met Ser Pro Ser Ser Ala Ser Ile Ala Trp Pro Gln
    355                 360                 365
Pro Asn Ile Pro Ala Leu His Leu Pro Gly Ser Asn Phe His Ser Ser
    370                 375                 380
Arg Leu Arg Ser Ser Leu Asn Ala Arg Asp Phe Ser Val Asp Asp Phe
385                 390                 395                 400
Asp Leu Leu Leu Pro Asp Tyr Asp His His His His Gln Gln Gln Gln
                405                 410                 415
Gln Gln Phe Leu Asn Glu Leu Ser Cys Leu Ser Pro His Ala Met Asn
        420                 425                 430
Cys Asn Thr Met Asn Arg Ser Gly Arg Met Lys Pro Leu Thr Pro Ser
    435                 440                 445
Asn Leu Asp Asp Leu Phe Ser Ala Glu Ser Ser Ser Pro Arg Tyr Ala
    450                 455                 460
Asp Pro Ala Leu Ala Ser Ala Val Phe Ser Pro Thr His Lys Ser Ala
465                 470                 475                 480
Val Phe Asn Gln Phe Gln His Gln Gln Ser Met Leu Ala Pro Leu Asn
                485                 490                 495
Thr Asn Phe Ala Ser Lys Asn Phe Glu His Pro Leu Leu Gln Ala Ser
        500                 505                 510
Leu Gly Met Ser Pro Arg Asn Val Glu Pro Ile Ser Pro Met Gly Ser
    515                 520                 525
Arg Ile Ser Met Leu Ala Gln Arg Glu Lys Gln Gln Phe Arg Ser Leu
    530                 535                 540
Ser Phe Arg Glu Leu Gly Ser Asn Ser Ala Ala Ala Ser Ala Asp Ser
545                 550                 555                 560
Trp Ser Lys Trp Gly Ser Pro Asn Val Lys Leu Asp Trp Pro Val Gly
                565                 570                 575
Ala Gly Glu Val Gly Lys Leu Arg Arg Ser Ser Ser Phe Glu Leu Gly
        580                 585                 590
Asn Asn Gly Glu Glu Pro Asp Leu Ser Trp Val Gln Ser Leu Val Lys
    595                 600                 605
Glu Ser Pro Ala Glu Val Lys Asp Lys Leu Ala Thr Thr Val Ser Tyr
    610                 615                 620
Val Ala Ala Ala Ala Ala Gly Ser Ser Ser Glu Gly Ser Asn Ile Ser
625                 630                 635                 640
Thr Gln Met Glu Ser Val Val Asp His Ala Val Leu Gly Ala Trp Leu
```

645 650 655
Glu Gln Met Gln Leu Asp His Leu Val Ala Gln Gln Asn
660 665

<210> 47
<211> 580
<212> PRT
<213> Arabidopsis thaliana

<400> 47

```
Met Cys Ser Gly Pro Lys Ser Asn Leu Cys Ser Ser Arg Thr Leu Thr
1               5                   10                  15
Glu Ile Glu Ser Arg Gln Lys Glu Glu Glu Thr Met Leu Leu Leu Glu
            20                  25                  30
Phe Ala Ala Cys Asp Asp Leu Asp Ser Phe Lys Arg Glu Val Glu Glu
        35                  40                  45
Lys Gly Leu Asp Leu Asp Glu Ser Gly Leu Trp Tyr Cys Arg Arg Val
    50                  55                  60
Gly Ser Lys Lys Met Gly Leu Glu Glu Arg Thr Pro Leu Met Val Ala
65                  70                  75                  80
Ala Met Tyr Gly Ser Ile Lys Val Leu Thr Phe Ile Val Ser Thr Gly
                85                  90                  95
Lys Ser Asp Val Asn Arg Ala Cys Gly Glu Glu Arg Val Thr Pro Leu
            100                 105                 110
His Cys Ala Val Ala Gly Cys Ser Val Asn Met Ile Glu Val Ile Asn
        115                 120                 125
Val Leu Leu Asp Ala Ser Ala Leu Val Asn Ser Val Asp Ala Asn Gly
    130                 135                 140
Asn Gln Pro Leu Asp Val Phe Val Arg Val Ser Arg Phe Val Ala Ser
145                 150                 155                 160
Pro Arg Arg Lys Ala Val Glu Leu Leu Leu Arg Gly Gly Gly Val Gly
                165                 170                 175
Gly Leu Ile Asp Glu Ala Val Glu Glu Glu Ile Lys Ile Val Ser Lys
            180                 185                 190
Tyr Pro Ala Asp Ala Ser Leu Pro Asp Ile Asn Glu Gly Val Tyr Gly
        195                 200                 205
Ser Asp Glu Phe Arg Met Tyr Ser Phe Lys Val Lys Pro Cys Ser Arg
    210                 215                 220
Ala Tyr Ser His Asp Trp Thr Glu Cys Ala Phe Val His Pro Gly Glu
225                 230                 235                 240
Asn Ala Arg Arg Arg Asp Pro Arg Lys Tyr Pro Tyr Thr Cys Val Pro
                245                 250                 255
Cys Pro Glu Phe Arg Lys Gly Ser Cys Pro Lys Gly Asp Ser Cys Glu
            260                 265                 270
Tyr Ala His Gly Val Phe Glu Ser Trp Leu His Pro Ala Gln Tyr Lys
        275                 280                 285
Thr Arg Leu Cys Lys Asp Glu Thr Gly Cys Ala Arg Lys Val Cys Phe
    290                 295                 300
Phe Ala His Lys Arg Glu Glu Met Arg Pro Val Asn Ala Ser Thr Gly
305                 310                 315                 320
Ser Ala Val Ala Gln Ser Pro Phe Ser Ser Leu Glu Met Met Pro Gly
                325                 330                 335
Leu Ser Pro Leu Ala Tyr Ser Ser Gly Val Ser Thr Pro Pro Val Ser
            340                 345                 350
Pro Met Ala Asn Gly Val Pro Ser Ser Pro Arg Asn Gly Gly Ser Trp
        355                 360                 365
Gln Asn Arg Val Asn Thr Leu Thr Pro Pro Ala Leu Gln Leu Asn Gly
    370                 375                 380
Gly Ser Arg Leu Lys Ser Thr Leu Ser Ala Arg Asp Ile Asp Met Glu
```

```
385                 390                 395                 400
Met Glu Met Glu Leu Arg Leu Arg Gly Phe Gly Asn Asn Val Glu Glu
                405                 410                 415
Thr Phe Gly Ser Tyr Val Ser Ser Pro Ser Arg Asn Ser Gln Met Gly
            420                 425                 430
Gln Asn Met Asn Gln His Tyr Pro Ser Ser Pro Val Arg Gln Pro Pro
        435                 440                 445
Ser Gln His Gly Phe Glu Ser Ser Ala Ala Ala Ala Val Ala Val Met
    450                 455                 460
Lys Ala Arg Ser Thr Ala Phe Ala Lys Arg Ser Leu Ser Phe Lys Pro
465                 470                 475                 480
Ala Thr Gln Ala Ala Pro Gln Ser Asn Leu Ser Asp Trp Gly Ser Pro
                485                 490                 495
Asn Gly Lys Leu Glu Trp Gly Met Lys Gly Glu Glu Leu Asn Lys Met
            500                 505                 510
Arg Arg Ser Val Ser Phe Gly Ile His Gly Asn Asn Asn Asn Asn Ala
        515                 520                 525
Ala Arg Asp Tyr Arg Asp Glu Pro Asp Val Ser Trp Val Asn Ser Leu
    530                 535                 540
Val Lys Asp Ser Thr Val Val Ser Glu Arg Ser Phe Gly Met Asn Glu
545                 550                 555                 560
Arg Val Arg Ile Met Ser Trp Ala Glu Gln Met Tyr Arg Glu Lys Glu
                565                 570                 575
Gln Thr Val Val
            580
```

<210> 48
<211> 719
<212> PRT
<213> Arabidopsis thaliana

<400> 48

```
Met Cys Cys Gly Ser Asp Arg Leu Asn Gln Ile Val Ser Ser Arg Ser
1               5                   10                  15
Ser Leu Pro Ile Ser Phe Glu Glu Asp Asn Asn Leu Val Thr Asn Thr
            20                  25                  30
Asp Met Asn His Ile Thr Val Glu Thr Glu Asp Thr Phe Ala Ser Leu
        35                  40                  45
Leu Glu Leu Ala Ala Asn Asn Asp Val Glu Gly Val Arg Leu Ser Ile
    50                  55                  60
Glu Arg Asp Pro Ser Cys Val Asp Glu Ala Gly Leu Trp Tyr Gly Arg
65                  70                  75                  80
Gln Lys Gly Ser Lys Ala Met Val Asn Asp Tyr Arg Thr Pro Leu Met
                85                  90                  95
Val Ala Ala Thr Tyr Gly Ser Ile Asp Val Ile Lys Leu Ile Val Ser
            100                 105                 110
Leu Thr Asp Ala Asp Val Asn Arg Ala Cys Gly Asn Asp Gln Thr Thr
        115                 120                 125
Ala Leu His Cys Ala Ala Ser Gly Gly Ala Val Asn Ala Ile Gln Val
    130                 135                 140
Val Lys Leu Leu Leu Ala Ala Gly Ala Asp Leu Asn Leu Leu Asp Ala
145                 150                 155                 160
Glu Gly Gln Arg Ala Gly Asp Val Ile Val Val Pro Pro Lys Leu Glu
                165                 170                 175
Gly Val Lys Leu Met Leu Gln Glu Leu Leu Ser Ala Asp Gly Ser Ser
            180                 185                 190
Thr Ala Glu Arg Asn Leu Arg Val Val Thr Asn Val Pro Asn Arg Ser
        195                 200                 205
Ser Ser Pro Cys His Ser Pro Thr Gly Glu Asn Gly Gly Ser Gly Ser
```

```
    210                 215                 220
Gly Ser Pro Leu Gly Ser Pro Phe Lys Leu Lys Ser Thr Glu Phe Lys
225                 230                 235                 240
Lys Glu Tyr Pro Val Asp Pro Ser Leu Pro Asp Ile Lys Asn Ser Ile
                245                 250                 255
Tyr Ala Thr Asp Glu Phe Arg Met Tyr Ser Phe Lys Val Arg Pro Cys
            260                 265                 270
Ser Arg Ala Tyr Ser His Asp Trp Thr Glu Cys Pro Phe Val His Pro
        275                 280                 285
Gly Glu Asn Ala Arg Arg Arg Asp Pro Arg Lys Phe His Tyr Ser Cys
    290                 295                 300
Val Pro Cys Pro Asp Phe Arg Lys Gly Ala Cys Arg Arg Gly Asp Met
305                 310                 315                 320
Cys Glu Tyr Ala His Gly Val Phe Glu Cys Trp Leu His Pro Ala Gln
                325                 330                 335
Tyr Arg Thr Arg Leu Cys Lys Asp Gly Thr Gly Cys Ala Arg Arg Val
            340                 345                 350
Cys Phe Phe Ala His Thr Pro Glu Glu Leu Arg Pro Leu Tyr Ala Ser
        355                 360                 365
Thr Gly Ser Ala Val Pro Ser Pro Arg Ser Asn Ala Asp Tyr Ala Ala
    370                 375                 380
Ala Leu Ser Leu Leu Pro Gly Ser Pro Ser Gly Val Ser Val Met Ser
385                 390                 395                 400
Pro Leu Ser Pro Ser Ala Ala Gly Asn Gly Met Ser His Ser Asn Met
                405                 410                 415
Ala Trp Pro Gln Pro Asn Val Pro Ala Leu His Leu Pro Gly Ser Asn
            420                 425                 430
Leu Gln Ser Ser Arg Leu Arg Ser Ser Leu Asn Ala Arg Asp Ile Pro
        435                 440                 445
Thr Asp Glu Phe Asn Met Leu Ala Asp Tyr Glu Gln Gln Gln Leu Leu
    450                 455                 460
Asn Glu Tyr Ser Asn Ala Leu Ser Arg Ser Gly Arg Met Lys Ser Met
465                 470                 475                 480
Pro Pro Ser Asn Leu Glu Asp Leu Phe Ser Ala Glu Gly Ser Ser Ser
                485                 490                 495
Pro Arg Phe Thr Asp Ser Ala Leu Ala Ser Ala Val Phe Ser Pro Thr
            500                 505                 510
His Lys Ser Ala Val Phe Asn Gln Phe Gln Gln Gln Gln Gln Gln Gln
        515                 520                 525
Gln Gln Gln Ser Met Leu Ser Pro Ile Asn Thr Ser Phe Ser Ser Pro
    530                 535                 540
Lys Ser Val Asp His Ser Leu Phe Ser Gly Gly Gly Arg Met Ser Pro
545                 550                 555                 560
Arg Asn Val Val Glu Pro Ile Ser Pro Met Ser Ala Arg Val Ser Met
                565                 570                 575
Leu Ala Gln Cys Val Lys Gln Gln Gln Gln Gln Gln Gln Gln Gln Gln
            580                 585                 590
Gln Gln Gln His Gln Phe Arg Ser Leu Ser Ser Arg Glu Leu Arg Thr
        595                 600                 605
Asn Ser Ser Pro Ile Val Gly Ser Pro Val Asn Asn Asn Thr Trp Ser
    610                 615                 620
Ser Lys Trp Gly Ser Ser Asn Gly Gln Pro Asp Trp Gly Met Ser Ser
625                 630                 635                 640
Glu Ala Leu Gly Lys Leu Arg Ser Ser Ser Ser Phe Asp Gly Asp Glu
                645                 650                 655
Pro Asp Val Ser Trp Val Gln Ser Leu Val Lys Glu Thr Pro Ala Glu
            660                 665                 670
Ala Lys Glu Lys Ala Ala Thr Ser Ser Ser Gly Glu His Val Met Lys
        675                 680                 685
```

Gln Pro Asn Pro Val Glu Pro Val Met Asp His Ala Gly Leu Glu Ala
690 695 700
Trp Ile Glu Gln Met Gln Leu Asp Gln Leu Val Ala Gln Gln Asn
705 710 715

<210> 49
<211> 686
<212> PRT
<213> Arabidopsis thaliana

<400> 49

```
Met Asn His Ile Thr Val Glu Thr Glu Asp Thr Phe Ala Ser Leu Leu
1               5                   10                  15
Glu Leu Ala Ala Asn Asn Asp Val Glu Gly Val Arg Leu Ser Ile Glu
            20                  25                  30
Arg Asp Pro Ser Cys Val Asp Glu Ala Gly Leu Trp Tyr Gly Arg Gln
        35                  40                  45
Lys Gly Ser Lys Ala Met Val Asn Asp Tyr Arg Thr Pro Leu Met Val
    50                  55                  60
Ala Ala Thr Tyr Gly Ser Ile Asp Val Ile Lys Leu Ile Val Ser Leu
65                  70                  75                  80
Thr Asp Ala Asp Val Asn Arg Ala Cys Gly Asn Asp Gln Thr Thr Ala
                85                  90                  95
Leu His Cys Ala Ala Ser Gly Gly Ala Val Asn Ala Ile Gln Val Val
            100                 105                 110
Lys Leu Leu Leu Ala Ala Gly Ala Asp Leu Asn Leu Leu Asp Ala Glu
        115                 120                 125
Gly Gln Arg Ala Gly Asp Val Ile Val Val Pro Pro Lys Leu Glu Gly
    130                 135                 140
Val Lys Leu Met Leu Gln Glu Leu Leu Ser Ala Asp Gly Ser Ser Thr
145                 150                 155                 160
Ala Glu Arg Asn Leu Arg Val Val Thr Asn Val Pro Asn Arg Ser Ser
                165                 170                 175
Ser Pro Cys His Ser Pro Thr Gly Glu Asn Gly Gly Ser Gly Ser Gly
            180                 185                 190
Ser Pro Leu Gly Ser Pro Phe Lys Leu Lys Ser Thr Glu Phe Lys Lys
        195                 200                 205
Glu Tyr Pro Val Asp Pro Ser Leu Pro Asp Ile Lys Asn Ser Ile Tyr
    210                 215                 220
Ala Thr Asp Glu Phe Arg Met Tyr Ser Phe Lys Val Arg Pro Cys Ser
225                 230                 235                 240
Arg Ala Tyr Ser His Asp Trp Thr Glu Cys Pro Phe Val His Pro Gly
                245                 250                 255
Glu Asn Ala Arg Arg Arg Asp Pro Arg Lys Phe His Tyr Ser Cys Val
            260                 265                 270
Pro Cys Pro Asp Phe Arg Lys Gly Ala Cys Arg Arg Gly Asp Met Cys
        275                 280                 285
Glu Tyr Ala His Gly Val Phe Glu Cys Trp Leu His Pro Ala Gln Tyr
    290                 295                 300
Arg Thr Arg Leu Cys Lys Asp Gly Thr Gly Cys Ala Arg Arg Val Cys
305                 310                 315                 320
Phe Phe Ala His Thr Pro Glu Glu Leu Arg Pro Leu Tyr Ala Ser Thr
                325                 330                 335
Gly Ser Ala Val Pro Ser Pro Arg Ser Asn Ala Asp Tyr Ala Ala Ala
            340                 345                 350
Leu Ser Leu Leu Pro Gly Ser Pro Ser Gly Val Ser Val Met Ser Pro
        355                 360                 365
Leu Ser Pro Ser Ala Ala Gly Asn Gly Met Ser His Ser Asn Met Ala
    370                 375                 380
```

```
Trp Pro Gln Pro Asn Val Pro Ala Leu His Leu Pro Gly Ser Asn Leu
385                 390                 395                 400
Gln Ser Ser Arg Leu Arg Ser Ser Leu Asn Ala Arg Asp Ile Pro Thr
                405                 410                 415
Asp Glu Phe Asn Met Leu Ala Asp Tyr Glu Gln Gln Gln Leu Leu Asn
            420                 425                 430
Glu Tyr Ser Asn Ala Leu Ser Arg Ser Gly Arg Met Lys Ser Met Pro
        435                 440                 445
Pro Ser Asn Leu Glu Asp Leu Phe Ser Ala Glu Gly Ser Ser Ser Pro
    450                 455                 460
Arg Phe Thr Asp Ser Ala Leu Ala Ser Ala Val Phe Ser Pro Thr His
465                 470                 475                 480
Lys Ser Ala Val Phe Asn Gln Phe Gln Gln Gln Gln Gln Gln Gln Gln
                485                 490                 495
Gln Gln Ser Met Leu Ser Pro Ile Asn Thr Ser Phe Ser Ser Pro Lys
            500                 505                 510
Ser Val Asp His Ser Leu Phe Ser Gly Gly Gly Arg Met Ser Pro Arg
        515                 520                 525
Asn Val Val Glu Pro Ile Ser Pro Met Ser Ala Arg Val Ser Met Leu
    530                 535                 540
Ala Gln Cys Val Lys Gln Gln Gln Gln Gln Gln Gln Gln Gln Gln Gln
545                 550                 555                 560
Gln Gln His Gln Phe Arg Ser Leu Ser Ser Arg Glu Leu Arg Thr Asn
                565                 570                 575
Ser Ser Pro Ile Val Gly Ser Pro Val Asn Asn Asn Thr Trp Ser Ser
            580                 585                 590
Lys Trp Gly Ser Ser Asn Gly Gln Pro Asp Trp Gly Met Ser Ser Glu
        595                 600                 605
Ala Leu Gly Lys Leu Arg Ser Ser Ser Ser Phe Asp Gly Asp Glu Pro
    610                 615                 620
Asp Val Ser Trp Val Gln Ser Leu Val Lys Glu Thr Pro Ala Glu Ala
625                 630                 635                 640
Lys Glu Lys Ala Ala Thr Ser Ser Ser Gly Glu His Val Met Lys Gln
                645                 650                 655
Pro Asn Pro Val Glu Pro Val Met Asp His Ala Gly Leu Glu Ala Trp
            660                 665                 670
Ile Glu Gln Met Gln Leu Asp Gln Leu Val Ala Gln Gln Asn
        675                 680                 685
```

<210> 50
<211> 633
<212> PRT
<213> Arabidopsis thaliana

<400> 50

```
Met Val Asn Asp Tyr Arg Thr Pro Leu Met Val Ala Ala Thr Tyr Gly
1               5                   10                  15
Ser Ile Asp Val Ile Lys Leu Ile Val Ser Leu Thr Asp Ala Asp Val
            20                  25                  30
Asn Arg Ala Cys Gly Asn Asp Gln Thr Thr Ala Leu His Cys Ala Ala
        35                  40                  45
Ser Gly Gly Ala Val Asn Ala Ile Gln Val Val Lys Leu Leu Leu Ala
    50                  55                  60
Ala Gly Ala Asp Leu Asn Leu Leu Asp Ala Glu Gly Gln Arg Ala Gly
65                  70                  75                  80
Asp Val Ile Val Val Pro Pro Lys Leu Glu Gly Val Lys Leu Met Leu
                85                  90                  95
Gln Glu Leu Leu Ser Ala Asp Gly Ser Ser Thr Ala Glu Arg Asn Leu
            100                 105                 110
```

```
Arg Val Val Thr Asn Val Pro Asn Arg Ser Ser Ser Pro Cys His Ser
        115                 120                 125
Pro Thr Gly Glu Asn Gly Gly Ser Gly Ser Gly Ser Pro Leu Gly Ser
    130                 135                 140
Pro Phe Lys Leu Lys Ser Thr Glu Phe Lys Lys Glu Tyr Pro Val Asp
145                 150                 155                 160
Pro Ser Leu Pro Asp Ile Lys Asn Ser Ile Tyr Ala Thr Asp Glu Phe
                165                 170                 175
Arg Met Tyr Ser Phe Lys Val Arg Pro Cys Ser Arg Ala Tyr Ser His
            180                 185                 190
Asp Trp Thr Glu Cys Pro Phe Val His Pro Gly Glu Asn Ala Arg Arg
        195                 200                 205
Arg Asp Pro Arg Lys Phe His Tyr Ser Cys Val Pro Cys Pro Asp Phe
    210                 215                 220
Arg Lys Gly Ala Cys Arg Arg Gly Asp Met Cys Glu Tyr Ala His Gly
225                 230                 235                 240
Val Phe Glu Cys Trp Leu His Pro Ala Gln Tyr Arg Thr Arg Leu Cys
                245                 250                 255
Lys Asp Gly Thr Gly Cys Ala Arg Arg Val Cys Phe Phe Ala His Thr
            260                 265                 270
Pro Glu Glu Leu Arg Pro Leu Tyr Ala Ser Thr Gly Ser Ala Val Pro
        275                 280                 285
Ser Pro Arg Ser Asn Ala Asp Tyr Ala Ala Ala Leu Ser Leu Leu Pro
    290                 295                 300
Gly Ser Pro Ser Gly Val Ser Val Met Ser Pro Leu Ser Pro Ser Ala
305                 310                 315                 320
Ala Gly Asn Gly Met Ser His Ser Asn Met Ala Trp Pro Gln Pro Asn
                325                 330                 335
Val Pro Ala Leu His Leu Pro Gly Ser Asn Leu Gln Ser Ser Arg Leu
            340                 345                 350
Arg Ser Ser Leu Asn Ala Arg Asp Ile Pro Thr Asp Glu Phe Asn Met
        355                 360                 365
Leu Ala Asp Tyr Glu Gln Gln Gln Leu Leu Asn Glu Tyr Ser Asn Ala
    370                 375                 380
Leu Ser Arg Ser Gly Arg Met Lys Ser Met Pro Pro Ser Asn Leu Glu
385                 390                 395                 400
Asp Leu Phe Ser Ala Glu Gly Ser Ser Ser Pro Arg Phe Thr Asp Ser
                405                 410                 415
Ala Leu Ala Ser Ala Val Phe Ser Pro Thr His Lys Ser Ala Val Phe
            420                 425                 430
Asn Gln Phe Gln Gln Gln Gln Gln Gln Gln Gln Gln Gln Ser Met Leu
        435                 440                 445
Ser Pro Ile Asn Thr Ser Phe Ser Ser Pro Lys Ser Val Asp His Ser
    450                 455                 460
Leu Phe Ser Gly Gly Gly Arg Met Ser Pro Arg Asn Val Val Glu Pro
465                 470                 475                 480
Ile Ser Pro Met Ser Ala Arg Val Ser Met Leu Ala Gln Cys Val Lys
                485                 490                 495
Gln Gln Gln Gln Gln Gln Gln Gln Gln Gln Gln Gln Gln His Gln Phe
            500                 505                 510
Arg Ser Leu Ser Ser Arg Glu Leu Arg Thr Asn Ser Ser Pro Ile Val
        515                 520                 525
Gly Ser Pro Val Asn Asn Asn Thr Trp Ser Ser Lys Trp Gly Ser Ser
    530                 535                 540
Asn Gly Gln Pro Asp Trp Gly Met Ser Ser Glu Ala Leu Gly Lys Leu
545                 550                 555                 560
Arg Ser Ser Ser Ser Phe Asp Gly Asp Glu Pro Asp Val Ser Trp Val
                565                 570                 575
Gln Ser Leu Val Lys Glu Thr Pro Ala Glu Ala Lys Glu Lys Ala Ala
```

```
                580                     585                     590
Thr Ser Ser Ser Gly Glu His Val Met Lys Gln Pro Asn Pro Val Glu
            595                     600                     605
Pro Val Met Asp His Ala Gly Leu Glu Ala Trp Ile Glu Gln Met Gln
    610                     615                     620
Leu Asp Gln Leu Val Ala Gln Gln Asn
625                 630
```

<210> 51
<211> 678
<212> PRT
<213> Arabidopsis thaliana

<400> 51

Met Asn Asp Ala Ala Glu Trp Glu His Ser Phe Ser Ala Leu Leu Glu
1               5                   10                  15
Phe Ala Ala Asp Asn Asp Val Glu Gly Phe Arg Arg Gln Leu Ser Asp
            20                  25                  30
Val Ser Cys Ile Asn Gln Met Gly Leu Trp Tyr Arg Arg Gln Arg Phe
        35                  40                  45
Val Arg Arg Met Val Leu Glu Gln Arg Thr Pro Leu Met Val Ala Ser
    50                  55                  60
Leu Tyr Gly Ser Leu Asp Val Val Lys Phe Ile Leu Ser Phe Pro Glu
65                  70                  75                  80
Ala Glu Leu Asn Leu Ser Cys Gly Pro Asp Lys Ser Thr Ala Leu His
                85                  90                  95
Cys Ala Ala Ser Gly Ala Ser Val Asn Ser Leu Asp Val Val Lys Leu
            100                 105                 110
Leu Leu Ser Val Gly Ala Asp Pro Asn Ile Pro Asp Ala His Gly Asn
        115                 120                 125
Arg Pro Val Asp Val Leu Val Val Ser Pro His Ala Pro Gly Leu Arg
    130                 135                 140
Thr Ile Leu Glu Glu Ile Leu Lys Lys Asp Glu Ile Ile Ser Glu Asp
145                 150                 155                 160
Leu His Ala Ser Ser Ser Ser Leu Gly Ser Ser Phe Arg Ser Leu Ser
                165                 170                 175
Ser Ser Pro Asp Asn Gly Ser Ser Leu Leu Ser Leu Asp Ser Val Ser
            180                 185                 190
Ser Pro Thr Lys Pro His Gly Thr Asp Val Thr Phe Ala Ser Glu Lys
        195                 200                 205
Lys Glu Tyr Pro Ile Asp Pro Ser Leu Pro Asp Ile Lys Ser Gly Ile
    210                 215                 220
Tyr Ser Thr Asp Glu Phe Arg Met Phe Ser Phe Lys Ile Arg Pro Cys
225                 230                 235                 240
Ser Arg Ala Tyr Ser His Asp Trp Thr Glu Cys Pro Phe Ala His Pro
                245                 250                 255
Gly Glu Asn Ala Arg Arg Arg Asp Pro Arg Lys Phe His Tyr Thr Cys
            260                 265                 270
Val Pro Cys Pro Asp Phe Lys Lys Gly Ser Cys Lys Gln Gly Asp Met
        275                 280                 285
Cys Glu Tyr Ala His Gly Val Phe Glu Cys Trp Leu His Pro Ala Gln
    290                 295                 300
Tyr Arg Thr Arg Leu Cys Lys Asp Gly Met Gly Cys Asn Arg Arg Val
305                 310                 315                 320
Cys Phe Phe Ala His Ala Asn Glu Glu Leu Arg Pro Leu Tyr Pro Ser
                325                 330                 335
Thr Gly Ser Gly Leu Pro Ser Pro Arg Ala Ser Ser Ala Val Ser Ala
            340                 345                 350
Ser Thr Met Asp Met Ala Ser Val Leu Asn Met Leu Pro Gly Ser Pro

```
        355                 360                 365
Ser Ala Ala Gln His Ser Phe Thr Pro Pro Ile Ser Pro Ser Gly Asn
    370                 375                 380
Gly Ser Met Pro His Ser Ser Met Gly Trp Pro Gln Gln Asn Ile Pro
385                 390                 395                 400
Ala Leu Asn Leu Pro Gly Ser Asn Ile Gln Leu Ser Arg Leu Arg Ser
                405                 410                 415
Ser Leu Asn Ala Arg Asp Ile Pro Ser Glu Gln Leu Ser Met Leu His
            420                 425                 430
Glu Phe Glu Met Gln Arg Gln Leu Ala Gly Asp Met His Ser Pro Arg
        435                 440                 445
Phe Met Asn His Ser Ala Arg Pro Lys Thr Leu Asn Pro Ser Asn Leu
    450                 455                 460
Glu Glu Leu Phe Ser Ala Glu Val Ala Ser Pro Arg Phe Ser Asp Gln
465                 470                 475                 480
Leu Ala Val Ser Ser Val Leu Ser Pro Ser His Lys Ser Ala Leu Leu
                485                 490                 495
Asn Gln Leu Gln Asn Asn Lys Gln Ser Met Leu Ser Pro Ile Lys Thr
            500                 505                 510
Asn Leu Met Ser Ser Pro Lys Asn Val Glu Gln His Ser Leu Leu Gln
        515                 520                 525
Gln Ala Ser Ser Pro Arg Gly Gly Glu Pro Ile Ser Pro Met Asn Ala
    530                 535                 540
Arg Met Lys Gln Gln Leu His Ser Arg Ser Leu Ser Ser Arg Asp Phe
545                 550                 555                 560
Gly Ser Ser Leu Pro Arg Asp Leu Met Pro Thr Asp Ser Gly Ser Pro
                565                 570                 575
Leu Ser Pro Trp Ser Ser Trp Asp Gln Thr His Gly Ser Lys Val Asp
            580                 585                 590
Trp Ser Val Gln Ser Asp Glu Leu Gly Arg Leu Arg Lys Ser His Ser
        595                 600                 605
Leu Ala Asn Asn Pro Asn Arg Glu Ala Asp Val Ser Trp Ala Gln Gln
    610                 615                 620
Met Leu Lys Asp Ser Ser Ser Pro Arg Asn Gly Asn Arg Val Val Asn
625                 630                 635                 640
Met Asn Gly Ala Arg Pro Leu Thr Gln Gly Gly Ser Ser Val Asn Pro
                645                 650                 655
His Asn Ser Asp Thr Arg Glu Ser Asp Ile Leu Asp Ala Trp Leu Glu
            660                 665                 670
Gln Leu His Leu Asp Arg
        675
```

<210> 52
<211> 640
<212> PRT
<213> Arabidopsis thaliana

<400> 52

```
Met Gly Leu Trp Tyr Arg Arg Gln Arg Phe Val Arg Arg Met Val Leu
1               5                   10                  15
Glu Gln Arg Thr Pro Leu Met Val Ala Ser Leu Tyr Gly Ser Leu Asp
            20                  25                  30
Val Val Lys Phe Ile Leu Ser Phe Pro Glu Ala Glu Leu Asn Leu Ser
        35                  40                  45
Cys Gly Pro Asp Lys Ser Thr Ala Leu His Cys Ala Ala Ser Gly Ala
    50                  55                  60
Ser Val Asn Ser Leu Asp Val Val Lys Leu Leu Leu Ser Val Gly Ala
65                  70                  75                  80
Asp Pro Asn Ile Pro Asp Ala His Gly Asn Arg Pro Val Asp Val Leu
```

```
                    85                  90                  95
Val Val Ser Pro His Ala Pro Gly Leu Arg Thr Ile Leu Glu Glu Ile
                100                 105                 110
Leu Lys Lys Asp Glu Ile Ile Ser Glu Asp Leu His Ala Ser Ser Ser
            115                 120                 125
Ser Leu Gly Ser Ser Phe Arg Ser Leu Ser Ser Ser Pro Asp Asn Gly
    130                 135                 140
Ser Ser Leu Leu Ser Leu Asp Ser Val Ser Ser Pro Thr Lys Pro His
145                 150                 155                 160
Gly Thr Asp Val Thr Phe Ala Ser Glu Lys Lys Glu Tyr Pro Ile Asp
                165                 170                 175
Pro Ser Leu Pro Asp Ile Lys Ser Gly Ile Tyr Ser Thr Asp Glu Phe
            180                 185                 190
Arg Met Phe Ser Phe Lys Ile Arg Pro Cys Ser Arg Ala Tyr Ser His
        195                 200                 205
Asp Trp Thr Glu Cys Pro Phe Ala His Pro Gly Glu Asn Ala Arg Arg
    210                 215                 220
Arg Asp Pro Arg Lys Phe His Tyr Thr Cys Val Pro Cys Pro Asp Phe
225                 230                 235                 240
Lys Lys Gly Ser Cys Lys Gln Gly Asp Met Cys Glu Tyr Ala His Gly
                245                 250                 255
Val Phe Glu Cys Trp Leu His Pro Ala Gln Tyr Arg Thr Arg Leu Cys
            260                 265                 270
Lys Asp Gly Met Gly Cys Asn Arg Arg Val Cys Phe Phe Ala His Ala
        275                 280                 285
Asn Glu Glu Leu Arg Pro Leu Tyr Pro Ser Thr Gly Ser Gly Leu Pro
    290                 295                 300
Ser Pro Arg Ala Ser Ser Ala Val Ser Ala Ser Thr Met Asp Met Ala
305                 310                 315                 320
Ser Val Leu Asn Met Leu Pro Gly Ser Pro Ser Ala Ala Gln His Ser
                325                 330                 335
Phe Thr Pro Pro Ile Ser Pro Ser Gly Asn Gly Ser Met Pro His Ser
            340                 345                 350
Ser Met Gly Trp Pro Gln Gln Asn Ile Pro Ala Leu Asn Leu Pro Gly
        355                 360                 365
Ser Asn Ile Gln Leu Ser Arg Leu Arg Ser Ser Leu Asn Ala Arg Asp
    370                 375                 380
Ile Pro Ser Glu Gln Leu Ser Met Leu His Glu Phe Glu Met Gln Arg
385                 390                 395                 400
Gln Leu Ala Gly Asp Met His Ser Pro Arg Phe Met Asn His Ser Ala
                405                 410                 415
Arg Pro Lys Thr Leu Asn Pro Ser Asn Leu Glu Glu Leu Phe Ser Ala
            420                 425                 430
Glu Val Ala Ser Pro Arg Phe Ser Asp Gln Leu Ala Val Ser Ser Val
        435                 440                 445
Leu Ser Pro Ser His Lys Ser Ala Leu Leu Asn Gln Leu Gln Asn Asn
    450                 455                 460
Lys Gln Ser Met Leu Ser Pro Ile Lys Thr Asn Leu Met Ser Ser Pro
465                 470                 475                 480
Lys Asn Val Glu Gln His Ser Leu Leu Gln Gln Ala Ser Ser Pro Arg
                485                 490                 495
Gly Gly Glu Pro Ile Ser Pro Met Asn Ala Arg Met Lys Gln Gln Leu
            500                 505                 510
His Ser Arg Ser Leu Ser Ser Arg Asp Phe Gly Ser Ser Leu Pro Arg
        515                 520                 525
Asp Leu Met Pro Thr Asp Ser Gly Ser Pro Leu Ser Pro Trp Ser Ser
    530                 535                 540
Trp Asp Gln Thr His Gly Ser Lys Val Asp Trp Ser Val Gln Ser Asp
545                 550                 555                 560
```

```
Glu Leu Gly Arg Leu Arg Lys Ser His Ser Leu Ala Asn Asn Pro Asn
                565                 570                 575
Arg Glu Ala Asp Val Ser Trp Ala Gln Gln Met Leu Lys Asp Ser Ser
            580                 585                 590
Ser Pro Arg Asn Gly Asn Arg Val Val Asn Met Asn Gly Ala Arg Pro
        595                 600                 605
Leu Thr Gln Gly Gly Ser Ser Val Asn Pro His Asn Ser Asp Thr Arg
    610                 615                 620
Glu Ser Asp Ile Leu Asp Ala Trp Leu Glu Gln Leu His Leu Asp Arg
625                 630                 635                 640
```

<210> 53
<211> 2158
<212> DNA
<213> Oryza sativa

<400> 53

```
tgctccttct tcattgcaaa gaaagcacca ccttttaaag aatcctcctc acactccatt      60
cttcttaaaa aacccaccac aacacaattc ccacttgttt cttcatcatc acctacttca     120
atcaaaaaac attccaactt tcttctcaat ttcattccag gatagtatta tgtgcagtgg     180
accaaagagc aatctctgct cttcaagaac cttaacagaa atcgaatcaa ggcaaaagga     240
agaagaaaca atgcttctcc tcgaattcgc tgcttgtgat gatcttgact cgttcaagag     300
agaggttgaa gagaaagggc ttgatttgga tgagtcaggg ttatggtatt gcagacgtgt     360
cggttctaag aagatgggtc ttgaagaaag aacaccttta atggttgcag ctatgtatgg     420
aagcataaag gttttgactt tcatcgtttc cactggaaaa tctgatgtga acagagcttg     480
tggtgaagag agagttactc cgcttcactg tgctgttgct ggctgttctg tgaatatgat     540
tgaagtcatc aatgtcttgc ttgatgcttc tgctttggtt aactctgttg atgctaatgg     600
gaatcaacct ttggatgtgt ttgttcgagt ttcgaggttt gtggctagtc cgaggaggaa     660
agcggttgag ttgttgctga gaggaggagg tgttggagga ttgatcgatg aggcggttga     720
agaagagatc aagattgtct ctaagtatcc agctgatgct tctttaccgg atataaacga     780
aggggtttat ggaagtgatg agtttaggat gtatagcttt aaggttaagc catgttctag     840
ggcttattct catgattgga ccgagtgtgc ttttgttcat ccgggagaaa atgcgaggag     900
gagagatccg aggaagtatc cttacacttg tgtcccctgt cccgagttcc gtaaaggatc     960
atgcccgaaa ggagattctt gcgagtatgc tcacggggtt ttcgagtcgt ggcttcaccc    1020
cgcgcagtat aaaacccggc tttgtaaaga tgaaacgggt tgtgcaagga aagtttgttt    1080
ctttgctcat aaacgcgaag agatgagacc tgttaatgct tcaactggct ctgccgtggc    1140
tcagtctccg tttagcagct tggagatgat gccagggttg tctcctcttg cttattcttc    1200
aggagtttcg actcctccgg tttctccaat ggctaatggt gttccttcct ctccaagaaa    1260
cggcggatca tggcagaaca gagtcaatac ccttactcca ccggctttgc agctcaatgg    1320
tggaagcaga ttgaagtcca cactgagcgc tagagatatc gatatggaga tggagatgga    1380
attgagactc cgcggttttg gcaacaatgt ggaagagacg ttcgggtctt atgtttcctc    1440
tccaagtagg aattctcaaa tgggtcaaaa catgaaccaa cattatccat cttccccggt    1500
gagacaaccg ccatctcaac acgggttcga atcttcagca gctgcagcgg ttgcagtgat    1560
gaaagcgaga tcaaccgcct ttgcgaaacg tagcttgagc ttcaaaccag ctactcaagc    1620
agcaccacag tcgaatctct cggattgggg atctccaaac gggaagctgg aatggggaat    1680
gaaaggagaa gagctgaata agatgagaag aagtgtttcc tttggaatcc atggaaacaa    1740
caacaataac gcagctagag actacaggga cgagccagat gtgtcatggg ttaactcttt    1800
agttaaagac agtactgtgg tgtctgagag aagctttgga atgaatgaga gggttcggat    1860
aatgtcgtgg gctgagcaaa tgtacagaga gaaggagcag actgtggtgt aaacacacac    1920
aaagatggtt tcttatatat attgcttttg ggccatctct gcaaatttga ttctttaatt    1980
tttgtgactt tctttagttg ttactgttat tagtagtata tggtttgttg tcactacgag    2040
tctacgtgat gaaaagatag aagttaattg cattagtttc tatattcgtt tctcatcctc    2100
ttgtaattta tcaaaccatg aaatggctaa gcaatccaaa ccgaaaaaaa aaaaaaa       2158
```

<210> 54
<211> 2193
<212> DNA
<213> Oryza sativa

<400> 54

```
aatccgaaaa gtttctgcac cgttttcacc ccctaactaa caatataggg aacgtgtgct     60
aaatataaaa tgagacctta tatatgtagc gctgataact agaactatgc aagaaaaact    120
catccaccta ctttagtggc aatcgggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
aaaaaatctt tctagctgaa ctcaatgggt aaagagagag atttttttta aaaaaataga    360
atgaagatat tctgaacgta ttggcaaaga tttaaacata taattatata attttatagt    420
ttgtgcattc gtcatatcgc acatcattaa ggacatgtct tactccatcc caatttttat    480
ttagtaatta aagacaattg acttattttt attatttatc ttttttcgat tagatgcaag    540
gtacttacgc acacactttg tgctcatgtg catgtgtgag tgcacctcct caatacacgt    600
tcaactagca acacatctct aatatcactc gcctatttaa tacatttagg tagcaatatc    660
tgaattcaag cactccacca tcaccagacc acttttaata atatctaaaa tacaaaaaat    720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa ccttttaaca gcaggctttg cggccaggag agaggaggag aggcaaagaa    960
aaccaagcat cctcctcctc ccatctataa attcctcccc ccttttcccc tctctatata   1020
ggaggcatcc aagccaagaa gagggagagc accaaggaca cgcgactagc agaagccgag   1080
cgaccgcctt cttcgatcca tatcttccgg tcgagttctt ggtcgatctc ttccctcctc   1140
cacctcctcc tcacagggta tgtgcccttc ggttgttctt ggatttattg ttctaggttg   1200
tgtagtacgg gcgttgatgt taggaaaggg gatctgtatc tgtgatgatt cctgttcttg   1260
gatttgggat agaggggttc ttgatgttgc atgttatcgg ttcggtttga ttagtagtat   1320
ggttttcaat cgtctggaga gctctatgga aatgaaatgg tttagggtac ggaatcttgc   1380
gattttgtga gtaccttttg tttgaggtaa aatcagagca ccggtgattt tgcttggtgt   1440
aataaaagta cggttgtttg gtcctcgatt ctggtagtga tgcttctcga tttgacgaag   1500
ctatcctttg tttattccct attgaacaaa aataatccaa ctttgaagac ggtcccgttg   1560
atgagattga atgattgatt cttaagcctg tccaaaattt cgcagctggc ttgtttagat   1620
acagtagtcc ccatcacgaa attcatggaa acagttataa tcctcaggaa caggggattc   1680
cctgttcttc cgatttgctt tagtcccaga atttttttc ccaaatatct taaaaagtca   1740
ctttctggtt cagttcaatg aattgattgc tacaaataat gcttttatag cgttatccta   1800
gctgtagttc agttaatagg taatacccct atagtttagt caggagaaga acttatccga   1860
tttctgatct ccatttttaa ttatatgaaa tgaactgtag cataagcagt attcatttgg   1920
attatttttt ttattagctc tcacccette attattctga gctgaaagtc tggcatgaac   1980
tgtcctcaat tttgttttca aattcacatc gattatctat gcattatcct cttgtatcta   2040
cctgtagaag tttctttttg gttattcctt gactgcttga ttacagaaag aaatttatga   2100
agctgtaatc gggatagtta tactgcttgt tcttatgatt catttccttt gtgcagttct   2160
tggtgtagct tgccactttc accagcaaag ttc                                2193
```

<210> 55
<211> 1827
<212> DNA
<213> Oryza sativa

<400> 55

```
gcttgagtca tagggagaaa acaaatcgat catatttgac tcttttccct ccatctctct      60
taccggcaaa aaaagtagta ctggtttata tgtaaagtaa gattctttaa ttatgtgaga     120
tccggcttaa tgcttttctt ttgtcacata tactgcattg caacaattgc catatattca     180
cttctgccat cccattatat agcaactcaa gaatggattg atatatcccc tattactaat     240
ctagacatgt taaggctgag ttgggcagtc catcttccca acccaccacc ttcgtttttc     300
gcgcacatac ttttcaaact actaaatggt gtgtttttta aaaatatttt caatacaaaa     360
gttgctttaa aaaattatat tgatccattt ttttaaaaaa aatagctaat acttaattaa     420
tcacgtgtta aaagaccgct ccgttttgcg tgcaggaggg ataggttcac atcctgcatt     480
accgaacaca gcctaaatct tgttgtctag attcgtagta ctggatatat taaatcatgt     540
tctaagttac tatatactga gatgaataga ataagtaaaa ttagacccac cttaagtctt     600
gatgaagtta ctactagctg cgtttgggag gacttcccaa aaaaaaaagt attagccatt     660
agcacgtgat taattaagta ctagtttaaa aaacttaaaa aataaattaa tatgattctc     720
ttaagtaact ctcctataga aaacttttac aaaattacac cgtttaatag tttggaaaat     780
atgtcagtaa aaaataagag agtagaagtt atgaaagtta gaaaaagaat tgttttagta     840

gtatacagtt ataaactatt ccctctgttc taaaacataa gggattatgg atggattcga     900
catgtaccag taccatgaat cgaatccaga caagtttttt atgcatattt attctactat     960
aatatatcac atctgctcta aatatcttat atttcgaggt ggagactgtc gctatgtttt    1020
tctgcccgtt gctaagcaca cgccaccccc gatgcgggga cgcctctggc cttcttgcca    1080
cgataattga atggaacttc cacattcaga ttcgataggt gaccgtcgac tccaagtgct    1140
ttgcacaaaa caactccggc ctcccggcca ccagtcacac gactcacggc actaccaccc    1200
ctgactccct gaggcggacc tgccactgtt ctgcatgcga agctatctaa aattctgaag    1260
caaagaaagc acagcacatg ctccgggaca cgcgccaccc ggcggaaaag ggctcggtgt    1320
ggcgatctca cagccgcata tcgcatttca caagccgccc atctccaccg gcttcacgag    1380
gctcatcgcg gcacgaccgc gcacggaacg cacgcggccg acccgcgcgc ctcgatgcgc    1440
gagcccatcc gccgcgtcct ccctttgcct ttgccgctat cctctcggtc gtatcccgtt    1500
tctctgtctt ttgctccccg gcgcgcgcca gttcggagta ccagcgaaac ccggacacct    1560
ggtacacctc cgccggccac aacgcgtgtc cccctacgtg gccgcgcagc acatgcccat    1620
gcgcgacacg tgcacctcct catccaaact ctcaagtctc aacggtccta taaatgcacg    1680
gatagcctca agctgctcgt cacaaggcaa gaggcaagag gcaagagcat ccgtattaac    1740
cagccttttg agacttgaga gtgtgtgtga ctcgatccag cgtagtttca gttcgtgtgt    1800
tggtgagtga ttccagccaa gtttgcg                                        1827
```

<210> 56
<211> 2194
<212> DNA
<213> Oryza sativa

<400> 56

```
aatccgaaaa gtttctgcac cgttttcacc ccctaactaa caatataggg aacgtgtgct     60
aaatataaaa tgagacctta tatatgtagc gctgataact agaactatgc aagaaaaact    120
catccaccta ctttagtggc aatcgggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
aaaaaatctt tctagctgaa ctcaatgggt aaagagagag attttttttta aaaaaataga    360
atgaagatat tctgaacgta ttggcaaaga tttaaacata taattatata attttatagt    420
ttgtgcattc gtcatatcgc acatcattaa ggacatgtct tactccatcc caatttttat    480
ttagtaatta aagacaattg acttattttt attatttatc ttttttcgat tagatgcaag    540
gtacttacgc acacactttg tgctcatgtg catgtgtgag tgcacctcct caatacacgt    600
tcaactagca acacatctct aatatcactc gcctatttaa tacatttagg tagcaatatc    660
tgaattcaag cactccacca tcaccagacc acttttaata atatctaaaa tacaaaaaat    720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa cctttttaaca gcaggctttg cggccaggag agaggaggag aggcaaagaa    960
aaccaagcat cctccttctc ccatctataa attcctcccc ccttttcccc tctctatata   1020
ggaggcatcc aagccaagaa gagggagagc accaaggaca cgcgactagc agaagccgag   1080
cgaccgcctt ctcgatccat atcttccggt cgagttcttg gtcgatctct tccctcctcc   1140
acctcctcct cacagggtat gtgcctccct tcggttgttc ttggatttat tgttctaggt   1200
tgtgtagtac gggcgttgat gttaggaaag gggatctgta tctgtgatga ttcctgttct   1260
tggatttggg atagaggggt tcttgatgtt gcatgttatc ggttcggttt gattagtagt   1320
atggttttca atcgtctgga gagctctatg gaaatgaaat ggtttaggga tcggaatctt   1380
gcgattttgt gagtaccttt tgtttgaggt aaaatcagag caccggtgat tttgcttggt   1440
gtaataaagt acggttgttt ggtcctcgat tctggtagtg atgcttctcg atttgacgaa   1500
gctatccttt gtttattccc tattgaacaa aaataatcca actttgaaga cggtcccgtt   1560
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aattttttttt cccaaatatc ttaaaaagtc   1740
actttctggt tcagttcaat gaattgattg ctacaaataa tgcttttata gcgttatcct   1800
agctgtagtt cagttaatag gtaataccc tatagtttag tcaggagaag aacttatccg   1860
atttctgatc tccattttta attatatgaa atgaactgta gcataagcag tattcatttg   1920
gattattttt tttattagct ctcacccctt cattattctg agctgaaagt ctggcatgaa   1980
ctgtcctcaa ttttgttttc aaattcacat cgattatcta tgcattatcc tcttgtatct   2040
acctgtagaa gtttcttttt ggttattcct tgactgcttg attacagaaa gaaatttatg   2100

aagctgtaat cgggatagtt atactgcttg ttcttatgat tcatttcctt tgtgcagttc   2160
ttggtgtagc ttgccacttt caccagcaaa gttc                                2194
```

<210> 57
<211> 46
<212> PRT
<213> Artificial sequence

<220>
<223> SNH domain

<220>
<221> VARIANT
<222> (3)..(3)
<223> /replace = "Lys"

<220>
<221> UNSURE
<222> (4)..(4)
<223> Xaa can be any naturally occurring amino acid

<220>

<221> VARIANT
<222> (6)..(6)
<223> /replace = "Glu"

<220>
<221> VARIANT
<222> (7)..(7)
<223> /replace = "Asp"

<220>
<221> VARIANT
<222> (9)..(9)
<223> /replace = "Asn"

<220>
<221> UNSURE
<222> (10)..(10)
<223> Xaa can be any naturally occurring amino acid

<220>
<221> UNSURE
<222> (13)..(13)
<223> Xaa can be any naturally occurring amino acid

<220>
<221> VARIANT
<222> (14)..(14)
<223> /replace = "Ala" /replace = "Lys"

<220>
<221> VARIANT
<222> (16)..(16)
<223> /replace = "Val" /replace = "Met"

<220>
<221> VARIANT
<222> (17)..(17)
<223> /replace = "Asp" /replace = "Ser"

<220>
<221> VARIANT
<222> (18)..(18)
<223> /replace = "Asn"

<220>
<221> VARIANT
<222> (19)..(19)
<223> /replace = "Leu"

<220>
<221> UNSURE
<222> (21)..(21)
<223> Xaa can be any naturally occurring amino acid

<220>
<221> VARIANT
<222> (23)..(23)
<223> /replace = "Arg"

<220>
<221> UNSURE
<222> (24)..(25)
<223> Xaa can be any naturally occurring amino acid

<220>
<221> VARIANT
<222> (28)..(28)
<223> /replace = "Asp" /replace = "Ser"

<220>
<221> UNSURE
<222> (29)..(30)
<223> Xaa can be any naturally occurring amino acid

<220>
<221> VARIANT
<222> (32)..(32)
<223> /replace = "Ser" /replace = "Gln"

<220>
<221> UNSURE
<222> (33)..(33)
<223> Xaa can be any naturally occurring amino acid

<220>
<221> VARIANT
<222> (35)..(35)
<223> /replace = "His"

<220>
<221> UNSURE
<222> (36)..(36)
<223> Xaa can be any naturally occurring amino acid

<220>
<221> VARIANT
<222> (39)..(39)
<223> /replace = "Leu" /replace = "Val"

<220>
<221> VARIANT
<222> (43)..(43)
<223> /replace = "Thr"

<400> 57

```
Ile Gln Gln Xaa Leu Asp Glu Asn Lys Xaa Leu Ile Xaa Cys Ile Leu
1               5                   10                  15
Glu Ser Gln Asn Xaa Gly Lys Xaa Xaa Glu Cys Ala Xaa Xaa Gln Ala
            20                  25                  30
Xaa Leu Gln Xaa Asn Leu Met Tyr Leu Ala Ala Ile Ala Asp
        35                  40                  45
```

<210> 58
<211> 46
<212> PRT
<213> Arabidopsis thaliana

<400> 58

Ile Gln Gln Tyr Leu Asp Glu Asn Lys Ser Leu Ile Leu Lys Ile Val
1 5 10 15
Glu Ser Gln Asn Ser Gly Lys Leu Ser Glu Cys Ala Glu Asn Gln Ala
20 25 30
Arg Leu Gln Arg Asn Leu Met Tyr Leu Ala Ala Ile Ala Asp
35 40 45

<210> 59
<211> 633
<212> DNA
<213> Arabidopsis thaliana

<400> 59

atgcaacagc acctgatgca gatgcagccc atgatggctg gttactaccc cagcaatgtt 60
acctctgatc atatccaaca gtacttggac gaaaacaaat cgttgattct gaagattgtt 120
gagtctcaaa actctggaaa gcttagcgaa tgcgccgaga atcaagcaag gcttcaacgc 180
aacctaatgt acctagctgc aatagcagat tctcagcctc agccaccaag tgtgcatagc 240
cagtatggat ctgctggtgg tgggatgatt cagggagaag gagggtcaca ctatttgcag 300
cagcaacaag cgactcaaca gcaacagatg actcagcagt ctctaatggc ggctcgatct 360
tcaatgttgt atgctcagca acagcagcag cagcagcctt acgcgacgct tcagcatcag 420
caattgcacc atagccagct tggaatgagc tcgagcagcg gaggaggagg aagcagtggt 480
ctccatatcc ttcagggaga ggctggtggg tttcatgatt ttggccgtgg gaagccggaa 540
atgggaagtg gtggtggcgg tgaaggcaga ggaggaagtt caggggatgg tggagaaacc 600
ctttacttga aatcatcaga tgatgggaat tga 633

<210> 60
<211> 210
<212> PRT
<213> Arabidopsis thaliana

<400> 60

Met Gln Gln His Leu Met Gln Met Gln Pro Met Met Ala Gly Tyr Tyr
1 5 10 15
Pro Ser Asn Val Thr Ser Asp His Ile Gln Gln Tyr Leu Asp Glu Asn
20 25 30
Lys Ser Leu Ile Leu Lys Ile Val Glu Ser Gln Asn Ser Gly Lys Leu
35 40 45

```
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Ser Val His Ser
65                  70                  75                  80
Gln Tyr Gly Ser Ala Gly Gly Gly Met Ile Gln Gly Glu Gly Gly Ser
                85                  90                  95
His Tyr Leu Gln Gln Gln Gln Ala Thr Gln Gln Gln Gln Met Thr Gln
            100                 105                 110
Gln Ser Leu Met Ala Ala Arg Ser Ser Met Leu Tyr Ala Gln Gln Gln
        115                 120                 125
Gln Gln Gln Gln Pro Tyr Ala Thr Leu Gln His Gln Gln Leu His His
    130                 135                 140
Ser Gln Leu Gly Met Ser Ser Ser Ser Gly Gly Gly Gly Ser Ser Gly
145                 150                 155                 160
Leu His Ile Leu Gln Gly Glu Ala Gly Gly Phe His Asp Phe Gly Arg
                165                 170                 175
Gly Lys Pro Glu Met Gly Ser Gly Gly Gly Gly Glu Gly Arg Gly Gly
            180                 185                 190
Ser Ser Gly Asp Gly Gly Glu Thr Leu Tyr Leu Lys Ser Ser Asp Asp
        195                 200                 205
Gly Asn
    210
```

<210> 61
<211> 588
<212> DNA
<213> Arabidopsis thaliana

<400> 61

```
atgcagcagc agcagtctcc gcaaatgttt ccgatggttc cgtcgattcc ccctgctaac     60
aacatcacta ccgaacagat ccaaaagtac cttgatgaga acaagaagct gattatggcc    120
atcatggaaa accagaatct cggtaaactt gctgagtgcg cccagtacca agctcttctc    180
cagaagaact tgatgtatct tgctgcaatt gctgatgctc aaccccccacc acctacgcca    240
ggaccttcac catctacagc tgtcgctgcc cagatggcaa caccgcattc tgggatgcaa    300
ccacctagct acttcatgca acacccacaa gcatcccctg cagggatttt cgctccaagg    360
ggtcctttac agtttggtag cccactccag tttcaggatc cgcaacagca gcagcagata    420
catcagcaag ctatgcaagg acacatgggg attagaccaa tgggtatgac caacaacggg    480
atgcagcatg cgatgcaaca accagaaacc ggtcttggag gaaacgtggg gcttagagga    540
ggaaagcaag atggagcaga tggacaagga aaagatgatg gcaagtga                 588
```

<210> 62
<211> 195
<212> PRT
<213> Arabidopsis thaliana

<400> 62

```
Met Gln Gln Gln Gln Ser Pro Gln Met Phe Pro Met Val Pro Ser Ile
1               5                   10                  15
Pro Pro Ala Asn Asn Ile Thr Thr Glu Gln Ile Gln Lys Tyr Leu Asp
            20                  25                  30
Glu Asn Lys Lys Leu Ile Met Ala Ile Met Glu Asn Gln Asn Leu Gly
        35                  40                  45
Lys Leu Ala Glu Cys Ala Gln Tyr Gln Ala Leu Leu Gln Lys Asn Leu
    50                  55                  60
Met Tyr Leu Ala Ala Ile Ala Asp Ala Gln Pro Pro Pro Pro Thr Pro
65                  70                  75                      80
Gly Pro Ser Pro Ser Thr Ala Val Ala Ala Gln Met Ala Thr Pro His
                85                  90                      95
Ser Gly Met Gln Pro Pro Ser Tyr Phe Met Gln His Pro Gln Ala Ser

            100                 105                 110
Pro Ala Gly Ile Phe Ala Pro Arg Gly Pro Leu Gln Phe Gly Ser Pro
        115                 120                 125
Leu Gln Phe Gln Asp Pro Gln Gln Gln Gln Ile His Gln Gln Ala
    130                 135                 140
Met Gln Gly His Met Gly Ile Arg Pro Met Gly Met Thr Asn Asn Gly
145                 150                 155                     160
Met Gln His Ala Met Gln Gln Pro Glu Thr Gly Leu Gly Gly Asn Val
                165                 170                     175
Gly Leu Arg Gly Gly Lys Gln Asp Gly Ala Asp Gly Gln Gly Lys Asp
            180                 185                 190
Asp Gly Lys
        195
```

<210> 63
<211> 672
<212> DNA
<213> Arabidopsis thaliana

<400> 63

```
atgcagcaat ctccacagat gattccgatg gttcttcctt catttccgcc caccaataat      60
atcaccaccg aacagatcca aaagtatctt gatgagaaca agaagctgat aatggcgatc     120
ttggaaaatc agaacctcgg taaacttgca gaatgtgctc agtatcaagc tcttctccag     180
aagaatttga tgtatctcgc tgcaattgcg gatgctcaac ctcagccacc agcagctaca     240
ctaacatcag gagccatgac tccccaagca atggctccta atccgtcatc aatgcagcca     300
ccaccaagct acttcatgca gcaacatcaa gctgtgggaa tggctcaaca aatacctcct     360
gggattttcc ctcctagagg tccattgcaa tttggtagcc cgcatcagtt tctggatccg     420
cagcaacagt tacatcaaca agctatgcaa gggcacatgg ggattagacc aatgggtttg     480
aataataaca acggactgca acatcaaatg caccaccatg aaactgctct tgccgcaaac     540
aatgcgggtc ctaacgatgc tagtggagga ggtaaaccgg atgggaccaa tatgagccag     600
agtggagctg atgggcaagg tggctcagcc gctagacatg gcggtggtga tgcaaaaact     660
gaaggaaaat ga                                                         672
```

<210> 64
<211> 223
<212> PRT
<213> Arabidopsis thaliana

<400> 64

```
Met Gln Gln Ser Pro Gln Met Ile Pro Met Val Leu Pro Ser Phe Pro
1               5                   10                  15
Pro Thr Asn Asn Ile Thr Thr Glu Gln Ile Gln Lys Tyr Leu Asp Glu
            20                  25                  30
Asn Lys Lys Leu Ile Met Ala Ile Leu Glu Asn Gln Asn Leu Gly Lys
        35                  40                  45
Leu Ala Glu Cys Ala Gln Tyr Gln Ala Leu Leu Gln Lys Asn Leu Met
    50                  55                  60
Tyr Leu Ala Ala Ile Ala Asp Ala Gln Pro Gln Pro Pro Ala Ala Thr
65                  70                  75                  80
Leu Thr Ser Gly Ala Met Thr Pro Gln Ala Met Ala Pro Asn Pro Ser
                85                  90                  95
Ser Met Gln Pro Pro Pro Ser Tyr Phe Met Gln Gln His Gln Ala Val
            100                 105                 110
Gly Met Ala Gln Gln Ile Pro Pro Gly Ile Phe Pro Pro Arg Gly Pro
        115                 120                 125
Leu Gln Phe Gly Ser Pro His Gln Phe Leu Asp Pro Gln Gln Gln Leu
    130                 135                 140
His Gln Gln Ala Met Gln Gly His Met Gly Ile Arg Pro Met Gly Leu
145                 150                 155                 160

Asn Asn Asn Asn Gly Leu Gln His Gln Met His His His Glu Thr Ala
                165                 170                 175
Leu Ala Ala Asn Asn Ala Gly Pro Asn Asp Ala Ser Gly Gly Gly Lys
            180                 185                 190
Pro Asp Gly Thr Asn Met Ser Gln Ser Gly Ala Asp Gly Gln Gly Gly
        195                 200                 205
Ser Ala Ala Arg His Gly Gly Gly Asp Ala Lys Thr Glu Gly Lys
    210                 215                 220
```

<210> 65
<211> 633
<212> DNA
<213> Aspergillus officinalis

<400> 65

```
atgcagcagc acctgatgca gatgcagccc atgatggcaa cctacggttc accgaatcag     60
gtcaccaccg atatcattca gcagtatctg gacgagaaca agcagttgat tctggctatt    120
cttgaaaacc aaaattcagg aaaagctgat gaatgtgctg agaatcaggc taagcttcag    180
aggaatctga tgtatcttgc agccattgcg gatagccagc cccaagttcc taccattgct    240
cagtatcctc ccaacgctgt tgctgctatg caatcgagtg ctcgctacat gcaacaacac    300
caagcagctc aacagatgac ccctcaatct ctcatggctg ctcgctcctc aatgctctac    360
tcacagtccc caatgtctgc actccagcag caacagcagc aagcagcaat gcatagccag    420
ctcgccatga gctccggagg caacaacagc agcaccggag gattcaccat tcttcatggt    480
gaagctagca taggaggcaa tggctcaatg aattctggtg gagtctttgg agattttgga    540
cggagcagcg gtgggaagca agagactggg agcgaagggc acgggacaga gactcctatg    600
tacctgaaag gctctgaaga agaaggaaac tga                                  633
```

<210> 66
<211> 210
<212> PRT
<213> Aspergillus officinalis

<400> 66

```
Met Gln Gln His Leu Met Gln Met Gln Pro Met Met Ala Thr Tyr Gly
1               5                   10                  15
Ser Pro Asn Gln Val Thr Thr Asp Ile Ile Gln Gln Tyr Leu Asp Glu
            20                  25                  30
Asn Lys Gln Leu Ile Leu Ala Ile Leu Glu Asn Gln Asn Ser Gly Lys
        35                  40                  45
Ala Asp Glu Cys Ala Glu Asn Gln Ala Lys Leu Gln Arg Asn Leu Met
    50                  55                  60
Tyr Leu Ala Ala Ile Ala Asp Ser Gln Pro Gln Val Pro Thr Ile Ala
65                  70                  75                  80
Gln Tyr Pro Pro Asn Ala Val Ala Ala Met Gln Ser Ser Ala Arg Tyr
                85                  90                  95
Met Gln Gln His Gln Ala Ala Gln Gln Met Thr Pro Gln Ser Leu Met
            100                 105                 110
Ala Ala Arg Ser Ser Met Leu Tyr Ser Gln Ser Pro Met Ser Ala Leu
        115                 120                 125
Gln Gln Gln Gln Gln Gln Ala Ala Met His Ser Gln Leu Ala Met Ser
    130                 135                 140
Ser Gly Gly Asn Asn Ser Ser Thr Gly Gly Phe Thr Ile Leu His Gly
145                 150                 155                 160
Glu Ala Ser Ile Gly Gly Asn Gly Ser Met Asn Ser Gly Gly Val Phe
                165                 170                 175
Gly Asp Phe Gly Arg Ser Ser Gly Gly Lys Gln Glu Thr Gly Ser Glu
            180                 185                 190
Gly His Gly Thr Glu Thr Pro Met Tyr Leu Lys Gly Ser Glu Glu Glu
        195                 200                 205

Gly Asn
    210
```

<210> 67
<211> 591
<212> DNA
<213> Brassica napus

<400> 67

```
atgcagccca tgatggctgg ttactacccc agcaatgtca cctctgatca tatccagcag      60
tacttggatg agaacaagtc tttgattctg aagatagttg agtctcaaaa ctcaggaaag     120
ctcagcgagt gtgccgagaa tcaggcaagg cttcaacgca acctcatgta cttggctgca     180
atagcagatt ctcagcctca acctccaagc gtgcatagcc agtatggatc tgctggtggt     240
gggttgattc agggagaagg agcgtcacac tatttgcagc agcaacaggc gactcaacag     300
cagcagatga ctcagcagtc tcttatggca gctcgttctt caatgatgta tcagcagcag     360
caacagcctt atgcaacgct tcagcatcag cagttgcacc atagccagct tgggatgagc     420
tctagcagcg gaggaggaag cagtggtctc catatccttc agggagaggc tggtgggttt     480
catgaatttg gccgtgggaa gccggagatg ggaagtggtg aaggcagggg tggaagctca     540
ggggatggtg gagaaacact ctacttgaag tcatcagatg atgggaactg a              591
```

<210> 68
<211> 203
<212> PRT
<213> Brassica napus

<400> 68

```
Met Gln Gln His Leu Met Gln Met Gln Pro Met Met Ala Gly Tyr Tyr
1               5                   10                  15
Pro Ser Asn Val Thr Ser Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Ser Val His Ser
65                  70                  75                  80
Gln Tyr Gly Ser Ala Gly Gly Gly Leu Ile Gln Gly Glu Gly Ala Ser
                85                  90                  95
His Tyr Leu Gln Gln Gln Gln Ala Thr Gln Gln Gln Gln Met Thr Gln
            100                 105                 110
Gln Ser Leu Met Ala Ala Arg Ser Ser Met Met Tyr Gln Gln Gln Gln
        115                 120                 125
Gln Pro Tyr Ala Thr Leu Gln His Gln Gln Leu His His Ser Gln Leu
    130                 135                 140
Gly Met Ser Ser Ser Ser Gly Gly Gly Ser Ser Gly Leu His Ile Leu
145                 150                 155                 160
Gln Gly Glu Ala Gly Gly Phe His Glu Phe Gly Arg Gly Lys Pro Glu
                165                 170                 175
Met Gly Ser Gly Glu Gly Arg Gly Gly Ser Ser Gly Asp Gly Gly Glu
            180                 185                 190
Thr Leu Tyr Leu Lys Ser Ser Asp Asp Gly Asn
        195                 200
```

<210> 69
<211> 663
<212> DNA
<213> Citrus sinensis

<400> 69

```
atgcaacagc acctgatgca gatgcagccc atgatggcag cttattatcc caacaacgtc      60
actactgacc acattcaaca gtatctagat gagaacaaat cattgatttt gaagattgtt     120
gagagccaga attcagggaa actgagcgag tgtgcagaga accaggcaag attgcagcgg     180
aatctcatgt acctggctgc tattgctgat gctcaacccc aaccacctag cgttcatgcc     240
cagttctctt ctggtggcat tatgcagcca ggagctcact atatgcaaca ccagcaatct     300
cagccaatga caccacagtc acttatggct gcacgctcat ccatggtgta ctctcaacag     360
caattttcag tgcttcagca acagcaagcc ttgcatggtc agcttggcat gagctctggt     420
ggtagctcag gacttcacat gctgcaaagt gagggtagta ctgcaggagg tagtggttca     480
cttgggggtg ggggattccc tgattttggc cgtggctcat ctggtgaagg cttgcactca     540
aggggaatgg ggagcaagca tgatataggc agttctggat ctgctgaagg acgaggaggg     600
agctcaggaa gccaagatgg aggcgaaact ctctacttga aggggctga tgatggaaat      660
taa                                                                   663
```

<210> 70
<211> 219
<212> PRT
<213> Citrus sinensis

<400> 70

```
Met Gln Gln His Leu Met Gln Met Gln Pro Met Met Ala Ala Tyr Tyr
1               5                   10                  15
Pro Asn Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ala Gln Pro Gln Pro Pro Ser Val His Ala
65                  70                  75                  80
Gln Phe Ser Ser Gly Gly Ile Met Gln Pro Gly Ala His Tyr Met Gln
                85                  90                  95
His Gln Gln Ser Gln Pro Met Thr Pro Gln Ser Leu Met Ala Ala Arg
            100                 105                 110
Ser Ser Met Val Tyr Ser Gln Gln Gln Phe Ser Val Leu Gln Gln Gln
        115                 120                 125
Gln Ala Leu His Gly Gln Leu Gly Met Ser Ser Gly Gly Ser Ser Gly
    130                 135                 140
Leu His Met Leu Gln Ser Glu Gly Ser Thr Ala Gly Gly Ser Gly Ser
145                 150                 155                 160
Leu Gly Gly Gly Gly Phe Pro Asp Phe Gly Arg Gly Ser Ser Gly Glu
                165                 170                 175
Gly Leu His Ser Arg Gly Met Gly Ser Lys His Asp Ile Gly Ser Ser
            180                 185                 190
Gly Ser Ala Glu Gly Arg Gly Gly Ser Ser Gly Ser Gln Asp Gly Gly
        195                 200                 205
Glu Thr Leu Tyr Leu Lys Gly Ala Asp Asp Gly
    210                 215
```

<210> 71
<211> 660
<212> DNA
<213> Gossypium arboreum

<220>
<221> misc_feature
<222> (309)..(309)
<223> n is a, c, g, or t

<400> 71

```
atgcagcagc acctgatgca gatgcagccc atgatggcag cttattatcc caacaacgtc     60
actactgatc atattcaaca gtatctcgat gagaacaagt cattgatctt aaagattgtt    120
gagagccaga attctgggaa attgagtgaa tgtgctgaga accaagcaag gctgcagcga    180
aacctcatgt acctggctgc cattgcggat tctcaacccc aaccacccac cgtgcatgca    240
cagtttccat ctggtggtat catgcagcaa ggagctgggc actacatgca gcaccaacaa    300
gctcaacana tgacacaaca gtcgcttatg gctgctcggt cctcaatgtt gtattctcag    360
caaccatttt ctgcactgca acaacaacaa caacaaggct ttgcacagtc agcttggcat    420
gagctctggc gggagcacag gcctttcata tgctgcaaac tgaatctagt actgcagggg    480
gcagtgagac accttgggcc cgagggttgt cctgatttgg acgggggtct tttggagagg    540
catccctggt ggcaggccaa tggccggggg aacaaccaaa aatccgggga ggccggctca    600
cctaagggcc gggaggagcc cttggggcag ggggggtga tgggggaac ctcttcttaa      660
```

<210> 72
<211> 219
<212> PRT
<213> Gossypium arboreum

<220>

<221> UNSURE
<222> (103)..(103)
<223> Xaa can be any naturally occurring amino acid

<400> 72

```
Met Gln Gln His Leu Met Gln Met Gln Pro Met Met Ala Ala Tyr Tyr
1               5                   10                  15
Pro Asn Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Thr Val His Ala
65                  70                  75                  80
Gln Phe Pro Ser Gly Gly Ile Met Gln Gln Gly Ala Gly His Tyr Met
                85                  90                  95
Gln His Gln Gln Ala Gln Xaa Met Thr Gln Gln Ser Leu Met Ala Ala
            100                 105                 110
Arg Ser Ser Met Leu Tyr Ser Gln Gln Pro Phe Ser Ala Leu Gln Gln
        115                 120                 125
Gln Gln Gln Gln Gly Phe Ala Gln Ser Ala Trp His Glu Leu Trp Arg
    130                 135                 140
Glu His Arg Pro Phe Ile Cys Cys Lys Leu Asn Leu Val Leu Gln Gly
145                 150                 155                 160
Ala Val Arg His Leu Gly Pro Glu Gly Cys Pro Asp Leu Asp Gly Gly
                165                 170                 175
Leu Leu Glu Arg His Pro Trp Trp Gln Ala Asn Gly Arg Gly Asn Asn
            180                 185                 190
Gln Lys Ser Gly Glu Ala Gly Ser Pro Lys Gly Arg Glu Glu Pro Leu
        195                 200                 205
Gly Gln Gly Gly Val Met Gly Gly Thr Ser Ser
    210                 215
```

<210> 73
<211> 636
<212> DNA
<213> Medicago trunculata

<400> 73

```
atgcagcagc acctgatgca gatgcagccc atgatggcag cttactatcc taacaacgtc     60

actactgatc atattcaaca gtatcttgat gagaacaagt ccttgattct caagattgtt    120
gaaagccaga acactggcaa gctcaccgag tgtgctgaga accaatcaag gcttcagaga    180
aatctcatgt acctagctgc aatagctgat tctcaacccc aaccacctac tatgcctggc    240
cagtaccctt caagtggaat gatgcagcag ggaggacact acatgcaggc tcaacaagct    300
cagcagatga cacaacaaca attaatggct gcacgttcct ctcttatgta tgctcaacag    360
cttcaacagc agcaagcctt gcaaagccaa cttggtatga attccagtgg aagtcaaggc    420
cttcacatgt tgcatagtga aggggctaat gttggaggca attcatctct aggggctggt    480
tttcctgatt ttggccgtag ctcagccggt gatggtttgc acggcagtgg taagcaagac    540
attggaagca ctgatggccg cggtggaagc tctagtggtc actctggtga tggcggcgaa    600
acactttacc tgaaatcttc tggtgatggg aattag                              636
```

<210> 74
<211> 211
<212> PRT

<213> Medicago truncatula

<400> 74

```
Met Gln Gln His Leu Met Gln Met Gln Pro Met Met Ala Ala Tyr Tyr
1               5                   10                  15
Pro Asn Asn Val Thr Thr Asp His Ile Gln Gln Tyr Leu Asp Glu Asn
            20                  25                  30
Lys Ser Leu Ile Leu Lys Ile Val Glu Ser Gln Asn Thr Gly Lys Leu
        35                  40                  45
Thr Glu Cys Ala Glu Asn Gln Ser Arg Leu Gln Arg Asn Leu Met Tyr
    50                  55                  60
Leu Ala Ala Ile Ala Asp Ser Gln Pro Gln Pro Pro Thr Met Pro Gly
65                  70                  75                  80
Gln Tyr Pro Ser Ser Gly Met Met Gln Gln Gly Gly His Tyr Met Gln
                85                  90                  95
Ala Gln Gln Ala Gln Gln Met Thr Gln Gln Gln Leu Met Ala Ala Arg
            100                 105                 110
Ser Ser Leu Met Tyr Ala Gln Gln Leu Gln Gln Gln Gln Ala Leu Gln
        115                 120                 125
Ser Gln Leu Gly Met Asn Ser Ser Gly Ser Gln Gly Leu His Met Leu
    130                 135                 140
His Ser Glu Gly Ala Asn Val Gly Gly Asn Ser Ser Leu Gly Ala Gly
145                 150                 155                 160
Phe Pro Asp Phe Gly Arg Ser Ser Ala Gly Asp Gly Leu His Gly Ser
                165                 170                 175
Gly Lys Gln Asp Ile Gly Ser Thr Asp Gly Arg Gly Gly Ser Ser Ser
            180                 185                 190
Gly His Ser Gly Asp Gly Gly Glu Thr Leu Tyr Leu Lys Ser Ser Gly
        195                 200                 205
Asp Gly Asn
    210
```

<210> 75
<211> 684
<212> DNA
<213> Oryza sativa

<400> 75

```
atgcagcagc aacacctgat gcagatgaac cagggcatga tgggggata tgcttcccct      60
accaccgtca ccactgatct cattcagcag tatctggatg agaacaagca gctgatcctg    120
gccatccttg acaaccagaa caatgggaag gtggaagagt gcgctcggaa ccaagctaag    180
ctccagcaca atctcatgta cctcgccgcc atcgccgaca gccagccgcc gcagacggcc    240
gccatgtccc agtatccgtc gaacctgatg atgcagtccg ggcgaggta catgccgcag    300
cagtcggcgc agatgatggc gccgcagtcg ctgatggcgg cgaggtcttc gatgatgtac    360
gcgcagccgg cgctgtcgcc gctccagcag cagcagcagc agcaggcggc ggcggcgcac    420

gggcagctgg gcatgggctc gggggcacc accagcgggt tcagcatcct ccacggcgag    480
gccagcatgg gcggcggcgg cggcggcggt ggcgccggta acagcatgat gaacgccggc    540
gtgttctccg acttcggacg cggcggcggc ggcggcggca aggaggggtc cacctcgctg    600
tccgtcgacg tccggggcgc caactccggc gcccagagcg gcgacgggga gtacctcaag    660
ggcaccgagg aggaaggcag ctag                                           684
```

<210> 76
<211> 227
<212> PRT
<213> Oryza sativa

<400> 76

```
Met Gln Gln Gln His Leu Met Gln Met Asn Gln Gly Met Met Gly Gly
1               5                   10                  15
Tyr Ala Ser Pro Thr Thr Val Thr Thr Asp Leu Ile Gln Gln Tyr Leu
            20                  25                  30
Asp Glu Asn Lys Gln Leu Ile Leu Ala Ile Leu Asp Asn Gln Asn Asn
        35                  40                  45
Gly Lys Val Glu Glu Cys Ala Arg Asn Gln Ala Lys Leu Gln His Asn
    50                  55                  60
Leu Met Tyr Leu Ala Ala Ile Ala Asp Ser Gln Pro Pro Gln Thr Ala
65                  70                  75                  80
Ala Met Ser Gln Tyr Pro Ser Asn Leu Met Met Gln Ser Gly Ala Arg
                85                  90                  95
Tyr Met Pro Gln Gln Ser Ala Gln Met Met Ala Pro Gln Ser Leu Met
            100                 105                 110
Ala Ala Arg Ser Ser Met Met Tyr Ala Gln Pro Ala Leu Ser Pro Leu
        115                 120                 125
Gln Gln Gln Gln Gln Gln Gln Ala Ala Ala Ala His Gly Gln Leu Gly
    130                 135                 140
Met Gly Ser Gly Gly Thr Thr Ser Gly Phe Ser Ile Leu His Gly Glu
145                 150                 155                 160
Ala Ser Met Gly Gly Gly Gly Gly Gly Gly Gly Ala Gly Asn Ser Met
                165                 170                 175
Met Asn Ala Gly Val Phe Ser Asp Phe Gly Arg Gly Gly Gly Gly Gly
            180                 185                 190
Gly Lys Glu Gly Ser Thr Ser Leu Ser Val Asp Val Arg Gly Ala Asn
        195                 200                 205
Ser Gly Ala Gln Ser Gly Asp Gly Glu Tyr Leu Lys Gly Thr Glu Glu
    210                 215                 220
Glu Gly Ser
225
```

<210> 77
<211> 558
<212> DNA
<213> Oryza sativa

<400> 77

```
atgcagcagc agccgatgcc gatgcccgcg caggcgccgc cgacggccgg aatcaccacc      60
gagcagatcc aaaagtatct ggatgaaaac aagcagctta ttttggctat tttggaaaat     120
cagaatctgg gaaagttggc agaatgtgct cagtatcaag cgcagcttca gaagaatctc     180
ttgtacttgg ctgcaattgc tgatactcaa ccgcagacca ctataagccg tccccagatg     240
gtgccgcatg gtgcatcgcc ggggttaggg gggcaataca tgtcgcaggt gccaatgttc     300
ccccccagga cccctctaac gccccagcag atgcaggagc agcagctgca gcaacagcaa     360
gcccagctgc tctcgttcgg cggtcagatg gttatgaggc ctggcgttgt gaatggcatt     420
cctcagcttc tgcaaggcga aatgcaccgc ggagcagatc accagaacgc tggcggggcc     480
acctcggagc cttccgagag ccacaggagc accggcaccg aaaatgacgg tggaagcgac     540
ttcggcgatc aatcctaa                                                   558
```

<210> 78
<211> 185
<212> PRT
<213> Oryza sativa

<400> 78

```
Met Gln Gln Gln Pro Met Pro Met Pro Ala Gln Ala Pro Pro Thr Ala
1               5                   10                  15
Gly Ile Thr Thr Glu Gln Ile Gln Lys Tyr Leu Asp Glu Asn Lys Gln
            20                  25                  30
Leu Ile Leu Ala Ile Leu Glu Asn Gln Asn Leu Gly Lys Leu Ala Glu
        35                  40                  45
Cys Ala Gln Tyr Gln Ala Gln Leu Gln Lys Asn Leu Leu Tyr Leu Ala
    50                  55                  60
Ala Ile Ala Asp Thr Gln Pro Gln Thr Thr Ile Ser Arg Pro Gln Met
65                  70                  75                  80
Val Pro His Gly Ala Ser Pro Gly Leu Gly Gly Gln Tyr Met Ser Gln
                85                  90                  95
Val Pro Met Phe Pro Pro Arg Thr Pro Leu Thr Pro Gln Gln Met Gln
            100                 105                 110
Glu Gln Gln Leu Gln Gln Gln Gln Ala Gln Leu Leu Ser Phe Gly Gly
        115                 120                 125
Gln Met Val Met Arg Pro Gly Val Val Asn Gly Ile Pro Gln Leu Leu
    130                 135                 140
Gln Gly Glu Met His Arg Gly Ala Asp His Gln Asn Ala Gly Gly Ala
145                 150                 155                 160
Thr Ser Glu Pro Ser Glu Ser His Arg Ser Thr Gly Thr Glu Asn Asp
                165                 170                 175
Gly Gly Ser Asp Phe Gly Asp Gln Ser
            180                 185
```

<210> 79
<211> 618
<212> DNA
<213> Oryza sativa

<400> 79

```
atgcagcagc agatggccat gccggcgggg gccgccgccg ccgcggtgcc gccggcggcc     60
ggcatcacca ccgagcagat ccaaaagtat ttggatgaaa ataaacagct aattttggcc    120
atcctggaaa atcaaaacct agggaagttg gctgaatgtg ctcagtacca agctcagctt    180
caaaagaatc tcttgtatct ggctgccatt gcagatgccc aaccacctca gaatccagga    240
agtcgccctc agatgatgca gcctggtgct accccaggtg ctgggcatta catgtcccaa    300
gtaccgatgt tccctccaag aactccctta accccacaac agatgcaaga gcagcagcag    360
cagcaactcc agcaacagca agctcaggct ctagccttcc ccggccagat gctaatgaga    420
ccaggtactg tcaatggcat gcaatctatc ccagttgctg accctgctcg cgcagccgat    480
cttcagacgg cagcaccggg ctcggtagat ggccgaggaa acaagcagga tgcaacctcg    540
gagccttccg ggaccgagag ccacaagagt gcgggagcag ataacgacgc aggcggtgac    600
atagcggaga agtcctga                                                  618
```

<210> 80
<211> 205
<212> PRT
<213> Oryza sativa

<400> 80

```
Met Gln Gln Gln Met Ala Met Pro Ala Gly Ala Ala Ala Ala Ala Val
1               5                   10                  15
Pro Pro Ala Ala Gly Ile Thr Thr Glu Gln Ile Gln Lys Tyr Leu Asp
```

```
                20                  25                  30
Glu Asn Lys Gln Leu Ile Leu Ala Ile Leu Glu Asn Gln Asn Leu Gly
            35                  40                  45
Lys Leu Ala Glu Cys Ala Gln Tyr Gln Ala Gln Leu Gln Lys Asn Leu
        50                  55                  60
Leu Tyr Leu Ala Ala Ile Ala Asp Ala Gln Pro Pro Gln Asn Pro Gly
65                  70                  75                  80
Ser Arg Pro Gln Met Met Gln Pro Gly Ala Thr Pro Gly Ala Gly His
                85                  90                  95
Tyr Met Ser Gln Val Pro Met Phe Pro Pro Arg Thr Pro Leu Thr Pro
            100                 105                 110
Gln Gln Met Gln Glu Gln Gln Gln Gln Gln Leu Gln Gln Gln Gln Ala
        115                 120                 125
Gln Ala Leu Ala Phe Pro Gly Gln Met Leu Met Arg Pro Gly Thr Val
    130                 135                 140
Asn Gly Met Gln Ser Ile Pro Val Ala Asp Pro Ala Arg Ala Ala Asp
145                 150                 155                 160
Leu Gln Thr Ala Ala Pro Gly Ser Val Asp Gly Arg Gly Asn Lys Gln
                165                 170                 175
Asp Ala Thr Ser Glu Pro Ser Gly Thr Glu Ser His Lys Ser Ala Gly
            180                 185                 190
Ala Asp Asn Asp Ala Gly Gly Asp Ile Ala Glu Lys Ser
        195                 200                 205
```

<210> 81
<211> 540
<212> DNA
<213> Solanum tuberosum

<400> 81

```
atgcagcagc agcacctgat gcagatgcag cccatgatgg cagcctatta tcccaacaat     60
gtcactactg atcatattca acagttcctg gatgagaaca aatcacttat tctgaagatt    120
gttgagagcc agaactctgg gaaaataagt gaatgtgcag agtcccaagc taaacttcag    180
agaaatctta tgtaccttgc agctattgct gattcacagc cccagcctcc tagtatgcat    240
tcacagttag cttctggtgg gatgatgcag ggagggggcac attatatgca gcaacaacaa    300
gctcaacaac tcacaacgca atcgcttatg gctgcagcaa gatcctcctc ctcaatgctc    360
tatggacaac aacaacaaca acaacaacaa caactatcat cattgcaaca acagcaagca    420
gcctttcata gccagcaact cggaatgagc agctctggtg gaggaagcag tagtggactt    480
cacatgctac aaagcgaaaa cactcatagt gctagcactg gtggtgggtg gtttccctga    540
```

<210> 82
<211> 179
<212> PRT
<213> Solanum tuberosum

<400> 82

```
Met Gln Gln Gln His Leu Met Gln Met Gln Pro Met Met Ala Ala Tyr
1               5                   10                  15
Tyr Pro Asn Asn Val Thr Thr Asp His Ile Gln Gln Phe Leu Asp Glu
            20                  25                  30
Asn Lys Ser Leu Ile Leu Lys Ile Val Glu Ser Gln Asn Ser Gly Lys
        35                  40                  45
Ile Ser Glu Cys Ala Glu Ser Gln Ala Lys Leu Gln Arg Asn Leu Met
    50                  55                  60
Tyr Leu Ala Ala Ile Ala Asp Ser Gln Pro Gln Pro Pro Ser Met His
65                  70                  75                  80
Ser Gln Leu Ala Ser Gly Gly Met Met Gln Gly Gly Ala His Tyr Met
                85                  90                  95
Gln Gln Gln Gln Ala Gln Gln Leu Thr Thr Gln Ser Leu Met Ala Ala

            100                 105                 110
Ala Arg Ser Ser Ser Ser Met Leu Tyr Gly Gln Gln Gln Gln Gln Gln
        115                 120                 125
Gln Gln Gln Leu Ser Ser Leu Gln Gln Gln Gln Ala Ala Phe His Ser
    130                 135                 140
Gln Gln Leu Gly Met Ser Ser Ser Gly Gly Gly Ser Ser Ser Gly Leu
145                 150                 155                 160
His Met Leu Gln Ser Glu Asn Thr His Ser Ala Ser Thr Gly Gly Gly
                165                 170                 175
Trp Phe Pro
```

<210> 83
<211> 684
<212> DNA
<213> Zea mays

<400> 83

```
atgcagcagc aacacctgat gcagatgaac cagaacatga tgggggcta cacctctcct      60
gccgccgtga ccaccgatct catccagcag cacctggacg agaacaagca gctgatcctg    120
gccatcctcg acaaccagaa caatggcaag gcggaggagt gcgaacggca ccaagctaag    180
ctccagcaca acctcatgta cctggccgcc atcgctgaca gccagccgcc acagaccgcg    240
ccactatcac agtacccgtc caacctgatg atgcagccgg gccctcggta catgccaccg    300
cagtccgggc agatgatgaa cccgcagtcg ctgatggcgg cgcggtcctc catgatgtac    360
gcgcacccgt ccctgtcgcc actccagcag cagcaggcgg cgcacggaca gctgggtatg    420
gctccagggg gcggcggtgg cggcacgacc agcgggttca gcatcctcca cggcgaggcc    480
agcatgggcg gtggtggtgc tggcgcaggc gccggcaaca acatgatgaa cgccggcatg    540
ttctcgggct ttggccgcag cggcagtggc gccaaggaag ggtcgacctc tctgtcggtt    600
gacgtccggg gtggaaccag ctccggcgcg cagagcgggg acggcgagta cctcaaagtc    660
ggcaccgagg aagaaggcag ttag                                            684
```

<210> 84
<211> 227
<212> PRT
<213> Zea mays

<400> 84

```
Met Gln Gln Gln His Leu Met Gln Met Asn Gln Asn Met Met Gly Gly
1               5                   10                  15
Tyr Thr Ser Pro Ala Ala Val Thr Thr Asp Leu Ile Gln Gln His Leu
            20                  25                  30
Asp Glu Asn Lys Gln Leu Ile Leu Ala Ile Leu Asp Asn Gln Asn Asn
        35                  40                  45
Gly Lys Ala Glu Glu Cys Glu Arg His Gln Ala Lys Leu Gln His Asn
    50                  55                  60
Leu Met Tyr Leu Ala Ala Ile Ala Asp Ser Gln Pro Pro Gln Thr Ala
65                  70                  75                  80
Pro Leu Ser Gln Tyr Pro Ser Asn Leu Met Met Gln Pro Gly Pro Arg
                85                  90                  95
Tyr Met Pro Pro Gln Ser Gly Gln Met Met Asn Pro Gln Ser Leu Met
            100                 105                 110
Ala Ala Arg Ser Ser Met Met Tyr Ala His Pro Ser Leu Ser Pro Leu
        115                 120                 125
Gln Gln Gln Gln Ala Ala His Gly Gln Leu Gly Met Ala Pro Gly Gly
    130                 135                 140
Gly Gly Gly Gly Thr Thr Ser Gly Phe Ser Ile Leu His Gly Glu Ala
145                 150                 155                 160
Ser Met Gly Gly Gly Gly Ala Gly Ala Gly Ala Gly Asn Asn Met Met
                165                 170                 175

Asn Ala Gly Met Phe Ser Gly Phe Gly Arg Ser Gly Ser Gly Ala Lys
            180                 185                 190
Glu Gly Ser Thr Ser Leu Ser Val Asp Val Arg Gly Gly Thr Ser Ser
        195                 200                 205
Gly Ala Gln Ser Gly Asp Gly Glu Tyr Leu Lys Val Gly Thr Glu Glu
    210                 215                 220
Glu Gly Ser
225
```

<210> 85
<211> 549
<212> DNA
<213> Zea mays

<400> 85

```
atgcagcagc cgatgcacat gcagccacag gcgccggcga taaccccagc tgccggaatc      60
agcacggagc agatccaaaa gtatctggat gagaataagc agcttatttt ggctatttttg    120
gaaaatcaga acctaggaaa attggcagaa tgtgctcagt atcaatcaca acttcagaag     180
aacctcttgt atctcgctgc aatcgcagat gctcaaccgc agactgctgt aagccgccct     240
cagatggcgc cgcctggtgg atcgcctgga gtagggcagt acatgtcaca ggtgcctatg     300
ttcccaccga ggacacctct tacaccccag cagatgcagg agcagcagct tcagcagcag     360
caggctcagt tgctaaactt cagtggccaa atggttgcta gaccaggcat ggtcaacggc     420
atggctcagt ccatgcaagc tcagctacca ccgggtgtga acaagcagga tgctggtggg     480
gtcgcctctg agccctcggg caccgagagc cacaggagca ctggtggtga cgatggtgga     540
agcgactag                                                             549
```

<210> 86
<211> 182
<212> PRT
<213> Zea mays

<400> 86

```
Met Gln Gln Pro Met His Met Gln Pro Gln Ala Pro Ala Ile Thr Pro
1               5                   10                  15
Ala Ala Gly Ile Ser Thr Glu Gln Ile Gln Lys Tyr Leu Asp Glu Asn
            20                  25                  30
Lys Gln Leu Ile Leu Ala Ile Leu Glu Asn Gln Asn Leu Gly Lys Leu
        35                  40                  45
Ala Glu Cys Ala Gln Tyr Gln Ser Gln Leu Gln Lys Asn Leu Leu Tyr
    50                  55                  60
Leu Ala Ala Ile Ala Asp Ala Gln Pro Gln Thr Ala Val Ser Arg Pro
65                  70                  75                  80
Gln Met Ala Pro Pro Gly Gly Ser Pro Gly Val Gly Gln Tyr Met Ser
                85                  90                  95
Gln Val Pro Met Phe Pro Pro Arg Thr Pro Leu Thr Pro Gln Gln Met
            100                 105                 110
Gln Glu Gln Gln Leu Gln Gln Gln Gln Ala Gln Leu Leu Asn Phe Ser
        115                 120                 125
Gly Gln Met Val Ala Arg Pro Gly Met Val Asn Gly Met Ala Gln Ser
    130                 135                 140
Met Gln Ala Gln Leu Pro Pro Gly Val Asn Lys Gln Asp Ala Gly Gly
145                 150                 155                 160
Val Ala Ser Glu Pro Ser Gly Thr Glu Ser His Arg Ser Thr Gly Gly
                165                 170                 175
Asp Asp Gly Gly Ser Asp
            180
```

<210> 87
<211> 1173
<212> DNA
<213> Homo sapiens

<400> 87

```
atgggcggca acatgtctgt ggctttcgcg gccccgaggc agcgaggcaa gggggagatc      60
actcccgctg cgattcagaa gatgttggat gacaataacc atcttattca gtgtataatg     120
gactctcaga ataaaggaaa gacctcagag tgttctcagt atcagcagat gttgcacaca     180
aacttggtat accttgctac aatagcagat tctaatcaaa atatgcagtc tcttttacca     240
gcaccaccca cacagaatat gcctatgggt cctggaggga tgaatcagag cggccctccc     300
ccacctccac gctctcacaa catgccttca gatggaatgg taggtggggg tcctcctgca     360
ccgcacatgc agaaccagat gaacggccag atgcctgggc ctaaccatat gcctatgcag     420
ggacctggac ccaatcaact caatatgaca aacagttcca tgaatatgcc ttcaagtagc     480
catggatcca tgggaggtta caaccattct gtgccatcat cacagagcat gccagtacag     540
aatcagatga caatgagtca gggacaacca atgggaaact atggtcccag accaaatatg     600
agtatgcagc caaaccaagg tccaatgatg catcagcagc ctccttctca gcaatacaat     660
atgccacagg gaggcggaca gcattaccaa ggacagcagc cacctatggg aatgatgggt     720
caagttaacc aaggcaatca tatgatgggt cagagacaga ttcctcccta tagacctcct     780
caacagggcc caccacagca gtactcaggc caggaagact attacgggga ccaatacagt     840
catggtggac aaggtcctcc agaaggcatg aaccagcaat attaccctga tggaaattca     900
cagtatggcc aacagcaaga tgcataccag ggaccacctc cacaacaggg atatccaccc     960
cagcagcagc agtacccagg gcagcaaggt tacccaggac agcagcaggg ctacggtcct    1020
tcacagggtg gtccaggtcc tcagtatcct aactacccac agggacaagg tcagcagtat    1080
ggaggatata gaccaacaca gcctggacca ccacagccac cccagcagag gccttatgga    1140
tatgaccagg gacagtatgg aaattaccag cag                                 1173
```

<210> 88
<211> 391
<212> PRT
<213> Homo sapiens

<400> 88

```
Met Gly Gly Asn Met Ser Val Ala Phe Ala Ala Pro Arg Gln Arg Gly
1               5                   10                  15
Lys Gly Glu Ile Thr Pro Ala Ala Ile Gln Lys Met Leu Asp Asp Asn
            20                  25                  30
Asn His Leu Ile Gln Cys Ile Met Asp Ser Gln Asn Lys Gly Lys Thr
        35                  40                  45
Ser Glu Cys Ser Gln Tyr Gln Gln Met Leu His Thr Asn Leu Val Tyr
    50                  55                  60
Leu Ala Thr Ile Ala Asp Ser Asn Gln Asn Met Gln Ser Leu Leu Pro
65                  70                  75                  80
Ala Pro Pro Thr Gln Asn Met Pro Met Gly Pro Gly Gly Met Asn Gln
                85                  90                  95
Ser Gly Pro Pro Pro Pro Pro Arg Ser His Asn Met Pro Ser Asp Gly
            100                 105                 110
Met Val Gly Gly Gly Pro Pro Ala Pro His Met Gln Asn Gln Met Asn
        115                 120                 125
Gly Gln Met Pro Gly Pro Asn His Met Pro Met Gln Gly Pro Gly Pro
    130                 135                 140
Asn Gln Leu Asn Met Thr Asn Ser Ser Met Asn Met Pro Ser Ser Ser
145                 150                 155                 160
His Gly Ser Met Gly Gly Tyr Asn His Ser Val Pro Ser Ser Gln Ser
                165                 170                 175
Met Pro Val Gln Asn Gln Met Thr Met Ser Gln Gly Gln Pro Met Gly
            180                 185                 190
Asn Tyr Gly Pro Arg Pro Asn Met Ser Met Gln Pro Asn Gln Gly Pro
        195                 200                 205
Met Met His Gln Gln Pro Pro Ser Gln Gln Tyr Asn Met Pro Gln Gly
    210                 215                 220

Gly Gly Gln His Tyr Gln Gly Gln Gln Pro Pro Met Gly Met Met Gly
225                 230                 235                 240
Gln Val Asn Gln Gly Asn His Met Met Gly Gln Arg Gln Ile Pro Pro
                245                 250                 255
Tyr Arg Pro Pro Gln Gln Gly Pro Pro Gln Gln Tyr Ser Gly Gln Glu
            260                 265                 270
Asp Tyr Tyr Gly Asp Gln Tyr Ser His Gly Gly Gln Gly Pro Pro Glu
        275                 280                 285
Gly Met Asn Gln Gln Tyr Tyr Pro Asp Gly Asn Ser Gln Tyr Gly Gln
    290                 295                 300
Gln Gln Asp Ala Tyr Gln Gly Pro Pro Pro Gln Gln Gly Tyr Pro Pro
305                 310                 315                 320
Gln Gln Gln Gln Tyr Pro Gly Gln Gln Gly Tyr Pro Gly Gln Gln Gln
                325                 330                 335
Gly Tyr Gly Pro Ser Gln Gly Gly Pro Gly Pro Gln Tyr Pro Asn Tyr
            340                 345                 350
Pro Gln Gly Gln Gly Gln Gln Tyr Gly Gly Tyr Arg Pro Thr Gln Pro
        355                 360                 365
Gly Pro Pro Gln Pro Pro Gln Gln Arg Pro Tyr Gly Tyr Asp Gln Gly
    370                 375                 380
Gln Tyr Gly Asn Tyr Gln Gln
385                 390
```

<210> 89
<211> 627
<212> DNA
<213> Allium cepa

<400> 89

```
atgcagcagc cgcagccagc gatgggaacc atgggctcgg tgccacctac tagcatcacc      60
accgaacaga ttcaaaggta cttggatgag aacaaacagt taatattggc aattttggat     120
aatcaaaatt taggaagact gaatgagtgt gctcaatatc aagctcagct tcaaaagaat     180
ctgctttacc tggcagcaat agctgatgct cagcctcagt ctcctgcggt gcgtctgcag     240
atgatgcctc aaggtgcagc tgccacgcct caagctggaa accaatttat gcagcagcag     300
agccctaatt tccctcccaa aacaggaatg caatttactc ctcaacaagt acaagaattg     360
cagcagcaac agctacaaca tcagccacat atgatgcctc catttcaagg tcaaatgggt     420
atgagaccta tgaatggaat gcaggcagca atgcatgcag attcatctct tgcttataac     480
actaacaata agcaagatgc aggaaacgca gcttatgaaa atactgctgc caacacagat     540
ggttccattc aaaagaaaac agcaaatgat gatttagacc cttctgcagc aaaccctaga     600
aggtctgaag atgccaaatc atcatga                                         627
```

<210> 90
<211> 208
<212> PRT
<213> Allium cepa

<400> 90

```
Met Gln Gln Pro Gln Pro Ala Met Gly Thr Met Gly Ser Val Pro Pro
1               5                   10                  15
Thr Ser Ile Thr Thr Glu Gln Ile Gln Arg Tyr Leu Asp Glu Asn Lys
            20                  25                  30
Gln Leu Ile Leu Ala Ile Leu Asp Asn Gln Asn Leu Gly Arg Leu Asn
        35                  40                  45
Glu Cys Ala Gln Tyr Gln Ala Gln Leu Gln Lys Asn Leu Leu Tyr Leu
    50                  55                  60
Ala Ala Ile Ala Asp Ala Gln Pro Gln Ser Pro Ala Val Arg Leu Gln
65                  70                  75                  80
Met Met Pro Gln Gly Ala Ala Ala Thr Pro Gln Ala Gly Asn Gln Phe
                85                  90                  95

Met Gln Gln Gln Ser Pro Asn Phe Pro Pro Lys Thr Gly Met Gln Phe
            100                 105                 110
Thr Pro Gln Gln Val Gln Glu Leu Gln Gln Gln Gln Leu Gln His Gln
        115                 120                 125
Pro His Met Met Pro Pro Phe Gln Gly Gln Met Gly Met Arg Pro Met
    130                 135                 140
Asn Gly Met Gln Ala Ala Met His Ala Asp Ser Ser Leu Ala Tyr Asn
145                 150                 155                 160
Thr Asn Asn Lys Gln Asp Ala Gly Asn Ala Ala Tyr Glu Asn Thr Ala
                165                 170                 175
Ala Asn Thr Asp Gly Ser Ile Gln Lys Lys Thr Ala Asn Asp Asp Leu
            180                 185                 190
Asp Pro Ser Ala Ala Asn Pro Arg Arg Ser Glu Asp Ala Lys Ser Ser
        195                 200                 205
```

<210> 91
<211> 633
<212> DNA
<213> Aquilegia formosa x Aquilegia pubescens

<400> 91

```
atgcaacaca tgcagatgca gcccatgatg ccaccttata gtgccaacag cgtcactact      60
gatcatatcc aacagtactt ggatgaaaat aaggcgttga ttctgaagat acttgagaac     120
caaaattcgg gaaaagttag tgaatgtgca gagaaccaag caagacttca acgaaatctt     180
atgtatctgg ctgcaattgc tgattctcaa ccacagcctc ccaatatgca tgctcagtac     240
tctaatgcgg gtataccacc tggtgcacat tacctacaac accaacaggc ccaacagatg     300
acacaacagt cgctcatggc tgctcgatca aatatgctgt atgctcagcc aatcacagga     360
atgcagcaac agcaagcaat gcatagccag cttggcatga gctctggtgg taacagtgga     420
ctccacatga tgcacaatga gggcagcatg ggaggtagtg ggcacttgg aagctattct     480
gattatggcc gtggcagtgg tggtggagta actatcgcta gcaaacaaga tggtggaagt     540
ggttctggtg aaggacgagg tggaaactct ggaggccaaa gtgcagatgg aggtgaatct     600
ctttacctga aaaacagtga cgaagggaac taa                                  633
```

<210> 92
<211> 210
<212> PRT
<213> Aquilegia formosa x Aquilegia pubescens

<400> 92

```
Met Gln His Met Gln Met Gln Pro Met Met Pro Pro Tyr Ser Ala Asn
1               5                   10                  15
Ser Val Thr Thr Asp His Ile Gln Gln Tyr Leu Asp Glu Asn Lys Ala
            20                  25                  30
Leu Ile Leu Lys Ile Leu Glu Asn Gln Asn Ser Gly Lys Val Ser Glu
        35                  40                  45
Cys Ala Glu Asn Gln Ala Arg Leu Gln Arg Asn Leu Met Tyr Leu Ala
    50                  55                  60
Ala Ile Ala Asp Ser Gln Pro Gln Pro Pro Asn Met His Ala Gln Tyr
65                  70                  75                  80
Ser Asn Ala Gly Ile Pro Pro Gly Ala His Tyr Leu Gln His Gln Gln
                85                  90                  95
Ala Gln Gln Met Thr Gln Gln Ser Leu Met Ala Ala Arg Ser Asn Met
            100                 105                 110
Leu Tyr Ala Gln Pro Ile Thr Gly Met Gln Gln Gln Gln Ala Met His
        115                 120                 125
Ser Gln Leu Gly Met Ser Ser Gly Gly Asn Ser Gly Leu His Met Met
    130                 135                 140
His Asn Glu Gly Ser Met Gly Gly Ser Gly Ala Leu Gly Ser Tyr Ser
145                 150                 155                 160

Asp Tyr Gly Arg Gly Ser Gly Gly Gly Val Thr Ile Ala Ser Lys Gln
                165                 170                 175
Asp Gly Gly Ser Gly Ser Gly Glu Gly Arg Gly Gly Asn Ser Gly Gly
            180                 185                 190
Gln Ser Ala Asp Gly Gly Glu Ser Leu Tyr Leu Lys Asn Ser Asp Glu
        195                 200                 205
Gly Asn
    210
```

<210> 93
<211> 615
<212> DNA
<213> Brachypodium distachyon

<400> 93

```
atgcagcagg cgatgtccat gtccccgggg tcggccggcg cggtgccgcc tccggccggc      60
atcaccacag agcagatcca aaagtatttg gatgaaaata agcaacttat tttggccatc     120
ctggaaaatc agaacctagg aaagttgact gaatgtgctc agtatcaagc tcaacttcag     180
aagaatctct tgtatctggc tgccattgcg gatgcccaac caccacagaa ccctggaagt     240
cgcccccaga tggtgcagcc tggtggtatg ccaggtgcag ggcattacat gtcgcaagta     300
ccaatgttcc ctccaagaac ccctttaacc ccacaacaga tgcaagagca acagcaccag     360
cagcttcagc agcagcaagc acaggctctt gctttcccca gccagatggt catgagacca     420
ggtactgtga acggcatgca gcctatgcaa gctgatctcc aagcagcagc agcagcacct     480
ggcctggcag acagccgagg aagtaagcag gacgcagcgg tagctggggc catctcggaa     540
ccttctggca ccgagagtca caagagtaca ggagcggatc atgaggcagg tggcgatgta     600
gctgagcaat cctaa                                                      615
```

<210> 94
<211> 204
<212> PRT
<213> Brachypodium distachyon

<400> 94

```
Met Gln Gln Ala Met Ser Met Ser Pro Gly Ser Ala Gly Ala Val Pro
1               5                   10                  15
Pro Pro Ala Gly Ile Thr Thr Glu Gln Ile Gln Lys Tyr Leu Asp Glu
            20                  25                  30
Asn Lys Gln Leu Ile Leu Ala Ile Leu Glu Asn Gln Asn Leu Gly Lys
        35                  40                  45
Leu Thr Glu Cys Ala Gln Tyr Gln Ala Gln Leu Gln Lys Asn Leu Leu
    50                  55                  60
Tyr Leu Ala Ala Ile Ala Asp Ala Gln Pro Pro Gln Asn Pro Gly Ser
65                  70                  75                  80
Arg Pro Gln Met Val Gln Pro Gly Gly Met Pro Gly Ala Gly His Tyr
                85                  90                  95
Met Ser Gln Val Pro Met Phe Pro Pro Arg Thr Pro Leu Thr Pro Gln
            100                 105                 110
Gln Met Gln Glu Gln Gln His Gln Gln Leu Gln Gln Gln Gln Ala Gln
        115                 120                 125
Ala Leu Ala Phe Pro Ser Gln Met Val Met Arg Pro Gly Thr Val Asn
    130                 135                 140
Gly Met Gln Pro Met Gln Ala Asp Leu Gln Ala Ala Ala Ala Ala Pro
145                 150                 155                 160
Gly Leu Ala Asp Ser Arg Gly Ser Lys Gln Asp Ala Ala Val Ala Gly
                165                 170                 175
Ala Ile Ser Glu Pro Ser Gly Thr Glu Ser His Lys Ser Thr Gly Ala
            180                 185                 190
Asp His Glu Ala Gly Gly Asp Val Ala Glu Gln Ser
        195                 200
```

<210> 95
<211> 636
<212> DNA
<213> Brassica napus

<400> 95

```
atgcagcagc agcagcagca gcagcagcag cctccgcaaa tgtttccgat ggctccttcg      60
atgccgccaa ctaacatcac caccgaacag atccaaaagt accttgagga gaacaagaag     120
ctgataatgg caatcatgga aaatcagaat cttggcaagc ttgcagagtg tgcacagtac     180
caagctcttc tccagaagaa cttaatgtac ctcgctgcta ttgctgatgc tcaacctcct     240
ccatctaccg ctggagctac accaccacca gctatggctt cccagatggg ggcaccgcat     300
cctgggatgc aaccgccgag ctactttatg caacacccac aagcttcagg gatggctcaa     360
caagcaccac ccgctggtat cttccctccg agaggtcctt tgcagtttgg tagcccacac     420
cagcttcagg atccgcaaca gcagcatatg catcaacagg ctatgcaagg acacatgggg     480
atgcgaccaa tgggtatcaa caacaacaat gggatgcagc atcagatgca gcaacaacaa     540
ccagaaacct ctcttggagg aagcgctgca aacgtggggc ttagaggtgg aaagcaagat     600
ggagcagatg gacaaggaaa agatgatggc aaatga                               636
```

<210> 96
<211> 203
<212> PRT
<213> Brassica napus

<400> 96

```
Met Gln Gln His Leu Met Gln Met Gln Pro Met Met Ala Gly Tyr Tyr
1               5                   10                  15
Pro Ser Asn Val Thr Ser Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Ser Val His Ser
65                  70                  75                  80
Gln Tyr Gly Ser Ala Gly Gly Gly Leu Ile Gln Gly Glu Gly Ala Ser
                85                  90                  95
His Tyr Leu Gln Gln Gln Gln Ala Thr Gln Gln Gln Gln Met Thr Gln
            100                 105                 110
Gln Ser Leu Met Ala Ala Arg Ser Ser Met Met Tyr Gln Gln Gln Gln
        115                 120                 125
Gln Pro Tyr Ala Thr Leu Gln His Gln Gln Leu His His Ser Gln Leu
    130                 135                 140
Gly Met Ser Ser Ser Ser Gly Gly Gly Ser Ser Gly Leu His Ile Leu
145                 150                 155                 160
Gln Gly Glu Ala Gly Gly Phe His Glu Phe Gly Arg Gly Lys Pro Glu
                165                 170                 175
Met Gly Ser Gly Glu Gly Arg Gly Gly Ser Ser Gly Asp Gly Gly Glu
            180                 185                 190
Thr Leu Tyr Leu Lys Ser Ser Asp Asp Gly Asn
        195                 200
```

<210> 97
<211> 636
<212> DNA
<213> Citrus sinensis

<400> 97

```
atgcagcagc caccgcaaat gatccctgtt atgccttcat ttccacccac caacatcacc      60
```

```
acagagcaga ttcaaaagta ccttgatgag aacaaaaagt tgattttggc aattttggac     120
aatcaaaatc ttggaaagct tacagaatgt gcccactatc aagctcagct tcaaaagaat     180
ttaatgtatt tagctgcaat tgctgatgca caaccacaag caccaacaat gcctcctcag     240
atggctccac atcctgcaat gcaagctagt gggtattaca tgcaacatcc tcaggcggca     300
gcaatggctc agcaacaagg aatctttccc caaaagatgc cattacaatt caataaccct     360
catcaactac aggatcctca acagcagcta caccaacatc aagccatgca agcacaaatg     420
ggaatgagac cgggtgccac taacaatggt atgcatccca tgcatgctga aagctctctt     480
ggaggtggca gcagtggagg accccccttca gcatcaggcc caggtgacat acgtggtgga     540
aataagcaag atgcctcgga ggctgggact actggtgctg atggccaggg cagttcggct     600
ggtgggcatg gtggggatgg agaggaggca aagtga                               636
```

<210> 98
<211> 211
<212> PRT
<213> Citrus sinensis

<400> 98

```
Met Gln Gln Pro Pro Gln Met Ile Pro Val Met Pro Ser Phe Pro Pro
1               5                   10                  15
Thr Asn Ile Thr Thr Glu Gln Ile Gln Lys Tyr Leu Asp Glu Asn Lys
            20                  25                  30
Lys Leu Ile Leu Ala Ile Leu Asp Asn Gln Asn Leu Gly Lys Leu Thr
        35                  40                  45
Glu Cys Ala His Tyr Gln Ala Gln Leu Gln Lys Asn Leu Met Tyr Leu
    50                  55                  60
Ala Ala Ile Ala Asp Ala Gln Pro Gln Ala Pro Thr Met Pro Pro Gln
65                  70                  75                  80
Met Ala Pro His Pro Ala Met Gln Ala Ser Gly Tyr Tyr Met Gln His
                85                  90                  95
Pro Gln Ala Ala Ala Met Ala Gln Gln Gln Gly Ile Phe Pro Gln Lys
            100                 105                 110
Met Pro Leu Gln Phe Asn Asn Pro His Gln Leu Gln Asp Pro Gln Gln
        115                 120                 125
Gln Leu His Gln His Gln Ala Met Gln Ala Gln Met Gly Met Arg Pro
    130                 135                 140
Gly Ala Thr Asn Asn Gly Met His Pro Met His Ala Glu Ser Ser Leu
145                 150                 155                 160
Gly Gly Gly Ser Ser Gly Gly Pro Pro Ser Ala Ser Gly Pro Gly Asp
                165                 170                 175
Ile Arg Gly Gly Asn Lys Gln Asp Ala Ser Glu Ala Gly Thr Thr Gly
            180                 185                 190
Ala Asp Gly Gln Gly Ser Ser Ala Gly Gly His Gly Gly Asp Gly Glu
        195                 200                 205
Glu Ala Lys
    210
```

<210> 99
<211> 597
<212> DNA
<213> Euphorbia esula

<400> 99

```
atgcagcagc aaccgcagat gatgcctatg atgccttcat atccaccagc aaacattacc     60
acggagcaaa tccaaaagta tcttgatgaa aataaaaaat tgattttggc gatcttggat    120
aatcaaaatc ttggaaaact cgctgagtgt gcacagtatc aagccctgct gcaaaaaaat    180
ctgatgtatt tagccgcaat tgctgatgca caaccccaga ccccacccat gccacctcag    240
atgtccccac atccggctat gcaacaagga gcatattaca tgcaacatcc tcaggctgca    300
gcagcagcaa tggctcatca gtcgggtatt ttcccaccaa agatgtctcc gttacaattc    360
aataatcctc atcaaataca ggacccccag cagttacatc aagcagccct ccaagggcaa    420

atgggaatga ggcccatggg gcccaataac gggatgcatc cgatgcaccc cgaggcaaat    480
cttggaggat ctaatgatgg tcgtggagga aacaaacagg atgctccgga gacgggagca    540
tcgggaggtg atgggcaagg caattctggt ggtgatgggg ctgaagatgg gaaatga       597
```

<210> 100
<211> 198
<212> PRT
<213> Euphorbia esula

<400> 100

```
Met Gln Gln Gln Pro Gln Met Met Pro Met Met Pro Ser Tyr Pro Pro
1               5                   10                  15
Ala Asn Ile Thr Thr Glu Gln Ile Gln Lys Tyr Leu Asp Glu Asn Lys
            20                  25                  30
Lys Leu Ile Leu Ala Ile Leu Asp Asn Gln Asn Leu Gly Lys Leu Ala
        35                  40                  45
Glu Cys Ala Gln Tyr Gln Ala Leu Leu Gln Lys Asn Leu Met Tyr Leu
    50                  55                  60
Ala Ala Ile Ala Asp Ala Gln Pro Gln Thr Pro Pro Met Pro Pro Gln
65                  70                  75                  80
Met Ser Pro His Pro Ala Met Gln Gln Gly Ala Tyr Tyr Met Gln His
                85                  90                  95
Pro Gln Ala Ala Ala Ala Ala Met Ala His Gln Ser Gly Ile Phe Pro
            100                 105                 110
Pro Lys Met Ser Pro Leu Gln Phe Asn Asn Pro His Gln Ile Gln Asp
        115                 120                 125
Pro Gln Gln Leu His Gln Ala Ala Leu Gln Gly Gln Met Gly Met Arg
    130                 135                 140
Pro Met Gly Pro Asn Asn Gly Met His Pro Met His Pro Glu Ala Asn
145                 150                 155                 160
Leu Gly Gly Ser Asn Asp Gly Arg Gly Gly Asn Lys Gln Asp Ala Pro
                165                 170                 175
Glu Thr Gly Ala Ser Gly Gly Asp Gly Gln Gly Asn Ser Gly Gly Asp
            180                 185                 190
Gly Ala Glu Asp Gly Lys
        195
```

<210> 101
<211> 642
<212> DNA
<213> Glycine max

<400> 101

```
atgcagcaga caccgccaat gattcctatg atgccttctt tcccacctac gaacataacc      60
accgagcaga ttcaaaaata ccttgatgag aacaagaagc tgattctggc aatattggac     120
aatcaaaatc ttggaaaact tgcagaatgt gcccagtacc aagctcagct tcaaaagaat     180
ttgatgtatt tagctgcaat tgctgatgcc cagcctcaaa ccccggccat gcctccgcag     240
atggcaccgc accctgccat gcaaccagga ttctatatgc aacatcctca ggctgctgca     300
gcagcaatgg ctcagcagca gcaaggaatg ttcccccaga aaatgccatt gcaatttggc     360
aatccacatc aaatgcagga acaacaacag cagctacacc agcaggccat ccaaggtcaa     420
atgggactta gacctggaga tataaataat ggcatgcatc caatgcacag tgaggctgct     480
cttggaggtg gaaacagcgg tggtccacct tcggctactg gtccaaacga tgcacgtggt     540
ggaagcaagc aagatgcctc tgaggctgga acagctggtg gagacggcca aggcagctcc     600
gcggctgctc ataacagtgg agatggtgaa gaggcaaagt ga                        642
```

<210> 102
<211> 213
<212> PRT
<213> Glycine max

<400> 102

```
Met Gln Gln Thr Pro Pro Met Ile Pro Met Met Pro Ser Phe Pro Pro
1               5                   10                  15
Thr Asn Ile Thr Thr Glu Gln Ile Gln Lys Tyr Leu Asp Glu Asn Lys
            20                  25                  30
Lys Leu Ile Leu Ala Ile Leu Asp Asn Gln Asn Leu Gly Lys Leu Ala
        35                  40                  45
Glu Cys Ala Gln Tyr Gln Ala Gln Leu Gln Lys Asn Leu Met Tyr Leu
    50                  55                  60
Ala Ala Ile Ala Asp Ala Gln Pro Gln Thr Pro Ala Met Pro Pro Gln
65                  70                  75                  80
Met Ala Pro His Pro Ala Met Gln Pro Gly Phe Tyr Met Gln His Pro
                85                  90                  95
Gln Ala Ala Ala Ala Ala Met Ala Gln Gln Gln Gln Gly Met Phe Pro
            100                 105                 110
Gln Lys Met Pro Leu Gln Phe Gly Asn Pro His Gln Met Gln Glu Gln
        115                 120                 125
Gln Gln Gln Leu His Gln Gln Ala Ile Gln Gly Gln Met Gly Leu Arg
    130                 135                 140
Pro Gly Asp Ile Asn Asn Gly Met His Pro Met His Ser Glu Ala Ala
145                 150                 155                 160
Leu Gly Gly Gly Asn Ser Gly Gly Pro Pro Ser Ala Thr Gly Pro Asn
                165                 170                 175
Asp Ala Arg Gly Gly Ser Lys Gln Asp Ala Ser Glu Ala Gly Thr Ala
            180                 185                 190
Gly Gly Asp Gly Gln Gly Ser Ser Ala Ala Ala His Asn Ser Gly Asp
        195                 200                 205
Gly Glu Glu Ala Lys
    210
```

<210> 103
<211> 633
<212> DNA
<213> Glycine soya

<400> 103

```
atgcagcaga caccgcctat gattcctatg atgccttcgt tcccacctac gaacataacc      60
accgagcaga ttcaaaaata ccttgatgag aacaagaagc tgattctggc aatattggac     120
aatcaaaatc ttggaaaact tgcagaatgt gcccagtacc aagctcagct tcaaaagaat     180
ttgatgtatt tagctgcaat tgctgatgcc cagcctcaaa caccagccat gcctccacag     240
atggcaccac accctgccat gcaaccagga ttctatatgc aacatcctca ggctgcagca     300
gcagcaatgg ctcagcagca gcagcaagga atgttccccc agaaaatgcc attgcaattt     360
ggcaatccac atcaaatgca ggaacaacag cagcagctac accagcaagc catccaaggt     420
caaatgggac tgagacctgg aggaataaat aatggcatgc atccaatgca caatgagggc     480
ggcaacagcg gtggtccacc ctcggctacc ggtccgaacg acgcacgtgg tggaagcaag     540
caagatgctt ctgaggctgg aacagctggt ggagatggcc aaggcagctc tgcagctgct     600
cataacagtg gagatggtga agaggcaaag tga                                  633
```

<210> 104
<211> 210
<212> PRT
<213> Glycine soya

<400> 104

```
Met Gln Gln Thr Pro Pro Met Ile Pro Met Met Pro Ser Phe Pro Pro
1               5                   10                  15
Thr Asn Ile Thr Thr Glu Gln Ile Gln Lys Tyr Leu Asp Glu Asn Lys
            20                  25                  30

Lys Leu Ile Leu Ala Ile Leu Asp Asn Gln Asn Leu Gly Lys Leu Ala
        35                  40                  45
Glu Cys Ala Gln Tyr Gln Ala Gln Leu Gln Lys Asn Leu Met Tyr Leu
    50                  55                  60
Ala Ala Ile Ala Asp Ala Gln Pro Gln Thr Pro Ala Met Pro Pro Gln
65                  70                  75                  80
Met Ala Pro His Pro Ala Met Gln Pro Gly Phe Tyr Met Gln His Pro
                85                  90                  95
Gln Ala Ala Ala Ala Ala Met Ala Gln Gln Gln Gln Gln Gly Met Phe
            100                 105                 110
Pro Gln Lys Met Pro Leu Gln Phe Gly Asn Pro His Gln Met Gln Glu
        115                 120                 125
Gln Gln Gln Gln Leu His Gln Gln Ala Ile Gln Gly Gln Met Gly Leu
    130                 135                 140
Arg Pro Gly Gly Ile Asn Asn Gly Met His Pro Met His Asn Glu Gly
145                 150                 155                 160
Gly Asn Ser Gly Gly Pro Pro Ser Ala Thr Gly Pro Asn Asp Ala Arg
                165                 170                 175
Gly Gly Ser Lys Gln Asp Ala Ser Glu Ala Gly Thr Ala Gly Gly Asp
            180                 185                 190
Gly Gln Gly Ser Ser Ala Ala Ala His Asn Ser Gly Asp Gly Glu Glu
        195                 200                 205
Ala Lys
    210
```

<210> 105
<211> 690
<212> DNA
<213> Gossypium hirsutum

<400> 105

```
atgcagcagc acctgatgca gatgcagccc atgatggcag cttattatcc caacaacgtc      60
actactgatc atattcaaca gtatctcgat gagaacaagt cattgatctt aaagattgtt     120
gagagccaga attctgggaa attgagtgaa tgtgctgaga accaagcaag gctgcagcga     180
aacctcatgt acctggctgc cattgcggat tctcaacccc aaccacccac cgtgcatgca     240
cagtttccat ctggtggtat catgcagcca ggagctgggc actacatgca gcaccaacaa     300
gctcaacaaa tgacacaaca gtcgcttatg gctgctcggt cctcaatgtt gtattctcag     360
caaccatttt ctgcactgca acaacaacag cagcaagctt tgcacagtca gcttggcatg     420
agctctggcg gaagcacagg ccttcatatg ctgcaaactg aatctagtac tgcaggtggc     480
agtggagcac ttggggccgg agggtttcct gattttggac gtggttcttc tggagaaggc     540
atccatggtg gcaggccaat ggcaggtgga agcaagcaag atatcgggag tgccggctca     600
gctgaaggtc gtggaggaag ctctggtggt cagggtggtg gtgatggggg tgaaaccctt     660
tacttaaaag cagccgatga tgggaactga                                      690
```

<210> 106
<211> 229
<212> PRT
<213> Gossypium hirsutum

<400> 106

```
Met Gln Gln His Leu Met Gln Met Gln Pro Met Met Ala Ala Tyr Tyr
1               5                   10                  15
Pro Asn Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Thr Val His Ala

65                  70                  75                  80
Gln Phe Pro Ser Gly Gly Ile Met Gln Pro Gly Ala Gly His Tyr Met
                85                  90                  95
Gln His Gln Gln Ala Gln Gln Met Thr Gln Gln Ser Leu Met Ala Ala
            100                 105                 110
Arg Ser Ser Met Leu Tyr Ser Gln Gln Pro Phe Ser Ala Leu Gln Gln
        115                 120                 125
Gln Gln Gln Gln Ala Leu His Ser Gln Leu Gly Met Ser Ser Gly Gly
    130                 135                 140
Ser Thr Gly Leu His Met Leu Gln Thr Glu Ser Ser Thr Ala Gly Gly
145                 150                 155                 160
Ser Gly Ala Leu Gly Ala Gly Gly Phe Pro Asp Phe Gly Arg Gly Ser
                165                 170                 175
Ser Gly Glu Gly Ile His Gly Gly Arg Pro Met Ala Gly Gly Ser Lys
            180                 185                 190
Gln Asp Ile Gly Ser Ala Gly Ser Ala Glu Gly Arg Gly Gly Ser Ser
        195                 200                 205
Gly Gly Gln Gly Gly Gly Asp Gly Gly Glu Thr Leu Tyr Leu Lys Ala
    210                 215                 220
Ala Asp Asp Gly Asn
225
```

<210> 107
<211> 642
<212> DNA
<213> Gossypium hirsutum

<400> 107

```
atgccgcagc caccgcaaat gattcctgtg atgccttcat atccacctac taatatcact      60
actgaacaga ttcagaagta ccttgatgag aataagaagt tgattttggc aattttggac     120
aatcagaatc ttggaaaact cgctgaatgc gcccagtatc aagctcagct gcaaaagaat     180
ttgatgtatt tagctgcaat tgcggatgct caacctcaat caacgccagc aatgtcgcct     240
cagatggcac cgcatccagc aatgcaaccc ggaggatatt ttatgcaaca tcctcaagct     300
gctgcaatgt cacagcaacc tggcatgtac cctcaaaagg tgccattgca attcaatagt     360
ccgcatcaaa tgcaggaccc tcagcacctc ctatatcagc agcatcaaca agcaatgcaa     420
ggtcaaatgg gaatcaggcc tgggggaccc aataatagca tgcatcccat gcattcagag     480
gctagccttg gaggcggcag cagtggtggt ccccctcaac cttcaggccc aagtgatgga     540
cgtgctggaa acaagcaaga gggctccgaa gctggtggta atgggcaggg cagcacaact     600
ggtgggcatg gtggcggtga tggagcggat gaggcaaagt ga                        642
```

<210> 108
<211> 213
<212> PRT
<213> Gossypium hirsutum

<400> 108

```
Met Pro Gln Pro Pro Gln Met Ile Pro Val Met Pro Ser Tyr Pro Pro
1               5                   10                  15
Thr Asn Ile Thr Thr Glu Gln Ile Gln Lys Tyr Leu Asp Glu Asn Lys
            20                  25                  30
Lys Leu Ile Leu Ala Ile Leu Asp Asn Gln Asn Leu Gly Lys Leu Ala
        35                  40                  45
Glu Cys Ala Gln Tyr Gln Ala Gln Leu Gln Lys Asn Leu Met Tyr Leu
    50                  55                  60
Ala Ala Ile Ala Asp Ala Gln Pro Gln Ser Thr Pro Ala Met Ser Pro
65                  70                  75                  80
Gln Met Ala Pro His Pro Ala Met Gln Pro Gly Gly Tyr Phe Met Gln
                85                  90                  95
His Pro Gln Ala Ala Ala Met Ser Gln Gln Pro Gly Met Tyr Pro Gln


            100                 105                 110
Lys Val Pro Leu Gln Phe Asn Ser Pro His Gln Met Gln Asp Pro Gln
        115                 120                 125
His Leu Leu Tyr Gln Gln His Gln Gln Ala Met Gln Gly Gln Met Gly
    130                 135                 140
Ile Arg Pro Gly Gly Pro Asn Asn Ser Met His Pro Met His Ser Glu
145                 150                 155                 160
Ala Ser Leu Gly Gly Gly Ser Ser Gly Gly Pro Pro Gln Pro Ser Gly
                165                 170                 175
Pro Ser Asp Gly Arg Ala Gly Asn Lys Gln Glu Gly Ser Glu Ala Gly
            180                 185                 190
Gly Asn Gly Gln Gly Ser Thr Thr Gly Gly His Gly Gly Gly Asp Gly
        195                 200                 205
Ala Asp Glu Ala Lys
    210
```

<210> 109
<211> 561
<212> DNA
<213> Hordeum vulgare

<400> 109

```
atgcagcaag cgatgcccat gccgccggcg gcggcggcgc ctgggatgcc tccttctgcc      60
ggcctcagca ccgagcagat ccaaaagtac ctggatgaaa ataaacaact aattttggct     120
atcttggaaa atcagaacct gggaaagttg gcggaatgtg ctcagtatca agctcagctt     180
cagaagaatc ttttgtattt ggctgcgatt gctgatactc agccacagac ctctgtaagc     240
cgtcctcaga tggcaccacc tgctgcatcc ccaggggcag ggcattacat gtcacaggtg     300
ccaatgttcc ctccgaggac ccctctaacg cctcagcaga tgcaggagca gcaactacag     360
caacaacagg ctcagatgct tccgtttgct ggtcaaatgg ttgcgagacc cggggctgtc     420
aatggcattc cccaggcccc tcaagttgaa caaccagcct atgcagcagg tggggccagt     480
tccgagcctt ctggcaccga gagccacagg agcactggcg ccgataacga tggtgggagc     540
ggcttggctg accagtccta a                                               561
```

<210> 110
<211> 186
<212> PRT
<213> Hordeum vulgare

<400> 110

```
Met Gln Gln Ala Met Pro Met Pro Pro Ala Ala Ala Ala Pro Gly Met
1               5                   10                  15
Pro Pro Ser Ala Gly Leu Ser Thr Glu Gln Ile Gln Lys Tyr Leu Asp
            20                  25                  30
Glu Asn Lys Gln Leu Ile Leu Ala Ile Leu Glu Asn Gln Asn Leu Gly
        35                  40                  45
Lys Leu Ala Glu Cys Ala Gln Tyr Gln Ala Gln Leu Gln Lys Asn Leu
    50                  55                  60
Leu Tyr Leu Ala Ala Ile Ala Asp Thr Gln Pro Gln Thr Ser Val Ser
65                  70                  75                  80
Arg Pro Gln Met Ala Pro Pro Ala Ala Ser Pro Gly Ala Gly His Tyr
                85                  90                  95
Met Ser Gln Val Pro Met Phe Pro Pro Arg Thr Pro Leu Thr Pro Gln
            100                 105                 110
Gln Met Gln Glu Gln Gln Leu Gln Gln Gln Gln Ala Gln Met Leu Pro
        115                 120                 125
Phe Ala Gly Gln Met Val Ala Arg Pro Gly Ala Val Asn Gly Ile Pro
    130                 135                 140
Gln Ala Pro Gln Val Glu Gln Pro Ala Tyr Ala Ala Gly Gly Ala Ser
145                 150                 155                 160

Ser Glu Pro Ser Gly Thr Glu Ser His Arg Ser Thr Gly Ala Asp Asn
                165                 170                 175
Asp Gly Gly Ser Gly Leu Ala Asp Gln Ser
            180                 185
```

<210> 111
<211> 555
<212> DNA
<213> Lactuca serriola

<220>
<221> misc_feature
<222> (253)..(253)
<223> n is a, c, g, or t

<400> 111

```
atgaagcagc cgatgatgcc gaatccaatg atgtcttctt cgtttcctcc tacaaacatc      60
accaccgatc agatccaaaa gttccttgat gaaaacaagc aactaattat agcaataatg     120
agcaacctaa atcttggaaa gcttgctgaa tgtgcccagt accaagctct actccaaaaa     180
aatttgatgt atctagcagc cattgcagat gctcaaccac ctacacctac accaacacta     240
aatatctctt atnagatggg cccggttcca catccaggga tgccacagca aggtggattt     300
tacatggcgc agcagcaccc tcaggcggct gtaatgacgg ctcagccacc ttctggtttt     360
ccacaaccga tgcctggtat gcaatttaac agcccacagg ctattcaagg gcagatgggc     420
gggaggtccg gtgggccgcc aagctcagcc gctagtgatg tctggagagg aagcatgcaa     480
gatggtggtg gtggtgctgc tgctgatggt ggtaaggatg gtcatgctgg cggtggacct     540
gaggaagcaa agtaa                                                      555
```

<210> 112
<211> 184
<212> PRT
<213> Lactuca serriola

<220>
<221> UNSURE
<222> (85)..(85)
<223> Xaa can be any naturally occurring amino acid

<400> 112

```
Met Lys Gln Pro Met Met Pro Asn Pro Met Met Ser Ser Ser Phe Pro
1               5                   10                  15
Pro Thr Asn Ile Thr Thr Asp Gln Ile Gln Lys Phe Leu Asp Glu Asn
            20                  25                  30
Lys Gln Leu Ile Ile Ala Ile Met Ser Asn Leu Asn Leu Gly Lys Leu
        35                  40                  45
Ala Glu Cys Ala Gln Tyr Gln Ala Leu Leu Gln Lys Asn Leu Met Tyr
    50                  55                  60
Leu Ala Ala Ile Ala Asp Ala Gln Pro Pro Thr Pro Thr Pro Thr Leu
65                  70                  75                  80
Asn Ile Ser Tyr Xaa Met Gly Pro Val Pro His Pro Gly Met Pro Gln
                85                  90                  95
Gln Gly Gly Phe Tyr Met Ala Gln Gln His Pro Gln Ala Ala Val Met
            100                 105                 110
Thr Ala Gln Pro Pro Ser Gly Phe Pro Gln Pro Met Pro Gly Met Gln
        115                 120                 125
Phe Asn Ser Pro Gln Ala Ile Gln Gly Gln Met Gly Gly Arg Ser Gly
    130                 135                 140
Gly Pro Pro Ser Ser Ala Ala Ser Asp Val Trp Arg Gly Ser Met Gln
145                 150                 155                 160
Asp Gly Gly Gly Gly Ala Ala Ala Asp Gly Gly Lys Asp Gly His Ala

                165                 170                 175
  Gly Gly Gly Pro Glu Glu Ala Lys
            180
```

<210> 113
<211> 627
<212> DNA
<213> Lycopersicon esculentum

<400> 113

```
atgcagcagc acctgatgca gatgcagccc atgatggcag cttactatcc aacgaacgtc      60
actactgacc atattcaaca gtatttggat gaaaacaaat cactcattct gaagattgtt     120
gagagccaga actctgggaa actcagtgaa tgtgcggaga accaagctag gcttcagagg     180
aatctgatgt accttgctgc gattgctgat tcacaacctc aaccttctag catgcattct     240
cagttctctt ctggtgggat gatgcagcca gggacacaca gttacttgca gcagcagcag     300
cagcaacaac aagcgcaaca aatggcaaca caacaactca tggctgcaag atcctcgtcg     360
atgctctatg gacaacagca gcagcaatct cagttatcgc aatatcaaca aggcttgcat     420
agtagccaac tcggcatgag ttctggcagt ggcggaagca ctggacttca tcacatgctt     480
caaagtgaat catcacctca tggtggtggt ttctctcatg acttcggccg cgcaaataag     540
caagacattg ggagtagtat gtctgctgaa gggcgcggcg gaagttcagg tggtgagaat     600
ctttatctga aagcttctga ggattga                                         627
```

<210> 114
<211> 208
<212> PRT
<213> Lycopersicon esculentum

<400> 114

```
Met Gln Gln His Leu Met Gln Met Gln Pro Met Met Ala Ala Tyr Tyr
1               5                   10                  15
Pro Thr Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Ser Ser Met His Ser
65                  70                  75                  80
Gln Phe Ser Ser Gly Gly Met Met Gln Pro Gly Thr His Ser Tyr Leu
                85                  90                  95
Gln Gln Gln Gln Gln Gln Gln Gln Ala Gln Gln Met Ala Thr Gln Gln
            100                 105                 110
Leu Met Ala Ala Arg Ser Ser Ser Met Leu Tyr Gly Gln Gln Gln Gln
        115                 120                 125
Gln Ser Gln Leu Ser Gln Tyr Gln Gln Gly Leu His Ser Ser Gln Leu
    130                 135                 140
Gly Met Ser Ser Gly Ser Gly Gly Ser Thr Gly Leu His His Met Leu
145                 150                 155                 160
Gln Ser Glu Ser Ser Pro His Gly Gly Gly Phe Ser His Asp Phe Gly
                165                 170                 175
Arg Ala Asn Lys Gln Asp Ile Gly Ser Ser Met Ser Ala Glu Gly Arg
            180                 185                 190
Gly Gly Ser Ser Gly Gly Glu Asn Leu Tyr Leu Lys Ala Ser Glu Asp
        195                 200                 205
```

<210> 115
<211> 624
<212> DNA
<213> Malus domestica

<400> 115

```
atgcagcagc caccacaaat gatccccgtc atgccttcat ttcctcccac caacatcacc     60
accgaacaaa ttcagaagta ccttgatgac aacaaaaagt tgattctggc aatattggat    120
aatcaaaatc ttggaaaact tgctgagtgt gctcagtacc aggctctgct tcaaaagaat    180
ctgatgtatt tagcagcaat tgccgatgcg caaccacagg caccagctgc cctccccag     240
atggccccac atcctgctat gcaacaggca ggatattaca tgcaacatcc tcaggcagca    300
gcaatggctc agcaacaggg tattttctcc ccaaagatgc cgatgcaatt caataacatg    360
catcaaatgc acgatccaca gcagcaccaa caagccatgc aagggcaaat gggaatgaga    420
cctggagggc ctaacggcat gccttccatg cttcatactg aggccacaca tggtggtggt    480
agtggcggcc caaattcagc tggagaccca aatgatgggc gtggaggaag caagcaagac    540
gcctctgagt ctggggcagg tggtgatggc caggggacct cagccggcgg gcgtggaact    600
ggtgatggag aggacggcaa gtga                                           624
```

<210> 116
<211> 207
<212> PRT
<213> Malus domestica

<400> 116

```
Met Gln Gln Pro Pro Gln Met Ile Pro Val Met Pro Ser Phe Pro Pro
1               5                   10                  15
Thr Asn Ile Thr Thr Glu Gln Ile Gln Lys Tyr Leu Asp Asp Asn Lys
            20                  25                  30
Lys Leu Ile Leu Ala Ile Leu Asp Asn Gln Asn Leu Gly Lys Leu Ala
        35                  40                  45
Glu Cys Ala Gln Tyr Gln Ala Leu Leu Gln Lys Asn Leu Met Tyr Leu
    50                  55                  60
Ala Ala Ile Ala Asp Ala Gln Pro Gln Ala Pro Ala Ala Pro Pro Gln
65                  70                  75                  80
Met Ala Pro His Pro Ala Met Gln Gln Ala Gly Tyr Tyr Met Gln His
                85                  90                  95
Pro Gln Ala Ala Ala Met Ala Gln Gln Gln Gly Ile Phe Ser Pro Lys
            100                 105                 110
Met Pro Met Gln Phe Asn Asn Met His Gln Met His Asp Pro Gln Gln
        115                 120                 125
His Gln Gln Ala Met Gln Gly Gln Met Gly Met Arg Pro Gly Gly Pro
    130                 135                 140
Asn Gly Met Pro Ser Met Leu His Thr Glu Ala Thr His Gly Gly Gly
145                 150                 155                 160
Ser Gly Gly Pro Asn Ser Ala Gly Asp Pro Asn Asp Gly Arg Gly Gly
                165                 170                 175
Ser Lys Gln Asp Ala Ser Glu Ser Gly Ala Gly Gly Asp Gly Gln Gly
            180                 185                 190
Thr Ser Ala Gly Gly Arg Gly Thr Gly Asp Gly Glu Asp Gly Lys
        195                 200                 205
```

<210> 117
<211> 639
<212> DNA
<213> Medicago trunculata

<400> 117

```
atgcagcaga cacctcaaat gattcctatg atgccttcat tcccacaaca aacaaacata     60
accactgagc agattcaaaa atatcttgat gagaacaaga agctgatcct ggcaatattg    120
gacaatcaaa atcttggaaa acttgcagaa tgtgcccagt accaagctca gcttcagaag    180
aatttgatgt atttagctgc aattgctgac gcgcagccac aaacaccggc cttgcctcca    240
cagatggccc cgcaccctgc gatgcaacaa ggattctata tgcaacatcc tcaggctgca    300
gcaatggctc agcaacaagg aatgttcccc caaaaaatgc caatgcagtt cggtaatccg    360

catcaaatgc aggatcagca gcatcagcag caacaacagc agctacatca gcaagctatg    420
caaggtcaaa tgggacttag acctggaggg ataaataacg gcatgcatcc aatgcacaac    480
gaggctgctc tcggaggtag cggcagtggt ggtcaaatga cgggcgtggt ggtggagcaa    540
gcaagatgct tcggagctgg gacagccggc ggtgatggtc aaggaacctc tgccgcagct    600
gcgcacaaca gtggagatgc ttcagaagaa ggaaagtaa                           639
```

<210> 118
<211> 213
<212> PRT
<213> Medicago trunculata

<400> 118

```
Met Gln Gln Thr Pro Gln Met Ile Pro Met Met Pro Ser Phe Pro Gln
1               5                   10                  15
Gln Thr Asn Ile Thr Thr Glu Gln Ile Gln Lys Tyr Leu Asp Glu Asn
            20                  25                  30
Lys Lys Leu Ile Leu Ala Ile Leu Asp Asn Gln Asn Leu Gly Lys Leu
        35                  40                  45
Ala Glu Cys Ala Gln Tyr Gln Ala Gln Leu Gln Lys Asn Leu Met Tyr
    50                  55                  60
Leu Ala Ala Ile Ala Asp Ala Gln Pro Gln Thr Pro Ala Leu Pro Pro
65                  70                  75                  80
Gln Met Ala Pro His Pro Ala Met Gln Gln Gly Phe Tyr Met Gln His
                85                  90                  95
Pro Gln Ala Ala Ala Met Ala Gln Gln Gln Gly Met Phe Pro Gln Lys
            100                 105                 110
Met Pro Met Gln Phe Gly Asn Pro His Gln Met Gln Asp Gln Gln His
        115                 120                 125
Gln Gln Gln Gln Gln Gln Leu His Gln Gln Ala Met Gln Gly Gln Met
    130                 135                 140
Gly Leu Arg Pro Gly Gly Ile Asn Asn Gly Met His Pro Met His Asn
145                 150                 155                 160
Glu Ala Ala Leu Gly Gly Ser Gly Ser Gly Gly Pro Asn Asp Gly Arg
                165                 170                 175
Gly Gly Gly Ser Lys Gln Asp Ala Ser Glu Ala Gly Thr Ala Gly Gly
            180                 185                 190
Asp Gly Gln Gly Thr Ser Ala Ala Ala Ala His Asn Ser Gly Asp Ala
        195                 200                 205
Ser Glu Glu Gly Lys
    210
```

<210> 119
<211> 624
<212> DNA
<213> Panicum virgatum

<400> 119

```
atgcagcagc agatgcccat gcagtcggcg cccccggcga ccggcatcac caccgagcag      60
atccaaaagt atttggatga aaataagcag cttattttgg ccatcctgga aaatcagaac     120
ttaggaaagt tggctgaatg tgctcagtat caagctcagc ttcaaaagaa tctcttgtac     180
ctggctgcga ttgcagatgc ccaaccccaa ccaccacaga accctgcaag tcgcccacag     240
atgatgcaac ctggcatggt accaggtgca gggcattaca tgtcccaagt accaatgttc     300
ccgccaagaa caccattaac cccgcaacag atgcaagaac agcagcagca gcagcagcag     360
cttcaacagc agcaagcaca ggctcttgct ttcccgggac agatggtcat gagacctacc     420
attaatggca tgcagcctat gcaagccgac cctgctgccg ccgccgccag cctacagcag     480
tcagcacctg gccctactga tgggcgagga ggcaagcaag atgcaactgc tggggtgagc     540
acagagcctt ctggcaccga gagccacaag agcacaaccg cagcagatca cgatgtgggc     600
actgatgtcg cggagaaatc ctaa                                            624
```

<210> 120
<211> 207
<212> PRT
<213> Panicum virgatum

<400> 120

```
Met Gln Gln Gln Met Pro Met Gln Ser Ala Pro Pro Ala Thr Gly Ile
1               5                   10                  15
Thr Thr Glu Gln Ile Gln Lys Tyr Leu Asp Glu Asn Lys Gln Leu Ile
            20                  25                  30
Leu Ala Ile Leu Glu Asn Gln Asn Leu Gly Lys Leu Ala Glu Cys Ala
        35                  40                  45
Gln Tyr Gln Ala Gln Leu Gln Lys Asn Leu Leu Tyr Leu Ala Ala Ile
    50                  55                  60
Ala Asp Ala Gln Pro Gln Pro Pro Gln Asn Pro Ala Ser Arg Pro Gln
65                  70                  75                  80
Met Met Gln Pro Gly Met Val Pro Gly Ala Gly His Tyr Met Ser Gln
                85                  90                  95
Val Pro Met Phe Pro Pro Arg Thr Pro Leu Thr Pro Gln Gln Met Gln
            100                 105                 110
Glu Gln Gln Gln Gln Gln Gln Gln Leu Gln Gln Gln Gln Ala Gln Ala
        115                 120                 125
Leu Ala Phe Pro Gly Gln Met Val Met Arg Pro Thr Ile Asn Gly Met
    130                 135                 140
Gln Pro Met Gln Ala Asp Pro Ala Ala Ala Ala Ala Ser Leu Gln Gln
145                 150                 155                 160
Ser Ala Pro Gly Pro Thr Asp Gly Arg Gly Gly Lys Gln Asp Ala Thr
                165                 170                 175
Ala Gly Val Ser Thr Glu Pro Ser Gly Thr Glu Ser His Lys Ser Thr
            180                 185                 190
Thr Ala Ala Asp His Asp Val Gly Thr Asp Val Ala Glu Lys Ser
        195                 200                 205
```

<210> 121
<211> 747
<212> DNA
<213> Picea sitchensis

<400> 121

```
atgcagcagc atctcatgca aatgcagccc atgatggcgg catacgcctc caacaacatc      60
accactgatc acatccagaa gtacctggat gagaacaagc agttgattct ggcaattctg     120
gacaaccaaa atcttggaaa gctcaatgag tgtgctcagt accaagcaaa acttcagcag     180
aatttgatgt atctggctgc gattgctgat tctcaaccac aagcacaaac tgcacatgct     240
cagattcctc ctaatgcagt gatgcagtct ggtgggcatt acatgcagca ccagcaggca     300
cagcaacaag tgactcctca gtctctgatg gcagctagat cttccatgct gtattctcag     360
cagccgatgg ctgctttgca tcaagctcag caacaacagc agcagcagca tcagcagcaa     420
caacaatctc ttcacagcca gcttggcata aattctggag gaagcagtgg attgcatatg     480
ttgcatggtg agacaaacat gggatgtaat gggcctctct catctggggg cttccctgaa     540
tttgggcgtg ggtctgctac ctctgctgaa ggtatgcagg ccaacagggg cttcactata     600
gatcgtggtt caaataagca ggatggagta ggatcagaga atgcccatcc aggtgctggt     660
gatggaagag ggagttcaac tggagggcag aatgcagatg agtcagaacc atcatacctg     720
aaagcctccg aagaagaagg aaactag                                         747
```

<210> 122
<211> 248
<212> PRT
<213> Picea sitchensis

<400> 122

```
Met Gln Gln His Leu Met Gln Met Gln Pro Met Met Ala Ala Tyr Ala
1               5                   10                  15
Ser Asn Asn Ile Thr Thr Asp His Ile Gln Lys Tyr Leu Asp Glu Asn
            20                  25                  30
Lys Gln Leu Ile Leu Ala Ile Leu Asp Asn Gln Asn Leu Gly Lys Leu
        35                  40                  45
Asn Glu Cys Ala Gln Tyr Gln Ala Lys Leu Gln Gln Asn Leu Met Tyr
    50                  55                  60
Leu Ala Ala Ile Ala Asp Ser Gln Pro Gln Ala Gln Thr Ala His Ala
65                  70                  75                  80
Gln Ile Pro Pro Asn Ala Val Met Gln Ser Gly Gly His Tyr Met Gln
                85                  90                  95
His Gln Gln Ala Gln Gln Gln Val Thr Pro Gln Ser Leu Met Ala Ala
            100                 105                 110
Arg Ser Ser Met Leu Tyr Ser Gln Gln Pro Met Ala Ala Leu His Gln
        115                 120                 125
Ala Gln Gln Gln Gln Gln Gln Gln His Gln Gln Gln Gln Gln Ser Leu
    130                 135                 140
His Ser Gln Leu Gly Ile Asn Ser Gly Gly Ser Ser Gly Leu His Met
145                 150                 155                 160
Leu His Gly Glu Thr Asn Met Gly Cys Asn Gly Pro Leu Ser Ser Gly
                165                 170                 175
Gly Phe Pro Glu Phe Gly Arg Gly Ser Ala Thr Ser Ala Glu Gly Met
            180                 185                 190
Gln Ala Asn Arg Gly Phe Thr Ile Asp Arg Gly Ser Asn Lys Gln Asp
        195                 200                 205
Gly Val Gly Ser Glu Asn Ala His Pro Gly Ala Gly Asp Gly Arg Gly
    210                 215                 220
Ser Ser Thr Gly Gly Gln Asn Ala Asp Glu Ser Glu Pro Ser Tyr Leu
225                 230                 235                 240
Lys Ala Ser Glu Glu Glu Gly Asn
                245
```

<210> 123
<211> 735
<212> DNA
<213> Pinus taeda

<400> 123

```
atgcagcagc acctcatgca aatgcagccc atgatggcgg cctacgcctc caacaatatc      60
accactgatc acatccagaa gtacctggat gagaacaagc agttgattct ggcaattttg     120
gacaaccaaa atctcggaaa gctcaatgag tgtgctcaat accaagcaaa acttcagcag     180
aatttgatgt atctggctgc tattgctgat tctcaacctc aagcacaaac tgcacatgct     240
cagattcctc caaatgcggt gatgcagtct ggtgggcatt acatgcagca tcaacaggca     300
cagcaacaag ttactcctca gtctctgatg gcagctagat cttccatact gtatgctcag     360
caacaacagc agcagcagca tcagcagcat cagcagcaac agcagcaaca acagtctctt     420
cacagccagc ttggcataaa ttctggagga agcagcggtt tgcatatgtt gcatggtgag     480
acaaacatgg gatgtaatgg gcctctgtca tctgggggat tccctgaatt tgggcgtggg     540
tctgctacct ctgctgatgg tatgcaggtg aacaggggct ttgctataga tcgtggttca     600
aacaagcagg atggagttgg atcagagaat gcccatgctg gtgctggtga tggaagaggg     660
agttcaactg gagggcagaa tgcagatgag tcagaaccat catacctgaa ggcctccgag     720
gaagaaggaa actag                                                      735
```

<210> 124
<211> 244
<212> PRT
<213> Pinus taeda

<400> 124

```
Met Gln Gln His Leu Met Gln Met Gln Pro Met Met Ala Ala Tyr Ala
1               5                   10                  15
Ser Asn Asn Ile Thr Thr Asp His Ile Gln Lys Tyr Leu Asp Glu Asn
            20                  25                  30
Lys Gln Leu Ile Leu Ala Ile Leu Asp Asn Gln Asn Leu Gly Lys Leu
        35                  40                  45
Asn Glu Cys Ala Gln Tyr Gln Ala Lys Leu Gln Gln Asn Leu Met Tyr
    50                  55                  60
Leu Ala Ala Ile Ala Asp Ser Gln Pro Gln Ala Gln Thr Ala His Ala
65                  70                  75                  80
Gln Ile Pro Pro Asn Ala Val Met Gln Ser Gly Gly His Tyr Met Gln
                85                  90                  95
His Gln Gln Ala Gln Gln Gln Val Thr Pro Gln Ser Leu Met Ala Ala
            100                 105                 110
Arg Ser Ser Ile Leu Tyr Ala Gln Gln Gln Gln Gln Gln Gln His Gln
        115                 120                 125
Gln His Gln Gln Gln Gln Gln Gln Gln Gln Ser Leu His Ser Gln Leu
    130                 135                 140
Gly Ile Asn Ser Gly Gly Ser Ser Gly Leu His Met Leu His Gly Glu
145                 150                 155                 160
Thr Asn Met Gly Cys Asn Gly Pro Leu Ser Ser Gly Gly Phe Pro Glu
                165                 170                 175
Phe Gly Arg Gly Ser Ala Thr Ser Ala Asp Gly Met Gln Val Asn Arg
            180                 185                 190
Gly Phe Ala Ile Asp Arg Gly Ser Asn Lys Gln Asp Gly Val Gly Ser
        195                 200                 205
Glu Asn Ala His Ala Gly Ala Gly Asp Gly Arg Gly Ser Ser Thr Gly
    210                 215                 220
Gly Gln Asn Ala Asp Glu Ser Glu Pro Ser Tyr Leu Lys Ala Ser Glu
225                 230                 235                 240
Glu Glu Gly Asn
```

<210> 125
<211> 663
<212> DNA
<213> Populus tremula

<400> 125

```
atgcaacagc acctgatgca gatgcagccc atgatggcag cctattaccc cagcaacgtc     60
actactgatc atattcaaca gtatctggac gaaaacaagt cattgatttt gaagattgtt    120
gagagccaga attcagggaa actcagtgag tgtgcagaga accaagcaag actgcaacaa    180
aatctcatgt acttggctgc aattgctgat tgtcagcccc aaccacctac catgcatgcc    240
cagttccctt ccagcggcat tatgcagcca ggagcacatt acatgcagca tcaacaagct    300
caacagatga caccacaagc ccttatggct gcacgctctt ctatgctgca gtatgctcaa    360
cagccattct cagcgcttca acaacagcaa gccttacaca gccagctcgg catgagctct    420
ggtggaagcg caggacttca tatgatgcaa agcgaggcta acactgcagg aggcagtgga    480
gctcttggtg ctggacgatt tcctgatttt ggcatggatg cctccagtag aggaatcgca    540
agtgggagca agcaagatat tcggagtgca gggtctagtg aagggcgagg aggaagctct    600
ggaggccagg gtggtgatgg aggtgaaacc ctttacttga aatctgctga tgatgggaac    660
tga                                                                  663
```

<210> 126
<211> 220
<212> PRT
<213> Populus tremula

<400> 126

```
Met Gln Gln His Leu Met Gln Met Gln Pro Met Met Ala Ala Tyr Tyr
1               5                   10                  15
Pro Ser Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Gln Asn Leu Met Tyr
    50                  55                  60
Leu Ala Ala Ile Ala Asp Cys Gln Pro Gln Pro Pro Thr Met His Ala
65                  70                  75                  80
Gln Phe Pro Ser Ser Gly Ile Met Gln Pro Gly Ala His Tyr Met Gln
                85                  90                  95
His Gln Gln Ala Gln Gln Met Thr Pro Gln Ala Leu Met Ala Ala Arg
            100                 105                 110
Ser Ser Met Leu Gln Tyr Ala Gln Gln Pro Phe Ser Ala Leu Gln Gln
        115                 120                 125
Gln Gln Ala Leu His Ser Gln Leu Gly Met Ser Ser Gly Gly Ser Ala
    130                 135                 140
Gly Leu His Met Met Gln Ser Glu Ala Asn Thr Ala Gly Gly Ser Gly
145                 150                 155                 160
Ala Leu Gly Ala Gly Arg Phe Pro Asp Phe Gly Met Asp Ala Ser Ser
                165                 170                 175
Arg Gly Ile Ala Ser Gly Ser Lys Gln Asp Ile Arg Ser Ala Gly Ser
            180                 185                 190
Ser Glu Gly Arg Gly Gly Ser Ser Gly Gly Gln Gly Gly Asp Gly Gly
        195                 200                 205
Glu Thr Leu Tyr Leu Lys Ser Ala Asp Asp Gly Asn
    210                 215                 220
```

<210> 127
<211> 678
<212> DNA
<213> Saccharum officinarum

<400> 127

```
atgcagcagc aacacctgat gcagatgaac cagaacatga ttgggggcta cacctctcct     60
gccgctgtga caaccgatct catccagcag tacctggatg agaacaagca gctgatcctg    120
gccatcctcg acaaccagaa caatggcaag gtggaggagt gcgaacggca ccaagctaag    180
ctccagcaca acctcatgta cctggccgcc atcgccgaca gccagccacc acagactgca    240
ccactatcac aatacccgtc caacctgatg atgcagccgg gccctcggta catgccaccg    300
cagtccgggc agatgatgag cccgcagtcg ctaatggcgg cgcggtcctc catgatgtac    360
gcgcacccgt ccatgtcacc actccagcag cagcaggcag cgcacgggca gctgggcatg    420
gcttcagggg gcggcggtgg cacgaccagt gggttcaaca tcctccatgg cgaggccagt    480
atgggcggtg ctggtggcgc ttgtgccggc aacaacatga tgaacgccgg catgttctca    540
ggctttggcc gcagcggcag tggcgccaag gagggatcga cctcgctgtc ggttgacgtc    600
cgtggtggca ccagctccgg cgcgcaaagc ggggacggcg agtacctgaa agcaggcacc    660
gaggaagaag gcagttaa                                                  678
```

<210> 128
<211> 225
<212> PRT
<213> Saccharum officinarum

<400> 128

```
Met Gln Gln Gln His Leu Met Gln Met Asn Gln Asn Met Ile Gly Gly
1               5                   10                  15
Tyr Thr Ser Pro Ala Ala Val Thr Thr Asp Leu Ile Gln Gln Tyr Leu
            20                  25                  30
Asp Glu Asn Lys Gln Leu Ile Leu Ala Ile Leu Asp Asn Gln Asn Asn
        35                  40                  45

Gly Lys Val Glu Glu Cys Glu Arg His Gln Ala Lys Leu Gln His Asn
    50                  55                  60
Leu Met Tyr Leu Ala Ala Ile Ala Asp Ser Gln Pro Pro Gln Thr Ala
65                  70                  75                  80
Pro Leu Ser Gln Tyr Pro Ser Asn Leu Met Met Gln Pro Gly Pro Arg
                85                  90                  95
Tyr Met Pro Pro Gln Ser Gly Gln Met Met Ser Pro Gln Ser Leu Met
            100                 105                 110
Ala Ala Arg Ser Ser Met Met Tyr Ala His Pro Ser Met Ser Pro Leu
        115                 120                 125
Gln Gln Gln Gln Ala Ala His Gly Gln Leu Gly Met Ala Ser Gly Gly
    130                 135                 140
Gly Gly Gly Thr Thr Ser Gly Phe Asn Ile Leu His Gly Glu Ala Ser
145                 150                 155                 160
Met Gly Gly Ala Gly Gly Ala Cys Ala Gly Asn Asn Met Met Asn Ala
                165                 170                 175
Gly Met Phe Ser Gly Phe Gly Arg Ser Gly Ser Gly Ala Lys Glu Gly
            180                 185                 190
Ser Thr Ser Leu Ser Val Asp Val Arg Gly Gly Thr Ser Ser Gly Ala
        195                 200                 205
Gln Ser Gly Asp Gly Glu Tyr Leu Lys Ala Gly Thr Glu Glu Glu Gly
    210                 215                 220
Ser
225
```

<210> 129
<211> 561
<212> DNA
<213> Saccharum officinarum

<400> 129

```
atgcagcagc cgatgcccat gcagccgcag gcgccggaga tgaccccggc cgccggaatc     60
accacggagc agatccaaaa gtatctggat gagaataagc agcttatttt ggctattttg    120
gaaaatcaga acctaggaaa attggcagaa tgtgctcagt atcaatcaca acttcagaag    180
aacctcttgt atctcgctgc aatcgcagat gcccaaccac agactgctgt aagccgccct    240
cagatggcgc cgcctggtgc attgcctgga gtagggcagt acatgtcaca ggtgcctatg    300
ttcccaccga ggacacctct aacaccccag cagatgcagg agcagcaact tcagcagcag    360
caggctcagc tgctaaattt cagtggccta atggttgcta gacctggcat ggtcaacggc    420
atgcctcagt ccattcaagt tcagcaagct cagccaccac cagcagggaa caaacaggat    480
gctggtgggg tcgcctcgga gccctcgggc attgagaacc acaggagcac tggtggtgat    540
aatgatggtg gaagcgacta g                                              561
```

<210> 130
<211> 186
<212> PRT
<213> Saccharum officinarum

<400> 130

```
Met Gln Gln Pro Met Pro Met Gln Pro Gln Ala Pro Glu Met Thr Pro
1               5                   10                  15
Ala Ala Gly Ile Thr Thr Glu Gln Ile Gln Lys Tyr Leu Asp Glu Asn
            20                  25                  30
Lys Gln Leu Ile Leu Ala Ile Leu Glu Asn Gln Asn Leu Gly Lys Leu
        35                  40                  45
Ala Glu Cys Ala Gln Tyr Gln Ser Gln Leu Gln Lys Asn Leu Leu Tyr
    50                  55                  60
Leu Ala Ala Ile Ala Asp Ala Gln Pro Gln Thr Ala Val Ser Arg Pro
65                  70                  75                  80
Gln Met Ala Pro Pro Gly Ala Leu Pro Gly Val Gly Gln Tyr Met Ser

                85                  90                  95
Gln Val Pro Met Phe Pro Pro Arg Thr Pro Leu Thr Pro Gln Gln Met
            100                 105                 110
Gln Glu Gln Gln Leu Gln Gln Gln Gln Ala Gln Leu Leu Asn Phe Ser
        115                 120                 125
Gly Leu Met Val Ala Arg Pro Gly Met Val Asn Gly Met Pro Gln Ser
    130                 135                 140
Ile Gln Val Gln Gln Ala Gln Pro Pro Pro Ala Gly Asn Lys Gln Asp
145                 150                 155                 160
Ala Gly Gly Val Ala Ser Glu Pro Ser Gly Ile Glu Asn His Arg Ser
                165                 170                 175
Thr Gly Gly Asp Asn Asp Gly Gly Ser Asp
            180                 185
```

<210> 131
<211> 642
<212> DNA
<213> Saccharum officinarum

<400> 131

```
atgcagcagc agatgcccat gccgccggcg cccgctgcgg cggcggcgcc cccggcggcc      60
ggcatcacca ccgagcagat ccaaaagtat ttggacgaaa ataagcaact tattttggcc     120
atcctggaaa atcagaactt aggaaagttg gctgaatgtg ctcagtatca agctcaactt     180
caaaagaacc tcttgtacct ggctgcgatt gctgatgccc aaccccagcc accacaaaac     240
cctgcaggtc gccctcagat gatgcaacct ggtatagtgc caggtgcggg gcattacatg     300
tcacaagtac caatgttccc tccaagaact ccattaaccc cacagcagat gcaagagcag     360
cagcagcaac agcttcagca gcagcaagcg caggctctta cattccctgg acagatggtc     420
atgagaccag ctaccatcaa cggcatacag cagcctatgc aagctgaccc tgcccgggca     480
gcggagctgc aacaaccacc acctatccca gctgacgggc gagtaagcaa gcagcaggac     540
acaacggctg gcgtgagctc agagccttct gccaatgaga gccacaagac cacaactgga     600
gcagatagtg aggcaggtgg tgacgtggcg gagaaatcct aa                        642
```

<210> 132
<211> 213
<212> PRT
<213> Saccharum officinarum

<400> 132

```
Met Gln Gln Gln Met Pro Met Pro Pro Ala Pro Ala Ala Ala Ala Ala
1               5                   10                  15
Pro Pro Ala Ala Gly Ile Thr Thr Glu Gln Ile Gln Lys Tyr Leu Asp
            20                  25                  30
Glu Asn Lys Gln Leu Ile Leu Ala Ile Leu Glu Asn Gln Asn Leu Gly
        35                  40                  45
Lys Leu Ala Glu Cys Ala Gln Tyr Gln Ala Gln Leu Gln Lys Asn Leu
    50                  55                  60
Leu Tyr Leu Ala Ala Ile Ala Asp Ala Gln Pro Gln Pro Pro Gln Asn
65                  70                  75                  80
Pro Ala Gly Arg Pro Gln Met Met Gln Pro Gly Ile Val Pro Gly Ala
                85                  90                  95
Gly His Tyr Met Ser Gln Val Pro Met Phe Pro Pro Arg Thr Pro Leu
            100                 105                 110
Thr Pro Gln Gln Met Gln Glu Gln Gln Gln Gln Gln Leu Gln Gln Gln
        115                 120                 125
Gln Ala Gln Ala Leu Thr Phe Pro Gly Gln Met Val Met Arg Pro Ala
    130                 135                 140
Thr Ile Asn Gly Ile Gln Gln Pro Met Gln Ala Asp Pro Ala Arg Ala
145                 150                 155                 160
Ala Glu Leu Gln Gln Pro Pro Pro Ile Pro Ala Asp Gly Arg Val Ser


                165                 170                 175
Lys Gln Gln Asp Thr Thr Ala Gly Val Ser Ser Glu Pro Ser Ala Asn
            180                 185                 190
Glu Ser His Lys Thr Thr Thr Gly Ala Asp Ser Glu Ala Gly Gly Asp
        195                 200                 205
Val Ala Glu Lys Ser
    210
```

<210> 133
<211> 645
<212> DNA
<213> Solanum tuberosum

<400> 133

```
atgcagcagc acctgatgca gatgcagccc atgatggcag cttactatcc aacgaacgtc      60
actactgacc atattcaaca gtatttggat gagaacaaat cactcattct gaaaattgtt     120
gagagccaaa actcgggaaa actcagtgaa tgtgcagaga accaagctag gcttcagagg     180
aatctgatgt accttgctgc tattgctgat tcacaacctc agccttctag catgcattct     240
cagttctctt ctggtgggat gatgcagcca gggacacaca gttacctgca gcagcagcag     300
cagcaacaac aagcgcaaca aatggcaaca caacaactca tggctgcaag atcctcatca     360
atgctctatg gacaacaaca gcagcagcag cagcagtctc agttatcaca atttcaacaa     420
ggcttgcata gtagccaact tggcatgagt tctggcagtg gtggaagcac tggacttcat     480
cacatgcttc aaagtgaatc atcacctcat ggtggtggtt tctctcatga cttcggccgt     540
gcaaataagc aagacattgg gagtagtatg tctgctgaag ggcgcggcgg aagctcaggt     600
ggtgatggtg gtgagaatct ttatctgaaa gcttctgagg attga                     645
```

<210> 134
<211> 214
<212> PRT
<213> Solanum tuberosum

<400> 134

```
Met Gln Gln His Leu Met Gln Met Gln Pro Met Met Ala Ala Tyr Tyr
1               5                   10                  15
Pro Thr Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Ser Ser Met His Ser
65                  70                  75                  80
Gln Phe Ser Ser Gly Gly Met Met Gln Pro Gly Thr His Ser Tyr Leu
                85                  90                  95
Gln Gln Gln Gln Gln Gln Gln Gln Ala Gln Gln Met Ala Thr Gln Gln
            100                 105                 110
Leu Met Ala Ala Arg Ser Ser Ser Met Leu Tyr Gly Gln Gln Gln Gln
        115                 120                 125
Gln Gln Gln Gln Ser Gln Leu Ser Gln Phe Gln Gln Gly Leu His Ser
    130                 135                 140
Ser Gln Leu Gly Met Ser Ser Gly Ser Gly Gly Ser Thr Gly Leu His
145                 150                 155                 160
His Met Leu Gln Ser Glu Ser Ser Pro His Gly Gly Gly Phe Ser His
                165                 170                 175
Asp Phe Gly Arg Ala Asn Lys Gln Asp Ile Gly Ser Ser Met Ser Ala
            180                 185                 190
Glu Gly Arg Gly Gly Ser Ser Gly Gly Asp Gly Gly Glu Asn Leu Tyr
        195                 200                 205
Leu Lys Ala Ser Glu Asp


    210
```

<210> 135
<211> 645
<212> DNA
<213> Sorghum bicolor

<400> 135

```
atgcagcagc agatgcccat gccgccggcg cccgctgcgg cggcggcgac ggcgcccccg      60
gcggccggca tcaccaccga gcagatccag aagtatttgg acgaaaataa gcaacttatt     120
ttggccatcc tagaaaatca gaacttagga aagttggctg aatgtgctca gtatcaagct     180
caacttcaaa agaacctctt gtacctggct gcgattgctg atgcccaacc ccgaccaccg     240
caaaaccctg caggtcgccc tcagatgatg caacctggta tagtgccagg tgcagggcat     300
tacatgtcac aagtaccaat gttccctcca agaactccat taaccccaca gcaaatgcaa     360
gagcagcagc agcaacagct tcagcagcag caagcgcagg ctcttgcatt ccctgggcag     420
atggtcatga gaccagctac catcaacggc atgcagcagc ctatgcaggc tgaccctgcc     480
cgggcagcgg agctgcaaca gccagcatct gtcccagccg acgggcgagt aagcaagcag     540
gacacagcgg ctggggtgag ctcagagcct tctgccaatg agagccacaa gaccacaacc     600
ggagcagata gtgaggcagg tggagacgtg gcggagaaat cctaa                     645
```

<210> 136
<211> 214
<212> PRT
<213> Sorghum bicolor

<400> 136

```
Met Gln Gln Gln Met Pro Met Pro Pro Ala Pro Ala Ala Ala Ala Ala
1               5                   10                  15
Thr Ala Pro Pro Ala Ala Gly Ile Thr Thr Glu Gln Ile Gln Lys Tyr
            20                  25                  30
Leu Asp Glu Asn Lys Gln Leu Ile Leu Ala Ile Leu Glu Asn Gln Asn
        35                  40                  45
Leu Gly Lys Leu Ala Glu Cys Ala Gln Tyr Gln Ala Gln Leu Gln Lys
    50                  55                  60
Asn Leu Leu Tyr Leu Ala Ala Ile Ala Asp Ala Gln Pro Arg Pro Pro
65                  70                  75                  80
Gln Asn Pro Ala Gly Arg Pro Gln Met Met Gln Pro Gly Ile Val Pro
                85                  90                  95
Gly Ala Gly His Tyr Met Ser Gln Val Pro Met Phe Pro Pro Arg Thr
            100                 105                 110
Pro Leu Thr Pro Gln Gln Met Gln Glu Gln Gln Gln Gln Gln Leu Gln
        115                 120                 125
Gln Gln Gln Ala Gln Ala Leu Ala Phe Pro Gly Gln Met Val Met Arg
    130                 135                 140
Pro Ala Thr Ile Asn Gly Met Gln Gln Pro Met Gln Ala Asp Pro Ala
145                 150                 155                 160
Arg Ala Ala Glu Leu Gln Gln Pro Ala Ser Val Pro Ala Asp Gly Arg
                165                 170                 175
Val Ser Lys Gln Asp Thr Ala Ala Gly Val Ser Ser Glu Pro Ser Ala
            180                 185                 190
Asn Glu Ser His Lys Thr Thr Thr Gly Ala Asp Ser Glu Ala Gly Gly
        195                 200                 205
Asp Val Ala Glu Lys Ser
    210
```

<210> 137
<211> 558
<212> DNA
<213> Triticum aestivum

<400> 137

```
atgcagcaag cgatgcccat gccgccggcg gcggcggcgc cggggatgcc tccgtctgct      60
ggcctcagca ccgagcagat ccaaaagtac ctggatgaaa ataagcaact aattttggct     120
atcttggaaa atcagaacct gggaaagttg gcggaatgtg ctcagtatca agctcagctt     180
cagaagaatc ttttgtattt ggctgcaatc gctgatactc agccacagac cactgtaagc     240
cgtcctcaga tggcaccacc tagtgcatcc ccaggggcag ggcattacat gtcacaggtg     300
ccaatgttcc ctccgaggac ccctctaacg cctcagcaga tgcaggagca gcaactacag     360
cagcaacagg ctcagatgct tccgtttgct ggtcaaatgg ttgcgagacc tggggctgtc     420
aatggcatgc ctcaggcccc tcaagttgaa ccagcctatg cagcaggtgg ggccagttct     480
gagccttctg gcactgagag ccacaggagc actggtgccg ataatgacgg ggggagcggc     540
tgggctgatc agtcctaa                                                   558
```

<210> 138
<211> 185
<212> PRT
<213> Triticum aestivum

<400> 138

```
Met Gln Gln Ala Met Pro Met Pro Pro Ala Ala Ala Ala Pro Gly Met
1               5                   10                  15
Pro Pro Ser Ala Gly Leu Ser Thr Glu Gln Ile Gln Lys Tyr Leu Asp
            20                  25                  30
Glu Asn Lys Gln Leu Ile Leu Ala Ile Leu Glu Asn Gln Asn Leu Gly
        35                  40                  45
Lys Leu Ala Glu Cys Ala Gln Tyr Gln Ala Gln Leu Gln Lys Asn Leu
    50                  55                  60
Leu Tyr Leu Ala Ala Ile Ala Asp Thr Gln Pro Gln Thr Thr Val Ser
65                  70                  75                  80
Arg Pro Gln Met Ala Pro Pro Ser Ala Ser Pro Gly Ala Gly His Tyr
                85                  90                  95
Met Ser Gln Val Pro Met Phe Pro Pro Arg Thr Pro Leu Thr Pro Gln
            100                 105                 110
Gln Met Gln Glu Gln Gln Leu Gln Gln Gln Ala Gln Met Leu Pro
        115                 120                 125
Phe Ala Gly Gln Met Val Ala Arg Pro Gly Ala Val Asn Gly Met Pro
    130                 135                 140
Gln Ala Pro Gln Val Glu Pro Ala Tyr Ala Ala Gly Gly Ala Ser Ser
145                 150                 155                 160
Glu Pro Ser Gly Thr Glu Ser His Arg Ser Thr Gly Ala Asp Asn Asp
                165                 170                 175
Gly Gly Ser Gly Trp Ala Asp Gln Ser
            180                 185
```

<210> 139
<211> 603
<212> DNA
<213> Triticum aestivum

<400> 139

```
atgcagcagg cgatgtcctt gcccccggga gcggtcggcg cggtgtcctc gccggccggc      60
atcaccaccg agcagatcca aaagtatttg gatgaaaata agcaacttat tttggccatc     120
cttgaaaatc agaacctagg aaagttggct gaatgtgctc agtatcaagc tcaactccaa     180
aagaatctct tgtatctagc tgctatcgcg gatgcccaac caccacagaa ccctacaagt     240
caccctcaga tggtgcagcc tggtagtatg caaggtgcag ggcattacat gtcacaagta     300
ccaatgttcc ctccaagaac gcctttaacc ccacagcaga tgcaagagca gcagcaccag     360
cagcttcagc agcagcaagc ccaggccctt tctttccccg cccaggtggt catgagacca     420
ggcaccgtca acggcatgca gcagcctatg caagcagccg gcgacctcca gccagcagca     480
gcacctggag ggagcaagca ggacgccgca gtggctgggg ccagctcgga accatctggc     540

accaagagcc acaagaacgc gggagcagag gaggtgggcg ctgatgtagc agaacaatcc     600
taa                                                                   603
```

<210> 140
<211> 200
<212> PRT
<213> Triticum aestivum

<400> 140

```
Met Gln Gln Ala Met Ser Leu Pro Pro Gly Ala Val Gly Ala Val Ser
1               5                   10                  15
Ser Pro Ala Gly Ile Thr Thr Glu Gln Ile Gln Lys Tyr Leu Asp Glu
            20                  25                  30
Asn Lys Gln Leu Ile Leu Ala Ile Leu Glu Asn Gln Asn Leu Gly Lys
        35                  40                  45
Leu Ala Glu Cys Ala Gln Tyr Gln Ala Gln Leu Gln Lys Asn Leu Leu
    50                  55                  60
Tyr Leu Ala Ala Ile Ala Asp Ala Gln Pro Pro Gln Asn Pro Thr Ser
65                  70                  75                  80
His Pro Gln Met Val Gln Pro Gly Ser Met Gln Gly Ala Gly His Tyr
                85                  90                  95
Met Ser Gln Val Pro Met Phe Pro Pro Arg Thr Pro Leu Thr Pro Gln
            100                 105                 110
Gln Met Gln Glu Gln Gln His Gln Gln Leu Gln Gln Gln Gln Ala Gln
        115                 120                 125
Ala Leu Ser Phe Pro Ala Gln Val Val Met Arg Pro Gly Thr Val Asn
    130                 135                 140
Gly Met Gln Gln Pro Met Gln Ala Ala Gly Asp Leu Gln Pro Ala Ala
145                 150                 155                 160
Ala Pro Gly Gly Ser Lys Gln Asp Ala Ala Val Ala Gly Ala Ser Ser
                165                 170                 175
Glu Pro Ser Gly Thr Lys Ser His Lys Asn Ala Gly Ala Glu Glu Val
            180                 185                 190
Gly Ala Asp Val Ala Glu Gln Ser
        195                 200
```

<210> 141
<211> 672
<212> DNA
<213> Vitis vinifera

<400> 141

```
atgcagcagc acctgatgca gatgcagccc atgatggcag cctattaccc cagcaacgtc      60
accactgatc acattcagca gtatcttgat gaaaacaagt cattgattct gaagattgtt     120
gagagccaga attcaggaaa attgactgaa tgtgcagaga accaggcaag actacagaga     180
aacctcatgt acctggctgc aattgctgat tctcaacccc aaccacccac catgcatgct     240
cagttccctc ctagtggcat tgttcagcca ggagctcact acatgcaaca ccaacaagct     300
caacaaatga caccacagtc gctcctggct gcacgctcct ccatgctgta cacccaacaa     360
ccattttcgg ccctgcaaca acaacaagcc atccatagcc agcttggcat gggctctggt     420
ggaagtgcag gacttcacat gctgcaaagc gaggggagta atccaggagg caatggaaca     480
ctggggactg gtgggtttcc tgatttcagc cgtggaactt ctggagaagg cctgcaggct     540
gcaggcaggg gaatggctgg tgggagcaag caagatatgg gaaatgcaga agggcgagga     600
gggaactcag gaggtcaggg tggggatgga ggtgagactc tttacttgaa agctgctgaa     660
gatgggaatt ga                                                         672
```

<210> 142
<211> 223
<212> PRT
<213> Vitis vinifera

<400> 142

```
Met Gln Gln His Leu Met Gln Met Gln Pro Met Met Ala Ala Tyr Tyr
1               5                   10                  15
Pro Ser Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Thr Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Thr Met His Ala
65                  70                  75                  80
Gln Phe Pro Pro Ser Gly Ile Val Gln Pro Gly Ala His Tyr Met Gln
                85                  90                  95
His Gln Gln Ala Gln Gln Met Thr Pro Gln Ser Leu Leu Ala Ala Arg
            100                 105                 110
Ser Ser Met Leu Tyr Thr Gln Gln Pro Phe Ser Ala Leu Gln Gln Gln
        115                 120                 125
Gln Ala Ile His Ser Gln Leu Gly Met Gly Ser Gly Gly Ser Ala Gly
    130                 135                 140
Leu His Met Leu Gln Ser Glu Gly Ser Asn Pro Gly Gly Asn Gly Thr
145                 150                 155                 160
Leu Gly Thr Gly Gly Phe Pro Asp Phe Ser Arg Gly Thr Ser Gly Glu
                165                 170                 175
Gly Leu Gln Ala Ala Gly Arg Gly Met Ala Gly Gly Ser Lys Gln Asp
            180                 185                 190
Met Gly Asn Ala Glu Gly Arg Gly Gly Asn Ser Gly Gly Gln Gly Gly
        195                 200                 205
Asp Gly Gly Glu Thr Leu Tyr Leu Lys Ala Ala Glu Asp Gly Asn
    210                 215                 220
```

<210> 143
<211> 663
<212> DNA
<213> Zea mays

<400> 143

```
atgcagcagc agatgcccat gccgccggcg cccgctgccg ccgcggcggc ggcgcccccg      60
gcggcaggca tcactaccga gcagatccag aagtatttgg acgaaaataa gcaacttatt     120
ttggccatcc tggaaaatca gaacttaggg aagttggctg aatgtgctca gtatcaagct     180
caacttcaaa agaacctctt gtacctggct gcgattgctg atgcccaacc ccagcctccg     240
caaaaccctg caggtcgccc tcagatgatg cagcctggta tagtgccagg tgcggggcat     300
tacatgtcac aagtaccaat gttccctcca agaaccccat taaccccaca gcagatgcag     360
gagcagcagc aacaacaaca gtttcagcag cagcagcagc aagtgcaggc tcttacattt     420
cctggacaga tggtcatgag accaggcacc atcaacggca tgcagcagca gcagcctatg     480
caggctgacc ctgcccgggc agcagcggag ctgcagcagg cagcacctat cccagctgac     540
gggcgaggaa gcaagcagga caccgcgggt ggggcgagct cagagccttc tgccaatgag     600
agccacaaga gcgccaccgg agcagatacc gaggcaggtg gcgacgtggc cgagaaatcc     660
taa                                                                   663
```

<210> 144
<211> 220
<212> PRT
<213> Zea mays

<400> 144

```
Met Gln Gln Gln Met Pro Met Pro Pro Ala Pro Ala Ala Ala Ala Ala
1               5                   10                  15
Ala Ala Pro Pro Ala Ala Gly Ile Thr Thr Glu Gln Ile Gln Lys Tyr


            20                  25                  30
Leu Asp Glu Asn Lys Gln Leu Ile Leu Ala Ile Leu Glu Asn Gln Asn
        35                  40                  45
Leu Gly Lys Leu Ala Glu Cys Ala Gln Tyr Gln Ala Gln Leu Gln Lys
    50                  55                  60
Asn Leu Leu Tyr Leu Ala Ala Ile Ala Asp Ala Gln Pro Gln Pro Pro
65                  70                  75                  80
Gln Asn Pro Ala Gly Arg Pro Gln Met Met Gln Pro Gly Ile Val Pro
                85                  90                  95
Gly Ala Gly His Tyr Met Ser Gln Val Pro Met Phe Pro Pro Arg Thr
            100                 105                 110
Pro Leu Thr Pro Gln Gln Met Gln Glu Gln Gln Gln Gln Gln Gln Phe
        115                 120                 125
Gln Gln Gln Gln Gln Gln Val Gln Ala Leu Thr Phe Pro Gly Gln Met
    130                 135                 140
Val Met Arg Pro Gly Thr Ile Asn Gly Met Gln Gln Gln Gln Pro Met
145                 150                 155                 160
Gln Ala Asp Pro Ala Arg Ala Ala Ala Glu Leu Gln Gln Ala Ala Pro
                165                 170                 175
Ile Pro Ala Asp Gly Arg Gly Ser Lys Gln Asp Thr Ala Gly Gly Ala
            180                 185                 190
Ser Ser Glu Pro Ser Ala Asn Glu Ser His Lys Ser Ala Thr Gly Ala
        195                 200                 205
Asp Thr Glu Ala Gly Gly Asp Val Ala Glu Lys Ser
    210                 215                 220
```

<210> 145
<211> 2193
<212> DNA
<213> Oryza sativa

<400> 145

```
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag attttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctcctcctc ccatctataa attcctcccc ccttttcccc tctctatata    1020
ggaggcatcc aagccaagaa gagggagagc accaaggaca cgcgactagc agaagccgag    1080
cgaccgcctt cttcgatcca tatcttccgg tcgagttctt ggtcgatctc ttccctcctc    1140
cacctcctcc tcacagggta tgtgcccttc ggttgttctt ggatttattg ttctaggttg    1200
tgtagtacgg gcgttgatgt taggaaaggg gatctgtatc tgtgatgatt cctgttcttg    1260
gatttgggat agaggggttc ttgatgttgc atgttatcgg ttcggtttga ttagtagtat    1320
ggttttcaat cgtctggaga gctctatgga aatgaaatgg tttagggtac ggaatcttgc    1380
gattttgtga gtaccttttg tttgaggtaa aatcagagca ccggtgattt tgcttggtgt    1440
aataaaagta cggttgtttg gtcctcgatt ctggtagtga tgcttctcga tttgacgaag    1500
ctatcctttg tttattccct attgaacaaa aataatccaa ctttgaagac ggtcccgttg    1560
atgagattga atgattgatt cttaagcctg tccaaaattt cgcagctggc ttgtttagat    1620


acagtagtcc ccatcacgaa attcatggaa acagttataa tcctcaggaa caggggattc    1680
cctgttcttc cgatttgctt tagtcccaga atttttttc ccaaatatct taaaaagtca    1740
ctttctggtt cagttcaatg aattgattgc tacaaataat gcttttatag cgttatccta    1800
gctgtagttc agttaatagg taatacccct atagtttagt caggagaaga acttatccga    1860
tttctgatct ccatttttaa ttatatgaaa tgaactgtag cataagcagt attcatttgg    1920
attattttt ttattagctc tcaccccttc attattctga gctgaaagtc tggcatgaac    1980
tgtcctcaat tttgttttca aattcacatc gattatctat gcattatcct cttgtatcta    2040
cctgtagaag tttcttttg gttattcctt gactgcttga ttacagaaag aaatttatga    2100
agctgtaatc gggatagtta tactgcttgt tcttatgatt catttccttt gtgcagttct    2160
tggtgtagct tgccactttc accagcaaag ttc                                 2193
```

<210> 146
<211> 12
<212> PRT
<213> Artificial sequence

<220>
<223> Box I

<220>
<221> VARIANT
<222> (3)..(3)
<223> /replace = "Lys"

<220>
<221> VARIANT
<222> (4)..(4)
<223> /replace = "Met" /replace = "Phe" /replace = "His"

<220>

<221> VARIANT
<222> (6)..(6)
<223> /replace = "Glu"

<220>
<221> VARIANT
<222> (7)..(7)
<223> /replace = "Asp"

<220>
<221> VARIANT
<222> (9)..(9)
<223> /replace = "Asn"

<220>
<221> VARIANT
<222> (10)..(10)
<223> Xaa can be any naturally occurring amino acid

<400> 146

```
Ile Gln Gln Tyr Leu Asp Glu Asn Lys Xaa Leu Ile
1               5                   10
```

<210> 147
<211> 10
<212> PRT
<213> Artificial sequence

<220>
<223> Box II

<220>
<221> VARIANT
<222> (3)..(3)
<223> /replace= "Leu" /replace= "Val"

<220>
<221> VARIANT
<222> (7)..(7)
<223> /replace= "Thr"

<400> 147

```
Asn Leu Met Tyr Leu Ala Ala Ile Ala Asp
1               5                   10
```

<210> 148
<211> 53
<212> DNA
<213> Artificial sequence

<220>
<223> primer: prm06681

<400> 148
ggggacaagt ttgtacaaaa aagcaggctt aaacaatgca acagcacctg atg 53

<210> 149
<211> 50
<212> DNA
<213> Artificial sequence

<220>
<223> primer: prm06682

<400> 149
ggggaccact ttgtacaaga aagctgggtc atcattaaga ttccttgtgc 50

<210> 150
<211> 615
<212> DNA
<213> Brassica napus

<400> 150

```
atgcaacagc acctgatgca gatgcagccc atgatggctg gttactaccc cagcaatgtc     60
acctctgatc atattcagca gtacttggac gagaacaaat cgttgattct gaagatagtt    120
gaatctcaaa actcgggaaa gctcagcgag tgtgccgaga accaggcaag gcttcaacgc    180
aacttaatgt acttagctgc aattgcagat tctcagcctc aacctccaag catgcatagc    240
cagtatggaa ctgctggtgg tggtgggttg atgcagggag aaggagggtc acactatttg    300
caacagcaac aggcaattca acagcagcag agtcagcagt ctctaatggc ggctcgatct    360
tcaatgttgt atgctcagca gcagcaacag ccttatgcaa cgcttcagca gcagcaattg    420
caccatagcc agcttgggat gagctcaagc agcggaggag gaagcagcgg tctccatatg    480
ctacagggag aggctggtgg gtttcatgat tttggccgtg agaagttgga aatgggaagt    540
ggtgaaggca gaggaggaag ctcaggggat ggtggagaaa ccctttactt gaagtcatca    600
gatgatggga actga                                                     615
```

<210> 151
<211> 204
<212> PRT
<213> Brassica napus

<400> 151

```
Met Gln Gln His Leu Met Gln Met Gln Pro Met Met Ala Gly Tyr Tyr
1               5                   10                  15
Pro Ser Asn Val Thr Ser Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Ser Met His Ser
65                  70                  75                  80
Gln Tyr Gly Thr Ala Gly Gly Gly Gly Leu Met Gln Gly Glu Gly Gly
                85                  90                  95
Ser His Tyr Leu Gln Gln Gln Gln Ala Ile Gln Gln Gln Gln Ser Gln
            100                 105                 110
Gln Ser Leu Met Ala Ala Arg Ser Ser Met Leu Tyr Ala Gln Gln Gln
        115                 120                 125
Gln Gln Pro Tyr Ala Thr Leu Gln Gln Gln Gln Leu His His Ser Gln
    130                 135                 140
Leu Gly Met Ser Ser Ser Ser Gly Gly Gly Ser Ser Gly Leu His Met
145                 150                 155                 160
Leu Gln Gly Glu Ala Gly Gly Phe His Asp Phe Gly Arg Glu Lys Leu
                165                 170                 175
Glu Met Gly Ser Gly Glu Gly Arg Gly Gly Ser Ser Gly Asp Gly Gly
            180                 185                 190
Glu Thr Leu Tyr Leu Lys Ser Ser Asp Asp Gly Asn
        195                 200
```

<210> 152
<211> 639
<212> DNA
<213> Glycine max

<400> 152

```
atgcagcagc acctgatgca gatgcagccc atgatggctg cctactaccc caacaacgtc      60
accactgatc acattcaaca gtacctggat gagaacaagt ccttgattct gaagattgtt     120
gaaagccaga attctggcaa gctgagcgag tgtgccgaga accaatcaag gctgcagaga     180
aatctcatgt acctagctgc aatagctgat tctcaaccac aaccatctcc attggctggt     240
cagtatcctt ctagtggact tgtgcagcag ggagcacact acatgcaggc tcaacaggct     300
cagcagatgt cacaacaaca gctaatggct tcgcgctcct cgctcctgta ctcccaacag     360
cctttctcag tgcttcaaca gcagcaaggc atgcacagcc aacttggcat gagctccagt     420
ggaagtcaag gcctccacat gctgcaaagt gaagccacta atgttggagg caatgcaacc     480
ataggaaccg gaggagggtt tccggacttt gtacgcattg gtagtggcaa gcaagatatt     540
ggaatctctg gtgaaggcag aggaggaaac tctagtggcc actctggtga tggtggtgag     600
acacttaatt acctgaaagc tgctggtgat ggaaactga                            639
```

<210> 153
<211> 212
<212> PRT
<213> Glycine max

<400> 153

```
Met Gln Gln His Leu Met Gln Met Gln Pro Met Met Ala Ala Tyr Tyr
1               5                   10                  15
Pro Asn Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
```

```
        35                  40                  45
Ser Glu Cys Ala Glu Asn Gln Ser Arg Leu Gln Arg Asn Leu Met Tyr
    50                  55                  60
Leu Ala Ala Ile Ala Asp Ser Gln Pro Gln Pro Ser Pro Leu Ala Gly
65                  70                  75                  80
Gln Tyr Pro Ser Ser Gly Leu Val Gln Gln Gly Ala His Tyr Met Gln
                85                  90                  95
Ala Gln Gln Ala Gln Gln Met Ser Gln Gln Gln Leu Met Ala Ser Arg
            100                 105                 110
Ser Ser Leu Leu Tyr Ser Gln Gln Pro Phe Ser Val Leu Gln Gln Gln
        115                 120                 125
Gln Gly Met His Ser Gln Leu Gly Met Ser Ser Ser Gly Ser Gln Gly
    130                 135                 140
Leu His Met Leu Gln Ser Glu Ala Thr Asn Val Gly Gly Asn Ala Thr
145                 150                 155                 160
Ile Gly Thr Gly Gly Gly Phe Pro Asp Phe Val Arg Ile Gly Ser Gly
                165                 170                 175
Lys Gln Asp Ile Gly Ile Ser Gly Glu Gly Arg Gly Gly Asn Ser Ser
            180                 185                 190
Gly His Ser Gly Asp Gly Gly Glu Thr Leu Asn Tyr Leu Lys Ala Ala
        195                 200                 205
Gly Asp Gly Asn
    210
```

## Description

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

Abiotic stresses such as drought stress, salinity stress, heat stress and cold stress, or a combination of one or more of these, are major limiting factors of plant growth and productivity (Boyer (1982) Science 218: 443-448). These stresses have as common theme important for plant growth water availability. Since high salt content in some soils results in less available water for cell intake, its effect is similar to those observed under drought conditions. Additionally, under freezing temperatures, plant cells loose water as a result of ice formation that starts from the apoplast and withdraws water from the symplast (McKersie and Leshem (1994) Stress and stress coping in cultivated plants, Kluwer Academic Publishers). During heat stress, stomata aperture is affected to adjust cooling by evapotranspiration, thereby affecting the water content of the plant. Commonly, a plant's molecular response mechanisms to each of these stress conditions is similar.

Plants are exposed during their entire life cycle to conditions of reduced environmental water content. Most plants have evolved strategies to protect themselves against these conditions. However, if the severity and duration of the stress conditions are too great, the effects on plant development, growth and yield of most crop plant are profound.

Continuous exposure to reduced environmental water availability causes major alterations in plant metabolism. These great changes in metabolism ultimately lead to cell death and consequently to yield losses. Crop losses and crop yield losses of major crops such as rice, maize (corn), and wheat caused by these stresses represent a significant economic and political factor and contribute to food shortages in many parts of the world.

Another example of abiotic environmental stress is the reduced availability of one or more nutrients that need to be assimilated by the plants for growth and development. Because of the strong influence of nutrition utilization efficiency on plant yield and product quality, a huge amount of fertilizer is poured onto fields to optimize plant growth and quality. Productivity of plants is limited by three primary nutrients: phosphorous, potassium and nitrogen, which are usually the rate-limiting elements in plant growth. The major nutritional element required for plant growth is nitrogen (N). It is a constituent of numerous important compounds found in living cells, including amino acids, proteins (enzymes), nucleic acids, and chlorophyll. 1.5% to 2% of plant dry matter is nitrogen and approximately 16% of total plant protein. Thus, nitrogen availability is a major limiting factor in crop plant growth and production (Frink et al. (1999) Proc Natl Acad Sci USA 96(4): 1175-1180), and has a major impact on protein accumulation and amino acid composition. Therefore, of great interest are crop plants with an increased yield when grown under nitrogen-limiting conditions.

Plant biomass is yield for forage crops like alfalfa, silage corn and hay. Many proxies for yield have been used in grain crops. Chief amongst these are estimates of plant size. Plant size can be measured in many ways depending on species and developmental stage, but include total plant dry weight, above-ground dry weight, above-ground fresh weight, leaf area, stem volume, plant height, rosette diameter, leaf length, root length, root mass, tiller number and leaf number. Many species maintain a conservative ratio between the size of different parts of the plant at a given developmental stage. These allometric relationships are used to extrapolate from one of these measures of size to another (e.g. Tittonell et al. (2005) Agric Ecosys & Environ 105: 213). Plant size at an early developmental stage will typically correlate with plant size later in development. A larger plant with a greater leaf area can typically absorb more light and carbon dioxide than a smaller plant and therefore will likely gain a greater weight during the same period (Fasoula & Tollenaar (2005) Maydica 50:39). This is in addition to the potential continuation of the micro-environmental or genetic advantage that the plant had to achieve the larger size initially. There is a strong genetic component to plant size and growth rate (e.g. ter Steege et al. (2005) Plant Physiology 139:1078), and so for a range of diverse genotypes plant size under one environmental condition is likely to correlate with size under another (Hittalmani et al. (2003) Theoretical Applied Genetics 107:679). In this way a standard environment is used as a proxy for the diverse and dynamic environments encountered at different locations and times by crops in the field.

Developing stress tolerant plants is a strategy that has the potential to solve or mediate at least some aspects of yield loss (McKersie and Leshem (1994) Stress and stress coping in cultivated plants, Kluwer Academic Publishers). However, traditional breeding strategies to develop new lines of plants that exhibit resistance (tolerance) to these types of stresses are relatively slow and require specific resistant lines for crossing with the desired line. Limited germplasm resources for stress tolerance and incompatibility in crosses between distantly related plant species represent significant problems encountered in conventional breeding. Furthermore, such selective breeding techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

SYT is a transcriptional co-activator that, in plants, forms a functional complex with transcription activators of the GRF (growth-regulating factor) family of proteins (Kim HJ, Kende H (2004) Proc Nat Acad Sc 101: 13374-9). SYT is called GIF for GRF-interacting factor in this paper, and AN3 for angustifolia3 in Horiguchi et al. (2005) Plant J 43: 68-78. The GRF transcription activators share structural domains (in the N-terminal region) with the SWI/SNF proteins of the chromatin-remodelling complexes in yeast (van der Knaap E et al., (2000) Plant Phys 122: 695-704). Transcriptional co-activators of these complexes are proposed to be involved in recruiting SWI/SNF complexes to enhancer and promoter regions to effect local chromatin remodelling (review Näär AM et al., (2001) Annu Rev Biochem 70: 475-501). The alteration in local chromatin structure modulates transcriptional activation. More precisely, SYT is proposed to interact with the plant SWI/SNF complex to affect transcriptional activation of GRF target gene(s) (Kim HJ, Kende H (2004) Proc Nat Acad Sc 101: 13374-9).

SYT belongs to a gene family of three members in *Arabidopsis.* The SYT polypeptide shares homology with the human SYT. The human SYT polypeptide was shown to be a transcriptional co-activator (Thaete et al. (1999) Hum Molec Genet 8: 585-591). Three domains characterize the mammalian SYT polypeptide:

(i) the N-terminal SNH (SYT N-terminal homology) domain, conserved in mammals, plants, nematodes and fish;
(ii) the C-terminal QPGY-rich domain, composed predominantly of glycine, proline, glutamine and tyrosine, occurring at variable intervals;
(iii) a methionine-rich (Met-rich) domain located between the two previous domains.

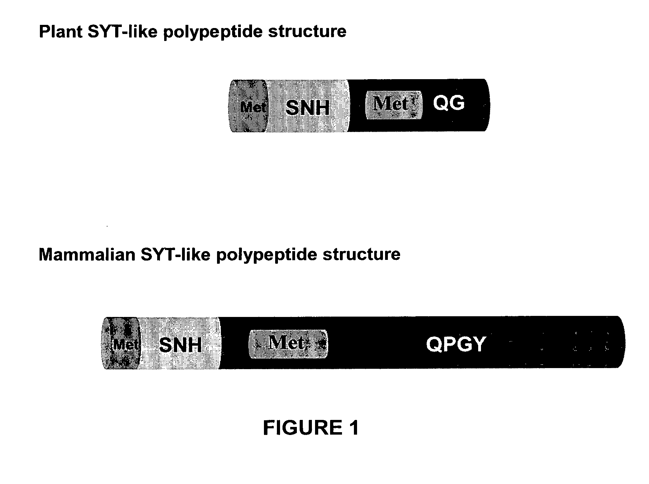

In plant SYT polypeptides, the SNH domain is well conserved. The C-terminal domain is rich in glycine and glutamine, but not in proline or tyrosine. It has therefore been named the QG-rich domain in contrast to the QPGY domain of mammals. As with mammalian SYT, a Met-rich domain may be identified N-terminally of the QG domain. The QG-rich domain may be taken to be substantially the C-terminal remainder of the polypeptide (minus the SHN domain); the Met-rich domain is typically comprised within the first half of the QG-rich (from the N-terminus to the C-terminus). A second Met-rich domain may precede the SNH domain in plant SYT polypeptides (see Fig 1).

A SYT loss-of function mutant and transgenic plants with reduced expression of SYT was reported to develop small and narrow leaves and petals, which have fewer cells (Kim HJ, Kende H (2004) Proc Nat Acad Sc 101: 13374-9).

Overexpression of AN3 in Arabidopsis thaliana resulted in plants with leaves that were 20-30% larger than those of the wild type (Horiguchi et al. (2005) Plant J 43: 68-78).

In Japanese patent application 2004-350553, a method for controlling the size of leaves in the horizontal direction is described, by controlling the expression of the AN3 gene.

WO2006/079655 discloses the use of SYT polypeptides for increasing certain yield-related traits in plants.

Surprisingly, it has now been found that modulating expression in a plant of a nucleic acid sequence encoding a SYT polypeptide gives plants having increased yield under reduced nutrient availability stress relative to control plants.

Therefore, the invention relates to a method for increasing plant yield under reduced nutrient availability stress relative to control plants, wherein said plant yield is any one or more of total seed yield, number of filled seeds, seed fill rate, TKW and harvest index, said harvest index being the ratio of seed yield divided by total biomass, wherein said method comprises modulating expression in a plant of a nucleic acid sequence encoding a synovial sarcoma translocation (SYT) polypeptide, and optionally selecting for plants having increased yield, wherein said modulated expression is effected by introducing and expressing in a plant, plant part or plant cell a nucleic acid sequence encoding a SYT polypeptide and wherein said SYT polypeptide comprises from N-terminal to C-terminal: (i) an SNH domain having at least 65% sequence identity to the SNH domain of SEQ ID NO: 58; and (ii) a Met-rich domain; and (iii) a QG-rich domain.

## Definitions

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid seauence(s)/nucleotide sequences)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | lie; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6x\log_{10}[Na^+]^a + 0.41x\%[G/C^b] - 500x[L^c]^{-1} - 0.61x\%\ formamide$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m$= 2 ($I_n$)
For 20-35 nucleotides: $T_m$= 22 + 1.46 ($I_n$)

[a] or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

<u>Splice variant</u>

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

<u>Allelic variant</u>

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

<u>Gene shuffling/Directed evolution</u>

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.Table 2a: Examples of constitutive promoters

| Gene Source | Reference |
|---|---|
| Actin | McElroy et al, Plant Cell, 2: 163-171, 1990 |

(continued)

| Gene Source | Reference |
|---|---|
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters are shown in Tables 2b to 2e below. Further examples of seed-specific promoters are given in Qing Qu and Takaiwa (Plant Biotechnol. J. 2, 113-125, 2004)..

**Table 2b:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat $\alpha$, $\beta$, $\gamma$-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice a-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice $\alpha$-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum $\alpha$-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136 rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |

(continued)

| Gene source | Reference |
|---|---|
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149; 1125-38,1998 |

**Table 2c:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2d:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2e:** Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Another example of a tissue-specific promoter is a meristem-specific promoter, which is transcriptionally active predominantly in meristematic tissue, substantially to the exclusion of any other parts of a plant, whilst still allowing for any leaky expression in these other plant parts.

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene as disclosed herein so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased epression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid as disclosed herein and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors as disclosed herein, all those constructions brought about by recombinant methods in which either

(a) the nucleic acid sequences encoding proteins useful in the methods of the invention, or
(b) genetic control sequence(s) which is operably linked with the nucleic acid sequence as disclosed herein, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the method of the present invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct as disclosed and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994). In the case of corn transformation, the preferred method is as described in either Ishida et al. (Nat. Biotechnol 14(6): 745-50, 1996) or Frame et al. (Plant Physiol 129(1): 13-22, 2002). Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens*, for example pBin19 (Bevan et al., Nucl. Acids Res. 12 (1984) 8711). Agrobacteria transformed by such a vector can then be used in known manner for the transformation of plants, such as plants used as a model, like *Arabidopsis* (*Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis*, intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

<u>T-DNA activation tagging</u>

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

<u>TILLING</u>

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer spp., Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., Cocos spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera*), *Eleusine coracana, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago safiva, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. (e.g. *Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., Poa spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma* cacao, *Trifolium* spp., *Triticosecale rimpaui, Triticum* spp. *(e.g. Triticum aestivum, Triticum durum, Triticum turgidum, Triticum hybernum, Triticum macha, Triticum sativum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

**Detailed description of the invention**

**Detailed description for the SYT polypeptide**

Surprisingly, it has now been found that modulating expression of a nucleic acid sequence encoding a SYT polypeptide increases plant yield and/or early vigour under abiotic stress relative to control plants.

Therefore, the invention relates to a method of increasing plant yield under reduced nutrient availability stress relative to control plants, wherein said plant yield is any one or more of total seed yield, number of filled seeds, seed fill rate, TKW and harvest index, said harvest index being the ratio of seed yield divided by total biomass, wherein said method comprises modulating expression in a plant of a nucleic acid sequence encoding a synovial sarcoma translocation (SYT) polypeptide, and optionally selecting for plants having increased yield, wherein said modulated expression is effected by introducing and expressing in a plant, plant part or plant cell a nucleic acid sequence encoding a SYT polypeptide and wherein said SYT polypeptide comprises from N-terminal to C-terminal: (i) an SNH domain having at least 65% sequence identity to the SNH domain of SEQ ID NO: 58; and (ii) a Met-rich domain; and (iii) a QG-rich domain.

Reference herein to "control plants" is taken to mean any suitable control plant or plants.

The "reference", "control", or "wild type" are used herein interchangeably and is preferably a subject, e.g. an organelle, a cell, a tissue, a plant, which is as similar to the subject matter of the invention as possible. The reference, control or wild type is in its genome, transcriptome, proteome or metabolome as similar as possible to the subject of the present invention. Preferably, the term "reference-" "control-" or "wild type-"-organelle, -cell, -tissue or plant, relates to an organelle, cell, tissue or plant, which is nearly genetically identical to the organelle, cell, tissue or plant, or to a part thereof, having in increasing order of preference 95%, 98%, 99,00%, 99,10%, 99,30%, 99,50%, 99,70%, 99,90%, 99,99%, 99, 999% or more genetic identity to the organelle, cell, tissue or plant, of the disclosure.. Most preferable the "reference", "control", or "wild type" is preferably a subject, e.g. an organelle, a cell, a tissue, a plant, which is genetically identical to the plant, tissue, cell, organelle used according to the method of the invention except that the nucleic acid sequences or the gene product in question is changed, modulated or modified according to the inventive method.

Preferably, any comparison between the control plants and the plants produced by the method of the invention is carried out under analogous conditions. The term "analogous conditions" means that all conditions such as culture or growing conditions, assay conditions (such as buffer composition, temperature, substrates, pathogen strain, concentrations and the like) are kept identical between the experiments to be compared.

Any reference hereinafter to a "protein useful in the methods of the invention" is taken to mean a SYT polypeptide as defined herein. Any reference hereinafter to a "nucleic acid useful in the methods of the invention" is taken to mean a nucleic acid capable of encoding such a SYT polypeptide. The nucleic acid to be introduced into a plant (and therefore useful in performing the methods of the invention) is any nucleic acid encoding the type of protein which will now be described, hereafter also named *"SYT* nucleic acid" or *"SYT* gene".

The term "sequence" relates to polynucleotides, nucleic acids, nucleic acid molecules, peptides, polypeptides and proteins, depending on the context in which the term "sequence" is used. A "coding sequence" is a nucleic acid sequence, which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleic acid sequences or genomic DNA, while introns may be present as well under certain circumstances. The term "expression" or "gene expression" is as defined above. The term "modulation" is defined above and means in relation to expression or gene expression, an increase in expression.

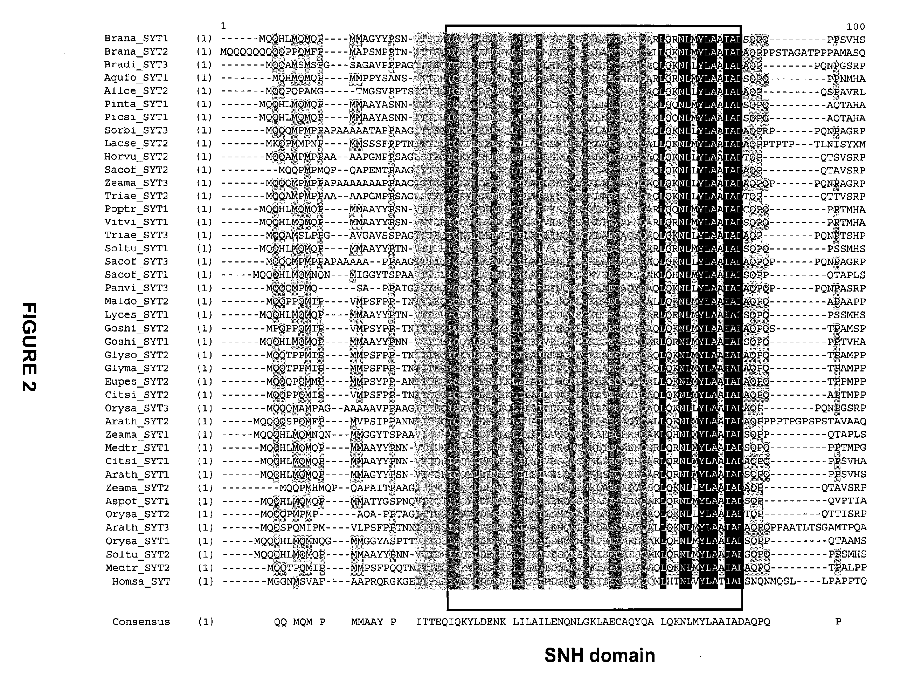

The term "SYT polypeptide" as defined herein refers to a polypeptide comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58; and (ii) a Met-rich domain; and (iii) a QG-rich domain. Preferably, the SNH domain comprises the residues shown in black in Figure 2. Further preferably, the SNH domain is as represented by SEQ ID NO: 57.

Additionally, the SYT polypeptide may comprise one or more of the following: (a) SEQ ID NO: 146; (b) SEQ ID NO: 147; and (c) a Met-rich domain at the N-terminal preceding the SNH domain.

A SYT polypeptide is encoded by a SYT nucleic acid sequence. Therefore the term "SYT nucleic acid sequence" as defined herein is any nucleic acid sequence encoding a SYT polypeptide as defined hereinabove.

The terms "motif" and "domain" are defined in the definitions section. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

SYT polypeptides may readily be identified using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Basic Local Alignment Search Tool; Altschul et al. (1990) J Mol Biol 215: 403-10) calculates percent sequence identity (%) and performs a statistical analysis of the similarity between the two sequences (E-value). Percentage identity refers to the number of identical nucleotides (or amino acids) between the two compared nucleic acid (or polypeptide) sequences over a particular length. The higher the similarity between two sequences, the lower the E-value (or in other words the lower the chance that the hit was found by chance). Computation of the E-value is well known in the art. The software for performing BLAST analysis is publicly available through the National Centre for Biotechnology Information. SYT polypeptides comprising an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58 may be identified this way. Alternatively, SYT polypeptides useful in the methods of the present invention have, in increasing order of preference, at least 30%, 35%, 40%, 45%, 50%, 55%, 60%, 65%, 70%, 75%, 80%, 85%, 90%, 95%, 96%, 97%, 98% or 99% sequence identity to the polypeptide of SEQ ID NO: 60. Minor manual editing may be performed to optimise alignment between conserved motifs, as would be apparent to a person skilled in the art. In some instances, default parameters may be adjusted to modify the stringency of the search. For example using BLAST, the statistical significance threshold (called "expect" value) for reporting matches against database sequences may be increased to show less stringent matches. In this way, short nearly exact matches may be identified. The presence of SEQ ID NO: 146 and of SEQ ID NO: 147 both comprised in the SYT polypeptides useful in the methods of the invention may be identified this way.

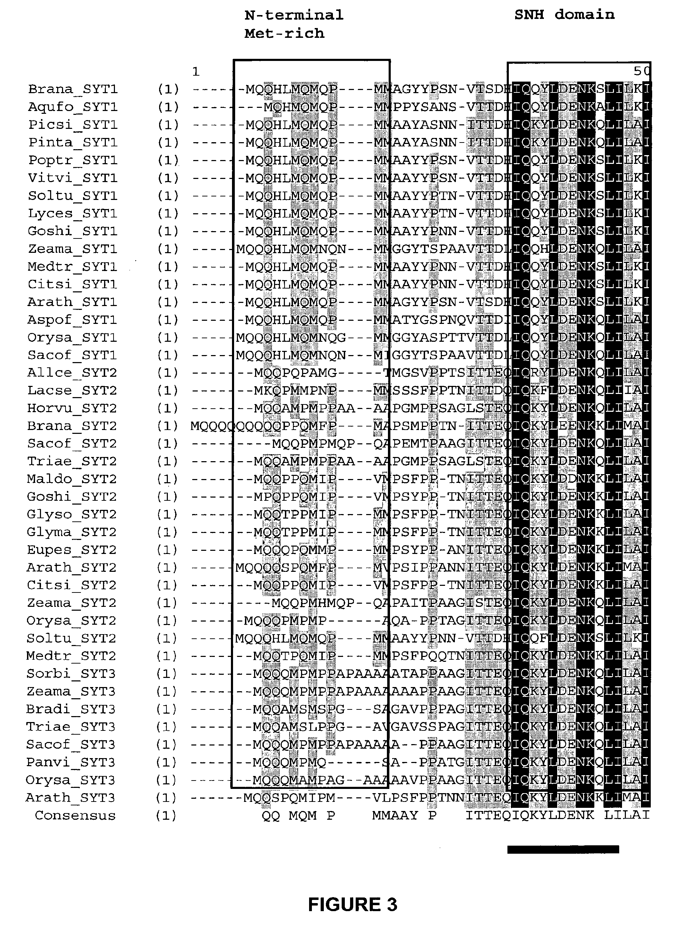

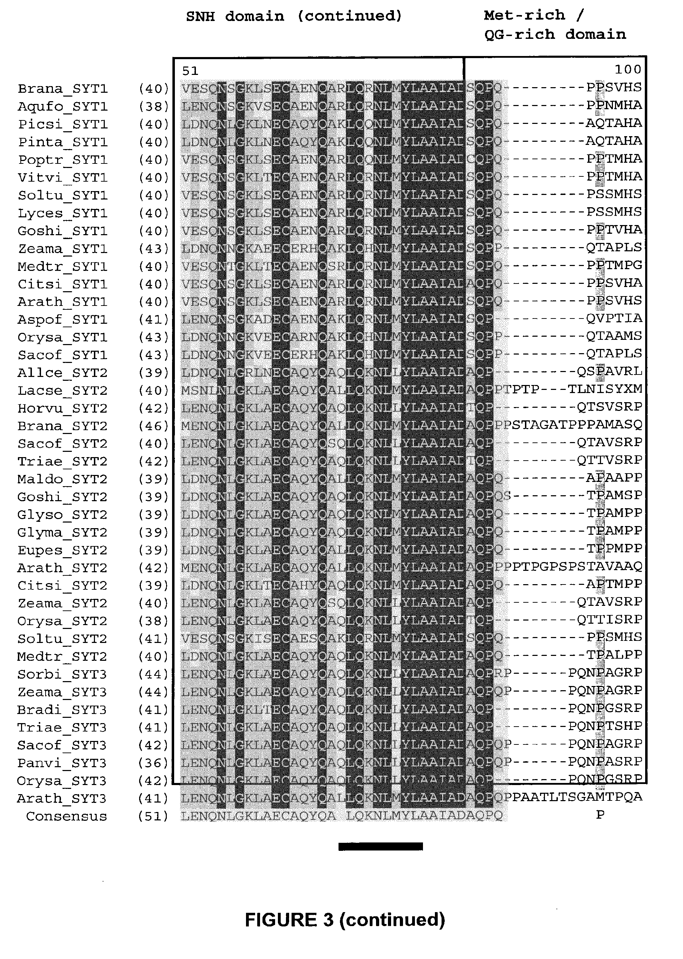

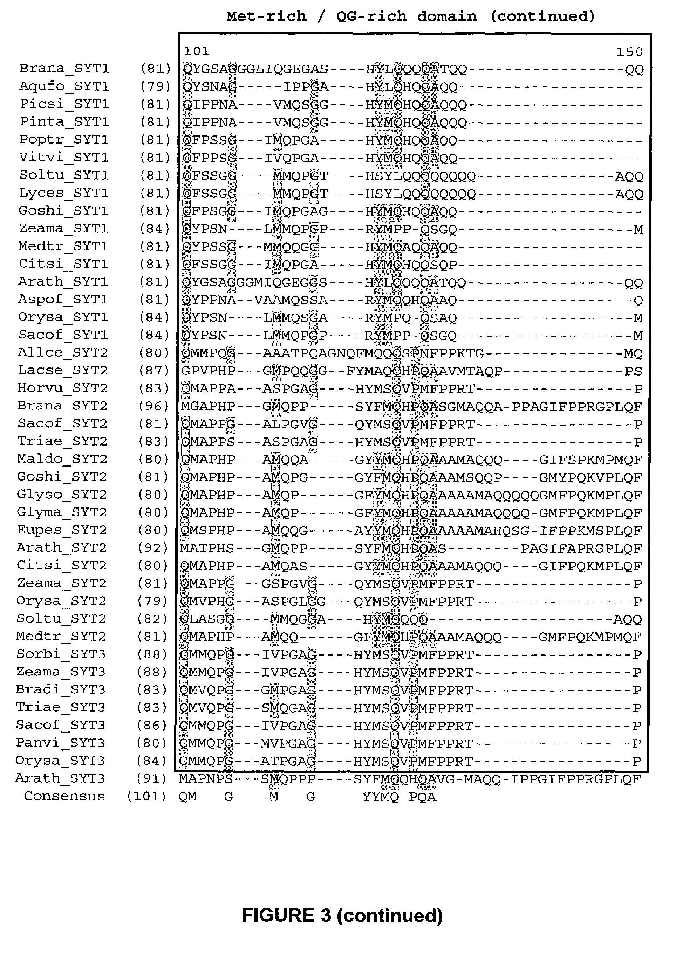

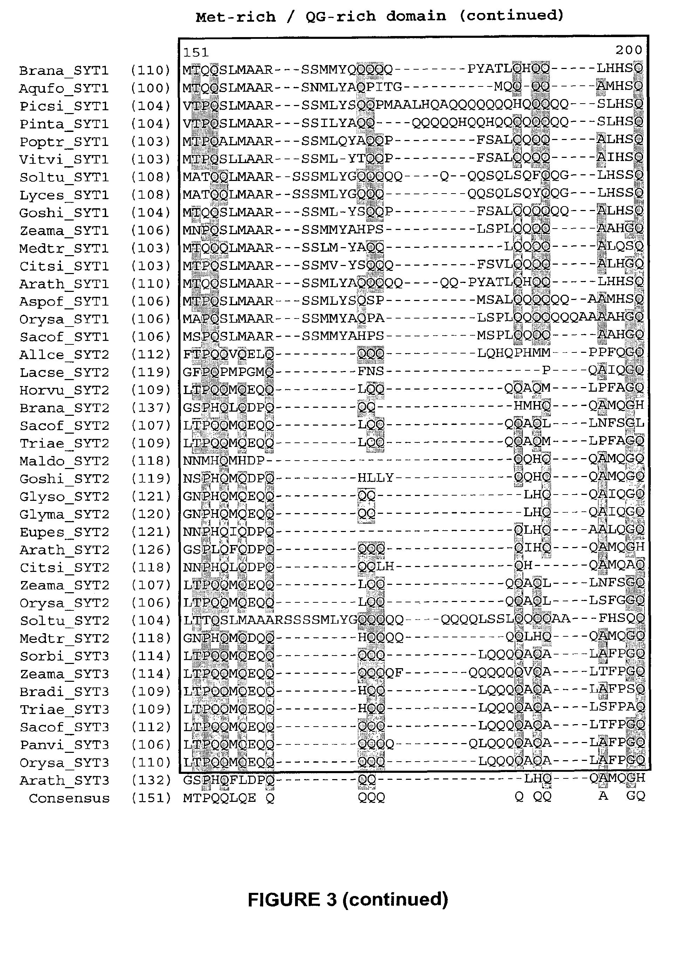

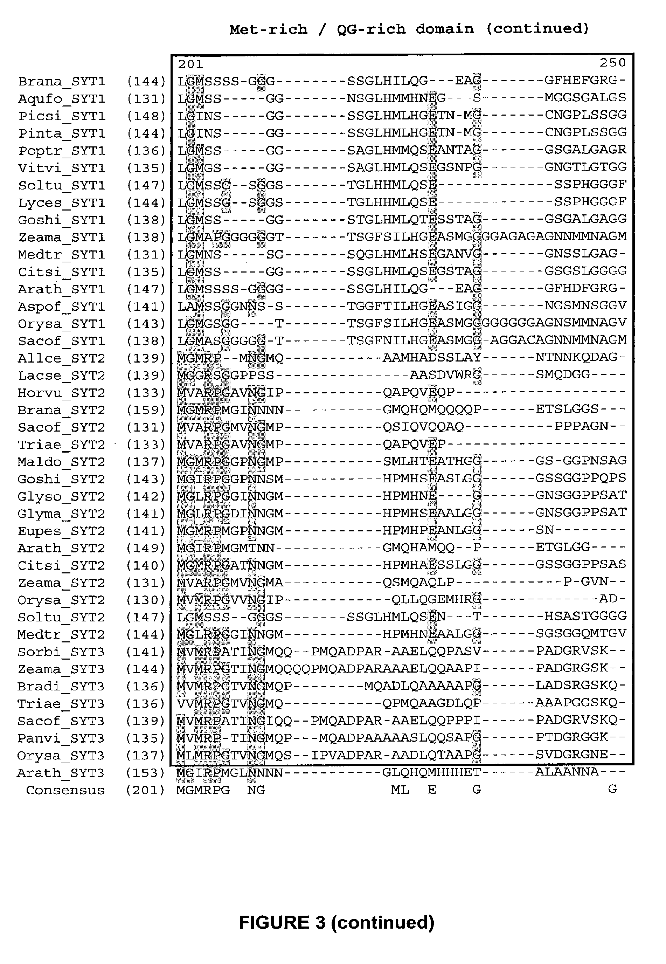

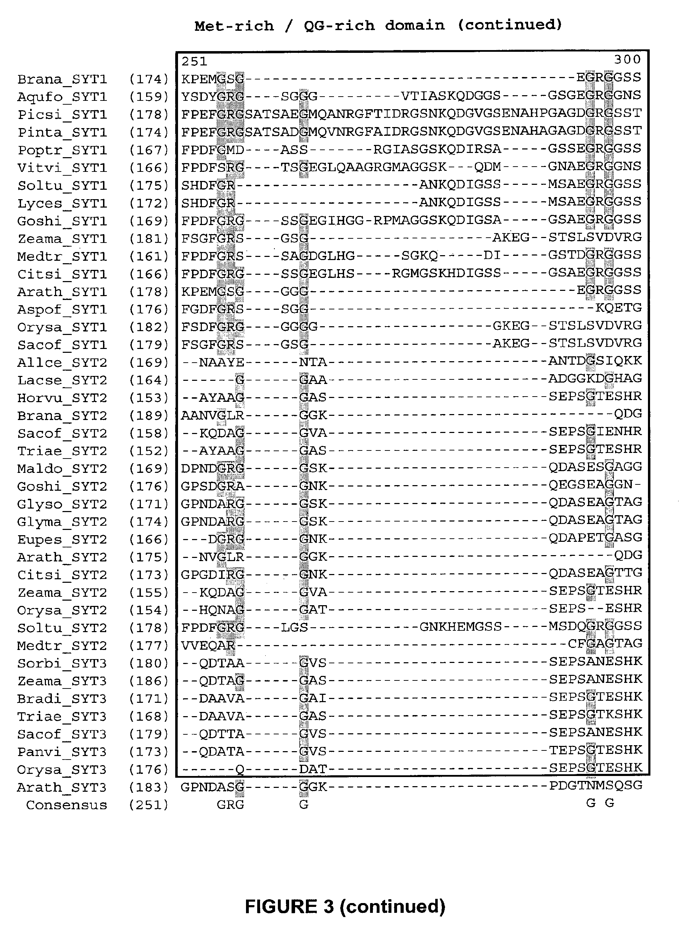

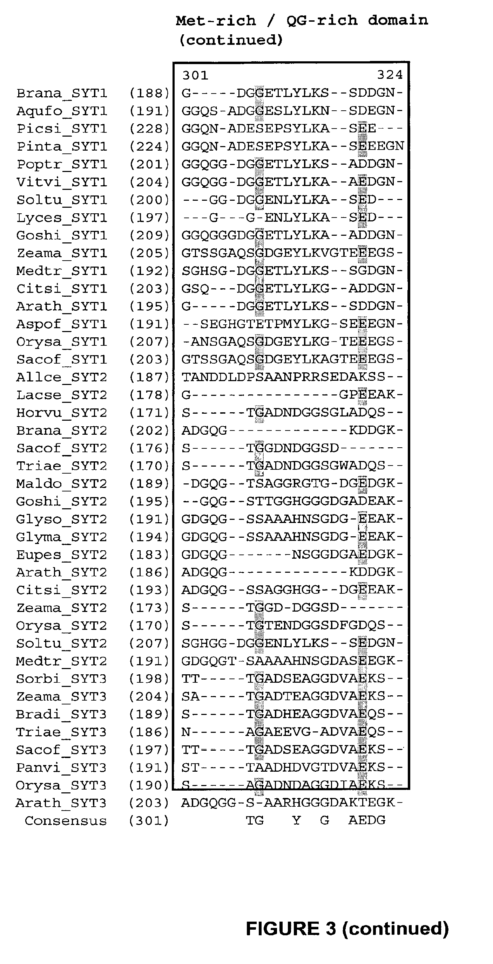

Furthermore, the presence of a Met-rich domain or a QG-rich domain may also readily be identified. As shown in Figure 3, the Met-rich domain and QG-rich domain follows the SNH domain. The QG-rich domain may be taken to be substantially the C-terminal remainder of the polypeptide (minus the SHN domain); the Met-rich domain is typically comprised within the first half of the QG-rich (from the N-term to the C-term). Primary amino acid composition (in %) to determine if a polypeptide domain is rich in specific amino acids may be calculated using software programs from the ExPASy server (Gasteiger E et al. (2003) ExPASy: the proteomics server for in-depth protein knowledge and analysis. Nucleic Acids Res 31:3784-3788), in particular the ProtParam tool. The composition of the polypeptide of interest may then be compared to the average amino acid composition (in %) in the Swiss-Prot Protein Sequence data bank (Table 3). Within this databank, the average Met (M) content is of 2.37%, the average Gln (Q) content is of 3.93% and the average Gly (G) content is of 6.93% (Table 3). As defined herein, a Met-rich domain or a QG-rich domain has Met content (in %) or a Gln and Gly content (in %) above the average amino acid composition (in %) in the Swiss-Prot Protein Sequence data bank. For example in SEQ ID NO: 60, the Met-rich domain at the N-terminal preceding the SNH domain (from amino acid positions 1 to 24) has Met content of 20.8 % and a QG-rich domain (from amino acid positions 71 to 200) has a Gln (Q) content of 18.6 % and a Gly (G) content of 21.4 %. Preferably, the Met domain as defined herein has a Met content (in %) that is at least 1.25, 1.5, 1.75, 2.0, 2.25, 2.5, 2.75, 3.0, 3.25, 3.5, 3.75, 4.0, 4.25, 4.5, 4.75, 5.0, 5.25, 5.0, 5.75, 6.0, 6.25, 6.5, 6.75, 7.0, 7.25, 7.5, 7.75, 8.0, 8.25, 8.5, 8.75, 9.0, 9.25, 9.5, 9.75, 10 or more as the average amino acid composition (in %) of said kind of protein sequences, which are included in the Swiss-Prot Protein Sequence data bank. Preferably, the QG-rich domain as defined herein has a Gln (Q) content and/or a Gly (G) content that is at least 1.25, 1.5, 1.75, 2.0, 2.25, 2.5, 2.75, 3.0, 3.25, 3.5, 3.75, 4.0, 4.25, 4.5, 4.75, 5.0, 5.25, 5.0, 5.75, 6.0, 6.25, 6.5, 6.75, 7.0, 7.25, 7.5, 7.75, 8.0, 8.25, 8.5, 8.75, 9.0, 9.25, 9.5, 9.75, 10 or more as much as the average amino acid composition (in %) of said kind of protein sequences, which are included in the Swiss-Prot Protein Sequence data bank.

Table 3: Mean amino acid composition (%) of proteins in SWISS PROT Protein Sequence data bank (July 2004):

| Residue | % | Residue | % |
|---|---|---|---|
| A = Ala | 7.80 | M = Met | 2.37 |
| C = Cys | 1.57 | N = Asn | 4.22 |
| D = Asp | 5.30 | P = Pro | 4.85 |
| E = Glu | 6.59 | Q = Gln | 3.93 |
| F = Phe | 4.02 | R = Arg | 5.29 |
| G = Gly | 6.93 | S = Ser | 6.89 |
| H = His | 2.27 | T = Thr | 5.46 |
| I = Ile | 5.91 | V = Val | 6.69 |
| K = Lys | 5.93 | W = Trp | 1.16 |
| L = Leu | 9.62 | Y = Tyr | 3.09 |

Examples of SYT polypeptides include (encoded by polynucleotide sequence accession number in parenthesis; see also Table 4 and mentioned in the sequence protocol): *Arabidopsis thaliana* Arath_SYT1 (AY102639.1) SEQ ID NO: 60, *Arabidopsis thaliana* Arath_SYT2 (AY102640.1) SEQ ID NO: 62, *Arabidopsis thaliana* Arath_SYT3 (AY102641.1) SEQ ID NO: *64, Aspergillus officinalis* Aspof_SYT (CV287542) SEQ ID NO: 66, *Brassica napus* Brana_SYT (CD823592) SEQ ID NO: 68, *Citrus sinensis* Citsi_SYT (CB290588) SEQ ID NO: 70, *Gossypium arboreum* Gosar_SYT (BM359324) SEQ ID NO: 72, *Medicago truncualta* Medtr_SYT (CA858507.1) SEQ ID NO: 74, *Oryza sativa* Orysa_SYT1 (AK058575) SEQ ID NO: 76, *Oryza sativa* Orysa_SYT2 (AK105366) SEQ ID NO: 78, *Oryza sativa* Orysa_SYT3 (BP185008) SEQ ID NO: 80, *Solanum tuberosum* Soltu_SYT (BG590990) SEQ ID NO: 82, *Zea mays* Zeama_SYT1 (BG874129.1, CA409022.1) SEQ ID NO: 84, Zea *mays* Zeama_SYT2 (AY106697) SEQ ID NO: 86, *Homo sapiens* Homsa_SYT (CAG46900) SEQ ID NO: 88, *Allium cepa* Allce_SYT2 (CF437485) SEQ ID NO: 90, *Aquilegia formosa x Aquilegia pubescens* Aqufo_SYT1 (DT758802) SEQ ID NO: 92, *Brachypodium distachyon* Bradi_SYT3 (DV480064) SEQ ID NO: 94, *Brassica napus* Brana_SYT2 (CN732814) SEQ ID NO: 96, *Citrus sinensis* Citsi_SYT2 (CV717501) SEQ ID NO: 98, *Euphorbia esula* Eupes_SYT2 (DV144834) SEQ ID NO: 100, *Glycine max* Glyma_SYT2 (BQ612648) SEQ ID NO: 102, *Glycine soya* Glyso_SYT2 (CA799921) SEQ ID NO: 104, *Gossypium hirsutum* Goshi_SYT1 (DT558852) SEQ ID NO: 106, *Gossypium hirsutum* Goshi_SYT2 (DT563805) SEQ ID NO: 108, *Hordeum vulgare* Horvu_SYT2 (CA032350) SEQ ID NO: 110, *Lactuca serriola* Lacse_SYT2 (DW110765) SEQ ID NO: 112, *Lycopersicon esculentum* Lyces_SYT1 (AW934450, BP893155) SEQ ID NO: 114, *Malus domestica* Maldo_SYT2 (CV084230, DR997566) SEQ ID NO: 116, *Medicago truncualta* Medtr_SYT2 (CA858743, BI310799, AL382135) SEQ ID NO: 118, *Panicum virgatum* Panvi_SYT3 (DN152517) SEQ ID NO: 120, *Picea sitchensis* Picsi_SYT1 (DR484100, DR478464) SEQ ID NO: 122, *Pinus taeda* Pinta_SYT1 (DT625916) SEQ ID NO: 124, *Populus tremula* Poptr_SYT1 (DT476906) SEQ ID NO: 126, *Saccharum officinarum* Sacof_SYT1 (CA078249, CA078630, CA082679, CA234526, CA239244, CA083312) SEQ ID NO: 128, *Saccharum officinarum.* Sacof_SYT2 (CA110367) SEQ ID NO: 130, *Saccharum officinarum* Sacof_SYT3 (CA161933, CA265085) SEQ ID NO: 132, *Solanum tuberosum* Soltu_SYT1 (CK265597) SEQ ID NO: 134, *Sorghum bicolor* Sorbi_SYT3 (CX611128) SEQ ID NO: 136, *Triticum aestivum* Triae_SYT2 (CD901951) SEQ ID NO: 138, *Triticum aestivum* Triae_SYT3 (BJ246754, BJ252709) SEQ ID NO: 140, *Vitis vinifera* Vitvi_SYT1 (DV219834) SEQ ID NO: 142, Zea *mays* Zeama_SYT3 (CO468901) SEQ ID NO: 144, *Brassica napus* Brana_SYT SEQ ID NO: 151, *Glycine max* Glyma_SYT SEQ ID NO: 153.

**Table 4:** Examples of nucleic acid sequences encoding SYT polypeptides

| Name | NCBI nucleotide accession number | Nucleic acid sequence SEQ ID NO | Translated polypeptide SEQ ID NO | Source |
|---|---|---|---|---|
| Arath_SYT1 | AY102639.1 | 59 | 60 | *Arabidopsis thaliana* |
| Arath_SYT2 | AY102640.1 | 61 | 62 | *Arabidopsis thaliana* |
| Arath_SYT3 | AY102641.1 | 63 | 64 | *Arabidopsis thaliana* |
| Aspof_SYT1 | CV287542 | 65 | 66 | *Aspergillus officinalis* |
| Brana_SYT1 | CD823592 | 67 | 68 | *Brassica napus* |
| Citsi_SYT1 | CB290588 | 69 | 70 | *Citrus sinensis* |
| Gosar_SYT1 | BM359324 | 71 | 72 | *Gossypium arboreum* |
| Medtr_SYT1 | CA858507.1 | 73 | 74 | *Medicago trunculata* |
| Orysa_SYT1 | AK058575 | 75 | 76 | *Oryza sativa* |
| Orysa_SYT2 | AK105366 | 77 | 78 | *Oryza sativa* |
| Orysa_SYT3 | BP185008 | 79 | 80 | *Oryza sativa* |
| Soltu_SYT2 | BG590990 | 81 | 82 | *Solanum tuberosum* |

(continued)

| Name | NCBI nucleotide accession number | Nucleic acid sequence SEQ ID NO | Translated polypeptide SEQ ID NO | Source |
|---|---|---|---|---|
| Zeama_SYT1 | BG874129.1 CA409022.1* | 83 | 84 | Zea *mays* |
| Zeama_SYT2 | AY106697 | 85 | 86 | Zea *mays* |
| Homsa_SYT | CR542103 | 87 | 88 | *Homo sapiens* |
| Alice_SYT2 | CF437485 | 89 | 90 | *Allium cepa* |
| Aqufo_SYT1 | DT758802.1 | 91 | 92 | *Aquilegia formosa x Aquilegia pubescens* |
| Bradi_SYT3 | DV480064.1 | 93 | 94 | *Brachypodium distachyon* |
| Brana_SYT2 | CN732814 | 95 | 96 | *Brassica napa* |
| Citsi_SYT2 | CV717501 | 97 | 98 | *Citrus sinensis* |
| Eupes_SYT2 | DV144834 | 99 | 100 | *Euphorbia esula* |
| Glyma_SYT2 | BQ612648 | 101 | 102 | *Glycine max* |
| Glyso_SYT2 | CA799921 | 103 | 104 | *Glycine soya* |
| Goshi_SYT1 | DT558852 | 105 | 106 | *Gossypium hirsutum* |
| Goshi_SYT2 | DT563805 | 107 | 108 | *Gossypium hirsutum* |
| Horvu_SYT2 | CA032350 | 109 | 110 | *Hordeum vulgare* |
| Lacse_SYT2 | DW110765 | 111 | 112 | *Lactuca serriola* |
| Lyces_SYT1 | AW934450.1 BP893155.1* | 113 | 114 | *Lycopersicon esculentum* |
| Maldo_SYT2 | CV084230 DR997566* | 115 | 116 | *Malus domestica* |
| Medtr_SYT2 | CA858743 B1310799.1 AL382135.1* | 117 | 118 | *Medicago trunculata* |
| Panvi_SYT3 | DN152517 | 119 | 120 | *Panicum virgatum* |
| Picsi_SYT1 | DR484100 DR478464.1 | 121 | 122 | *Picea sitchensis* |
| Pinta_SYT1 | DT625916 | 123 | 124 | *Pinus faeda* |

(continued)

| Name | NCBI nucleotide accession number | Nucleic acid sequence SEQ ID NO | Translated polypeptide SEQ ID NO | Source |
|---|---|---|---|---|
| Poptr_SYT1 | DT476906 | 125 | 126 | *Populus tremula* |
| Sacof_SYT1 | CA078249.1<br><br>CA078630<br>CA082679<br>CA234526<br>CA239244<br>CA083312* | 127 | 128 | *Saccharum officinarum* |
| Sacof_SYT2 | CA110367 | 129 | 130 | *Saccharum officinarum* |
| Sacof_SYT3 | CA161933.1<br><br>CA265085* | 131 | 132 | *Saccharum officinarum* |
| Soltu_SYT1 | CK265597 | 133 | 134 | *Solanum tuberosum* |
| Sorbi_SYT3 | CX611128 | 135 | 136 | *Sorghum bicolor* |
| Triae_SYT2 | CD901951 | 137 | 138 | *Triticum aestivum* |
| Triae_SYT3 | BJ246754<br><br>BJ252709* | 139 | 140 | *Triticum aestivum* |
| Vitvi_SYT1 | DV219834 | 141 | 142 | *Vitis vinifera* |
| Zeama_SYT3 | CO468901 | 143 | 144 | *Zea mays* |
| Brana_SYT | NA | 150 | 151 | *Brassica napus* |
| Glyma_SYT | NA | 152 | 153 | *Glycine max* |
| *Compiled from cited accessions<br>NA: not available (proprietary) | | | | |

Examples of nucleic acids encoding SYT polypeptides are given in Table 4 above. Such nucleic acids are useful in performing the methods of the invention. The amino acid sequences given in Table 4 above are example sequences of orthologues and paralogues of the SYT polypeptide represented by SEQ ID NO: 58, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table 4 above) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 59 or SEQ ID NO: 60, the second BLAST would therefore be against *Arabidopsis* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

It is to be understood that sequences falling under the definition of a "SYT polypeptide" are not to be limited to the polypeptides given in Table 4 (and mentioned in the sequence protocol), but that any polypeptide comprising from N-terminal to C-terminal: (i) an SNH domain having at least 65%, 70%, 75%, 80%, 85%, 90%, 91%, 92%, 93%, 94%, 95%, 96%, 97%, 98%, 99% sequence identity to the SNH domain of SEQ ID NO: 58; and (ii) a Met-rich domain; and (iii) a QG-rich domain may be suitable in performing the methods of the invention. Preferably, the SNH domain comprises the residues shown in black in Figure 2. Additionally, the SYT polypeptide may comprise one or more of the following: (a) SEQ ID NO: 146; (b) SEQ ID NO: 147; and (c) a Met-rich domain at the N-terminal preceding the SNH domain. Most preferably, the SYT polypeptide is as represented by SEQ ID NO: 60, SEQ ID NO: 151 or SEQ ID NO: 153.

A SYT polypeptide typically interacts with GRF (growth-regulating factor) polypeptides in yeast two-hybrid systems. Yeast two-hybrid interaction assays are well known in the art (see Field et al. (1989) Nature 340(6230): 245-246). For example, the SYT polypeptide as represented by SEQ ID NO: 4 is capable of interacting with AtGRF5 and with AtGRF9.

In a further embodiment the disclosure provides an isolated nucleic acid sequence comprising a nucleic acid sequence selected from the group consisting of:

(a) an isolated nucleic acid sequence as depicted in SEQ ID NO: 150 and SEQ ID NO: 152;
(b) an isolated nucleic acid sequence encoding the polypeptide as depicted in SEQ ID NO: 151 and SEQ ID NO: 153;
(c) an isolated nucleic acid sequence whose sequence can be deduced from a polypeptide as depicted in SEQ ID NO: 151 and SEQ ID NO: 153 as a result of the degeneracy of the genetic code;
(d) an isolated nucleic acid sequence which encodes a polypeptide which has at least 70% identity with the polypeptide encoded by the nucleic acid sequence of (a) to (c);
(e) an isolated nucleic acid sequence encoding a homologue, derivative or active fragment of the polypeptide as depicted in SEQ ID NO: 151 and SEQ ID NO: 153, which homologue, derivative or fragment is of plant origin and comprises advantageously from N-terminal to C-terminal:

    (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58;
    (ii) a Met-rich domain;
    (iii) a QG-rich domain;

(f) an isolated nucleic acid sequence capable of hybridising with a nucleic acid of (a) to (c) above, or its complement, wherein the hybridising sequence or the complement thereof encodes the plant protein of (a) to (e);

whereby modified expression of the nucleic acid sequence increases yield and/or early vigour in plants under abiotic stress compared to control plants.

The nucleic acid sequences encoding SYT polypeptides as given in Table 4, or orthologues or paralogues of any of the aforementioned SEQ ID NOs need not be full-length nucleic acid sequences, since performance of the methods of the invention does not rely on the use of full-length nucleic acid sequences.

SYT nucleic acid variants may also be suitable in practising the methods of the invention. Variant SYT nucleic acid sequences typically are those having the same function as a naturally occurring SYT nucleic acid sequence, which can be the same biological function or the function of increasing yield and/or early vigour when expression of the nucleic acid sequence is modulated in a plant under abiotic stress relative to control plants. Such variants include portions of a SYT nucleic acid sequence, splice variants of a SYT nucleic acid sequence, allelic variants of a SYT nucleic acid sequence, variants of a SYT nucleic acid sequence obtained by gene shuffling and/or nucleic acid sequences capable of hybridising with a SYT nucleic acid sequence as defined below. Preferably, the nucleic acid variant is a variant of a nucleic acid sequence is as represented by SEQ ID NO: 59, SEQ ID NO: 150 or SEQ ID NO: 152. The terms hybridising sequence, splice variant, allelic variant and gene shuffling are as described herein.

Nucleic acids encoding SYT polypeptides need not be full-length nucleic acids, since performance of the methods of the invention does not rely on the use of full-length nucleic acid sequences. According to the present invention, there is provided a method for enhancing yield and/or early vigour in plants under abiotic stress, comprising introducing and expressing in a plant a portion of any one of the nucleic acid sequences given in Table 4, or a portion of a nucleic acid encoding an orthologue, paralogue or homologue of any of the amino acid sequences given in Table 4.

The term portion as used herein refers to a piece of DNA encoding a polypeptide comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58 and (ii) a Met-rich domain; and (iii) a QG-rich domain. A portion may be prepared, for example, by making one or more deletions to a SYT nucleic acid sequence. The portions may be used in isolated form or they may be fused to other coding (or non coding) sequences in order to, for example, produce a polypeptide that combines several activities. When fused to other coding sequences, the resulting polypeptide produced upon translation may be bigger than that predicted for the SYT fragment. Preferably, the portion is a portion of a nucleic acid sequence as represented by any one given in Table 4, or orthologues or paralogues of any of the aforementioned SEQ ID NOs. Most preferably the portion is a portion of a nucleic acid sequence is as represented by SEQ ID NO: 59, SEQ ID NO: 150 or SEQ ID NO: 152.

Another variant of a SYT nucleic acid sequence is a nucleic acid sequence capable of hybridising under reduced stringency conditions, preferably under stringent conditions, with a SYT nucleic acid sequence as hereinbefore defined, which hybridising sequence encodes a SYT polypeptide or a portion as defined hereinabove.

According to the present invention, there is provided a method for enhancing yield and/or early vigour in plants under abiotic stress, comprising introducing and expressing in a plant a nucleic acid capable of hybridizing to any one of the nucleic acids given in Table 4, or comprising introducing and expressing in a plant a nucleic acid capable of hybridising to a nucleic acid encoding an orthologue, paralogue or homologue of any of the nucleic acid sequences given in Table 4.

Hybridising sequences useful in the methods of the invention encode a SYT polypeptide comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58 and (ii) a Met-rich domain; and (iii) a QG-rich domain. Preferably, the hybridising sequence is one that is capable of hybridising to a nucleic acid sequence as given in Table 4, or orthologues or paralogues of any of the aforementioned SEQ ID NOs, or to a portion of any of the aforementioned sequences as defined hereinabove. Most preferably the hybridizing sequence is one that is capable of hybridising to a nucleic acid sequence is as represented by SEQ ID NO: 59, SEQ ID NO: 150, SEQ ID NO: 152, or to portions (or probes) thereof. Methods for designing probes are well known in the art. Probes are generally less than 1000 bp in length, preferably less than 500 bp in length. Commonly, probe lengths for DNA-DNA hybridisations such as Southern blotting, vary between 100 and 500 bp, whereas the hybridising region in probes for DNA-DNA hybridisations such as in PCR amplification generally are shorter than 50 but longer than 10 nucleotides. The hybridising sequence is typically at least 100, 125, 150, 175, 200 or 225 nucleotides in length, preferably at least 250, 275, 300, 325, 350, 375, 400, 425, 450 or 475 nucleotides in length, further preferably least 500, 525, 550, 575, 600, 625, 650, 675, 700 or 725 nucleotides in length, or as long as a full length SYT cDNA.

Another nucleic acid variant useful in the methods of the invention is a splice variant encoding a SYT polypeptide as defined hereinabove. Preferred splice variants are splice variants of the SYT nucleic acid sequences as given in Table 4, or orthologues or paralogues of any of the aforementioned SEQ ID NOs. Most preferred is a splice variant of a SYT nucleic acid sequence as represented by SEQ ID NO: 59, SEQ ID NO: 150 or SEQ ID NO: 152.

According to the present invention, there is provided a method for enhancing yield and/or early vigour in plants under abiotic stress, comprising introducing and expressing in a plant a splice variant of any one of the nucleic acid sequences given in Table 4, or a splice variant of a nucleic acid encoding an orthologue, paralogue or homologue of any of the amino acid sequences given in Table 4.

Another nucleic acid variant useful in performing the methods of the invention is an allelic variant of a nucleic acid encoding a SYT polypeptide as defined hereinabove. Allelic variants exist in nature, and encompassed within the methods of the present invention is the use of these natural alleles. Preferred allelic variants are allelic variants of the SYT nucleic acid sequences as given in Table 4, or orthologues or paralogues of any of the aforementioned SEQ ID NOs. Most preferred is a splice variant of a SYT nucleic acid sequence as represented by SEQ ID NO: 59.

According to the present invention, there is provided a method for enhancing yield and/or early vigour in plants under abiotic stress, comprising introducing and expressing in a plant an allelic variant of any one of the nucleic acids given in Table 4, or comprising introducing and expressing in a plant an allelic variant of a nucleic acid encoding an orthologue, paralogue or homologue of any of the amino acid sequences given in Table 4.

A further nucleic acid variant useful in the methods of the invention is a nucleic acid variant encoding a SYT polypeptide obtained by gene shuffling (or directed evolution).

Another nucleic acid variant useful in the methods of the invention is a nucleic acid variant encoding a SYT polypeptide obtained by site-directed mutagenesis. Site-directed mutagenesis may be used to generate variants of SYT nucleic acid sequences. Several methods are available to achieve site-directed mutagenesis, the most common being PCR based methods (Current Protocols in Molecular Biology. Wiley Eds).

According to the present invention, there is provided a method for enhancing yield and/or early vigour in plants under abiotic stress, comprising introducing and expressing in a plant a variant of any one of the nucleic acid sequences given in Table 4, or comprising introducing and expressing in a plant a variant of a nucleic acid encoding an orthologue, paralogue or homologue of any of the amino acid sequences given in Table 4, which variant nucleic acid is obtained by gene shuffling or site-directed mutagenesis.

The following SYT nucleic acid variants are examples of variants suitable in practising the methods of the invention:

(i) a portion of a SYT nucleic acid sequence;
(ii) a nucleic acid sequence capable of hybridising with a SYT nucleic acid sequence;
(iii) a splice variant of a SYT nucleic acid sequence;
(iv) an allelic variant of a SYT nucleic acid sequence;
(v) a SYT nucleic acid sequence obtained by gene shuffling;
(vi) a SYT nucleic acid sequence obtained by site-directed mutagenesis.

Also useful in the methods of the invention are nucleic acids encoding homologues of SYT polypeptides as given in Table 4, or orthologues or paralogues of any of the aforementioned SEQ ID NOs.

**[0140]** Also useful in the methods of the invention are nucleic acids encoding derivatives of any one of the SYT nucleic acid sequences as given in Table 4, or orthologues or paralogues of any of the aforementioned SEQ ID NOs. Derivatives of orthologues or paralogues of any of the aforementioned SEQ ID NOs are further examples that may be suitable for use in the methods of the invention.

SYT nucleic acid sequences may be derived from any artificial source or natural source, such as plants, algae, fungi or animals. The nucleic acid sequence may be modified from its native form in composition and/or genomic environment through deliberate human manipulation. Preferably the nucleic acid sequence encoding a SYT polypeptide is of plant origin. The nucleic acid sequence may be isolated from a dicotyledonous species, preferably from the family Brassicaceae, further preferably from *Arabidopsis thaliana* or *Brassica napus.* Alternatively, nucleic acid sequence may be isolated from the family Fabaceae, preferably from *Glycine max.* More preferably, the SYT nucleic acid sequence isolated from:

(a) *Arabidopsis thaliana* is as represented by SEQ ID NO: 59 and the SYT polypeptide as represented by SEQ ID NO: 60;
(b) *Brassica napus* is as represented by SEQ ID NO: 150 and the SYT polypeptide as represented by SEQ ID NO: 151;
(c) *Glycine max* is as represented by SEQ ID NO: 152 and the SYT polypeptide as represented by SEQ ID NO: 153.

The terms "yield", "seed yield" and "early vigour" are defined above. The terms "increased", "improved", "enhanced", "amplified", "extended", or "rised" are interchangeable and are defined hereinabove. Taking corn as an example, a yield increase may be manifested as one or more of the following: increase in the number of plants per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may be manifested by an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

An increase in yield may also result in modified architecture, or may occur as a result of modified architecture.

According to a preferred feature, performance of the methods of the invention result in plants having increased biomass, increased seed yield and/or early vigour under abiotic stress relative to control plants. Therefore, according to the present invention, there is provided a method for increasing biomass, seed yield and/or early vigour in a plant under abiotic stress relative to control plants, which method comprises modulating expression in a plant of a nucleic acid sequence encoding a SYT polypeptide. Preferably, by increased biomass is herein taken to mean the aboveground part (or leafy biomass) during plant development and at maturity. Preferably, by increased seed yield is herein taken to mean any one of the following: total seed yield, number of filled seeds, seed fill rate, TKW and harvest index.

Since the transgenic plants according to the present disclosure have increased yield under abiotic stress, it is likely that these plants exhibit an increased growth rate under abiotic stress (during at least part of their life cycle, for example at seedling stage for early vigour), relative to the growth rate of control plants at a corresponding stage in their life cycle. The increased growth rate may be specific to one or more parts of a plant (including seeds), or may be throughout substantially the whole plant. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible. If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the planting and harvesting of corn plants followed by, for example, the planting and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others. The growth rate of plants is measured under abiotic stress such as salt stress; water stress (drought or excess water); reduced nutrient availability stress; temperature stresses caused by atypical hot or cold/freezing temperatures; oxidative stress; metal stress; chemical toxicity stress; or combinations thereof.

Performance of the methods of the invention gives plants having an increased growth rate under abiotic stress relative to control plants. Therefore, according to the present invention, there is provided a method for increasing growth rate in plants under abiotic stress relative to control plants, which method comprises modulating expression in a plant of a nucleic acid sequence encoding a SYT polypeptide.

Plants typically respond to exposure to stress by growing more slowly. In conditions of severe stress, the plant may even stop growing altogether. Mild stress leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the typical stresses to which a plant may be exposed. These stresses may be the everyday biotic and/or abiotic (environmental) stresses to which a plant is exposed. Biotic stresses are typically those stresses caused by pathogens, such as bacteria, viruses, fungi, nematodes and insects. Typical abiotic or environmental stresses comprise any one or more of: salt stress, water stress (drought or excess water), reduced nutrient availability stress, temperature stresses caused by atypical hot or cold/freezing temperatures, oxidative stress, metal stress or chemical toxicity stress.

Performance of the methods according to the present invention results in plants having increased yield and/or early vigour under abiotic stress relative to control plants. As reported in Wang et al., (Planta (2003) 218: 1-14), abiotic stress leads to a series of morphological, physiological, biochemical and molecular changes that adversely affect plant growth and productivity. Drought, salinity, extreme temperatures and oxidative stress are often interconnected and may induce growth and cellular damage through similar mechanisms. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturation of functional and structural proteins. Reduced nutrient availability, in particular reduced nitrogen availability, is a major limiting factor for plant growth, for example through the reduced availability of amino acids for protein synthesis. As a consequence, these diverse environmental stresses often activate similar cell signaling pathways and cellular responses, such as the production of stress proteins, up-regulation of anti-oxidants, accumulation of compatible solutes and growth arrest.

Since diverse environmental stresses activate similar pathways, the exemplification with salt stress should not be seen as a limitation to salt stress, but more as a screen to indicate the involvement of SYT polypeptides in abiotic stresses in general. A review in TRENDS in Plant Science (Jian-Kang Zhu, Vol. 6, No. 2, Feb. 2001) confirms that transgenic plants performing better under salt stress often also perform better under other stresses including chilling, freezing, heat and drought. A particularly high degree of "cross talk" is reported between drought stress and high-salinity stress (Rabbani et al., Plant Physiology, December 2003, Vol. 133, pp. 1755-1767). Therefore, it would be apparent that a SYT polypeptide (as defined herein) would, along with its usefulness in increasing yield and/or early vigour in plants under salt stress, also find use in increasing yield and/or early vigour of the plant under various other abiotic stresses.

The term "abiotic stress" as defined herein is taken to mean any one or more of: salt stress, water stress (drought or excess water), reduced nutrient availability stress, temperature stresses caused by atypical hot or cold/freezing temperatures, oxidative stress, metal stress or chemical toxicity stress. The term salt stress is not restricted to common salt (NaCl), but may be any one or more of: NaCl, KCl, LiCl, $MgCl_2$, $CaCl_2$, amongst others.

Performance of the methods of the invention gives plants having increased yield under reduced nutrient availability stress relative to control plants.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

A preferred method for introducing a genetic modification is to introduce and express in a plant a nucleic acid sequence encoding a SYT polypeptide. A SYT polypeptide is defined as a polypeptide comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58; and (ii) a Met-rich domain; and (iii) a QG-rich domain. Preferably, the SNH domain comprises the residues shown in black in Figure 2. Further preferably, the SNH domain is represented by SEQ ID NO: 57.

According to a preferred aspect of the present invention, the modulated expression of a SYT nucleic acid sequence is increased expression. The increase in expression may lead to raised SYT mRNA or polypeptide levels, which could equate to raised activity of the SYT polypeptide; or the activity may also be raised when there is no change in polypeptide levels, or even when there is a reduction in polypeptide levels. This may occur when the intrinsic properties of the SYT polypeptide are altered, for example, by making mutant versions that are more active that the wild type polypeptide. Methods for increasing or reducing expression of genes or gene products are known in the art.

The diclosure also provides genetic constructs and vectors to facilitate introduction and/or expression of the nucleotide sequences useful in the methods according to the invention.

Therefore, there is provided a genetic construct comprising:

(i) A nucleic acid sequence encoding a SYT polypeptide, as defined hereinabove, or a nucleic acid sequence as represented by SEQ ID NO: 150 or SEQ ID NO: 152;
(ii) One or more control sequences capable of driving expression of the nucleic acid sequence of (i); and optionally
(iii) A transcription termination sequence.

A preferred construct is one where the control sequence is a promoter derived from a plant, preferably from a monocotyledonous plant if a monocotyledonous is to be transformed.

Constructs useful in the methods according to the present invention may be constructed using recombinant DNA technology well known to persons skilled in the art. The genetic constructs may be inserted into vectors, which may be commercially available, suitable for transforming into plants and suitable for expression of the gene of interest in the transformed cells.

Plants are transformed with a vector comprising the sequence of interest (i.e., a nucleic acid sequence encoding a SYT polypeptide). The sequence of interest is operably linked to one or more control sequences (at least to a promoter).

Advantageously, any type of promoter may be used to drive expression of the nucleic acid sequence.

Useful promoters are constitutive promoters (Benfey et al., EMBO J. 8 (1989) 2195-2202), such as those which originate from plant viruses, such as 35S CAMV (Franck et al., Cell 21 (1980) 285-294), 19S CaMV (see also US 5352605 and WO 84/02913), 34S FMV (Sanger et al., Plant. Mol. Biol., 14, 1990: 433-443), the parsley ubiquitin promoter, or plant promoters such as the Rubisco small subunit promoter described in US 4,962,028 or the plant promoters PRP1 [Ward et al., Plant. Mol. Biol. 22 (1993)], SSU, PGEL1, OCS [Leisner (1988) Proc Natl Acad Sci USA 85(5): 2553-2557], lib4, usp, mas [Comai (1990) Plant Mol Biol 15 (3):373-381], STLS1, ScBV (Schenk (1999) Plant Mol Biol 39(6):1221-1230), B33, SAD1 or SAD2 (flax promoters, Jain et al., Crop Science, 39 (6), 1999: 1696-1701) or nos [Shaw et al. (1984) Nucleic Acids Res. 12(20):7831-7846]. Further examples of constitutive plant promoters are the sugarbeet V-ATPase promoters (WO 01/14572). Examples of synthetic constitutive promoters are the Super promoter (WO 95/14098) and promoters derived from G-boxes (WO 94/12015). If appropriate, chemical inducible promoters may furthermore also be used, compare EP-A 388186, EP-A 335528, WO 97/06268. Stable, constitutive expression of the polypeptides according to the disclosure a plant can be advantageous. However, inducible expression of the polypeptide of the disclosure is advantageous, if a late expression before the harvest is of advantage, as metabolic manipulation may lead to plant growth retardation.

The expression of plant genes can also be facilitated via a chemical inducible promoter (for a review, see Gatz 1997, Annu. Rev. Plant Physiol. Plant Mol. Biol., 48:89-108). Chemically inducible promoters are particularly suitable when it is desired to express the gene in a time-specific manner. Examples of such promoters are a salicylic acid inducible promoter (WO 95/19443), and abscisic acid-inducible promoter (EP 335 528), a tetracyclin-inducible promoter (Gatz et al. (1992) Plant J. 2, 397-404), a cyclohexanol- or ethanol-inducible promoter (WO 93/21334) or others as described herein.

Other suitable promoters are those that react to biotic or abiotic stress, for example the pathogen-induced PRP1 gene promoter (Ward et al., Plant. Mol. Biol. 22 (1993) 361-366), the tomato heat-inducible hsp80 promoter (US 5,187,267), the potato chill-inducible alpha-amylase promoter (WO 96/12814) or the wound-inducible pinII promoter (EP-A-0 375 091) or others as described herein.

Preferred promoters are in particular those which bring gene expression in tissues and organs, in seed cells, such as endosperm cells and cells of the developing embryo. Suitable promoters are the oilseed rape napin gene promoter (US 5,608,152), the Vicia faba USP promoter (Baeumlein et al., Mol Gen Genet, 1991, 225 (3): 459-67), the Arabidopsis oleosin promoter (WO 98/45461), the Phaseolus vulgaris phaseolin promoter (US 5,504,200), the Brassica Bce4 promoter (WO 91/13980), the bean arc5 promoter, the carrot DcG3 promoter, or the Legumin B4 promoter (LeB4; Baeumlein et al., 1992, Plant Journal, 2 (2): 233-9), and promoters which bring about the seed-specific expression in monocotyledonous plants such as maize, barley, wheat, rye, rice and the like. Advantageous seed-specific promoters are the sucrose binding protein promoter (WO 00/26388), the phaseolin promoter and the napin promoter. Suitable promoters which must be considered are the barley lpt2 or lpt1 gene promoter (WO 95/15389 and WO 95/23230), and the promoters described in WO 99/16890 (promoters from the barley hordein gene, the rice glutelin gene, the rice oryzin gene, the rice prolamin gene, the wheat gliadin gene, the wheat glutelin gene, the maize zein gene, the oat glutelin gene, the sorghum kasirin gene and the rye secalin gene). Further suitable promoters are Amy32b, Amy 6-6 and Aleurain [US 5,677,474], Bce4 (oilseed rape) [US 5,530,149], glycinin (soya) [EP 571 741], phosphoenolpyruvate carboxylase (soya) [JP 06/62870], ADR12-2 (soya) [WO 98/08962], isocitrate lyase (oilseed rape) [US 5,689,040] or $\alpha$-amylase (barley) [EP 781 849]. Other promoters which are available for the expression of genes in plants are leaf-specific promoters such as those described in DE-A 19644478 or light-regulated promoters such as, for example, the pea petE promoter.

Further suitable plant promoters are the cytosolic FBPase promoter or the potato ST-LSI promoter (Stockhaus et al., EMBO J. 8, 1989, 2445), the Glycine max phosphoribosylpyrophosphate amidotransferase promoter (GenBank Accession No. U87999) or the node-specific promoter described in EP-A-0 249 676.

In one embodiment, the SYT nucleic acid sequence is operably linked to a constitutive promoter. A constitutive promoter is transcriptionally active during most, but not necessarily all, phases of its growth and development and is substantially ubiquitously expressed. Preferably the promoter is derived from a plant, more preferably the promoter is from a monocotyledonous plant if a monocotyledonous plant is to be transformed. Further preferably, the constitutive promoter is a GOS2 promoter that is represented by a nucleic acid sequence substantially similar to SEQ ID NO: 145 or SEQ ID NO: 56. Most preferably the GOS2 promoter is as represented by SEQ ID NO: 56 or SEQ ID NO: 145 . It should be clear that the applicability of the present invention is not restricted to the SYT nucleic acid sequence represented by SEQ ID NO: 59, nor is the applicability of the invention restricted to expression of a SYT nucleic acid sequence when driven by a GOS2 promoter. Examples of other constitutive promoters that may also be used to drive expression of a SYT nucleic acid sequence are shown in the definitions section.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The disclosure also encompasses plants obtainable by the methods according to the present invention. The disclosure therefore provides plants, plant parts and plant cells obtainable by the methods according to the present invention, which plants have introduced therein a SYT nucleic acid sequence and which plants, plant parts and plant cells are preferably from a crop plant, further preferably from a monocotyledonous plant.

The invention also provides a method for the production of transgenic plants having increased yield and/or early vigour under abiotic stress, comprising introduction and expression in a plant of a SYT nucleic acid sequence.

More specifically, the present invention provides a method for the production of transgenic plants, preferably monocotyledonous plants, having any one or more of increased total seed yield, increased number of filled seeds, increased seed fill rate, increased TKW and increased harvest index, said harvest index being the ratio of seed yield divided by total biomass, relative to corresponding wild type plants under reduced nutrient availability stress, which method comprises:

(i) introducing and expressing in a plant or plant cell a nucleic acid sequence encoding a SYT polypeptide as defined in any of claims 1-2, 4, 5 and 7; and
(ii) cultivating the plant cell under conditions promoting plant growth and development.

The nucleic acid of (i) may be any of the nucleic acids capable of encoding a SYT polypeptide, as defined in any of claims 1-2, 4, 5 and 7, such as nucleic acid sequence as represented by SEQ ID NO: 150 or SEQ ID NO: 152.

Subsequent generations of the plants obtained from cultivating step (ii) may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The nucleic acid sequence may be introduced directly into a plant cell or into the plant itself (including introduction into a tissue, organ or any other part of a plant). According to a preferred feature of method of the present invention, the nucleic acid sequence is introduced into a plant by transformation.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present disclosure clearly extends to any plant cell or plant produced by any of the methods described herein, and to all plant parts and propagules thereof. The present disclosure extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention. The disclosure also includes host cells containing an isolated SYT nucleic acid sequence. Preferred host cells according to the disclosure are plant cells. The disclosure also extends to harvestable parts of a plant such as, but not limited to seeds, leaves, fruits, flowers, stem cultures, roots, rhizomes, tubers and bulbs. The disclosure furthermore relates to products derived, preferably directly derived, from a harvestable part of such a plant, such as dry pellets or powders, meal, oil, fat and fatty acids, starch or proteins.

According to a preferred feature of the method of the invention, the modulated expression is increased expression. Methods for increasing expression of nucleic acids or genes, or gene products, are well documented in the art and examples are provided in the definitions section.

Alternatively, the expression of a nucleic acid sequence encoding a SYT polypeptide may be modulated by introducing a genetic modification, for example, by any one (or more) of the following techniques: T-DNA activation, TILLING, homologous recombination, or by introducing and expressing in a plant a nucleic acid sequence encoding a SYT polypeptide. Following introduction of the genetic modification, there follows a step of selecting for modulated expression of a nucleic acid sequence encoding a SYT polypeptide, which modulated expression gives plants having increased yield and/or early vigour under abiotic stress.

One such technique is T-DNA activation tagging. The promoter to be introduced may be any promoter capable of driving expression of a gene in the desired organism, in this case a plant. For example, constitutive, tissue-preferred, cell type-preferred and inducible promoters are all suitable for use in T-DNA activation. The effects of the invention may also be reproduced using the technique of TILLING (Targeted Induced Local Lesions In Genomes). The effects of the invention may also be reproduced using homologous recombination.

The present invention also encompasses use of SYT nucleic acid sequence, or use of a SYT polypeptide as defined in any of claims 1-2, 4, 5 and 7, in improving yield under reduced nutrient availability stress relative to control plants and/or wherein said increased yield is one or more of the following: total seed yield, number of filled seeds, seed fill rate, TKW and harvest index, wherein the harvest index is the ratio of seed yield divided by total biomass.

SYT nucleic acid sequences or SYT polypeptides may find use in breeding programmes in which a DNA marker is identified that may be genetically linked to a SYT. The SYT nucleic acid sequences or SYT polypeptides may be used to define a molecular marker. This DNA or polypeptide marker may then be used in breeding programmes to select plants having increased yield. The SYT gene may, for example, be a nucleic acid sequence as represented by any one of the SYT nucleic acid sequences as given in Table 4, or orthologues or paralogues of any of the aforementioned SEQ ID NOs.

**[0186]** Allelic variants of a SYT nucleic acid sequence may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question, for example, different allelic variants of any one of the SYT nucleic acid sequences as given in Table 4, or orthologues or paralogues of any of the aforementioned SEQ ID NOs. Growth performance may be monitored in a greenhouse or in the field. Further optional steps include crossing plants, in which the superior allelic variant was identified, with another plant. This could be used, for example, to make a combination of interesting phenotypic features.

SYT nucleic acid sequences may also be used as probes for genetically and physically mapping the genes that they are a part of, and as markers for traits linked to those genes. Such information may be useful in plant breeding in order to develop lines with desired phenotypes. Such use of SYT nucleic acid sequences requires only a nucleic acid sequence of at least 15 nucleotides in length. The SYT nucleic acid sequences may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the SYT nucleic acid sequences. The resulting banding patterns may then be subjected to genetic analyses using computer programs such as MapMaker (Lander et al. (1987) Genomics 1: 174-181) in order to construct a genetic map. In addition, the nucleic acid sequences may be used to probe Southern blots containing restriction endonuclease-treated genomic DNAs of a set of individuals representing parent and progeny of a defined genetic cross. Segregation of the DNA polymorphisms is noted and used to calculate the position of the SYT nucleic acid sequence in the genetic map previously obtained using this population (Botstein et al. (1980) Am. J. Hum. Genet. 32:314-331).

The production and use of plant gene-derived probes for use in genetic mapping is described in Bematzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridization (FISH) mapping (Trask (1991) Trends Genet. 7:149-154). Although current methods of FISH mapping favor use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acid sequences. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the nucleic acid sequence is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having increased yield under abiotic stress, as described hereinbefore. These yield-enhancing traits may also be combined with other economically advantageous traits, such as further yield-enhancing traits, tolerance to various stresses, traits modifying various architectural features and/or biochemical and/or physiological features.

## Description of figures

**Fig. 1** shows the typical domain structure of SYT polypeptides from plants and mammals. The conserved SNH domain is located at the N-terminal end of the polypeptide. The C-terminal remainder of the polypeptide consists of a QG-rich domain in plant SYT polypeptides, and of a QPGY-rich domain in mammalian SYT polypeptides. A Met-rich domain is typically comprised within the first half of the QG-rich (from the N-term to the C-term) in plants or QPGY-rich in mammals. A second Met-rich domain may precede the SNH domain in plant SYT polypeptides

**Fig. 2** shows a multiple alignment of the N-terminal end of several SYT polypeptides, using VNTI AlignX multiple alignment program, based on a modified ClustalW algorithm (InforMax, Bethesda, MD, http://www.informaxinc.com), with default settings for gap opening penalty of 10 and a gap extension of 0.05). The SNH domain is boxed across the plant and human SYT polypeptides. The last line in the alignment consists of a consensus sequence derived from the aligned sequences.

**Fig. 3** shows a multiple alignment of several plant SYT polypeptides, using VNTI AlignX multiple alignment program, based on a modified ClustalW algorithm (InforMax, Bethesda, MD, http://www.informaxinc.com), with default settings for gap opening penalty of 10 and a gap extension of 0.05). The two main domains, from N-terminal to C-terminal, are boxed and identified as SNH domain and the Met-rich/QG-rich domain. Additionally, the N-terminal Met-rich domain is also boxed, and the positions of SEQ ID NO: 90 and SEQ ID NO 91 are underlined in bold.

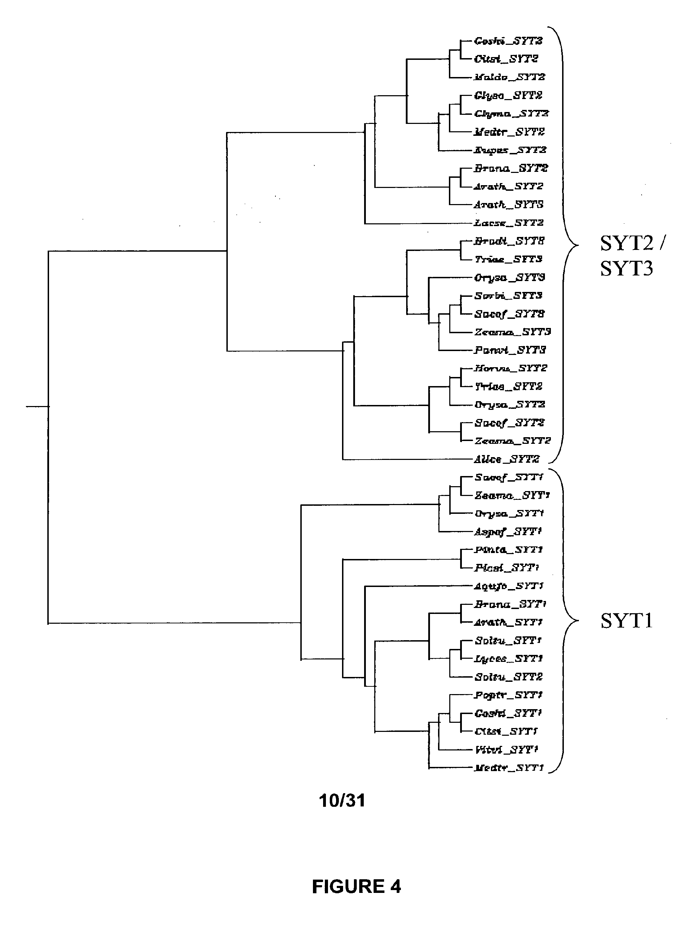

**Fig. 4** shows a Neighbour joining tree resulting from the alignment of multiple SYT polypeptides using CLUSTALW 1.83 (http://align.genome.jp/sit-bin/clustalw). The SYT1 and SYT2/SYT3 clades are identified with brackets.

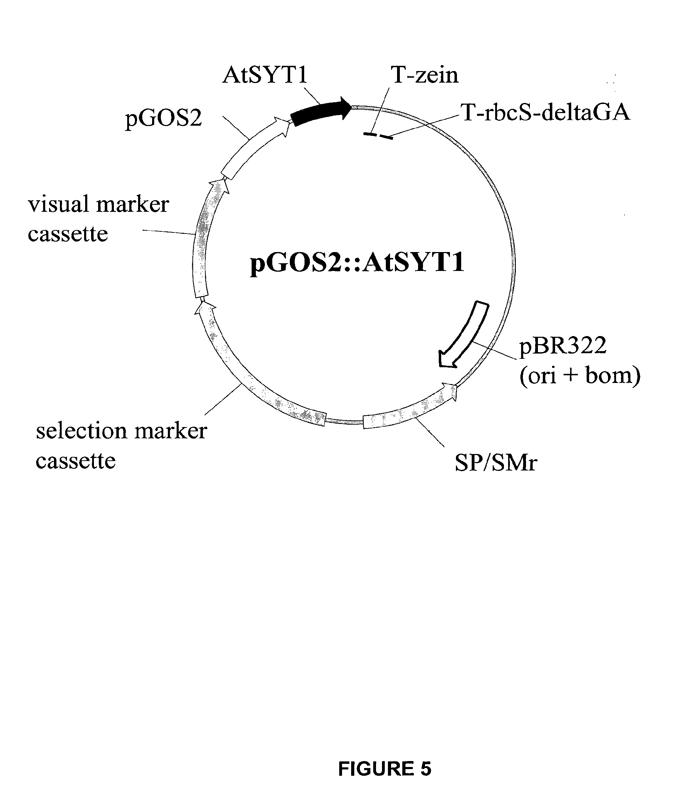

**Fig. 5** shows a binary vector p0523, for expression in *Oryza sativa* of an *Arabidopsis thaliana* AtSYT1 under the control of a GOS2 promoter (internal reference PR00129).

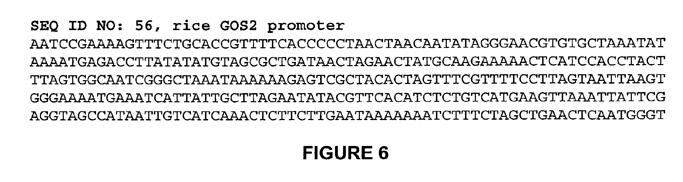

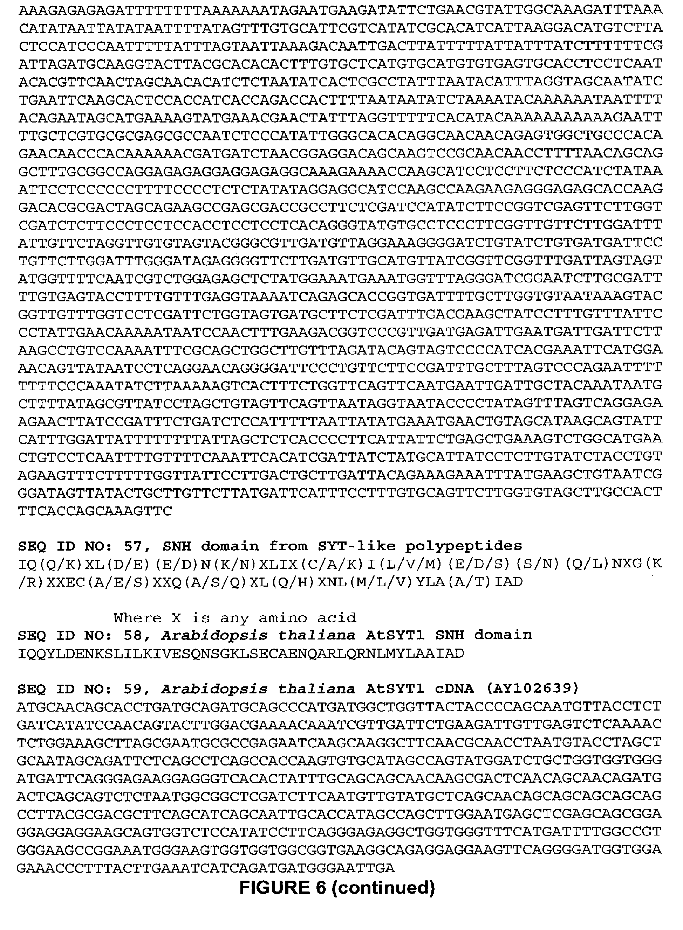

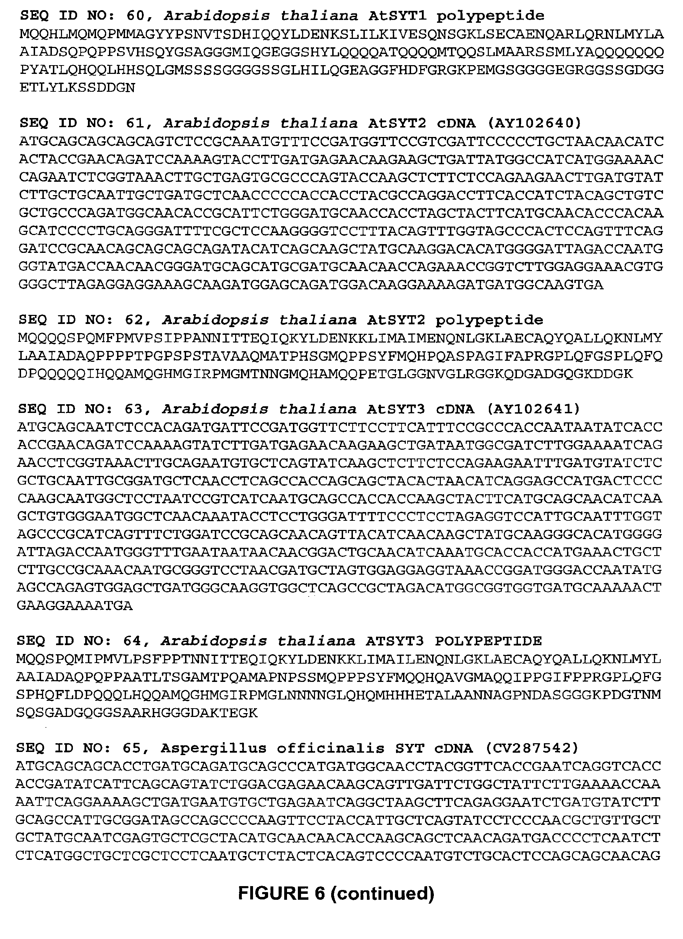

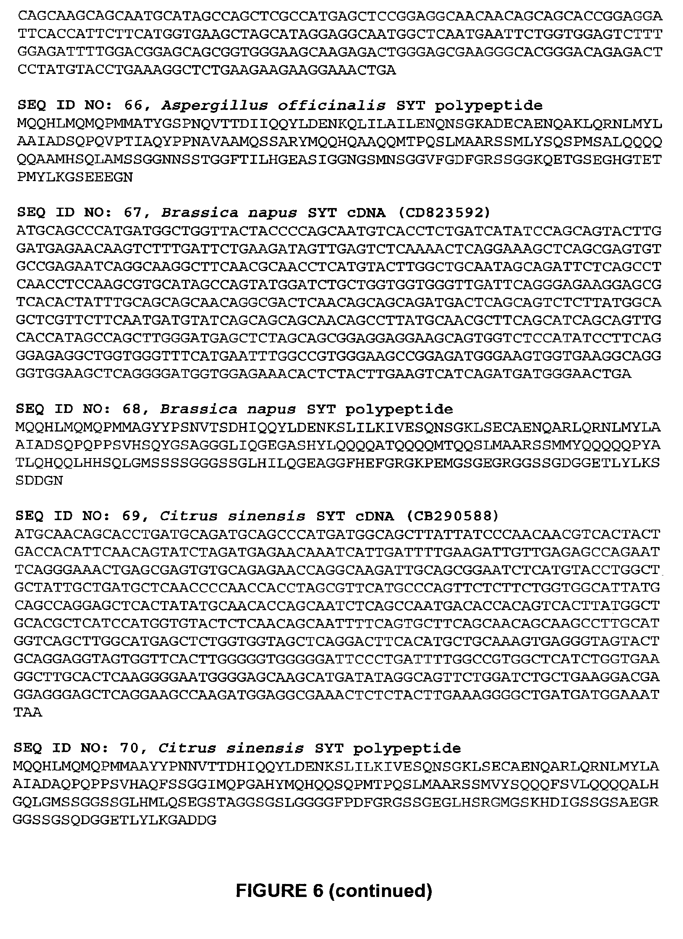

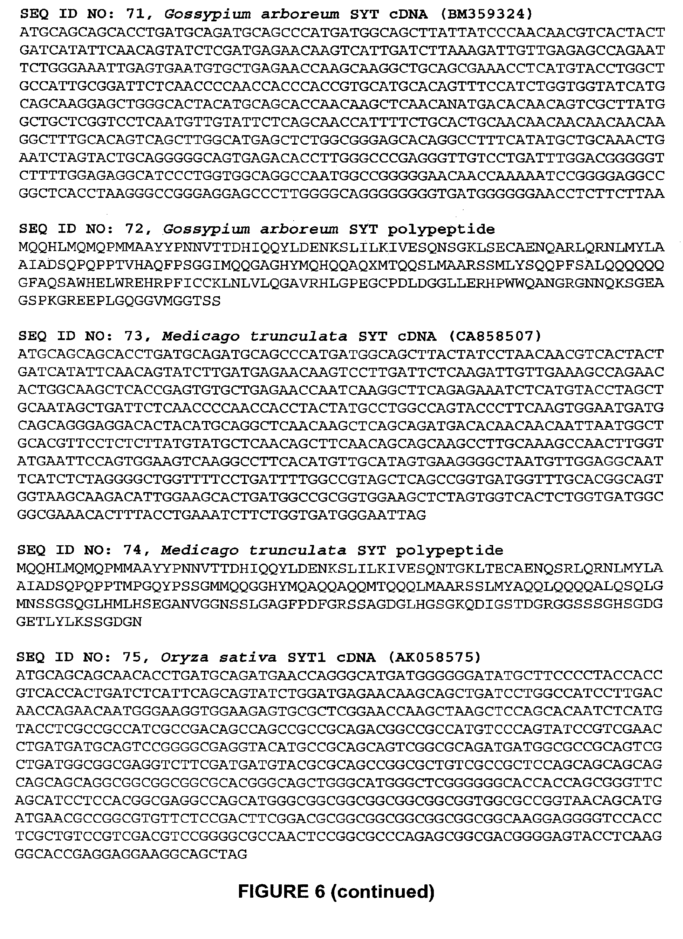

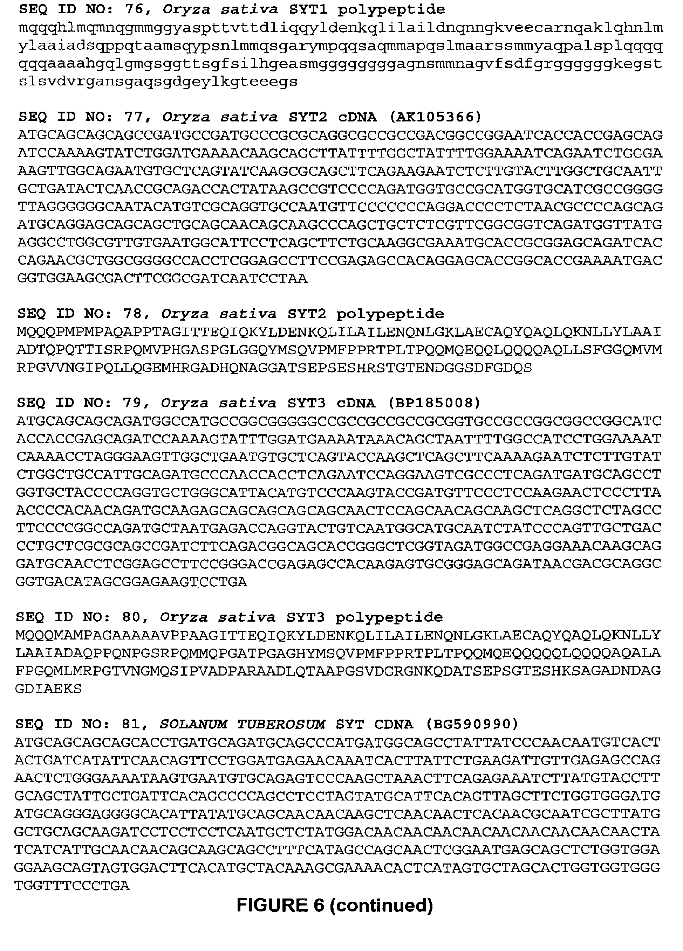

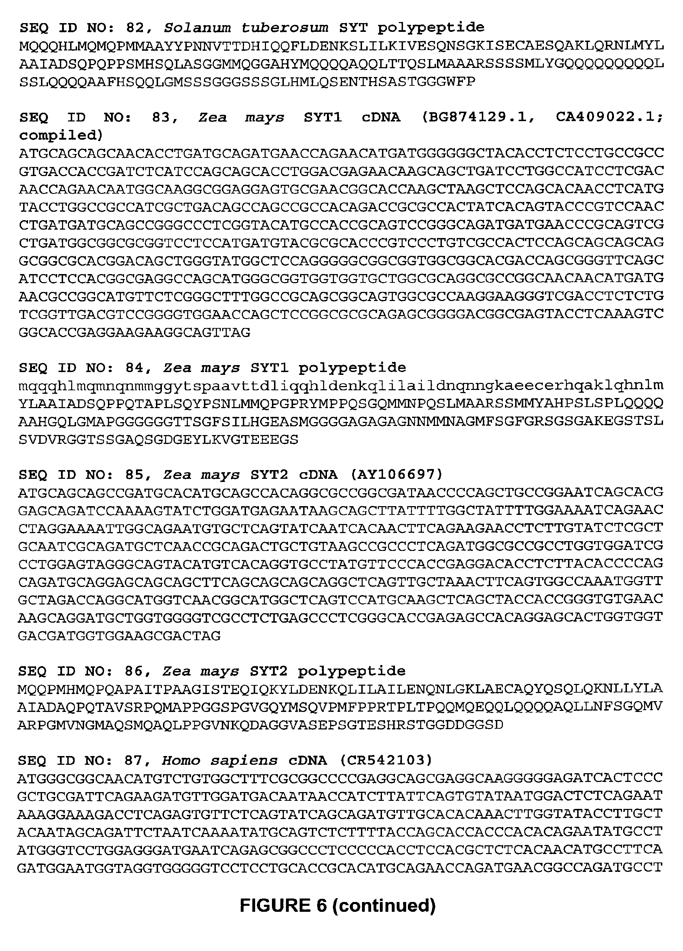

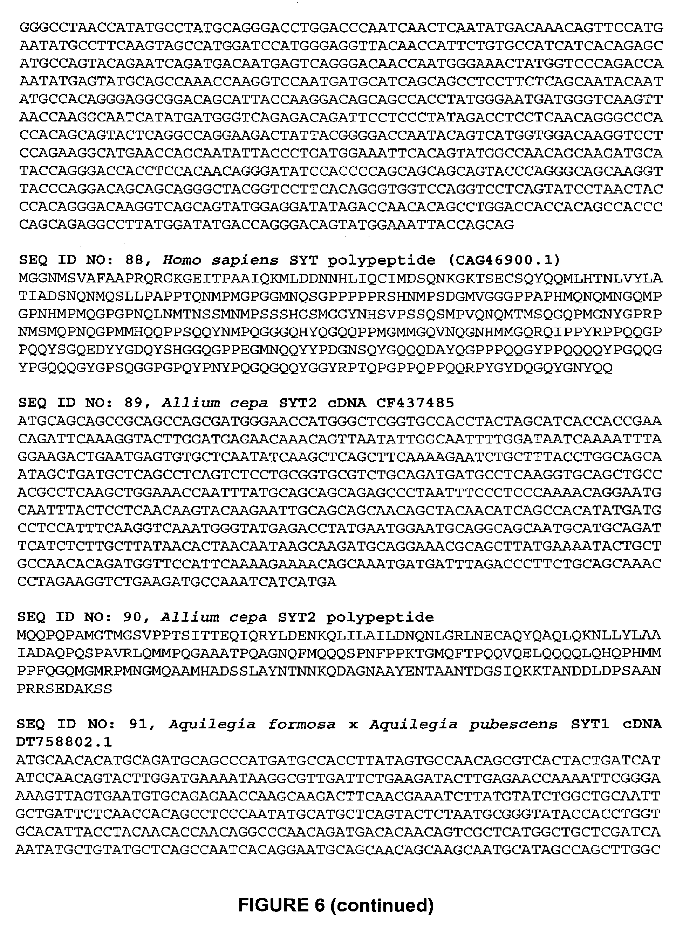

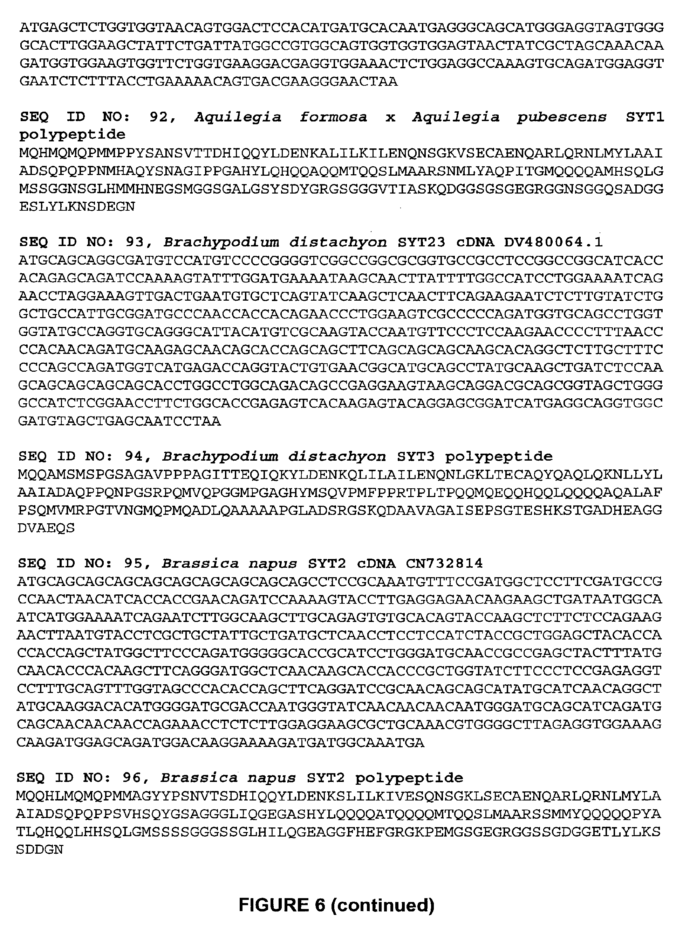

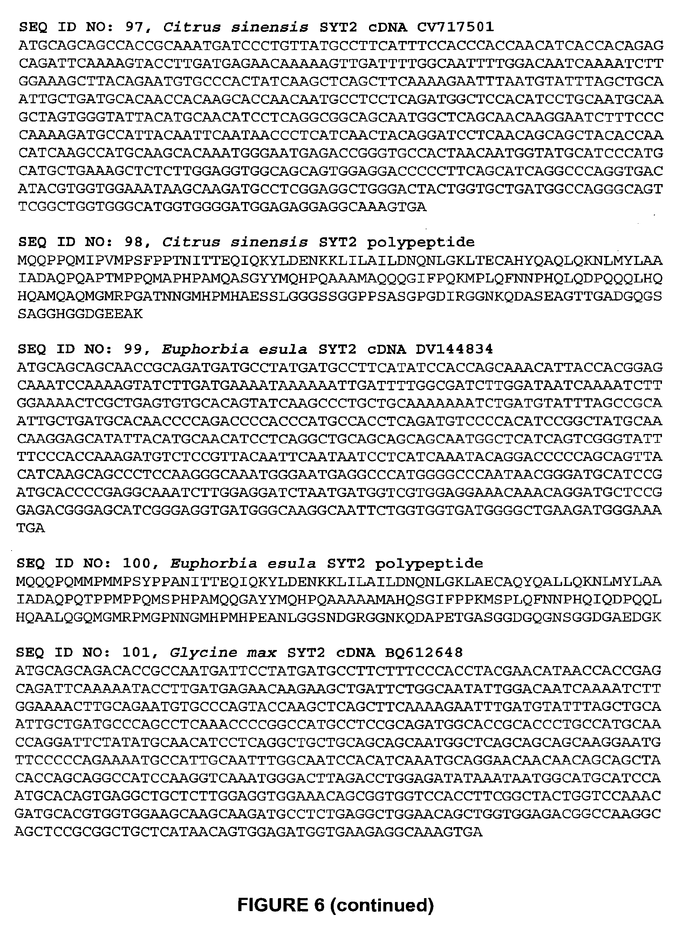

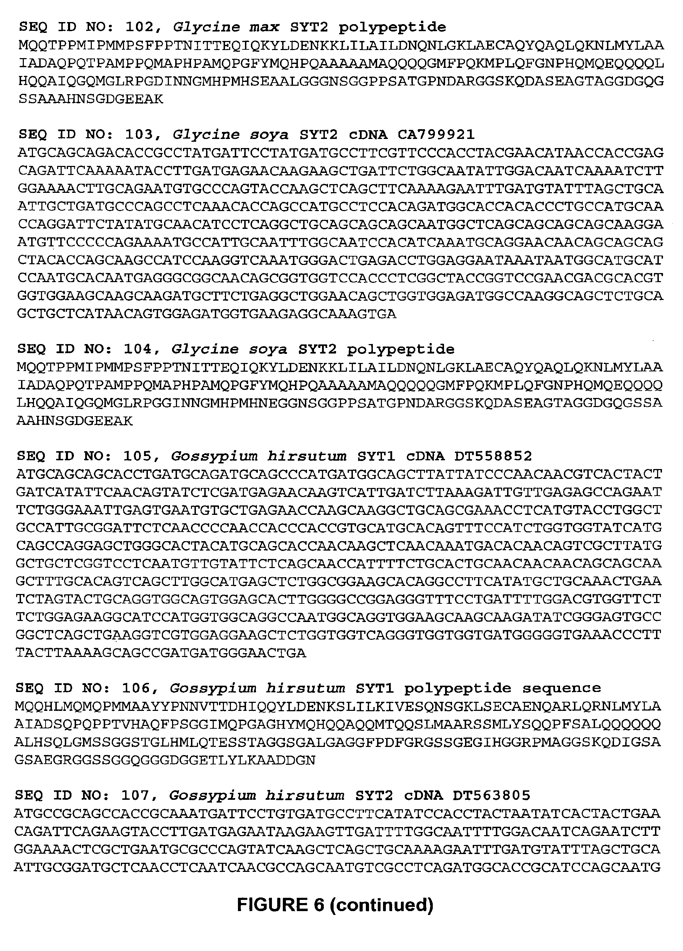

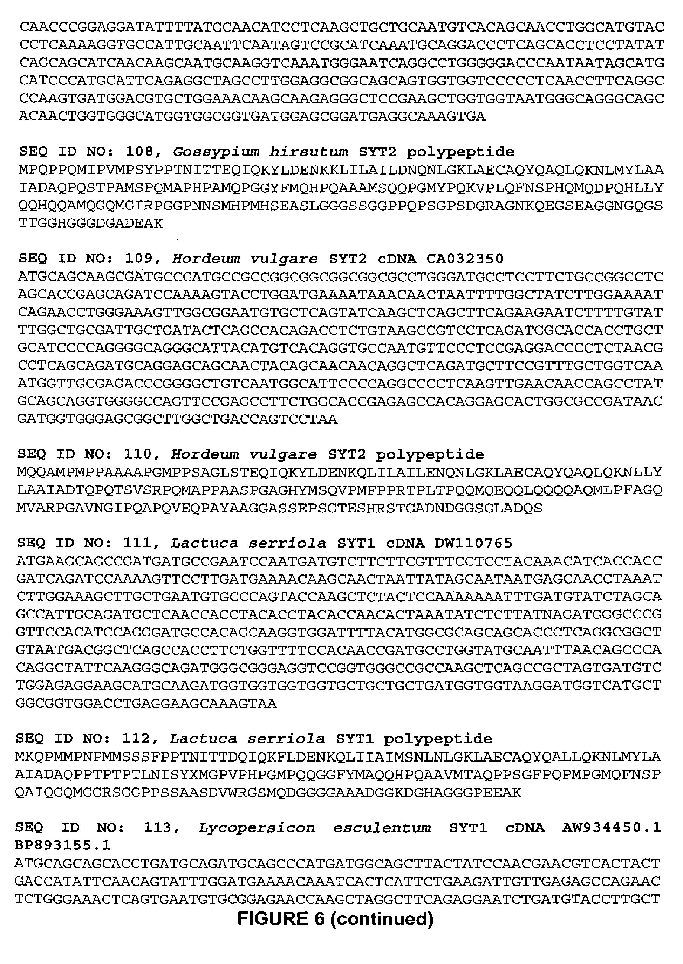

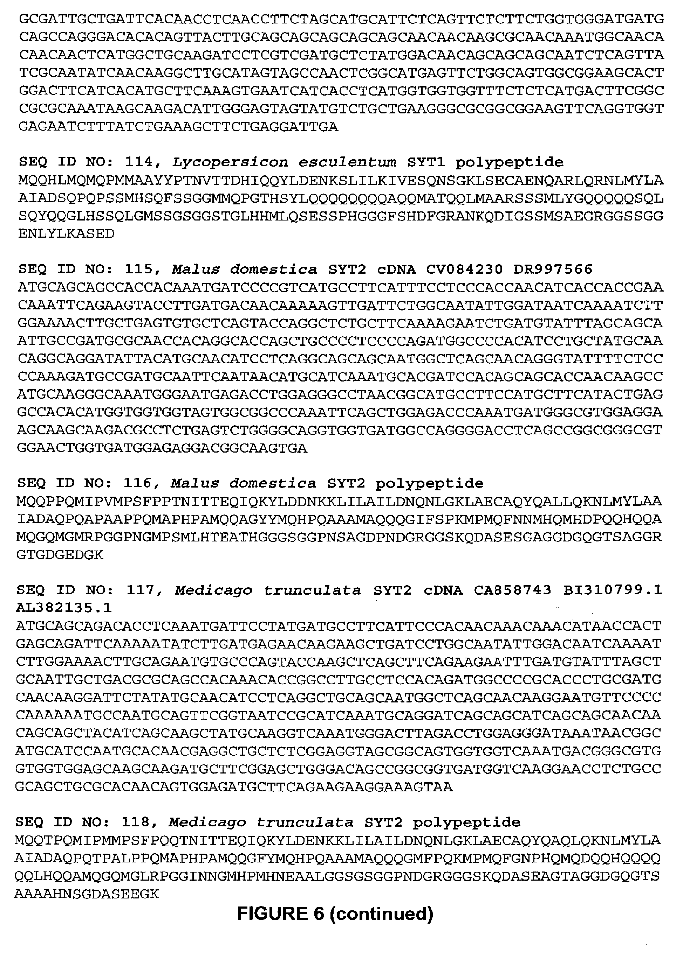

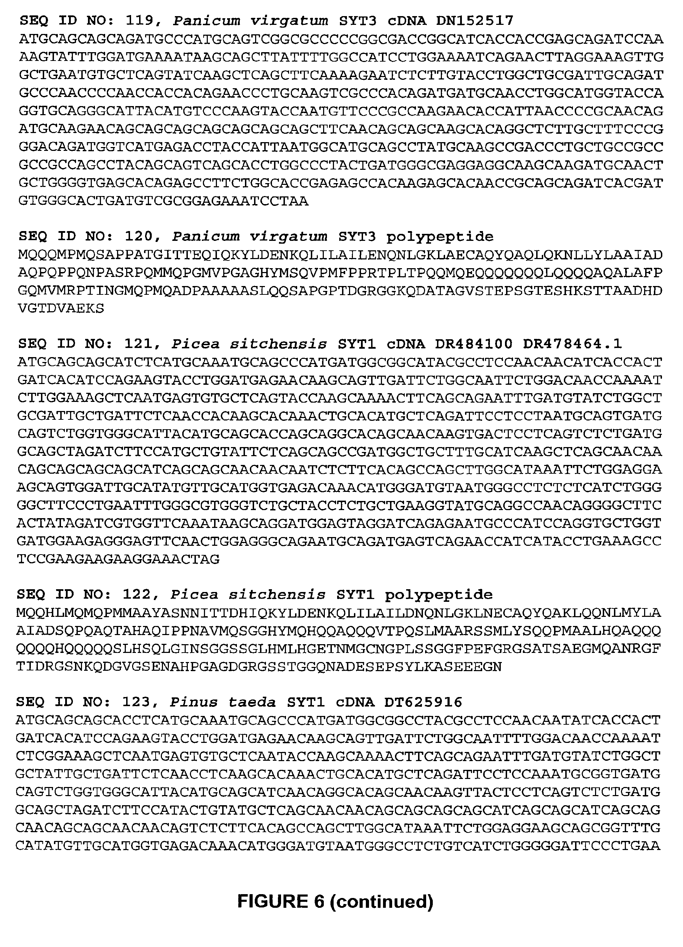

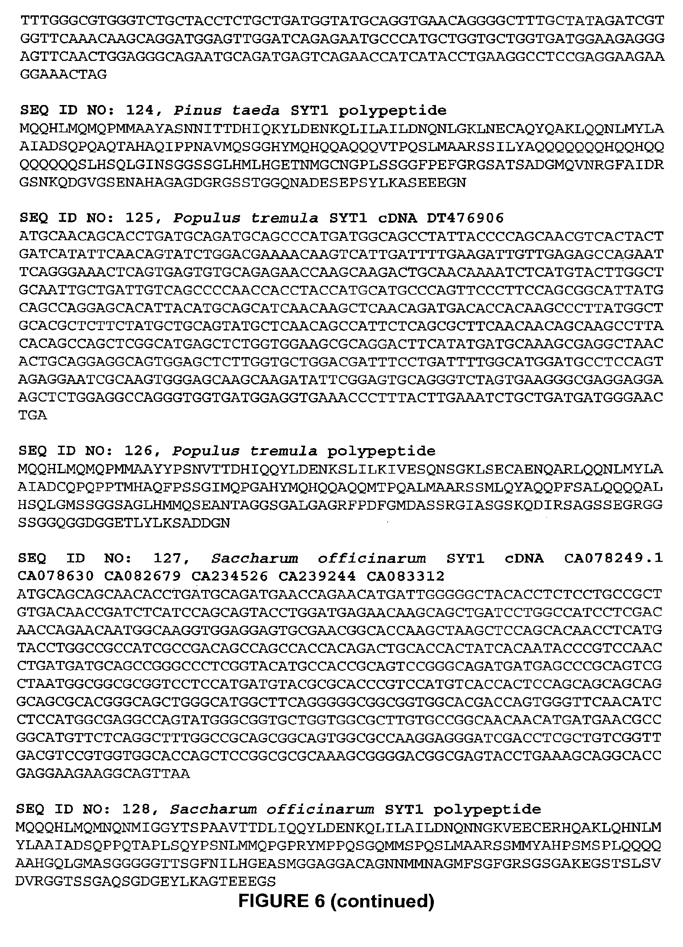

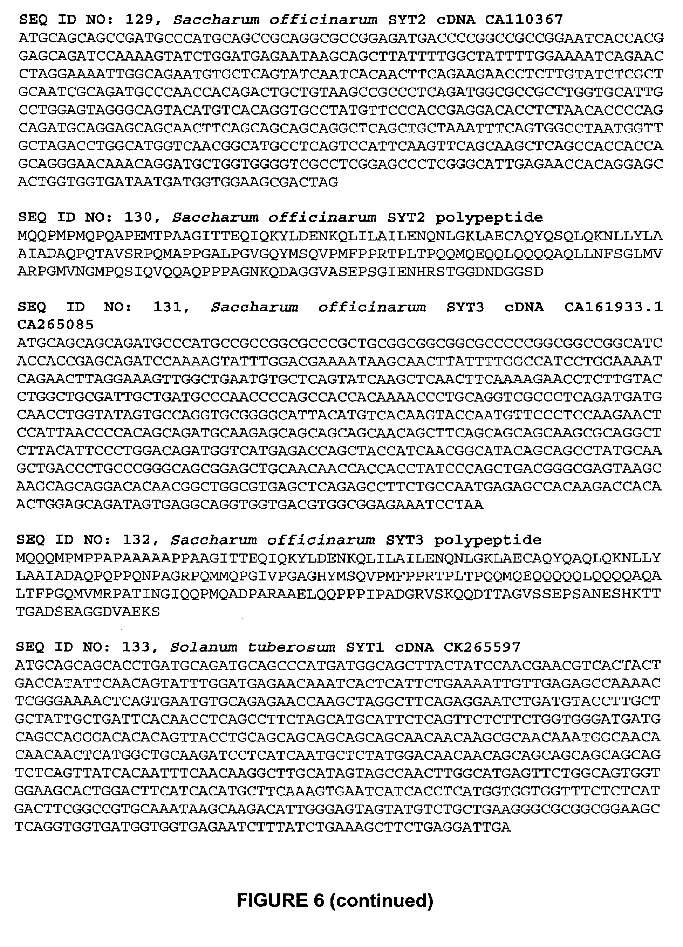

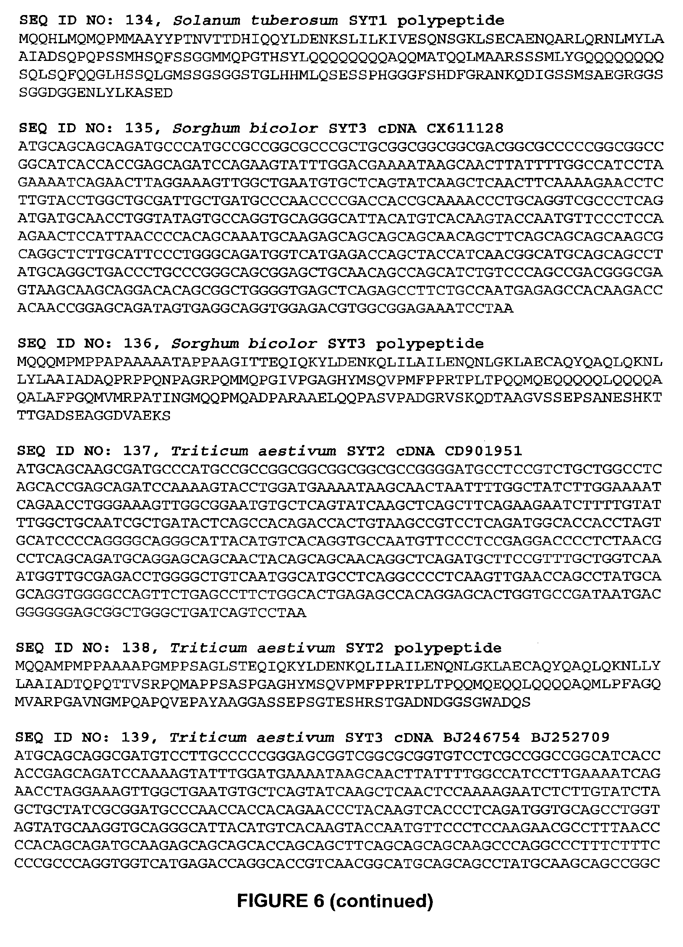

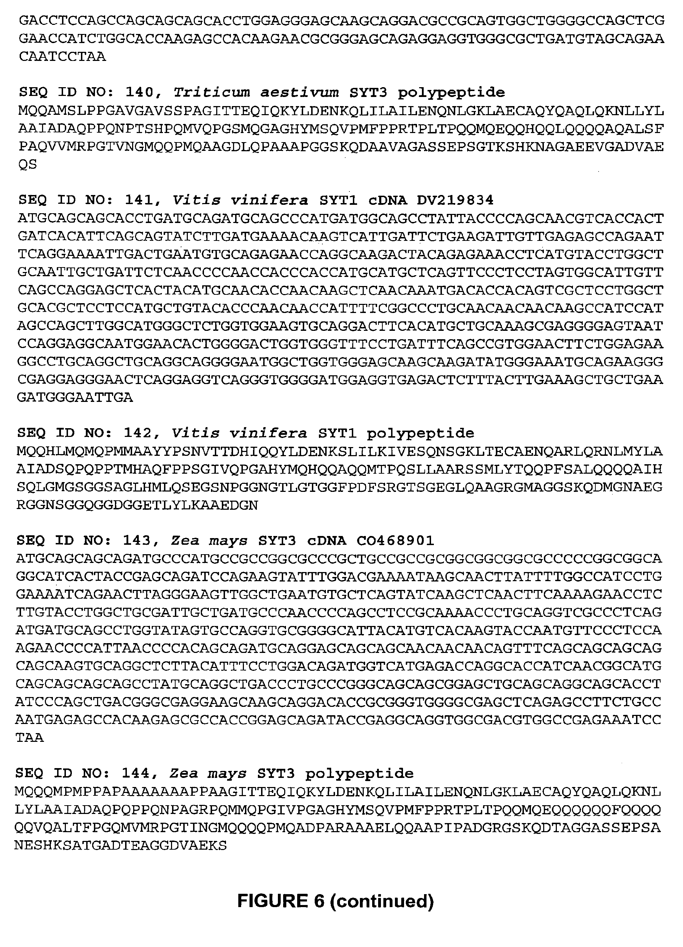

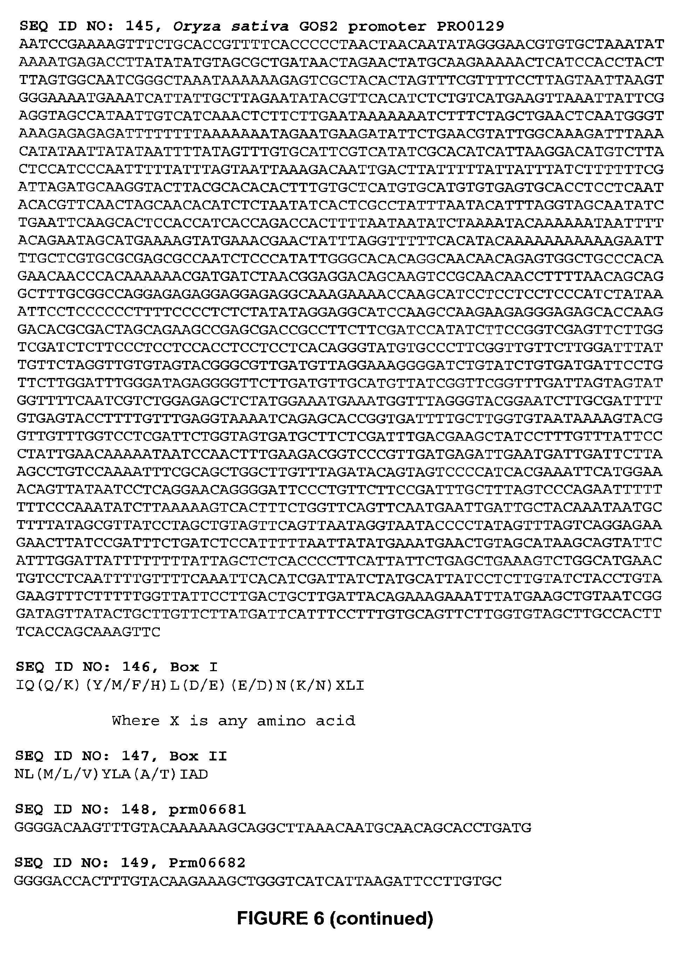

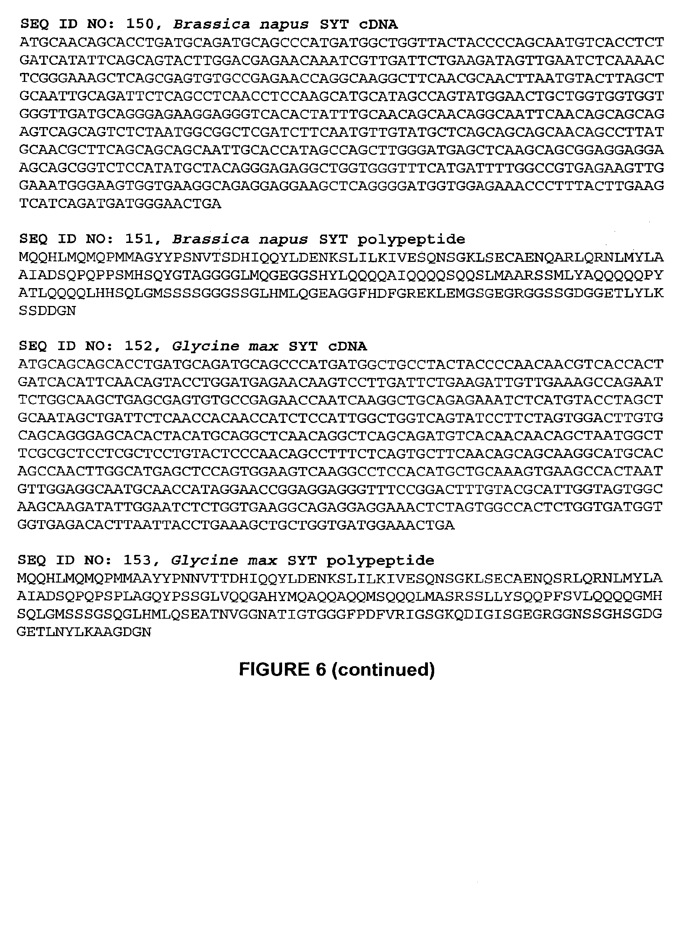

**Fig. 6** details examples of sequences. SEQ ID NO: 56 to 153 relate to SYT sequences. SYT nucleic acid sequences are presented from start to stop. The majority of these sequences are derived from EST sequencing, which is of lower quality. Therefore, nucleic acid substitutions may be encountered.

## Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### *Example 1: Gene Cloning of AtSYT1*

The *Arabidopsis thaliana* AtSYT1 gene was amplified by PCR using as template an *Arabidopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb and the original number of clones was of the order of $1.59 \times 10^7$ cfu. Original titer was determined to be $9.6 \times 10^5$ cfu/ml after first amplification of $6 \times 10^{11}$ cfu/ml. After plasmid extraction, 200 ng of template was used in a 50 μl PCR mix. Primers prm06681 (SEQ ID NO: 148; sense, start codon in bold, AttB1 site in italic: 5'-*GGGGACAAGTTTGTACAAAAAAGCAGG*CTTAAACA**ATG**CAACAGCACCTGATG -3') and prm06682 (SEQ ID NO: 149; reverse, complementary, AttB2 site in italic: 5'-*GGGGACCACTTTGTACAAGAAAGCTGGGTCAT*CATTAAGATTCCTTGTGC-3'), which include the AttB sites for Gateway recombination, were used for PCR amplification. PCR was performed using Hifi Taq DNA polymerase in standard conditions. A PCR fragment of 727 bp (including attB sites) was amplified and purified also using standard methods. The first step of the Gateway procedure, the BP reaction, was then performed, during which the PCR fragment recombines *in vivo* with the pDONR201 plasmid to produce, according to the Gateway terminology, an "entry clone", p07466. Plasmid pDONR201 was purchased from Invitrogen, as part of the Gateway® technology.

***Example 2: Vector Construction***

The entry clone p07466 was subsequently used in an LR reaction with p00640, a destination vector used for plant (*Oryza sativa*) transformation. This vector contains as functional elements within the T-DNA borders: a plant selectable marker; a screenable marker expression cassette; and a Gateway cassette intended for LR *in vivo* recombination with the sequence of interest already cloned in the entry clone. A rice GOS2 promoter (SEQ ID NO: 145) for constitutive expression (PRO0129) was located upstream of this Gateway cassette.

Many different binary (and super binary) vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription.

After the LR recombination step, the resulting expression vector pGOS2::AtSYT1 (Figure 5) was transformed into *Agrobacterium* strain LBA4044 using heat shock or electroporation protocols. Transformed colonies were grown on YEP media and selected by respective antibiotics for two days at 28°C. These *Agrobacterium* cultures were used for the plant transformation.

Other *Agrobacterium tumefaciens* strains can be used for plant transformation and are well known in the art. Examples of such strains are C58C1 or EHA105.

***Example 3: Plant transformation***

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2%$HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for cocultivation. *Agrobacterium* was inoculated on AB medium with the appropriate antibiotics and cultured for 3 days at 28°C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

*Corn transformation*

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for tansformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with Agrobacterium, the embryos are grown *in vitro* on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

***Example 4: Evaluation setup of AtSYT1 transgenic rice plants under abiotic stress***

Four events, of which the T1 progeny segregated 3:1 for presence/absence of the transgene, were retained. For each of these events, approximately 15 T1 seedlings containing the transgene (hetero- and homo-zygotes) and approximately 15 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), with temperatures on average 28°C in the light and 22°C in the dark, and a relative humidity on average of 70%.

*Salt stress screen*

Plants were grown on a substrate made of coco fibers and argex (3 to 1 ratio). A normal nutrient solution was used during the first two weeks after transplanting the plantlets in the greenhouse. After the first two weeks, 25 mM of salt (NaCl) was added to the nutrient solution, until the plants were harvested. Seed-related parameters were then measured.

*Drought screen*

Plants are grown in potting soil under normal conditions until they approach the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Seed-related parameters are then measured

An alternative method to impose water stress on the transgenic plants is by treatment with water containing an osmolyte such as polyethylene glycol (PEG) at specific water potential. Since PEG may be toxic, the plants are given only a short-term exposure and then normal watering is resumed.

*Reduced nutrient (nitrogen) availability screen*

The rice plants are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Seed-related parameters are then measured

**Statistical analysis: F-test**

A two factor ANOVA (analysis of variants) was used as a statistical model for the overall evaluation of plant phenotypic characteristics. An F-test was carried out on all the parameters measured of all the plants of all the events transformed with the gene as set forth in the method of present invention. The F-test was carried out to check for an effect of the gene over all the transformation events and for an overall effect of the gene, also known as a global gene effect. The threshold for significance for a true global gene effect was set at a 5% probability level for the F-test. A significant F-test value points to a gene effect, meaning that it is not only the presence or position of the gene that is causing the differences in phenotype.

**Biomass-related parameter measurement**

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the time point at which the plant had reached its maximal leafy biomass. The early vigour is the plant (seedling) aboveground area three weeks post-germination.

**Seed-related parameter measurements**

The mature primary panicles were harvested, counted, bagged, barcode-labeled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand kernel weight (TKW) is extrapolated from the number of filled seeds counted and their total weight. The harvest index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Example 5: Results of the evaluation of AtSYT1 transgenic rice plants under abiotic stress (salt stress)***

The results of the evaluation of AtSYT1 transgenic rice plants submitted to salt stress are presented in Table A. The percentage difference between the transgenics and the corresponding nullizygotes is also shown, with a P value from the F test below 0.05.

Aboveground biomass, early vigour, total seed yield, number of filled seeds, seed fill rate, TKW and harvest index are significantly increased in the AtSYT1 transgenic plants compared to the control plants (in this case, the nullizygotes), under abiotic stress.

**Table A:** Results of the evaluation of AtSYT1 transgenic rice plants under abiotic stress.

| **Trait** | **% Difference** |
|---|---|
| Aboveground biomass | 19 |
| Early vigour | 18 |
| Total seed yield | 30 |
| Nbr of filled seeds | 18 |
| Fill rate | 15 |
| TKW | 10 |
| Harvest index | 16 |

## Claims

1. Method for increasing plant yield under reduced nutrient availability stress relative to control plants, wherein said plant yield is any one or more of total seed yield, number of filled seeds, seed fill rate, TKW and harvest index, said harvest index being the ratio of seed yield divided by total biomass, wherein said method comprises modulating expression in a plant of a nucleic acid sequence encoding a synovial sarcoma translocation (SYT) polypeptide, and optionally selecting for plants having increased yield, wherein said modulated expression is effected by introducing and expressing in a plant, plant part or plant cell a nucleic acid sequence encoding a SYT polypeptide and wherein said SYT polypeptide comprises from N-terminal to C-terminal: (i) an SNH domain having at least 65% sequence identity to the SNH domain of SEQ ID NO: 58; and (ii) a Met-rich domain; and (iii) a QG-rich domain.

2. Method according to claim 1, wherein said SNH domain comprises the residues shown in black in Figure 6 and/or wherein said SNH domain is represented by SEQ ID NO: 57 and/or wherein said SYT polypeptide further comprises one or more of the following: (i) SEQ ID NO: 146; (ii) SEQ ID NO: 147; (iii) a Met-rich domain at the N-terminus preceding the SNH domain.

3. Method according to claim 1 or 2, wherein said reduced nutrient availability stress is reduced nitrogen availability.

4. Method according to any of claims 1 to 3, wherein said nucleic acid sequence is one or more of the following SYT nucleic acid variants:

   (i) a portion of a SYT nucleic acid sequence;
   (ii) a nucleic acid sequence capable of hybridising with a SYT nucleic acid sequence;
   (iii) a splice variant of a SYT nucleic acid sequence;
   (iv) an allelic variant of a SYT nucleic acid sequence;
   (v) a SYT nucleic acid sequence obtained by gene shuffling;
   (vi) a SYT nucleic acid sequence obtained by site-directed mutagenesis.

5. Method according to any of claims 1 to 4, wherein said nucleic acid sequence encodes any one of the SYT polypeptides given in Table 6, or orthologues or paralogues of any of the aforementioned SEQ ID NOs and/or wherein said nucleic acid sequence encodes the SYT polypeptides as represented by SEQ ID NO: 60, SEQ ID NO: 151 or SEQ ID NO: 153.

6. Method according to any one of claims 1 to 5, wherein said nucleic acid sequence encoding a SYT polypeptide is overexpressed in a plant.

7. Method according to any one of claims 1 to 6, wherein said nucleic acid sequence encoding a SYT polypeptide is of plant origin.

8. Method according to any one of claims 1 to 7, wherein said nucleic acid sequence encoding a SYT polypeptide is operably linked to a constitutive promoter, preferably wherein said constitutive promoter is plant-derived, preferably from a monocotyledonous plant, preferably wherein said constitutive promoter is a GOS2 promoter.

9. Method for the production of a transgenic plant, preferably a monocotyledonous plant, having any one or more of increased total seed yield, increased number of filled seeds, increased seed fill rate, increased TKW and increased harvest index, said harvest index being the ratio of seed yield divided by total biomass, relative to corresponding wild type plants under reduced nutrient availability stress, which method comprises:

(i) introducing and expressing in a plant or plant cell a nucleic acid sequence encoding a SYT polypeptide as defined in any of claims 1-2, 4, 5 and 7;
(ii) cultivating the plant cell under conditions promoting plant growth and development.

10. Use of a SYT nucleic acid sequence, or use of a SYT polypeptide as defined in any of claims 1-2, 4, 5 and 7, in improving yield under reduced nutrient availability stress relative to control plants and/or wherein said increased yield is one or more of the following: total seed yield, number of filled seeds, seed fill rate, TKW and harvest index, wherein the harvest index is the ratio of seed yield divided by total biomass.

## Patentansprüche

1. Verfahren zum Erhöhen des Pflanzenertrags unter Nährstoffmangelstress im Vergleich zu Kontrollpflanzen, wobei es sich bei dem Pflanzenertrag um eines oder mehrere der Merkmale Samengesamtertrag, Anzahl gefüllte Samen, Samenfüllrate, TKG und Harvest Index handelt, wobei der Harvest Index das Verhältnis von Samenertrag dividiert durch Gesamtbiomasse ist, wobei das Verfahren umfasst, dass man die Expression einer Nukleinsäuresequenz, die für ein Synovialsarkomtranslokations (SYT) -Polypeptid codiert, in einer Pflanze moduliert und gegebenenfalls auf Pflanzen mit erhöhtem Ertrag selektiert, wobei die modulierte Expression dadurch bewirkt wird, dass man eine Nukleinsäuresequenz, die für ein SYT-Polypeptid codiert, in eine Pflanze, einen Pflanzenteil oder eine Pflanzenzelle einführt und darin exprimiert, und wobei das SYT-Polypeptid von N-terminal nach C-terminal Folgendes umfasst: (i) eine SNH-Domäne mit mindestens 65% Sequenzidentität zu der SNH-Domäne von SEQ ID NO: 58; und (ii) eine Met-reiche Domäne; und (iii) eine QG-reiche Domäne.

2. Verfahren nach Anspruch 1, wobei die SNH-Domäne die in Fig. 6 in schwarz gezeigten Reste umfasst und/oder wobei die SNH-Domäne durch SEQ ID NO: 57 dargestellt ist und/oder wobei das SYT-Polypeptid weiterhin eine oder mehrere der Folgenden umfasst: (i) SEQ ID NO: 146; (ii) SEQ ID NO: 147; (iii) eine Met-reiche Domäne am N-terminalen Ende, die der SNH-Domäne vorangestellt ist.

3. Verfahren nach Anspruch 1 oder 2, wobei der Nährstoffmangelstress Stickstoffmangel ist.

4. Verfahren nach einem der Ansprüche 1 bis 3, wobei die Nukleinsäuresequenz eine oder mehrere der folgenden SYT-Nukleinsäurevarianten ist:

(i) ein Abschnitt einer SYT-Nukleinsäuresequenz;
(ii) eine Nukleinsäuresequenz, die fähig ist, mit einer SYT-Nukleinsäuresequenz zu hybridisieren;
(iii) eine Spleißvariante einer SYT-Nukleinsäuresequenz;
(iv) eine Allelvariante einer SYT-Nukleinsäuresequenz;
(v) eine durch Gene Shuffling erhaltene SYT-Nukleinsäuresequenz;
(vi) eine durch ortsgerichtete Mutagene erhaltene SYT-Nukleinsäuresequenz.

5. Verfahren nach einem der Ansprüche 1 bis 4, wobei die Nukleinsäuresequenz für eines der in Tabelle 6 angegebenen SYT-Polypeptide oder für Orthologe oder Paraloge von beliebigen der oben genannten SEQ ID NOs codiert und/oder wobei die Nukleinsäuresequenz für die SYT-Polypeptide gemäß SEQ ID NO: 60, SEQ ID NO: 151 oder SEQ ID NO: 153 codiert.

6. Verfahren nach einem der Ansprüche 1 bis 5, wobei die Nukleinsäuresequenz, die für ein SYT-Polypeptid codiert, in einer Pflanze überexprimiert wird.

7. Verfahren nach einem der Ansprüche 1 bis 6, wobei die Nukleinsäuresequenz, die für ein SYT-Polypeptid codiert, pflanzlichen Ursprungs ist.

8. Verfahren nach einem der Ansprüche 1 bis 7, wobei die Nukleinsäuresequenz, die für ein SYT-Polypeptid codiert, operativ mit einem konstitutiven Promoter verknüpft ist, vorzugsweise wobei der konstitutive Promoter von einer Pflanze abstammt, vorzugsweise von einer monokotylen Pflanze, vorzugsweise wobei es sich bei dem konstitutiven Promoter um einen GOS2-Promoter handelt.

9. Verfahren zur Herstellung einer transgenen Pflanze, vorzugsweise einer monokotylen Pflanze, die eine oder mehrere der Merkmale im Vergleich zu entsprechenden Wildtyppflanzen unter Nährstoffmangelstress erhöhter Samengesamtertrag, erhöhte Anzahl gefüllte Samen, erhöhte Samenfüllrate, erhöhtes TKG und erhöhten Harvest Index aufweist, wobei der Harvest Index das Verhältnis von Samenertrag dividiert durch Gesamtbiomasse ist, wobei das Verfahren Folgendes umfasst:

   (i) Einführen und Exprimieren einer Nukleinsäuresequenz, die für ein SYT-Polypeptid wie in einem der Ansprüche 1-2, 4, 5 und 7 definiert in eine(r) Pflanze oder Pflanzenzelle;
   (ii) Kultivieren der Pflanzenzelle unter Bedingungen, die das Pflanzenwachstum und die Pflanzenentwicklung fördern.

10. Verwendung einer SYT-Nukleinsäuresequenz, oder Verwendung eines SYT-Polypeptids wie in einem der Ansprüche 1-2, 4, 5 und 7 definiert, bei der Verbesserung des Ertrags unter Nährstoffmangel- stress im Vergleich zu Kontrollpflanzen und/oder wobei es sich bei dem erhöhten Ertrag um eines oder mehrere der folgenden Merkmale handelt: Samengesamtertrag, Anzahl gefüllte Samen, Samenfüllrate, TKG und Harvest Index, wobei es sich bei dem Harvest Index um das Verhältnis von Samenertrag dividiert durch Gesamtbiomasse handelt.

## Revendications

1. Méthode d' augmentation du rendement de plantes sous stress de disponibilité réduite en nutriments, par rapport à des plantes témoins, où ledit rendement de plantes est l' un ou plusieurs parmi le rendement en graines total, le nombre de graines remplies, le taux de remplissage des graines, le PMG et l'indice de moisson, ledit indice de moisson étant le rapport du rendement en graines divisé par la biomasse totale, **caractérisée en ce que** ladite méthode comprend la modulation de l'expression chez une plante d' une séquence d' acide nucléique codant pour un polypeptide de translocation de sarcome synovial (SYT), et la sélection éventuelle de plantes ayant un rendement accru, où ladite expression modulée est effectuée par l'introduction et l'expression dans une plante, une partie de plante ou une cellule végétale, d' une séquence d' acide nucléique codant pour un polypeptide SYT et où ledit polypeptide SYT comprend, de l'extrémité N-terminale à C-terminale : (i) un domaine SNH ayant une identité de séquence d' au moins 65% avec le domaine SNH de SEQ ID n° 58 ; et (ii) un domaine riche en Met ; et (iii) un domaine riche en QG.

2. Méthode selon la revendication 1, **caractérisée en ce que** ledit domaine SNH comprend les résidus illustrés en noir dans la Figure 6 et/ou **en ce que** ledit domaine SNH est représenté par SEQ ID n° 57 et/ou **en ce que** ledit polypeptide SYT comprend en outre un ou plusieurs parmi ce qui suit : (i) SEQ ID n° 146 ; (ii) SEQ ID n° 147 ; (iii) un domaine riche en Met au niveau de l'extrémité N-terminale précédant le domaine SNH.

3. Méthode selon la revendication 1 ou 2, **caractérisée en ce que** ledit stress de disponibilité réduite en nutriments est une disponibilité réduite en azote.

4. Méthode selon l'une quelconque des revendications 1 à 3, **caractérisée en ce que** ladite séquence d' acide nucléique est l'une ou plusieurs parmi les variants d' acide nucléique SYT suivants :

   (i) une portion d' une séquence d' acide nucléique SYT ;
   (ii) une séquence d' acide nucléique capable de s' hybrider avec une séquence d' acide nucléique SYT ;

(iii) un variant d' épissage d' une séquence d' acide nucléique SYT ;
(iv) un variant allélique d' une séquence d' acide nucléique SYT ;
(v) une séquence d' acide nucléique SYT obtenue par réarrangement de gène ;
(vi) une séquence d' acide nucléique SYT obtenue par mutagenèse dirigée.

**5.** Méthode selon l'une quelconque des revendications 1 à 4, **caractérisée en ce que** ladite séquence d' acide nucléique code pour l'un quelconque parmi les polypeptides SYT donnés dans le Tableau 6, ou des orthologues ou paralogues de l'une quelconque parmi les SEQ ID susmentionnées et/ou **en ce que** ladite séquence d' acide nucléique code pour les polypeptides SYT tels que représentés par SEQ ID n° 60, SEQ ID n° 151 ou SEQ ID n° 153.

**6.** Méthode selon l'une quelconque des revendications 1 à 5, **caractérisée en ce que** ladite séquence d' acide nucléique codant pour un polypeptide SYT est surexprimée dans une plante.

**7.** Méthode selon l'une quelconque des revendications 1 à 6, **caractérisée en ce que** ladite séquence d' acide nucléique codant pour un polypeptide SYT est d' origine végétale.

**8.** Méthode selon l'une quelconque des revendications 1 à 7, **caractérisée en ce que** ladite séquence d' acide nucléique codant pour un polypeptide SYT est liée de manière opérante à un promoteur constitutif, préférablement où ledit promoteur constitutif est dérivé d' une plante, préférablement d' une plante monocotylédone, préférablement où ledit promoteur constitutif est un promoteur GOS2.

**9.** Méthode de production d' une plante transgénique, préférablement d' une plante monocotylédone, ayant une ou plusieurs caractéristiques parmi un rendement accru en graines total, un nombre accru de graines remplies, un taux de remplissage accru des graines, un PMG accru et un indice de moisson accru, ledit indice de moisson étant le rapport du rendement en graines divisé par la biomasse totale, par rapport à des plantes de type sauvage correspondantes, sous stress de disponibilité réduite en nutriments, laquelle méthode comprend :

(i) l'introduction et l'expression dans une plante ou un cellule végétale d' une séquence d' acide nucléique codant pour un polypeptide SYT tel que défini selon l'une quelconque des revendications 1-2, 4, 5 et 7 ;
(ii) la culture de la cellule végétale dans des conditions favorisant la croissance et le développement de la plante.

**10.** Utilisation d' une séquence d' acide nucléique SYT, ou utilisation d' un polypeptide SYT tel que défini selon l'une quelconque des revendications 1-2, 4, 5 et 7, pour améliorer le rendement sous stress de disponibilité réduite en nutriments par rapport à des plantes témoins et/ou où ledit rendement accru est l'un ou plusieurs parmi les caractéristiques suivantes : le rendement en graines total, le nombre de graines remplies, le taux de remplissage des graines, le PMG et l'indice de moisson, où l'indice de moisson est le rapport du rendement en graines divisé par la biomasse totale.

Plant SYT-like polypeptide structure

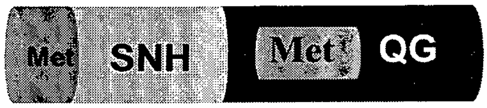


Mammalian SYT-like polypeptide structure

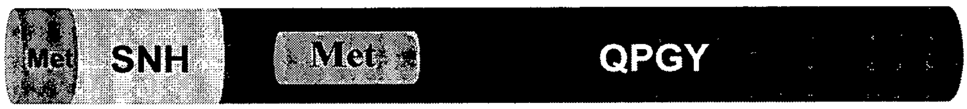


FIGURE 1

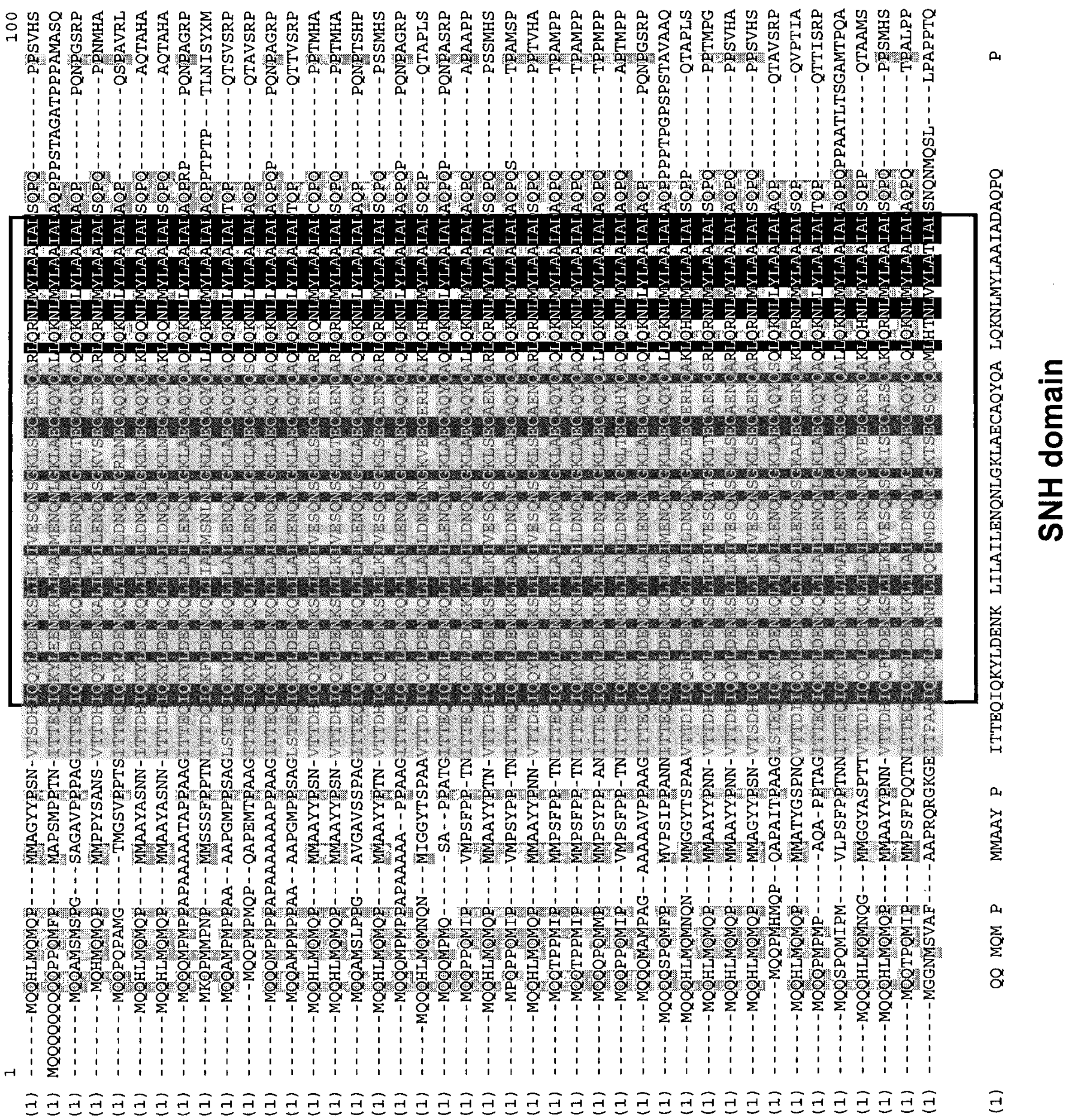


FIGURE 2

```
                        N-terminal                    SNH domain
                        Met-rich

                  1                                                50
Brana_SYT1   (1)  ------MQQHLMQMQP----MMAGYYPSN-VTSDHIQQYLDENKSLILKI
Aqufo_SYT1   (1)  --------MQHMQMQP----MMPPYSANS-VTTDHIQQYLDENKALILKI
Picsi_SYT1   (1)  ------MQQHLMQMQP----MMAAYASNN-ITTDHIQKYLDENKQLILAI
Pinta_SYT1   (1)  ------MQQHLMQMQP----MMAAYASNN-ITTDHIQKYLDENKQLILAI
Poptr_SYT1   (1)  ------MQQHLMQMQP----MMAAYYPSN-VTTDHIQQYLDENKSLILKI
Vitvi_SYT1   (1)  ------MQQHLMQMQP----MMAAYYPSN-VTTDHIQQYLDENKSLILKI
Soltu_SYT1   (1)  ------MQQHLMQMQP----MMAAYYPTN-VTTDHIQQYLDENKSLILKI
Lyces_SYT1   (1)  ------MQQHLMQMQP----MMAAYYPTN-VTTDHIQQYLDENKSLILKI
Goshi_SYT1   (1)  ------MQQHLMQMQP----MMAAYYPNN-VTTDHIQQYLDENKSLILKI
Zeama_SYT1   (1)  -----MQQQHLMQMNQN---MMGGYTSPAAVTTDLIQQHLDENKQLILAI
Medtr_SYT1   (1)  ------MQQHLMQMQP----MMAAYYPNN-VTTDHIQQYLDENKSLILKI
Citsi_SYT1   (1)  ------MQQHLMQMQP----MMAAYYPNN-VTTDHIQQYLDENKSLILKI
Arath_SYT1   (1)  ------MQQHLMQMQP----MMAGYYPSN-VTSDHIQQYLDENKSLILKI
Aspof_SYT1   (1)  ------MQQHLMQMQP----MMATYGSPNQVTTDIIQQYLDENKQLILAI
Orysa_SYT1   (1)  -----MQQQHLMQMNQG---MMGGYASPTTVTTDLIQQYLDENKQLILAI
Sacof_SYT1   (1)  -----MQQQHLMQMNQN---MIGGYTSPAAVTTDLIQQYLDENKQLILAI
Allce_SYT2   (1)  -------MQQPQPAMG-----TMGSVPPTSITTEQIQRYLDENKQLILAI
Lacse_SYT2   (1)  -------MKQPMMPNP----MMSSSFPPTNITTDQIQKFLDENKQLIIAI
Horvu_SYT2   (1)  -------MQQAMPMPPAA--AAPGMPPSAGLSTEQIQKYLDENKQLILAI
Brana_SYT2   (1)  MQQQQQQQQQPPQMFP----MAPSMPPTN-ITTEQIQKYLEENKKLIMAI
Sacof_SYT2   (1)  ---------MQQPMPMQP--QAPEMTPAAGITTEQIQKYLDENKQLILAI
Triae_SYT2   (1)  -------MQQAMPMPPAA--AAPGMPPSAGLSTEQIQKYLDENKQLILAI
Maldo_SYT2   (1)  -------MQQPPQMIP----VMPSFPP-TNITTEQIQKYLDDNKKLILAI
Goshi_SYT2   (1)  -------MPQPPQMIP----VMPSYPP-TNITTEQIQKYLDENKKLILAI
Glyso_SYT2   (1)  -------MQQTPPMIP----MMPSFPP-TNITTEQIQKYLDENKKLILAI
Glyma_SYT2   (1)  -------MQQTPPMIP----MMPSFPP-TNITTEQIQKYLDENKKLILAI
Eupes_SYT2   (1)  -------MQQQPQMMP----MMPSYPP-ANITTEQIQKYLDENKKLILAI
Arath_SYT2   (1)  -----MQQQQSPQMFP----MVPSIPPANNITTEQIQKYLDENKKLIMAI
Citsi_SYT2   (1)  -------MQQPPQMIP----VMPSFPP-TNITTEQIQKYLDENKKLILAI
Zeama_SYT2   (1)  ---------MQQPMHMQP--QAPAITPAAGISTEQIQKYLDENKQLILAI
Orysa_SYT2   (1)  ------MQQQPMPMP------AQA-PPTAGITTEQIQKYLDENKQLILAI
Soltu_SYT2   (1)  -----MQQQHLMQMQP----MMAAYYPNN-VTTDHIQQFLDENKSLILKI
Medtr_SYT2   (1)  -------MQQTPQMIP----MMPSFPQQTNITTEQIQKYLDENKKLILAI
Sorbi_SYT3   (1)  -------MQQQMPMPPAPAAAAATAPPAAGITTEQIQKYLDENKQLILAI
Zeama_SYT3   (1)  -------MQQQMPMPPAPAAAAAAAPPAAGITTEQIQKYLDENKQLILAI
Bradi_SYT3   (1)  -------MQQAMSMSPG---SAGAVPPPAGITTEQIQKYLDENKQLILAI
Triae_SYT3   (1)  -------MQQAMSLPPG---AVGAVSSPAGITTEQIQKYLDENKQLILAI
Sacof_SYT3   (1)  -------MQQQMPMPPAPAAAAA--PPAAGITTEQIQKYLDENKQLILAI
Panvi_SYT3   (1)  -------MQQQMPMQ------SA--PPATGITTEQIQKYLDENKQLILAI
Orysa_SYT3   (1)  -------MQQQMAMPAG--AAAAAVPPAAGITTEQIQKYLDENKQLILAI
Arath_SYT3   (1)  ------MQQSPQMIPM----VLPSFPPTNNITTEQIQKYLDENKKLIMAI
 Consensus   (1)        QQ MQM P    MMAAY P    ITTEQIQKYLDENK LILAI
```

**FIGURE 3**

```
                          SNH domain (continued)            Met-rich /
                                                            QG-rich domain
                          51                                               100
Brana_SYT1   (40)  VESQNSGKLSECAENQARLQRNLMYLAAIADSQPQ---------PPSVHS
Aqufo_SYT1   (38)  LENQNSGKVSECAENQARLQRNLMYLAAIADSQPQ---------PPNMHA
Picsi_SYT1   (40)  LDNQNLGKLNECAQYQAKLQQNLMYLAAIADSQPQ---------AQTAHA
Pinta_SYT1   (40)  LDNQNLGKLNECAQYQAKLQQNLMYLAAIADSQPQ---------AQTAHA
Poptr_SYT1   (40)  VESQNSGKLSECAENQARLQQNLMYLAAIADCQPQ---------PPTMHA
Vitvi_SYT1   (40)  VESQNSGKLTECAENQARLQRNLMYLAAIADSQPQ---------PPTMHA
Soltu_SYT1   (40)  VESQNSGKLSECAENQARLQRNLMYLAAIADSQPQ---------PSSMHS
Lyces_SYT1   (40)  VESQNSGKLSECAENQARLQRNLMYLAAIADSQPQ---------PSSMHS
Goshi_SYT1   (40)  VESQNSGKLSECAENQARLQRNLMYLAAIADSQPQ---------PPTVHA
Zeama_SYT1   (43)  LDNQNNGKAEECERHQAKLQHNLMYLAAIADSQPP---------QTAPLS
Medtr_SYT1   (40)  VESQNTGKLTECAENQSRLQRNLMYLAAIADSQPQ---------PPTMPG
Citsi_SYT1   (40)  VESQNSGKLSECAENQARLQRNLMYLAAIADAQPQ---------PPSVHA
Arath_SYT1   (40)  VESQNSGKLSECAENQARLQRNLMYLAAIADSQPQ---------PPSVHS
Aspof_SYT1   (41)  LENQNSGKADECAENQAKLQRNLMYLAAIADSQP----------QVPTIA
Orysa_SYT1   (43)  LDNQNNGKVEECARNQAKLQHNLMYLAAIADSQPP---------QTAAMS
Sacof_SYT1   (43)  LDNQNNGKVEECERHQAKLQHNLMYLAAIADSQPP---------QTAPLS
Allce_SYT2   (39)  LDNQNLGRLNECAQYQAQLQKNLLYLAAIADAQP---------QSPAVRL
Lacse_SYT2   (40)  MSNLNLGKLAECAQYQALLQKNLMYLAAIADAQPPTPTP---TLNISYXM
Horvu_SYT2   (42)  LENQNLGKLAECAQYQAQLQKNLLYLAAIADTQP---------QTSVSRP
Brana_SYT2   (46)  MENQNLGKLAECAQYQALLQKNLMYLAAIADAQPPPSTAGATPPPAMASQ
Sacof_SYT2   (40)  LENQNLGKLAECAQYQSQLQKNLLYLAAIADAQP---------QTAVSRP
Triae_SYT2   (42)  LENQNLGKLAECAQYQAQLQKNLLYLAAIADTQP---------QTTVSRP
Maldo_SYT2   (39)  LDNQNLGKLAECAQYQALLQKNLMYLAAIADAQPQ---------APAAPP
Goshi_SYT2   (39)  LDNQNLGKLAECAQYQAQLQKNLMYLAAIADAQPQS--------TPAMSP
Glyso_SYT2   (39)  LDNQNLGKLAECAQYQAQLQKNLMYLAAIADAQPQ---------TPAMPP
Glyma_SYT2   (39)  LDNQNLGKLAECAQYQAQLQKNLMYLAAIADAQPQ---------TPAMPP
Eupes_SYT2   (39)  LDNQNLGKLAECAQYQALLQKNLMYLAAIADAQPQ---------TPPMPP
Arath_SYT2   (42)  MENQNLGKLAECAQYQALLQKNLMYLAAIADAQPPPPTPGPSPSTAVAAQ
Citsi_SYT2   (39)  LDNQNLGKLTECAHYQAQLQKNLMYLAAIADAQPQ---------APTMPP
Zeama_SYT2   (40)  LENQNLGKLAECAQYQSQLQKNLLYLAAIADAQP---------QTAVSRP
Orysa_SYT2   (38)  LENQNLGKLAECAQYQAQLQKNLLYLAAIADTQP---------QTTISRP
Soltu_SYT2   (41)  VESQNSGKISECAESQAKLQRNLMYLAAIADSQPQ---------PPSMHS
Medtr_SYT2   (40)  LDNQNLGKLAECAQYQAQLQKNLMYLAAIADAQPQ---------TPALPP
Sorbi_SYT3   (44)  LENQNLGKLAECAQYQAQLQKNLLYLAAIADAQPRP------PQNPAGRP
Zeama_SYT3   (44)  LENQNLGKLAECAQYQAQLQKNLLYLAAIADAQPQP------PQNPAGRP
Bradi_SYT3   (41)  LENQNLGKLTECAQYQAQLQKNLLYLAAIADAQP--------PQNPGSRP
Triae_SYT3   (41)  LENQNLGKLAECAQYQAQLQKNLLYLAAIADAQP--------PQNPTSHP
Sacof_SYT3   (42)  LENQNLGKLAECAQYQAQLQKNLLYLAAIADAQPQP------PQNPAGRP
Panvi_SYT3   (36)  LENQNLGKLAECAQYQAQLQKNLLYLAAIADAQPQP------PQNPASRP
Orysa_SYT3   (42)  LENQNLGKLAECAQYQAQLQKNLLYLAAIADAQP--------PQNPGSRP
Arath_SYT3   (41)  LENQNLGKLAECAQYQALLQKNLMYLAAIADAQPQPPAATLTSGAMTPQA
 Consensus   (51)  LENQNLGKLAECAQYQA LQKNLMYLAAIADAQPQ          P
```

**FIGURE 3 (continued)**

**Met-rich / QG-rich domain (continued)**

```
                   101                                              150
Brana_SYT1   (81)  QYGSAGGGLIQGEGAS----HYLQQQQATQQ----------------QQ
Aqufo_SYT1   (79)  QYSNAG-----IPPGA----HYLQHQQAQQ-------------------
Picsi_SYT1   (81)  QIPPNA----VMQSGG----HYMQHQQAQQQ------------------
Pinta_SYT1   (81)  QIPPNA----VMQSGG----HYMQHQQAQQQ------------------
Poptr_SYT1   (81)  QFPSSG---IMQPGA-----HYMQHQQAQQ-------------------
Vitvi_SYT1   (81)  QFPPSG---IVQPGA-----HYMQHQQAQQ-------------------
Soltu_SYT1   (81)  QFSSGG----MMQPGT----HSYLQQQQQQQ---------------AQQ
Lyces_SYT1   (81)  QFSSGG----MMQPGT----HSYLQQQQQQQ---------------AQQ
Goshi_SYT1   (81)  QFPSGG---IMQPGAG----HYMQHQQAQQ-------------------
Zeama_SYT1   (84)  QYPSN----LMMQPGP----RYMPP-QSGQ------------------M
Medtr_SYT1   (81)  QYPSSG---MMQQGG-----HYMQAQQAQQ-------------------
Citsi_SYT1   (81)  QFSSGG---IMQPGA-----HYMQHQQSQP-------------------
Arath_SYT1   (81)  QYGSAGGGMIQGEGGS----HYLQQQQATQQ----------------QQ
Aspof_SYT1   (81)  QYPPNA--VAAMQSSA----RYMQQHQAAQ------------------Q
Orysa_SYT1   (84)  QYPSN----LMMQSGA----RYMPQ-QSAQ------------------M
Sacof_SYT1   (84)  QYPSN----LMMQPGP----RYMPP-QSGQ------------------M
Allce_SYT2   (80)  QMMPQG---AAATPQAGNQFMQQQSPNFPPKTG---------------MQ
Lacse_SYT2   (87)  GPVPHP---GMPQQGG--FYMAQQHPQAAVMTAQP-------------PS
Horvu_SYT2   (83)  QMAPPA---ASPGAG----HYMSQVPMFPPRT----------------P
Brana_SYT2   (96)  MGAPHP---GMQPP-----SYFMQHPQASGMAQQA-PPAGIFPPRGPLQF
Sacof_SYT2   (81)  QMAPPG---ALPGVG----QYMSQVPMFPPRT----------------P
Triae_SYT2   (83)  QMAPPS---ASPGAG----HYMSQVPMFPPRT----------------P
Maldo_SYT2   (80)  QMAPHP---AMQQA-----GYYMQHPQAAAMAQQQ----GIFSPKMPMQF
Goshi_SYT2   (81)  QMAPHP---AMQPG-----GYFMQHPQAAAMSQQP----GMYPQKVPLQF
Glyso_SYT2   (80)  QMAPHP---AMQP------GFYMQHPQAAAAAMAQQQQQGMFPQKMPLQF
Glyma_SYT2   (80)  QMAPHP---AMQP------GFYMQHPQAAAAAMAQQQQ-GMFPQKMPLQF
Eupes_SYT2   (80)  QMSPHP---AMQQG-----AYYMQHPQAAAAAMAHQSG-IFPPKMSPLQF
Arath_SYT2   (92)  MATPHS---GMQPP-----SYFMQHPQAS--------PAGIFAPRGPLQF
Citsi_SYT2   (80)  QMAPHP---AMQAS-----GYYMQHPQAAAMAQQQ----GIFPQKMPLQF
Zeama_SYT2   (81)  QMAPPG---GSPGVG----QYMSQVPMFPPRT----------------P
Orysa_SYT2   (79)  QMVPHG---ASPGLGG---QYMSQVPMFPPRT----------------P
Soltu_SYT2   (82)  QLASGG----MMQGGA----HYMQQQQ-------------------AQQ
Medtr_SYT2   (81)  QMAPHP---AMQQ------GFYMQHPQAAAMAQQQ----GMFPQKMPMQF
Sorbi_SYT3   (88)  QMMQPG---IVPGAG----HYMSQVPMFPPRT----------------P
Zeama_SYT3   (88)  QMMQPG---IVPGAG----HYMSQVPMFPPRT----------------P
Bradi_SYT3   (83)  QMVQPG---GMPGAG----HYMSQVPMFPPRT----------------P
Triae_SYT3   (83)  QMVQPG---SMQGAG----HYMSQVPMFPPRT----------------P
Sacof_SYT3   (86)  QMMQPG---IVPGAG----HYMSQVPMFPPRT----------------P
Panvi_SYT3   (80)  QMMQPG---MVPGAG----HYMSQVPMFPPRT----------------P
Orysa_SYT3   (84)  QMMQPG---ATPGAG----HYMSQVPMFPPRT----------------P
Arath_SYT3   (91)  MAPNPS---SMQPPP----SYFMQQHQAVG-MAQQ-IPPGIFPPRGPLQF
Consensus   (101)  QM   G    M  G      YYMQ PQA
```

**FIGURE 3 (continued)**

**Met-rich / QG-rich domain (continued)**

```
                         151                                              200
Brana_SYT1     (110)     MTQQSLMAAR---SSMMYQQQQQ--------PYATLQHQQ-----LHHSQ
Aqufo_SYT1     (100)     MTQQSLMAAR---SNMLYAQPITG----------MQQ-QQ-----AMHSQ
Picsi_SYT1     (104)     VTPQSLMAAR---SSMLYSQQPMAALHQAQQQQQQQHQQQQQ---SLHSQ
Pinta_SYT1     (104)     VTPQSLMAAR---SSILYAQQ----QQQQQHQQHQQQQQQQQ---SLHSQ
Poptr_SYT1     (103)     MTPQALMAAR---SSMLQYAQQP---------FSALQQQQ-----ALHSQ
Vitvi_SYT1     (103)     MTPQSLLAAR---SSML-YTQQP---------FSALQQQQ-----AIHSQ
Soltu_SYT1     (108)     MATQQLMAAR--SSSMLYGQQQQQ----Q--QQSQLSQFQQG---LHSSQ
Lyces_SYT1     (108)     MATQQLMAAR--SSSMLYGQQQ---------QQSQLSQYQQG---LHSSQ
Goshi_SYT1     (104)     MTQQSLMAAR---SSML-YSQQP---------FSALQQQQQQ---ALHSQ
Zeama_SYT1     (106)     MNPQSLMAAR---SSMMYAHPS----------LSPLQQQQ-----AAHGQ
Medtr_SYT1     (103)     MTQQQLMAAR---SSLM-YAQQ-------------LQQQQ-----ALQSQ
Citsi_SYT1     (103)     MTPQSLMAAR---SSMV-YSQQQ---------FSVLQQQQ-----ALHGQ
Arath_SYT1     (110)     MTQQSLMAAR---SSMLYAQQQQQ----QQ-PYATLQHQQ-----LHHSQ
Aspof_SYT1     (106)     MTPQSLMAAR---SSMLYSQSP----------MSALQQQQQQ--AAMHSQ
Orysa_SYT1     (106)     MAPQSLMAAR---SSMMYAQPA----------LSPLQQQQQQQAAAAHGQ
Sacof_SYT1     (106)     MSPQSLMAAR---SSMMYAHPS----------MSPLQQQQ-----AAHGQ
Allce_SYT2     (112)     FTPQQVQELQ---------QQQ----------LQHQPHMM----PPFQGQ
Lacse_SYT2     (119)     GFPQPMPGMQ---------FNS-----------------P----QAIQGQ
Horvu_SYT2     (109)     LTPQQMQEQQ---------LQQ-------------QQAQM----LPFAGQ
Brana_SYT2     (137)     GSPHQLQDPQ---------QQ---------------HMHQ----QAMQGH
Sacof_SYT2     (107)     LTPQQMQEQQ---------LQQ-------------QQAQL----LNFSGL
Triae_SYT2     (109)     LTPQQMQEQQ---------LQQ-------------QQAQM----LPFAGQ
Maldo_SYT2     (118)     NNMHQMHDP---------------------------QQHQ----QAMQGQ
Goshi_SYT2     (119)     NSPHQMQDPQ---------HLLY-------------QQHQ----QAMQGQ
Glyso_SYT2     (121)     GNPHQMQEQQ---------QQ----------------LHQ----QAIQGQ
Glyma_SYT2     (120)     GNPHQMQEQQ---------QQ----------------LHQ----QAIQGQ
Eupes_SYT2     (121)     NNPHQIQDPQ--------------------------QLHQ----AALQGQ
Arath_SYT2     (126)     GSPLQFQDPQ---------QQQ--------------QIHQ----QAMQGH
Citsi_SYT2     (118)     NNPHQLQDPQ---------QQLH-------------QH------QAMQAQ
Zeama_SYT2     (107)     LTPQQMQEQQ---------LQQ-------------QQAQL----LNFSGQ
Orysa_SYT2     (106)     LTPQQMQEQQ---------LQQ-------------QQAQL----LSFGGQ
Soltu_SYT2     (104)     LTTQSLMAAARSSSSMLYGQQQQQ----QQQQLSSLQQQQAA---FHSQQ
Medtr_SYT2     (118)     GNPHQMQDQQ---------HQQQQ-----------QQLHQ----QAMQGQ
Sorbi_SYT3     (114)     LTPQQMQEQQ---------QQQ----------LQQQQAQA----LAFPGQ
Zeama_SYT3     (114)     LTPQQMQEQQ---------QQQQF-------QQQQQQVQA----LTFPGQ
Bradi_SYT3     (109)     LTPQQMQEQQ---------HQQ----------LQQQQAQA----LAFPSQ
Triae_SYT3     (109)     LTPQQMQEQQ---------HQQ----------LQQQQAQA----LSFPAQ
Sacof_SYT3     (112)     LTPQQMQEQQ---------QQQ----------LQQQQAQA----LTFPGQ
Panvi_SYT3     (106)     LTPQQMQEQQ---------QQQQ--------QLQQQQAQA----LAFPGQ
Orysa_SYT3     (110)     LTPQQMQEQQ---------QQQ----------LQQQQAQA----LAFPGQ
Arath_SYT3     (132)     GSPHQFLDPQ---------QQ----------------LHQ----QAMQGH
 Consensus     (151)     MTPQQLQE Q         QQQ              Q QQ     A  GQ
```

**FIGURE 3 (continued)**

**Met-rich / QG-rich domain (continued)**

```
                      201                                               250
Brana_SYT1    (144)  LGMSSSS-GGG--------SSGLHILQG---EAG-------GFHEFGRG-
Aqufo_SYT1    (131)  LGMSS-----GG-------NSGLHMMHNEG---S-------MGGSGALGS
Picsi_SYT1    (148)  LGINS-----GG-------SSGLHMLHGETN-MG-------CNGPLSSGG
Pinta_SYT1    (144)  LGINS-----GG-------SSGLHMLHGETN-MG-------CNGPLSSGG
Poptr_SYT1    (136)  LGMSS-----GG-------SAGLHMMQSEANTAG-------GSGALGAGR
Vitvi_SYT1    (135)  LGMGS-----GG-------SAGLHMLQSEGSNPG-------GNGTLGTGG
Soltu_SYT1    (147)  LGMSSG--SGGS-------TGLHHMLQSE-------------SSPHGGGF
Lyces_SYT1    (144)  LGMSSG--SGGS-------TGLHHMLQSE-------------SSPHGGGF
Goshi_SYT1    (138)  LGMSS-----GG-------STGLHMLQTESSTAG-------GSGALGAGG
Zeama_SYT1    (138)  LGMAPGGGGGGT-------TSGFSILHGEASMGGGGAGAGAGNNMMNAGM
Medtr_SYT1    (131)  LGMNS-----SG-------SQGLHMLHSEGANVG-------GNSSLGAG-
Citsi_SYT1    (135)  LGMSS-----GG-------SSGLHMLQSEGSTAG-------GSGSLGGGG
Arath_SYT1    (147)  LGMSSSS-GGGG-------SSGLHILQG---EAG-------GFHDFGRG-
Aspof_SYT1    (141)  LAMSSGGNNS-S-------TGGFTILHGEASIGG-------NGSMNSGGV
Orysa_SYT1    (143)  LGMGSGG----T-------TSGFSILHGEASMGGGGGGGGAGNSMMNAGV
Sacof_SYT1    (138)  LGMASGGGGG-T-------TSGFNILHGEASMGG-AGGACAGNNMMNAGM
Allce_SYT2    (139)  MGMRP--MNGMQ-----------AAMHADSSLAY------NTNNKQDAG-
Lacse_SYT2    (139)  MGGRSGGPPSS---------------AASDVWRG------SMQDGG----
Horvu_SYT2    (133)  MVARPGAVNGIP-----------QAPQVEQP-------------------
Brana_SYT2    (159)  MGMRPMGINNNN-----------GMQHQMQQQQP------ETSLGGS---
Sacof_SYT2    (131)  MVARPGMVNGMP-----------QSIQVQQAQ----------PPPAGN--
Triae_SYT2    (133)  MVARPGAVNGMP-----------QAPQVEP--------------------
Maldo_SYT2    (137)  MGMRPGGPNGMP-----------SMLHTEATHGG------GS-GGPNSAG
Goshi_SYT2    (143)  MGIRPGGPNNSM-----------HPMHSEASLGG------GSSGGPPQPS
Glyso_SYT2    (142)  MGLRPGGINNGM-----------HPMHNE----G------GNSGGPPSAT
Glyma_SYT2    (141)  MGLRPGDINNGM-----------HPMHSEAALGG------GNSGGPPSAT
Eupes_SYT2    (141)  MGMRPMGPNNGM-----------HPMHPEANLGG------SN--------
Arath_SYT2    (149)  MGIRPMGMTNN------------GMQHAMQQ--P------ETGLGG----
Citsi_SYT2    (140)  MGMRPGATNNGM-----------HPMHAESSLGG------GSSGGPPSAS
Zeama_SYT2    (131)  MVARPGMVNGMA-----------QSMQAQLP------------P-GVN--
Orysa_SYT2    (130)  MVMRPGVVNGIP------------QLLQGEMHRG-------------AD-
Soltu_SYT2    (147)  LGMSSS--GGGS-------SSGLHMLQSEN---T-------HSASTGGGG
Medtr_SYT2    (144)  MGLRPGGINNGM-----------HPMHNEAALGG------SGSGGQMTGV
Sorbi_SYT3    (141)  MVMRPATINGMQQ--PMQADPAR-AAELQQPASV------PADGRVSK--
Zeama_SYT3    (144)  MVMRPGTINGMQQQQPMQADPARAAAELQQAAPI------PADGRGSK--
Bradi_SYT3    (136)  MVMRPGTVNGMQP--------MQADLQAAAAAPG------LADSRGSKQ-
Triae_SYT3    (136)  VVMRPGTVNGMQ-----------QPMQAAGDLQP------AAAPGGSKQ-
Sacof_SYT3    (139)  MVMRPATINGIQQ--PMQADPAR-AAELQQPPPI------PADGRVSKQ-
Panvi_SYT3    (135)  MVMRP-TINGMQP---MQADPAAAAASLQQSAPG------PTDGRGGK--
Orysa_SYT3    (137)  MLMRPGTVNGMQS--IPVADPAR-AADLQTAAPG------SVDGRGNE--
Arath_SYT3    (153)  MGIRPMGLNNNN-----------GLQHQMHHHET------ALAANNA---
 Consensus    (201)  MGMRPG  NG             ML  E    G                G
```

**FIGURE 3 (continued)**

Met-rich / QG-rich domain (continued)

| | | 251 300 |
|---|---|---|
| Brana_SYT1 | (174) | KPEMGSG-----------------------------------EGRGGSS |
| Aqufo_SYT1 | (159) | YSDYGRG----SGGG---------VTIASKQDGGS-----GSGEGRGGNS |
| Picsi_SYT1 | (178) | FPEFGRGSATSAEGMQANRGFTIDRGSNKQDGVGSENAHPGAGDGRGSST |
| Pinta_SYT1 | (174) | FPEFGRGSATSADGMQVNRGFAIDRGSNKQDGVGSENAHAGAGDGRGSST |
| Poptr_SYT1 | (167) | FPDFGMD----ASS-------RGIASGSKQDIRSA-----GSSEGRGGSS |
| Vitvi_SYT1 | (166) | FPDFSRG----TSGEGLQAAGRGMAGGSK---QDM-----GNAEGRGGNS |
| Soltu_SYT1 | (175) | SHDFGR-------------------ANKQDIGSS-----MSAEGRGGSS |
| Lyces_SYT1 | (172) | SHDFGR-------------------ANKQDIGSS-----MSAEGRGGSS |
| Goshi_SYT1 | (169) | FPDFGRG----SSGEGIHGG-RPMAGGSKQDIGSA-----GSAEGRGGSS |
| Zeama_SYT1 | (181) | FSGFGRS----GSG-------------------AKEG--STSLSVDVRG |
| Medtr_SYT1 | (161) | FPDFGRS----SAGDGLHG-----SGKQ-----DI-----GSTDGRGGSS |
| Citsi_SYT1 | (166) | FPDFGRG----SSGEGLHS----RGMGSKHDIGSS-----GSAEGRGGSS |
| Arath_SYT1 | (178) | KPEMGSG----GGG-----------------------------EGRGGSS |
| Aspof_SYT1 | (176) | FGDFGRS----SGG--------------------------------KQETG |
| Orysa_SYT1 | (182) | FSDFGRG----GGGG------------------GKEG--STSLSVDVRG |
| Sacof_SYT1 | (179) | FSGFGRS----GSG-------------------AKEG--STSLSVDVRG |
| Allce_SYT2 | (169) | --NAAYE------NTA----------------------ANTDGSIQKK |
| Lacse_SYT2 | (164) | ------G------GAA----------------------ADGGKDGHAG |
| Horvu_SYT2 | (153) | --AYAAG------GAS----------------------SEPSGTESHR |
| Brana_SYT2 | (189) | AANVGLR------GGK-------------------------------QDG |
| Sacof_SYT2 | (158) | --KQDAG------GVA----------------------SEPSGIENHR |
| Triae_SYT2 | (152) | --AYAAG------GAS----------------------SEPSGTESHR |
| Maldo_SYT2 | (169) | DPNDGRG------GSK----------------------QDASESGAGG |
| Goshi_SYT2 | (176) | GPSDGRA------GNK----------------------QEGSEAGGN- |
| Glyso_SYT2 | (171) | GPNDARG------GSK----------------------QDASEAGTAG |
| Glyma_SYT2 | (174) | GPNDARG------GSK----------------------QDASEAGTAG |
| Eupes_SYT2 | (166) | ---DGRG------GNK----------------------QDAPETGASG |
| Arath_SYT2 | (175) | --NVGLR------GGK-------------------------------QDG |
| Citsi_SYT2 | (173) | GPGDIRG------GNK----------------------QDASEAGTTG |
| Zeama_SYT2 | (155) | --KQDAG------GVA----------------------SEPSGTESHR |
| Orysa_SYT2 | (154) | --HQNAG------GAT----------------------SEPS--ESHR |
| Soltu_SYT2 | (178) | FPDFGRG----LGS------------GNKHEMGSS-----MSDQGRGGSS |
| Medtr_SYT2 | (177) | VVEQAR----------------------------------CFGAGTAG |
| Sorbi_SYT3 | (180) | --QDTAA------GVS----------------------SEPSANESHK |
| Zeama_SYT3 | (186) | --QDTAG------GAS----------------------SEPSANESHK |
| Bradi_SYT3 | (171) | --DAAVA------GAI----------------------SEPSGTESHK |
| Triae_SYT3 | (168) | --DAAVA------GAS----------------------SEPSGTKSHK |
| Sacof_SYT3 | (179) | --QDTTA------GVS----------------------SEPSANESHK |
| Panvi_SYT3 | (173) | --QDATA------GVS----------------------TEPSGTESHK |
| Orysa_SYT3 | (176) | ------Q------DAT----------------------SEPSGTESHK |
| Arath_SYT3 | (183) | GPNDASG------GGK----------------------PDGTNMSQSG |
| Consensus | (251) | GRG G G G |

FIGURE 3 (continued)

**Met-rich / QG-rich domain (continued)**

```
                         301                    324
Brana_SYT1     (188)  G-----DGGETLYLKS--SDDGN-
Aqufo_SYT1     (191)  GGQS-ADGGESLYLKN--SDEGN-
Picsi_SYT1     (228)  GGQN-ADESEPSYLKA--SEE---
Pinta_SYT1     (224)  GGQN-ADESEPSYLKA--SEEEGN
Poptr_SYT1     (201)  GGQGG-DGGETLYLKS--ADDGN-
Vitvi_SYT1     (204)  GGQGG-DGGETLYLKA--AEDGN-
Soltu_SYT1     (200)  ---GG-DGGENLYLKA--SED---
Lyces_SYT1     (197)  ---G---G-ENLYLKA--SED---
Goshi_SYT1     (209)  GGQGGGDGGETLYLKA--ADDGN-
Zeama_SYT1     (205)  GTSSGAQSGDGEYLKVGTEEEGS-
Medtr_SYT1     (192)  SGHSG-DGGETLYLKS--SGDGN-
Citsi_SYT1     (203)  GSQ---DGGETLYLKG--ADDGN-
Arath_SYT1     (195)  G-----DGGETLYLKS--SDDGN-
Aspof_SYT1     (191)  --SEGHGTETPMYLKG-SEEEGN-
Orysa_SYT1     (207)  -ANSGAQSGDGEYLKG-TEEEGS-
Sacof_SYT1     (203)  GTSSGAQSGDGEYLKAGTEEEGS-
Allce_SYT2     (187)  TANDDLDPSAANPRRSEDAKSS--
Lacse_SYT2     (178)  G---------------GPEEAK-
Horvu_SYT2     (171)  S------TGADNDGGSGLADQS--
Brana_SYT2     (202)  ADGQG-------------KDDGK-
Sacof_SYT2     (176)  S------TGGDNDGGSD-------
Triae_SYT2     (170)  S------TGADNDGGSGWADQS--
Maldo_SYT2     (189)  -DGQG--TSAGGRGTG-DGEDGK-
Goshi_SYT2     (195)  --GQG--STTGGHGGGDGADEAK-
Glyso_SYT2     (191)  GDGQG--SSAAAHNSGDG-EEAK-
Glyma_SYT2     (194)  GDGQG--SSAAAHNSGDG-EEAK-
Eupes_SYT2     (183)  GDGQG-------NSGGDGAEDGK-
Arath_SYT2     (186)  ADGQG-------------KDDGK-
Citsi_SYT2     (193)  ADGQG--SSAGGHGG--DGEEAK-
Zeama_SYT2     (173)  S------TGGD-DGGSD-------
Orysa_SYT2     (170)  S------TGTENDGGSDFGDQS--
Soltu_SYT2     (207)  SGHGG-DGGENLYLKS--SEDGN-
Medtr_SYT2     (191)  GDGQGT-SAAAAHNSGDASEEGK-
Sorbi_SYT3     (198)  TT-----TGADSEAGGDVAEKS--
Zeama_SYT3     (204)  SA-----TGADTEAGGDVAEKS--
Bradi_SYT3     (189)  S------TGADHEAGGDVAEQS--
Triae_SYT3     (186)  N------AGAEEVG-ADVAEQS--
Sacof_SYT3     (197)  TT-----TGADSEAGGDVAEKS--
Panvi_SYT3     (191)  ST-----TAADHDVGTDVAEKS--
Orysa_SYT3     (190)  S------AGADNDAGGDIAEKS--
Arath_SYT3     (203)  ADGQGG-S-AARHGGGDAKTEGK-
 Consensus     (301)         TG    Y  G  AEDG
```

## FIGURE 3 (continued)

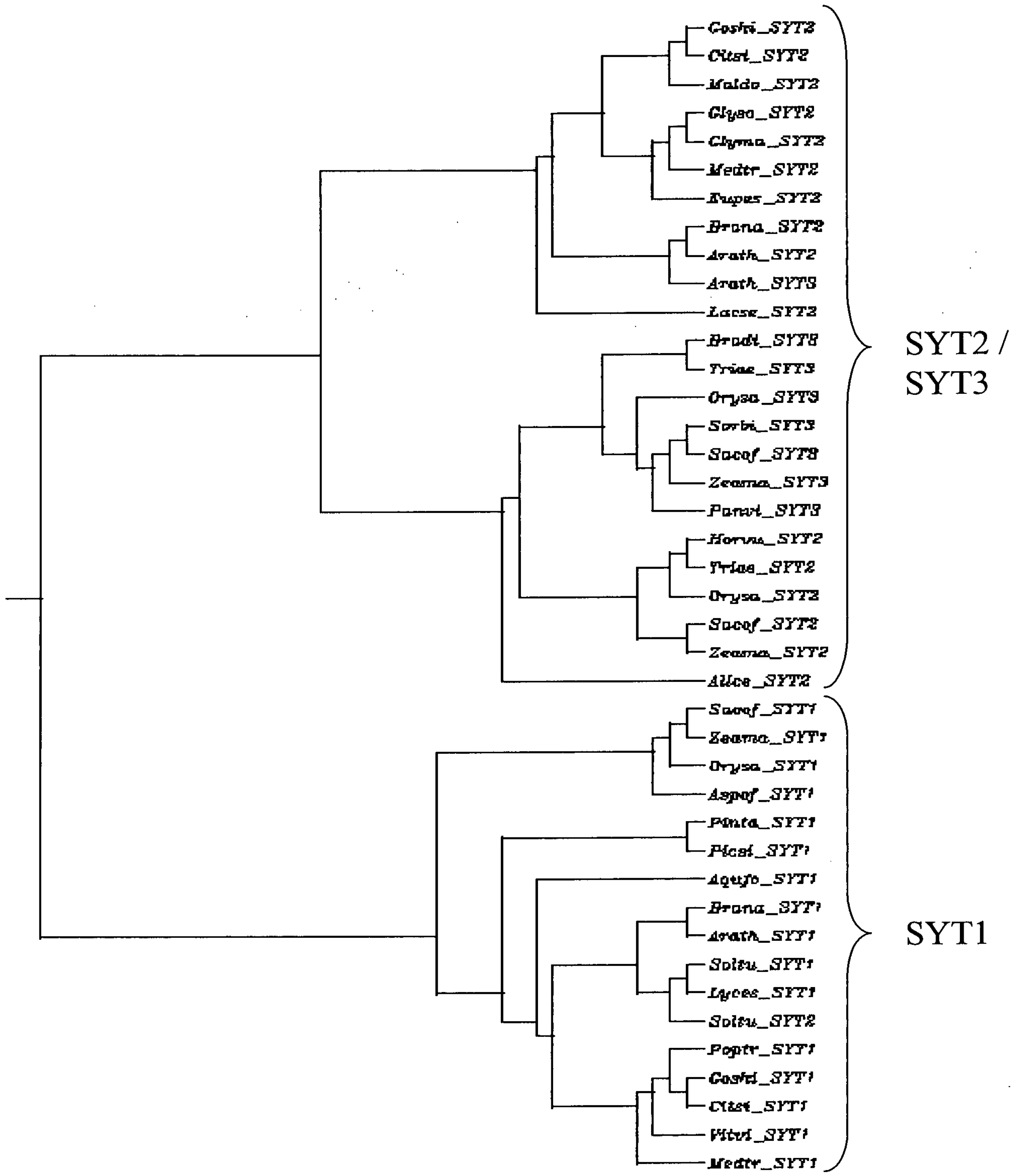


**10/31**

**FIGURE 4**

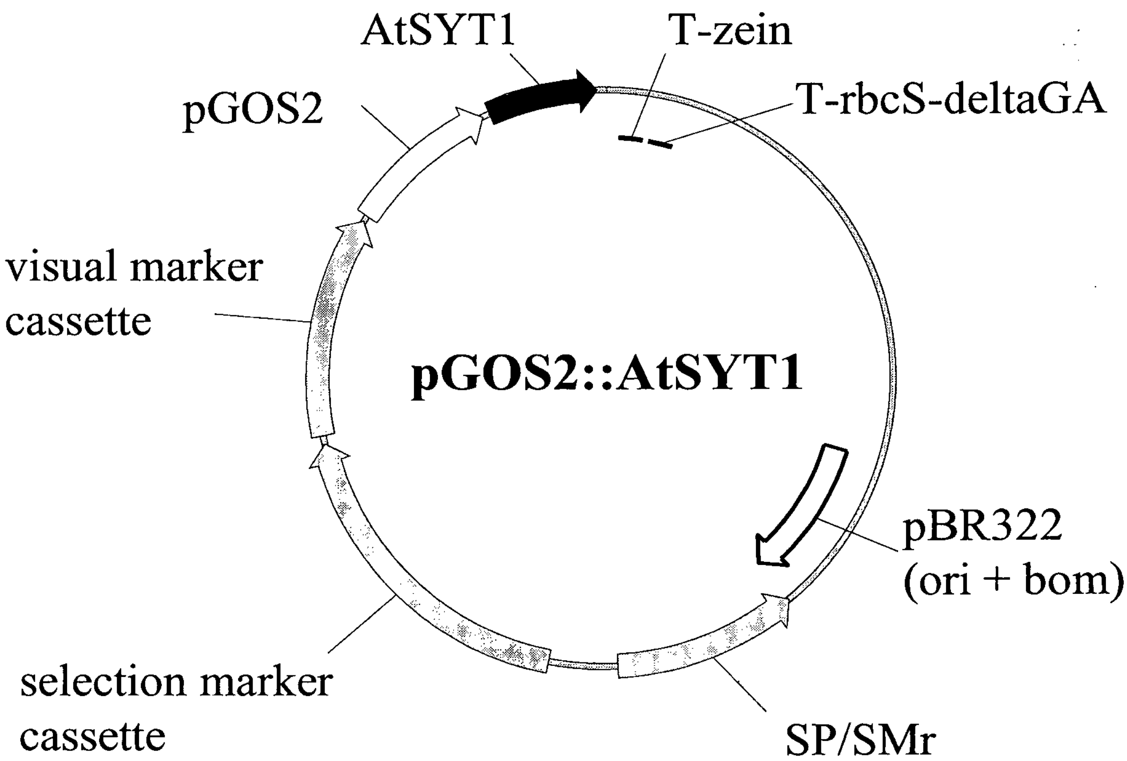
AtSYT1
T-zein
pGOS2
T-rbcS-deltaGA
visual marker
cassette
pGOS2::AtSYT1
pBR322
(ori + bom)
selection marker
cassette
SP/SMr

FIGURE 5

**SEQ ID NO: 56, rice GOS2 promoter**

AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT

## FIGURE 6

AAAGAGAGAGATTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTTCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTCGATCCATATCTTCCGGTCGAGTTCTTGGT
CGATCTCTTCCCTCCTCCACCTCCTCCTCACAGGGTATGTGCCTCCCTTCGGTTGTTCTTGGATTT
ATTGTTCTAGGTTGTGTAGTACGGGCGTTGATGTTAGGAAAGGGGATCTGTATCTGTGATGATTCC
TGTTCTTGGATTTGGGATAGAGGGGTTCTTGATGTTGCATGTTATCGGTTCGGTTTGATTAGTAGT
ATGGTTTTCAATCGTCTGGAGAGCTCTATGGAAATGAAATGGTTTAGGGATCGGAATCTTGCGATT
TTGTGAGTACCTTTTGTTTGAGGTAAAATCAGAGCACCGGTGATTTTGCTTGGTGTAATAAAGTAC
GGTTGTTTGGTCCTCGATTCTGGTAGTGATGCTTCTCGATTTGACGAAGCTATCCTTTGTTTATTC
CCTATTGAACAAAAATAATCCAACTTTGAAGACGGTCCCGTTGATGAGATTGAATGATTGATTCTT
AAGCCTGTCCAAAATTTCGCAGCTGGCTTGTTTAGATACAGTAGTCCCCATCACGAAATTCATGGA
AACAGTTATAATCCTCAGGAACAGGGGATTCCCTGTTCTTCCGATTTGCTTTAGTCCCAGAATTTT
TTTTCCCAAATATCTTAAAAAGTCACTTTCTGGTTCAGTTCAATGAATTGATTGCTACAAATAATG
CTTTTATAGCGTTATCCTAGCTGTAGTTCAGTTAATAGGTAATACCCCTATAGTTTAGTCAGGAGA
AGAACTTATCCGATTTCTGATCTCCATTTTTAATTATATGAAATGAACTGTAGCATAAGCAGTATT
CATTTGGATTATTTTTTTATTAGCTCTCACCCCTTCATTATTCTGAGCTGAAAGTCTGGCATGAA
CTGTCCTCAATTTTGTTTTCAAATTCACATCGATTATCTATGCATTATCCTCTTGTATCTACCTGT
AGAAGTTTCTTTTTGGTTATTCCTTGACTGCTTGATTACAGAAAGAAATTTATGAAGCTGTAATCG
GGATAGTTATACTGCTTGTTCTTATGATTCATTTCCTTTGTGCAGTTCTTGGTGTAGCTTGCCACT
TTCACCAGCAAAGTTC

**SEQ ID NO: 57, SNH domain from SYT-like polypeptides**
IQ(Q/K)XL(D/E)(E/D)N(K/N)XLIX(C/A/K)I(L/V/M)(E/D/S)(S/N)(Q/L)NXG(K
/R)XXEC(A/E/S)XXQ(A/S/Q)XL(Q/H)XNL(M/L/V)YLA(A/T)IAD

Where X is any amino acid

**SEQ ID NO: 58, *Arabidopsis thaliana* AtSYT1 SNH domain**
IQQYLDENKSLILKIVESQNSGKLSECAENQARLQRNLMYLAAIAD

**SEQ ID NO: 59, *Arabidopsis thaliana* AtSYT1 cDNA (AY102639)**
ATGCAACAGCACCTGATGCAGATGCAGCCCATGATGGCTGGTTACTACCCCAGCAATGTTACCTCT
GATCATATCCAACAGTACTTGGACGAAAACAAATCGTTGATTCTGAAGATTGTTGAGTCTCAAAAC
TCTGGAAAGCTTAGCGAATGCGCCGAGAATCAAGCAAGGCTTCAACGCAACCTAATGTACCTAGCT
GCAATAGCAGATTCTCAGCCTCAGCCACCAAGTGTGCATAGCCAGTATGGATCTGCTGGTGGTGGG
ATGATTCAGGGAGAAGGAGGGTCACACTATTTGCAGCAGCAACAAGCGACTCAACAGCAACAGATG
ACTCAGCAGTCTCTAATGGCGGCTCGATCTTCAATGTTGTATGCTCAGCAACAGCAGCAGCAGCAG
CCTTACGCGACGCTTCAGCATCAGCAATTGCACCATAGCCAGCTTGGAATGAGCTCGAGCAGCGGA
GGAGGAGGAAGCAGTGGTCTCCATATCCTTCAGGGAGAGGCTGGTGGGTTTCATGATTTTGGCCGT
GGGAAGCCGGAAATGGGAAGTGGTGGTGGCGGTGAAGGCAGAGGAGGAAGTTCAGGGGATGGTGGA
GAAACCCTTTACTTGAAATCATCAGATGATGGGAATTGA

**FIGURE 6 (continued)**

**SEQ ID NO: 60, *Arabidopsis thaliana* AtSYT1 polypeptide**
MQQHLMQMQPMMAGYYPSNVTSDHIQQYLDENKSLILKIVESQNSGKLSECAENQARLQRNLMYLA
AIADSQPQPPSVHSQYGSAGGGMIQGEGGSHYLQQQQATQQQQMTQQSLMAARSSMLYAQQQQQQQ
PYATLQHQQLHHSQLGMSSSSGGGGSSGLHILQGEAGGFHDFGRGKPEMGSGGGGEGRGGSSGDGG
ETLYLKSSDDGN

**SEQ ID NO: 61, *Arabidopsis thaliana* AtSYT2 cDNA (AY102640)**
ATGCAGCAGCAGCAGTCTCCGCAAATGTTTCCGATGGTTCCGTCGATTCCCCCTGCTAACAACATC
ACTACCGAACAGATCCAAAAGTACCTTGATGAGAACAAGAAGCTGATTATGGCCATCATGGAAAAC
CAGAATCTCGGTAAACTTGCTGAGTGCGCCCAGTACCAAGCTCTTCTCCAGAAGAACTTGATGTAT
CTTGCTGCAATTGCTGATGCTCAACCCCCACCACCTACGCCAGGACCTTCACCATCTACAGCTGTC
GCTGCCCAGATGGCAACACCGCATTCTGGGATGCAACCACCTAGCTACTTCATGCAACACCCACAA
GCATCCCCTGCAGGGATTTTCGCTCCAAGGGGTCCTTTACAGTTTGGTAGCCCACTCCAGTTTCAG
GATCCGCAACAGCAGCAGCAGATACATCAGCAAGCTATGCAAGGACACATGGGGATTAGACCAATG
GGTATGACCAACAACGGGATGCAGCATGCGATGCAACAACCAGAAACCGGTCTTGGAGGAAACGTG
GGGCTTAGAGGAGGAAAGCAAGATGGAGCAGATGGACAAGGAAAAGATGATGGCAAGTGA

**SEQ ID NO: 62, *Arabidopsis thaliana* AtSYT2 polypeptide**
MQQQQSPQMFPMVPSIPPANNITTEQIQKYLDENKKLIMAIMENQNLGKLAECAQYQALLQKNLMY
LAAIADAQPPPPTPGPSPSTAVAAQMATPHSGMQPPSYFMQHPQASPAGIFAPRGPLQFGSPLQFQ
DPQQQQQIHQQAMQGHMGIRPMGMTNNGMQHAMQQPETGLGGNVGLRGGKQDGADGQGKDDGK

**SEQ ID NO: 63, *Arabidopsis thaliana* AtSYT3 cDNA (AY102641)**
ATGCAGCAATCTCCACAGATGATTCCGATGGTTCTTCCTTCATTTCCGCCCACCAATAATATCACC
ACCGAACAGATCCAAAAGTATCTTGATGAGAACAAGAAGCTGATAATGGCGATCTTGGAAAATCAG
AACCTCGGTAAACTTGCAGAATGTGCTCAGTATCAAGCTCTTCTCCAGAAGAATTTGATGTATCTC
GCTGCAATTGCGGATGCTCAACCTCAGCCACCAGCAGCTACACTAACATCAGGAGCCATGACTCCC
CAAGCAATGGCTCCTAATCCGTCATCAATGCAGCCACCACCAAGCTACTTCATGCAGCAACATCAA
GCTGTGGGAATGGCTCAACAAATACCTCCTGGGATTTTCCCTCCTAGAGGTCCATTGCAATTTGGT
AGCCCGCATCAGTTTCTGGATCCGCAGCAACAGTTACATCAACAAGCTATGCAAGGGCACATGGGG
ATTAGACCAATGGGTTTGAATAATAACAACGGACTGCAACATCAAATGCACCACCATGAAACTGCT
CTTGCCGCAAACAATGCGGGTCCTAACGATGCTAGTGGAGGAGGTAAACCGGATGGGACCAATATG
AGCCAGAGTGGAGCTGATGGGCAAGGTGGCTCAGCCGCTAGACATGGCGGTGGTGATGCAAAAACT
GAAGGAAAATGA

**SEQ ID NO: 64, *Arabidopsis thaliana* ATSYT3 POLYPEPTIDE**
MQQSPQMIPMVLPSFPPTNNITTEQIQKYLDENKKLIMAILENQNLGKLAECAQYQALLQKNLMYL
AAIADAQPQPPAATLTSGAMTPQAMAPNPSSMQPPPSYFMQQHQAVGMAQQIPPGIFPPRGPLQFG
SPHQFLDPQQQLHQQAMQGHMGIRPMGLNNNNGLQHQMHHHETALAANNAGPNDASGGGKPDGTNM
SQSGADGQGGSAARHGGGDAKTEGK

**SEQ ID NO: 65, Aspergillus officinalis SYT cDNA (CV287542)**
ATGCAGCAGCACCTGATGCAGATGCAGCCCATGATGGCAACCTACGGTTCACCGAATCAGGTCACC
ACCGATATCATTCAGCAGTATCTGGACGAGAACAAGCAGTTGATTCTGGCTATTCTTGAAAACCAA
AATTCAGGAAAAGCTGATGAATGTGCTGAGAATCAGGCTAAGCTTCAGAGGAATCTGATGTATCTT
GCAGCCATTGCGGATAGCCAGCCCCAAGTTCCTACCATTGCTCAGTATCCTCCCAACGCTGTTGCT
GCTATGCAATCGAGTGCTCGCTACATGCAACAACACCAAGCAGCTCAACAGATGACCCCTCAATCT
CTCATGGCTGCTCGCTCCTCAATGCTCTACTCACAGTCCCCAATGTCTGCACTCCAGCAGCAACAG

## FIGURE 6 (continued)

CAGCAAGCAGCAATGCATAGCCAGCTCGCCATGAGCTCCGGAGGCAACAACAGCAGCACCGGAGGA
TTCACCATTCTTCATGGTGAAGCTAGCATAGGAGGCAATGGCTCAATGAATTCTGGTGGAGTCTTT
GGAGATTTTGGACGGAGCAGCGGTGGGAAGCAAGAGACTGGGAGCGAAGGGCACGGGACAGAGACT
CCTATGTACCTGAAAGGCTCTGAAGAAGAAGGAAACTGA

**SEQ ID NO: 66, *Aspergillus officinalis* SYT polypeptide**
MQQHLMQMQPMMATYGSPNQVTTDIIQQYLDENKQLILAILENQNSGKADECAENQAKLQRNLMYL
AAIADSQPQVPTIAQYPPNAVAAMQSSARYMQQHQAAQQMTPQSLMAARSSMLYSQSPMSALQQQQ
QQAAMHSQLAMSSGGNNSSTGGFTILHGEASIGGNGSMNSGGVFGDFGRSSGGKQETGSEGHGTET
PMYLKGSEEEGN

**SEQ ID NO: 67, *Brassica napus* SYT cDNA (CD823592)**
ATGCAGCCCATGATGGCTGGTTACTACCCCAGCAATGTCACCTCTGATCATATCCAGCAGTACTTG
GATGAGAACAAGTCTTTGATTCTGAAGATAGTTGAGTCTCAAAACTCAGGAAAGCTCAGCGAGTGT
GCCGAGAATCAGGCAAGGCTTCAACGCAACCTCATGTACTTGGCTGCAATAGCAGATTCTCAGCCT
CAACCTCCAAGCGTGCATAGCCAGTATGGATCTGCTGGTGGTGGGTTGATTCAGGGAGAAGGAGCG
TCACACTATTTGCAGCAGCAACAGGCGACTCAACAGCAGCAGATGACTCAGCAGTCTCTTATGGCA
GCTCGTTCTTCAATGATGTATCAGCAGCAGCAACAGCCTTATGCAACGCTTCAGCATCAGCAGTTG
CACCATAGCCAGCTTGGGATGAGCTCTAGCAGCGGAGGAGGAAGCAGTGGTCTCCATATCCTTCAG
GGAGAGGCTGGTGGGTTTCATGAATTTGGCCGTGGGAAGCCGGAGATGGGAAGTGGTGAAGGCAGG
GGTGGAAGCTCAGGGGATGGTGGAGAAACACTCTACTTGAAGTCATCAGATGATGGGAACTGA

**SEQ ID NO: 68, *Brassica napus* SYT polypeptide**
MQQHLMQMQPMMAGYYPSNVTSDHIQQYLDENKSLILKIVESQNSGKLSECAENQARLQRNLMYLA
AIADSQPQPPSVHSQYGSAGGGLIQGEGASHYLQQQQATQQQQMTQQSLMAARSSMMYQQQQQPYA
TLQHQQLHHSQLGMSSSSGGGSSGLHILQGEAGGFHEFGRGKPEMGSGEGRGGSSGDGGETLYLKS
SDDGN

**SEQ ID NO: 69, *Citrus sinensis* SYT cDNA (CB290588)**
ATGCAACAGCACCTGATGCAGATGCAGCCCATGATGGCAGCTTATTATCCCAACAACGTCACTACT
GACCACATTCAACAGTATCTAGATGAGAACAAATCATTGATTTTGAAGATTGTTGAGAGCCAGAAT
TCAGGGAAACTGAGCGAGTGTGCAGAGAACCAGGCAAGATTGCAGCGGAATCTCATGTACCTGGCT
GCTATTGCTGATGCTCAACCCCAACCACCTAGCGTTCATGCCCAGTTCTCTTCTGGTGGCATTATG
CAGCCAGGAGCTCACTATATGCAACACCAGCAATCTCAGCCAATGACACCACAGTCACTTATGGCT
GCACGCTCATCCATGGTGTACTCTCAACAGCAATTTTCAGTGCTTCAGCAACAGCAAGCCTTGCAT
GGTCAGCTTGGCATGAGCTCTGGTGGTAGCTCAGGACTTCACATGCTGCAAAGTGAGGGTAGTACT
GCAGGAGGTAGTGGTTCACTTGGGGGTGGGGGATTCCCTGATTTTGGCCGTGGCTCATCTGGTGAA
GGCTTGCACTCAAGGGGAATGGGGAGCAAGCATGATATAGGCAGTTCTGGATCTGCTGAAGGACGA
GGAGGGAGCTCAGGAAGCCAAGATGGAGGCGAAACTCTCTACTTGAAAGGGGCTGATGATGGAAAT
TAA

**SEQ ID NO: 70, *Citrus sinensis* SYT polypeptide**
MQQHLMQMQPMMAAYYPNNVTTDHIQQYLDENKSLILKIVESQNSGKLSECAENQARLQRNLMYLA
AIADAQPQPPSVHAQFSSGGIMQPGAHYMQHQQSQPMTPQSLMAARSSMVYSQQQFSVLQQQQALH
GQLGMSSGGSSGLHMLQSEGSTAGGSGSLGGGGFPDFGRGSSGEGLHSRGMGSKHDIGSSGSAEGR
GGSSGSQDGGETLYLKGADDG

## FIGURE 6 (continued)

**SEQ ID NO: 71, *Gossypium arboreum* SYT cDNA (BM359324)**
ATGCAGCAGCACCTGATGCAGATGCAGCCCATGATGGCAGCTTATTATCCCAACAACGTCACTACT
GATCATATTCAACAGTATCTCGATGAGAACAAGTCATTGATCTTAAAGATTGTTGAGAGCCAGAAT
TCTGGGAAATTGAGTGAATGTGCTGAGAACCAAGCAAGGCTGCAGCGAAACCTCATGTACCTGGCT
GCCATTGCGGATTCTCAACCCCAACCACCCACCGTGCATGCACAGTTTCCATCTGGTGGTATCATG
CAGCAAGGAGCTGGGCACTACATGCAGCACCAACAAGCTCAACANATGACACAACAGTCGCTTATG
GCTGCTCGGTCCTCAATGTTGTATTCTCAGCAACCATTTTCTGCACTGCAACAACAACAACAACAA
GGCTTTGCACAGTCAGCTTGGCATGAGCTCTGGCGGGAGCACAGGCCTTTCATATGCTGCAAACTG
AATCTAGTACTGCAGGGGGCAGTGAGACACCTTGGGCCCGAGGGTTGTCCTGATTTGGACGGGGGT
CTTTTGGAGAGGCATCCCTGGTGGCAGGCCAATGGCCGGGGGAACAACCAAAAATCCGGGGAGGCC
GGCTCACCTAAGGGCCGGGAGGAGCCCTTGGGGCAGGGGGGGGTGATGGGGGGAACCTCTTCTTAA

**SEQ ID NO: 72, *Gossypium arboreum* SYT polypeptide**
MQQHLMQMQPMMAAYYPNNVTTDHIQQYLDENKSLILKIVESQNSGKLSECAENQARLQRNLMYLA
AIADSQPQPPTVHAQFPSGGIMQQGAGHYMQHQQAQXMTQQSLMAARSSMLYSQQPFSALQQQQQQ
GFAQSAWHELWREHRPFICCKLNLVLQGAVRHLGPEGCPDLDGGLLERHPWWQANGRGNNQKSGEA
GSPKGREEPLGQGGVMGGTSS

**SEQ ID NO: 73, *Medicago truncu1ata* SYT cDNA (CA858507)**
ATGCAGCAGCACCTGATGCAGATGCAGCCCATGATGGCAGCTTACTATCCTAACAACGTCACTACT
GATCATATTCAACAGTATCTTGATGAGAACAAGTCCTTGATTCTCAAGATTGTTGAAAGCCAGAAC
ACTGGCAAGCTCACCGAGTGTGCTGAGAACCAATCAAGGCTTCAGAGAAATCTCATGTACCTAGCT
GCAATAGCTGATTCTCAACCCCAACCACCTACTATGCCTGGCCAGTACCCTTCAAGTGGAATGATG
CAGCAGGGAGGACACTACATGCAGGCTCAACAAGCTCAGCAGATGACACAACAACAATTAATGGCT
GCACGTTCCTCTCTTATGTATGCTCAACAGCTTCAACAGCAGCAAGCCTTGCAAAGCCAACTTGGT
ATGAATTCCAGTGGAAGTCAAGGCCTTCACATGTTGCATAGTGAAGGGGCTAATGTTGGAGGCAAT
TCATCTCTAGGGGCTGGTTTTCCTGATTTTGGCCGTAGCTCAGCCGGTGATGGTTTGCACGGCAGT
GGTAAGCAAGACATTGGAAGCACTGATGGCCGCGGTGGAAGCTCTAGTGGTCACTCTGGTGATGGC
GGCGAAACACTTTACCTGAAATCTTCTGGTGATGGGAATTAG

**SEQ ID NO: 74, *Medicago truncu1ata* SYT polypeptide**
MQQHLMQMQPMMAAYYPNNVTTDHIQQYLDENKSLILKIVESQNTGKLTECAENQSRLQRNLMYLA
AIADSQPQPPTMPGQYPSSGMMQQGGHYMQAQQAQQMTQQQLMAARSSLMYAQQLQQQQALQSQLG
MNSSGSQGLHMLHSEGANVGGNSSLGAGFPDFGRSSAGDGLHGSGKQDIGSTDGRGGSSSGHSGDG
GETLYLKSSGDGN

**SEQ ID NO: 75, *Oryza sativa* SYT1 cDNA (AK058575)**
ATGCAGCAGCAACACCTGATGCAGATGAACCAGGGCATGATGGGGGGATATGCTTCCCCTACCACC
GTCACCACTGATCTCATTCAGCAGTATCTGGATGAGAACAAGCAGCTGATCCTGGCCATCCTTGAC
AACCAGAACAATGGGAAGGTGGAAGAGTGCGCTCGGAACCAAGCTAAGCTCCAGCACAATCTCATG
TACCTCGCCGCCATCGCCGACAGCCAGCCGCCGCAGACGGCCGCCATGTCCCAGTATCCGTCGAAC
CTGATGATGCAGTCCGGGGCGAGGTACATGCCGCAGCAGTCGGCGCAGATGATGGCGCCGCAGTCG
CTGATGGCGGCGAGGTCTTCGATGATGTACGCGCAGCCGGCGCTGTCGCCGCTCCAGCAGCAGCAG
CAGCAGCAGGCGGCGGCGGCGCACGGGCAGCTGGGCATGGGCTCGGGGGGCACCACCAGCGGGTTC
AGCATCCTCCACGGCGAGGCCAGCATGGGCGGCGGCGGCGGCGGCGGTGGCGCCGGTAACAGCATG
ATGAACGCCGGCGTGTTCTCCGACTTCGGACGCGGCGGCGGCGGCGGCGGCAAGGAGGGGTCCACC
TCGCTGTCCGTCGACGTCCGGGGCGCCAACTCCGGCGCCCAGAGCGGCGACGGGGAGTACCTCAAG
GGCACCGAGGAGGAAGGCAGCTAG

## FIGURE 6 (continued)

**SEQ ID NO: 76, *Oryza sativa* SYT1 polypeptide**
mqqqhlmqmnqgmmggyaspttvttdliqqyldenkqlilaildnqnngkveecarnqaklqhnlm
ylaaiadsqppqtaamsqypsnlmmqsgarympqqsaqmmapqslmaarssmmyaqpalsplqqqq
qqqaaaahgqlgmgsggttsgfsilhgeasmgggggggagnsmmnagvfsdfgrggggggkegst
slsvdvrgansgaqsgdgeylkgteeegs

**SEQ ID NO: 77, *Oryza sativa* SYT2 cDNA (AK105366)**
ATGCAGCAGCAGCCGATGCCGATGCCCGCGCAGGCGCCGCCGACGGCCGGAATCACCACCGAGCAG
ATCCAAAAGTATCTGGATGAAAACAAGCAGCTTATTTTGGCTATTTTGGAAAATCAGAATCTGGGA
AAGTTGGCAGAATGTGCTCAGTATCAAGCGCAGCTTCAGAAGAATCTCTTGTACTTGGCTGCAATT
GCTGATACTCAACCGCAGACCACTATAAGCCGTCCCCAGATGGTGCCGCATGGTGCATCGCCGGGG
TTAGGGGGGCAATACATGTCGCAGGTGCCAATGTTCCCCCCCAGGACCCCTCTAACGCCCCAGCAG
ATGCAGGAGCAGCAGCTGCAGCAACAGCAAGCCCAGCTGCTCTCGTTCGGCGGTCAGATGGTTATG
AGGCCTGGCGTTGTGAATGGCATTCCTCAGCTTCTGCAAGGCGAAATGCACCGCGGAGCAGATCAC
CAGAACGCTGGCGGGGCCACCTCGGAGCCTTCCGAGAGCCACAGGAGCACCGGCACCGAAAATGAC
GGTGGAAGCGACTTCGGCGATCAATCCTAA

**SEQ ID NO: 78, *Oryza sativa* SYT2 polypeptide**
MQQQPMPMPAQAPPTAGITTEQIQKYLDENKQLILAILENQNLGKLAECAQYQAQLQKNLLYLAAI
ADTQPQTTISRPQMVPHGASPGLGGQYMSQVPMFPPRTPLTPQQMQEQQLQQQQAQLLSFGGQMVM
RPGVVNGIPQLLQGEMHRGADHQNAGGATSEPSESHRSTGTENDGGSDFGDQS

**SEQ ID NO: 79, *Oryza sativa* SYT3 cDNA (BP185008)**
ATGCAGCAGCAGATGGCCATGCCGGCGGGGGCCGCCGCCGCCGCGGTGCCGCCGGCGGCCGGCATC
ACCACCGAGCAGATCCAAAAGTATTTGGATGAAAATAAACAGCTAATTTTGGCCATCCTGGAAAAT
CAAAACCTAGGGAAGTTGGCTGAATGTGCTCAGTACCAAGCTCAGCTTCAAAAGAATCTCTTGTAT
CTGGCTGCCATTGCAGATGCCCAACCACCTCAGAATCCAGGAAGTCGCCCTCAGATGATGCAGCCT
GGTGCTACCCCAGGTGCTGGGCATTACATGTCCCAAGTACCGATGTTCCCTCCAAGAACTCCCTTA
ACCCCACAACAGATGCAAGAGCAGCAGCAGCAGCAACTCCAGCAACAGCAAGCTCAGGCTCTAGCC
TTCCCCGGCCAGATGCTAATGAGACCAGGTACTGTCAATGGCATGCAATCTATCCCAGTTGCTGAC
CCTGCTCGCGCAGCCGATCTTCAGACGGCAGCACCGGGCTCGGTAGATGGCCGAGGAAACAAGCAG
GATGCAACCTCGGAGCCTTCCGGGACCGAGAGCCACAAGAGTGCGGGAGCAGATAACGACGCAGGC
GGTGACATAGCGGAGAAGTCCTGA

**SEQ ID NO: 80, *Oryza sativa* SYT3 polypeptide**
MQQQMAMPAGAAAAAVPPAAGITTEQIQKYLDENKQLILAILENQNLGKLAECAQYQAQLQKNLLY
LAAIADAQPPQNPGSRPQMMQPGATPGAGHYMSQVPMFPPRTPLTPQQMQEQQQQLQQQQAQALA
FPGQMLMRPGTVNGMQSIPVADPARAADLQTAAPGSVDGRGNKQDATSEPSGTESHKSAGADNDAG
GDIAEKS

**SEQ ID NO: 81, *SOLANUM TUBEROSUM* SYT CDNA (BG590990)**
ATGCAGCAGCAGCACCTGATGCAGATGCAGCCCATGATGGCAGCCTATTATCCCAACAATGTCACT
ACTGATCATATTCAACAGTTCCTGGATGAGAACAAATCACTTATTCTGAAGATTGTTGAGAGCCAG
AACTCTGGGAAAATAAGTGAATGTGCAGAGTCCCAAGCTAAACTTCAGAGAAATCTTATGTACCTT
GCAGCTATTGCTGATTCACAGCCCCAGCCTCCTAGTATGCATTCACAGTTAGCTTCTGGTGGGATG
ATGCAGGGAGGGGCACATTATATGCAGCAACAACAAGCTCAACAACTCACAACGCAATCGCTTATG
GCTGCAGCAAGATCCTCCTCCTCAATGCTCTATGGACAACAACAACAACAACAACAACAACAACTA
TCATCATTGCAACAACAGCAAGCAGCCTTTCATAGCCAGCAACTCGGAATGAGCAGCTCTGGTGGA
GGAAGCAGTAGTGGACTTCACATGCTACAAAGCGAAAACACTCATAGTGCTAGCACTGGTGGTGGG
TGGTTTCCCTGA

**FIGURE 6 (continued)**

**SEQ ID NO: 82, *Solanum tuberosum* SYT polypeptide**
MQQQHLMQMQPMMAAYYPNNVTTDHIQQFLDENKSLILKIVESQNSGKISECAESQAKLQRNLMYL
AAIADSQPQPPSMHSQLASGGMMQGGAHYMQQQQAQQLTTQSLMAAARSSSSMLYGQQQQQQQQL
SSLQQQQAAFHSQQLGMSSSGGGSSSGLHMLQSENTHSASTGGGWFP

**SEQ ID NO: 83, *Zea mays* SYT1 cDNA (BG874129.1, CA409022.1; compiled)**
ATGCAGCAGCAACACCTGATGCAGATGAACCAGAACATGATGGGGGGCTACACCTCTCCTGCCGCC
GTGACCACCGATCTCATCCAGCAGCACCTGGACGAGAACAAGCAGCTGATCCTGGCCATCCTCGAC
AACCAGAACAATGGCAAGGCGGAGGAGTGCGAACGGCACCAAGCTAAGCTCCAGCACAACCTCATG
TACCTGGCCGCCATCGCTGACAGCCAGCCGCCACAGACCGCGCCACTATCACAGTACCCGTCCAAC
CTGATGATGCAGCCGGGCCCTCGGTACATGCCACCGCAGTCCGGGCAGATGATGAACCCGCAGTCG
CTGATGGCGGCGCGGTCCTCCATGATGTACGCGCACCCGTCCCTGTCGCCACTCCAGCAGCAGCAG
GCGGCGCACGGACAGCTGGGTATGGCTCCAGGGGGCGGCGGTGGCGGCACGACCAGCGGGTTCAGC
ATCCTCCACGGCGAGGCCAGCATGGGCGGTGGTGGTGCTGGCGCAGGCGCCGGCAACAACATGATG
AACGCCGGCATGTTCTCGGGCTTTGGCCGCAGCGGCAGTGGCGCCAAGGAAGGGTCGACCTCTCTG
TCGGTTGACGTCCGGGGTGGAACCAGCTCCGGCGCGCAGAGCGGGGACGGCGAGTACCTCAAAGTC
GGCACCGAGGAAGAAGGCAGTTAG

**SEQ ID NO: 84, *Zea mays* SYT1 polypeptide**
mqqqhlmqmnqnmmggytspaavttdliqqhldenkqlilaildnqnngkaeecerhqaklqhnlm
YLAAIADSQPPQTAPLSQYPSNLMMQPGPRYMPPQSGQMMNPQSLMAARSSMMYAHPSLSPLQQQQ
AAHGQLGMAPGGGGGGTTSGFSILHGEASMGGGGAGAGAGNNMMNAGMFSGFGRSGSGAKEGSTSL
SVDVRGGTSSGAQSGDGEYLKVGTEEEGS

**SEQ ID NO: 85, *Zea mays* SYT2 cDNA (AY106697)**
ATGCAGCAGCCGATGCACATGCAGCCACAGGCGCCGGCGATAACCCCAGCTGCCGGAATCAGCACG
GAGCAGATCCAAAAGTATCTGGATGAGAATAAGCAGCTTATTTTGGCTATTTTGGAAAATCAGAAC
CTAGGAAAATTGGCAGAATGTGCTCAGTATCAATCACAACTTCAGAAGAACCTCTTGTATCTCGCT
GCAATCGCAGATGCTCAACCGCAGACTGCTGTAAGCCGCCCTCAGATGGCGCCGCCTGGTGGATCG
CCTGGAGTAGGGCAGTACATGTCACAGGTGCCTATGTTCCCACCGAGGACACCTCTTACACCCCAG
CAGATGCAGGAGCAGCAGCTTCAGCAGCAGCAGGCTCAGTTGCTAAACTTCAGTGGCCAAATGGTT
GCTAGACCAGGCATGGTCAACGGCATGGCTCAGTCCATGCAAGCTCAGCTACCACCGGGTGTGAAC
AAGCAGGATGCTGGTGGGGTCGCCTCTGAGCCCTCGGGCACCGAGAGCCACAGGAGCACTGGTGGT
GACGATGGTGGAAGCGACTAG

**SEQ ID NO: 86, *Zea mays* SYT2 polypeptide**
MQQPMHMQPQAPAITPAAGISTEQIQKYLDENKQLILAILENQNLGKLAECAQYQSQLQKNLLYLA
AIADAQPQTAVSRPQMAPPGGSPGVGQYMSQVPMFPPRTPLTPQQMQEQQLQQQQAQLLNFSGQMV
ARPGMVNGMAQSMQAQLPPGVNKQDAGGVASEPSGTESHRSTGGDDGGSD

**SEQ ID NO: 87, *Homo sapiens* cDNA (CR542103)**
ATGGGCGGCAACATGTCTGTGGCTTTCGCGGCCCCGAGGCAGCGAGGCAAGGGGGAGATCACTCCC
GCTGCGATTCAGAAGATGTTGGATGACAATAACCATCTTATTCAGTGTATAATGGACTCTCAGAAT
AAAGGAAAGACCTCAGAGTGTTCTCAGTATCAGCAGATGTTGCACACAAACTTGGTATACCTTGCT
ACAATAGCAGATTCTAATCAAAATATGCAGTCTCTTTTACCAGCACCACCCACACAGAATATGCCT
ATGGGTCCTGGAGGGATGAATCAGAGCGGCCCTCCCCCACCTCCACGCTCTCACAACATGCCTTCA
GATGGAATGGTAGGTGGGGGTCCTCCTGCACCGCACATGCAGAACCAGATGAACGGCCAGATGCCT

## FIGURE 6 (continued)

GGGCCTAACCATATGCCTATGCAGGGACCTGGACCCAATCAACTCAATATGACAAACAGTTCCATG
AATATGCCTTCAAGTAGCCATGGATCCATGGGAGGTTACAACCATTCTGTGCCATCATCACAGAGC
ATGCCAGTACAGAATCAGATGACAATGAGTCAGGGACAACCAATGGGAAACTATGGTCCCAGACCA
AATATGAGTATGCAGCCAAACCAAGGTCCAATGATGCATCAGCAGCCTCCTTCTCAGCAATACAAT
ATGCCACAGGGAGGCGGACAGCATTACCAAGGACAGCAGCCACCTATGGGAATGATGGGTCAAGTT
AACCAAGGCAATCATATGATGGGTCAGAGACAGATTCCTCCCTATAGACCTCCTCAACAGGGCCCA
CCACAGCAGTACTCAGGCCAGGAAGACTATTACGGGGACCAATACAGTCATGGTGGACAAGGTCCT
CCAGAAGGCATGAACCAGCAATATTACCCTGATGGAAATTCACAGTATGGCCAACAGCAAGATGCA
TACCAGGGACCACCTCCACAACAGGGATATCCACCCCAGCAGCAGCAGTACCCAGGGCAGCAAGGT
TACCCAGGACAGCAGCAGGGCTACGGTCCTTCACAGGGTGGTCCAGGTCCTCAGTATCCTAACTAC
CCACAGGGACAAGGTCAGCAGTATGGAGGATATAGACCAACACAGCCTGGACCACCACAGCCACCC
CAGCAGAGGCCTTATGGATATGACCAGGGACAGTATGGAAATTACCAGCAG

**SEQ ID NO: 88, *Homo sapiens* SYT polypeptide (CAG46900.1)**
MGGNMSVAFAAPRQRGKGEITPAAIQKMLDDNNHLIQCIMDSQNKGKTSECSQYQQMLHTNLVYLA
TIADSNQNMQSLLPAPPTQNMPMGPGGMNQSGPPPPPRSHNMPSDGMVGGGPPAPHMQNQMNGQMP
GPNHMPMQGPGPNQLNMTNSSMNMPSSSHGSMGGYNHSVPSSQSMPVQNQMTMSQGQPMGNYGPRP
NMSMQPNQGPMMHQQPPSQQYNMPQGGGQHYQGQQPPMGMMGQVNQGNHMMGQRQIPPYRPPQQGP
PQQYSGQEDYYGDQYSHGGQGPPEGMNQQYYPDGNSQYGQQQDAYQGPPPQQGYPPQQQQYPGQQG
YPGQQQGYGPSQGGPGPQYPNYPQGQGQQYGGYRPTQPGPPQPPQQRPYGYDQGQYGNYQQ

**SEQ ID NO: 89, *Allium cepa* SYT2 cDNA CF437485**
ATGCAGCAGCCGCAGCCAGCGATGGGAACCATGGGCTCGGTGCCACCTACTAGCATCACCACCGAA
CAGATTCAAAGGTACTTGGATGAGAACAAACAGTTAATATTGGCAATTTTGGATAATCAAAATTTA
GGAAGACTGAATGAGTGTGCTCAATATCAAGCTCAGCTTCAAAAGAATCTGCTTTACCTGGCAGCA
ATAGCTGATGCTCAGCCTCAGTCTCCTGCGGTGCGTCTGCAGATGATGCCTCAAGGTGCAGCTGCC
ACGCCTCAAGCTGGAAACCAATTTATGCAGCAGCAGAGCCCTAATTTCCCTCCCAAAACAGGAATG
CAATTTACTCCTCAACAAGTACAAGAATTGCAGCAGCAACAGCTACAACATCAGCCACATATGATG
CCTCCATTTCAAGGTCAAATGGGTATGAGACCTATGAATGGAATGCAGGCAGCAATGCATGCAGAT
TCATCTCTTGCTTATAACACTAACAATAAGCAAGATGCAGGAAACGCAGCTTATGAAAATACTGCT
GCCAACACAGATGGTTCCATTCAAAAGAAAACAGCAAATGATGATTTAGACCCTTCTGCAGCAAAC
CCTAGAAGGTCTGAAGATGCCAAATCATCATGA

**SEQ ID NO: 90, *Allium cepa* SYT2 polypeptide**
MQQPQPAMGTMGSVPPTSITTEQIQRYLDENKQLILAILDNQNLGRLNECAQYQAQLQKNLLYLAA
IADAQPQSPAVRLQMMPQGAAATPQAGNQFMQQQSPNFPPKTGMQFTPQQVQELQQQQLQHQPHMM
PPFQGQMGMRPMNGMQAAMHADSSLAYNTNNKQDAGNAAYENTAANTDGSIQKKTANDDLDPSAAN
PRRSEDAKSS

**SEQ ID NO: 91, *Aquilegia formosa* x *Aquilegia pubescens* SYT1 cDNA DT758802.1**
ATGCAACACATGCAGATGCAGCCCATGATGCCACCTTATAGTGCCAACAGCGTCACTACTGATCAT
ATCCAACAGTACTTGGATGAAAATAAGGCGTTGATTCTGAAGATACTTGAGAACCAAAATTCGGGA
AAAGTTAGTGAATGTGCAGAGAACCAAGCAAGACTTCAACGAAATCTTATGTATCTGGCTGCAATT
GCTGATTCTCAACCACAGCCTCCCAATATGCATGCTCAGTACTCTAATGCGGGTATACCACCTGGT
GCACATTACCTACAACACCAACAGGCCCAACAGATGACACAACAGTCGCTCATGGCTGCTCGATCA
AATATGCTGTATGCTCAGCCAATCACAGGAATGCAGCAACAGCAAGCAATGCATAGCCAGCTTGGC

**FIGURE 6 (continued)**

ATGAGCTCTGGTGGTAACAGTGGACTCCACATGATGCACAATGAGGGCAGCATGGGAGGTAGTGGG
GCACTTGGAAGCTATTCTGATTATGGCCGTGGCAGTGGTGGTGGAGTAACTATCGCTAGCAAACAA
GATGGTGGAAGTGGTTCTGGTGAAGGACGAGGTGGAAACTCTGGAGGCCAAAGTGCAGATGGAGGT
GAATCTCTTTACCTGAAAAACAGTGACGAAGGGAACTAA

**SEQ ID NO: 92, *Aquilegia formosa* x *Aquilegia pubescens* SYT1 polypeptide**

MQHMQMQPMMPPYSANSVTTDHIQQYLDENKALILKILENQNSGKVSECAENQARLQRNLMYLAAI
ADSQPQPPNMHAQYSNAGIPPGAHYLQHQQAQQMTQQSLMAARSNMLYAQPITGMQQQQAMHSQLG
MSSGGNSGLHMMHNEGSMGGSGALGSYSDYGRGSGGGVTIASKQDGGSGSGEGRGGNSGGQSADGG
ESLYLKNSDEGN

**SEQ ID NO: 93, *Brachypodium distachyon* SYT23 cDNA DV480064.1**

ATGCAGCAGGCGATGTCCATGTCCCCGGGGTCGGCCGGCGCGGTGCCGCCTCCGGCCGGCATCACC
ACAGAGCAGATCCAAAAGTATTTGGATGAAAATAAGCAACTTATTTTGGCCATCCTGGAAAATCAG
AACCTAGGAAAGTTGACTGAATGTGCTCAGTATCAAGCTCAACTTCAGAAGAATCTCTTGTATCTG
GCTGCCATTGCGGATGCCCAACCACCACAGAACCCTGGAAGTCGCCCCCAGATGGTGCAGCCTGGT
GGTATGCCAGGTGCAGGGCATTACATGTCGCAAGTACCAATGTTCCCTCCAAGAACCCCTTTAACC
CCACAACAGATGCAAGAGCAACAGCACCAGCAGCTTCAGCAGCAGCAAGCACAGGCTCTTGCTTTC
CCCAGCCAGATGGTCATGAGACCAGGTACTGTGAACGGCATGCAGCCTATGCAAGCTGATCTCCAA
GCAGCAGCAGCAGCACCTGGCCTGGCAGACAGCCGAGGAAGTAAGCAGGACGCAGCGGTAGCTGGG
GCCATCTCGGAACCTTCTGGCACCGAGAGTCACAAGAGTACAGGAGCGGATCATGAGGCAGGTGGC
GATGTAGCTGAGCAATCCTAA

**SEQ ID NO: 94, *Brachypodium distachyon* SYT3 polypeptide**

MQQAMSMSPGSAGAVPPPAGITTEQIQKYLDENKQLILAILENQNLGKLTECAQYQAQLQKNLLYL
AAIADAQPPQNPGSRPQMVQPGGMPGAGHYMSQVPMFPPRTPLTPQQMQEQQHQQLQQQQAQALAF
PSQMVMRPGTVNGMQPMQADLQAAAAAPGLADSRGSKQDAAVAGAISEPSGTESHKSTGADHEAGG
DVAEQS

**SEQ ID NO: 95, *Brassica napus* SYT2 cDNA CN732814**

ATGCAGCAGCAGCAGCAGCAGCAGCAGCAGCCTCCGCAAATGTTTCCGATGGCTCCTTCGATGCCG
CCAACTAACATCACCACCGAACAGATCCAAAAGTACCTTGAGGAGAACAAGAAGCTGATAATGGCA
ATCATGGAAAATCAGAATCTTGGCAAGCTTGCAGAGTGTGCACAGTACCAAGCTCTTCTCCAGAAG
AACTTAATGTACCTCGCTGCTATTGCTGATGCTCAACCTCCTCCATCTACCGCTGGAGCTACACCA
CCACCAGCTATGGCTTCCCAGATGGGGGCACCGCATCCTGGGATGCAACCGCCGAGCTACTTTATG
CAACACCCACAAGCTTCAGGGATGGCTCAACAAGCACCACCCGCTGGTATCTTCCCTCCGAGAGGT
CCTTTGCAGTTTGGTAGCCCACACCAGCTTCAGGATCCGCAACAGCAGCATATGCATCAACAGGCT
ATGCAAGGACACATGGGGATGCGACCAATGGGTATCAACAACAACAATGGGATGCAGCATCAGATG
CAGCAACAACAACCAGAAACCTCTCTTGGAGGAAGCGCTGCAAACGTGGGGCTTAGAGGTGGAAAG
CAAGATGGAGCAGATGGACAAGGAAAAGATGATGGCAAATGA

**SEQ ID NO: 96, *Brassica napus* SYT2 polypeptide**

MQQHLMQMQPMMAGYYPSNVTSDHIQQYLDENKSLILKIVESQNSGKLSECAENQARLQRNLMYLA
AIADSQPQPPSVHSQYGSAGGGLIQGEGASHYLQQQQATQQQMTQQSLMAARSSMMYQQQQQPYA
TLQHQQLHHSQLGMSSSSGGGSSGLHILQGEAGGFHEFGRGKPEMGSGEGRGGSSGDGGETLYLKS
SDDGN

## FIGURE 6 (continued)

**SEQ ID NO: 97, *Citrus sinensis* SYT2 cDNA CV717501**
ATGCAGCAGCCACCGCAAATGATCCCTGTTATGCCTTCATTTCCACCCACCAACATCACCACAGAG
CAGATTCAAAAGTACCTTGATGAGAACAAAAAGTTGATTTTGGCAATTTTGGACAATCAAAATCTT
GGAAAGCTTACAGAATGTGCCCACTATCAAGCTCAGCTTCAAAAGAATTTAATGTATTTAGCTGCA
ATTGCTGATGCACAACCACAAGCACCAACAATGCCTCCTCAGATGGCTCCACATCCTGCAATGCAA
GCTAGTGGGTATTACATGCAACATCCTCAGGCGGCAGCAATGGCTCAGCAACAAGGAATCTTTCCC
CAAAAGATGCCATTACAATTCAATAACCCTCATCAACTACAGGATCCTCAACAGCAGCTACACCAA
CATCAAGCCATGCAAGCACAAATGGGAATGAGACCGGGTGCCACTAACAATGGTATGCATCCCATG
CATGCTGAAAGCTCTCTTGGAGGTGGCAGCAGTGGAGGACCCCCTTCAGCATCAGGCCCAGGTGAC
ATACGTGGTGGAAATAAGCAAGATGCCTCGGAGGCTGGGACTACTGGTGCTGATGGCCAGGGCAGT
TCGGCTGGTGGGCATGGTGGGGATGGAGAGGAGGCAAAGTGA

**SEQ ID NO: 98, *Citrus sinensis* SYT2 polypeptide**
MQQPPQMIPVMPSFPPTNITTEQIQKYLDENKKLILAILDNQNLGKLTECAHYQAQLQKNLMYLAA
IADAQPQAPTMPPQMAPHPAMQASGYYMQHPQAAAMAQQQGIFPQKMPLQFNNPHQLQDPQQQLHQ
HQAMQAQMGMRPGATNNGMHPMHAESSLGGGSSGGPPSASGPGDIRGGNKQDASEAGTTGADGQGS
SAGGHGGDGEEAK

**SEQ ID NO: 99, *Euphorbia esula* SYT2 cDNA DV144834**
ATGCAGCAGCAACCGCAGATGATGCCTATGATGCCTTCATATCCACCAGCAAACATTACCACGGAG
CAAATCCAAAAGTATCTTGATGAAAATAAAAAATTGATTTTGGCGATCTTGGATAATCAAAATCTT
GGAAAACTCGCTGAGTGTGCACAGTATCAAGCCCTGCTGCAAAAAAATCTGATGTATTTAGCCGCA
ATTGCTGATGCACAACCCCAGACCCCACCCATGCCACCTCAGATGTCCCCACATCCGGCTATGCAA
CAAGGAGCATATTACATGCAACATCCTCAGGCTGCAGCAGCAGCAATGGCTCATCAGTCGGGTATT
TTCCCACCAAAGATGTCTCCGTTACAATTCAATAATCCTCATCAAATACAGGACCCCCAGCAGTTA
CATCAAGCAGCCCTCCAAGGGCAAATGGGAATGAGGCCCATGGGGCCCAATAACGGGATGCATCCG
ATGCACCCCGAGGCAAATCTTGGAGGATCTAATGATGGTCGTGGAGGAAACAAACAGGATGCTCCG
GAGACGGGAGCATCGGGAGGTGATGGGCAAGGCAATTCTGGTGGTGATGGGGCTGAAGATGGGAAA
TGA

**SEQ ID NO: 100, *Euphorbia esula* SYT2 polypeptide**
MQQQPQMMPMMPSYPPANITTEQIQKYLDENKKLILAILDNQNLGKLAECAQYQALLQKNLMYLAA
IADAQPQTPPMPPQMSPHPAMQQGAYYMQHPQAAAAAMAHQSGIFPPKMSPLQFNNPHQIQDPQQL
HQAALQGQMGMRPMGPNNGMHPMHPEANLGGSNDGRGGNKQDAPETGASGGDGQGNSGGDGAEDGK

**SEQ ID NO: 101, *Glycine max* SYT2 cDNA BQ612648**
ATGCAGCAGACACCGCCAATGATTCCTATGATGCCTTCTTTCCCACCTACGAACATAACCACCGAG
CAGATTCAAAAATACCTTGATGAGAACAAGAAGCTGATTCTGGCAATATTGGACAATCAAAATCTT
GGAAAACTTGCAGAATGTGCCCAGTACCAAGCTCAGCTTCAAAAGAATTTGATGTATTTAGCTGCA
ATTGCTGATGCCCAGCCTCAAACCCCGGCCATGCCTCCGCAGATGGCACCGCACCCTGCCATGCAA
CCAGGATTCTATATGCAACATCCTCAGGCTGCTGCAGCAGCAATGGCTCAGCAGCAGCAAGGAATG
TTCCCCCAGAAAATGCCATTGCAATTTGGCAATCCACATCAAATGCAGGAACAACAACAGCAGCTA
CACCAGCAGGCCATCCAAGGTCAAATGGGACTTAGACCTGGAGATATAAATAATGGCATGCATCCA
ATGCACAGTGAGGCTGCTCTTGGAGGTGGAAACAGCGGTGGTCCACCTTCGGCTACTGGTCCAAAC
GATGCACGTGGTGGAAGCAAGCAAGATGCCTCTGAGGCTGGAACAGCTGGTGGAGACGGCCAAGGC
AGCTCCGCGGCTGCTCATAACAGTGGAGATGGTGAAGAGGCAAAGTGA

**FIGURE 6 (continued)**

**SEQ ID NO: 102, *Glycine max* SYT2 polypeptide**
MQQTPPMIPMMPSFPPTNITTEQIQKYLDENKKLILAILDNQNLGKLAECAQYQAQLQKNLMYLAA
IADAQPQTPAMPPQMAPHPAMQPGFYMQHPQAAAAAMAQQQQGMFPQKMPLQFGNPHQMQEQQQQL
HQQAIQGQMGLRPGDINNGMHPMHSEAALGGGNSGGPPSATGPNDARGGSKQDASEAGTAGGDGQG
SSAAAHNSGDGEEAK

**SEQ ID NO: 103, *Glycine soya* SYT2 cDNA CA799921**
ATGCAGCAGACACCGCCTATGATTCCTATGATGCCTTCGTTCCCACCTACGAACATAACCACCGAG
CAGATTCAAAAATACCTTGATGAGAACAAGAAGCTGATTCTGGCAATATTGGACAATCAAAATCTT
GGAAAACTTGCAGAATGTGCCCAGTACCAAGCTCAGCTTCAAAAGAATTTGATGTATTTAGCTGCA
ATTGCTGATGCCCAGCCTCAAACACCAGCCATGCCTCCACAGATGGCACCACACCCTGCCATGCAA
CCAGGATTCTATATGCAACATCCTCAGGCTGCAGCAGCAGCAATGGCTCAGCAGCAGCAGCAAGGA
ATGTTCCCCCAGAAAATGCCATTGCAATTTGGCAATCCACATCAAATGCAGGAACAACAGCAGCAG
CTACACCAGCAAGCCATCCAAGGTCAAATGGGACTGAGACCTGGAGGAATAAATAATGGCATGCAT
CCAATGCACAATGAGGGCGGCAACAGCGGTGGTCCACCCTCGGCTACCGGTCCGAACGACGCACGT
GGTGGAAGCAAGCAAGATGCTTCTGAGGCTGGAACAGCTGGTGGAGATGGCCAAGGCAGCTCTGCA
GCTGCTCATAACAGTGGAGATGGTGAAGAGGCAAAGTGA

**SEQ ID NO: 104, *Glycine soya* SYT2 polypeptide**
MQQTPPMIPMMPSFPPTNITTEQIQKYLDENKKLILAILDNQNLGKLAECAQYQAQLQKNLMYLAA
IADAQPQTPAMPPQMAPHPAMQPGFYMQHPQAAAAAMAQQQQQGMFPQKMPLQFGNPHQMQEQQQQ
LHQQAIQGQMGLRPGGINNGMHPMHNEGGNSGGPPSATGPNDARGGSKQDASEAGTAGGDGQGSSA
AAHNSGDGEEAK

**SEQ ID NO: 105, *Gossypium hirsutum* SYT1 cDNA DT558852**
ATGCAGCAGCACCTGATGCAGATGCAGCCCATGATGGCAGCTTATTATCCCAACAACGTCACTACT
GATCATATTCAACAGTATCTCGATGAGAACAAGTCATTGATCTTAAAGATTGTTGAGAGCCAGAAT
TCTGGGAAATTGAGTGAATGTGCTGAGAACCAAGCAAGGCTGCAGCGAAACCTCATGTACCTGGCT
GCCATTGCGGATTCTCAACCCCAACCACCCACCGTGCATGCACAGTTTCCATCTGGTGGTATCATG
CAGCCAGGAGCTGGGCACTACATGCAGCACCAACAAGCTCAACAAATGACACAACAGTCGCTTATG
GCTGCTCGGTCCTCAATGTTGTATTCTCAGCAACCATTTTCTGCACTGCAACAACAACAGCAGCAA
GCTTTGCACAGTCAGCTTGGCATGAGCTCTGGCGGAAGCACAGGCCTTCATATGCTGCAAACTGAA
TCTAGTACTGCAGGTGGCAGTGGAGCACTTGGGGCCGGAGGGTTTCCTGATTTTGGACGTGGTTCT
TCTGGAGAAGGCATCCATGGTGGCAGGCCAATGGCAGGTGGAAGCAAGCAAGATATCGGGAGTGCC
GGCTCAGCTGAAGGTCGTGGAGGAAGCTCTGGTGGTCAGGGTGGTGGTGATGGGGGTGAAACCCTT
TACTTAAAAGCAGCCGATGATGGGAACTGA

**SEQ ID NO: 106, *Gossypium hirsutum* SYT1 polypeptide sequence**
MQQHLMQMQPMMAAYYPNNVTTDHIQQYLDENKSLILKIVESQNSGKLSECAENQARLQRNLMYLA
AIADSQPQPPTVHAQFPSGGIMQPGAGHYMQHQQAQQMTQQSLMAARSSMLYSQQPFSALQQQQQQ
ALHSQLGMSSGGSTGLHMLQTESSTAGGSGALGAGGFPDFGRGSSGEGIHGGRPMAGGSKQDIGSA
GSAEGRGGSSGGQGGGDGGETLYLKAADDGN

**SEQ ID NO: 107, *Gossypium hirsutum* SYT2 cDNA DT563805**
ATGCCGCAGCCACCGCAAATGATTCCTGTGATGCCTTCATATCCACCTACTAATATCACTACTGAA
CAGATTCAGAAGTACCTTGATGAGAATAAGAAGTTGATTTTGGCAATTTTGGACAATCAGAATCTT
GGAAAACTCGCTGAATGCGCCCAGTATCAAGCTCAGCTGCAAAAGAATTTGATGTATTTAGCTGCA
ATTGCGGATGCTCAACCTCAATCAACGCCAGCAATGTCGCCTCAGATGGCACCGCATCCAGCAATG

**FIGURE 6 (continued)**

CAACCCGGAGGATATTTTATGCAACATCCTCAAGCTGCTGCAATGTCACAGCAACCTGGCATGTAC
CCTCAAAAGGTGCCATTGCAATTCAATAGTCCGCATCAAATGCAGGACCCTCAGCACCTCCTATAT
CAGCAGCATCAACAAGCAATGCAAGGTCAAATGGGAATCAGGCCTGGGGGACCCAATAATAGCATG
CATCCCATGCATTCAGAGGCTAGCCTTGGAGGCGGCAGCAGTGGTGGTCCCCCTCAACCTTCAGGC
CCAAGTGATGGACGTGCTGGAAACAAGCAAGAGGGCTCCGAAGCTGGTGGTAATGGGCAGGGCAGC
ACAACTGGTGGGCATGGTGGCGGTGATGGAGCGGATGAGGCAAAGTGA

**SEQ ID NO: 108, *Gossypium hirsutum* SYT2 polypeptide**
MPQPPQMIPVMPSYPPTNITTEQIQKYLDENKKLILAILDNQNLGKLAECAQYQAQLQKNLMYLAA
IADAQPQSTPAMSPQMAPHPAMQPGGYFMQHPQAAAMSQQPGMYPQKVPLQFNSPHQMQDPQHLLY
QQHQQAMQGQMGIRPGGPNNSMHPMHSEASLGGGSSGGPPQPSGPSDGRAGNKQEGSEAGGNGQGS
TTGGHGGGDGADEAK

**SEQ ID NO: 109, *Hordeum vulgare* SYT2 cDNA CA032350**
ATGCAGCAAGCGATGCCCATGCCGCCGGCGGCGGCGGCGCCTGGGATGCCTCCTTCTGCCGGCCTC
AGCACCGAGCAGATCCAAAAGTACCTGGATGAAAATAAACAACTAATTTTGGCTATCTTGGAAAAT
CAGAACCTGGGAAAGTTGGCGGAATGTGCTCAGTATCAAGCTCAGCTTCAGAAGAATCTTTTGTAT
TTGGCTGCGATTGCTGATACTCAGCCACAGACCTCTGTAAGCCGTCCTCAGATGGCACCACCTGCT
GCATCCCCAGGGGCAGGGCATTACATGTCACAGGTGCCAATGTTCCCTCCGAGGACCCCTCTAACG
CCTCAGCAGATGCAGGAGCAGCAACTACAGCAACAACAGGCTCAGATGCTTCCGTTTGCTGGTCAA
ATGGTTGCGAGACCCGGGGCTGTCAATGGCATTCCCCAGGCCCCTCAAGTTGAACAACCAGCCTAT
GCAGCAGGTGGGGCCAGTTCCGAGCCTTCTGGCACCGAGAGCCACAGGAGCACTGGCGCCGATAAC
GATGGTGGGAGCGGCTTGGCTGACCAGTCCTAA

**SEQ ID NO: 110, *Hordeum vulgare* SYT2 polypeptide**
MQQAMPMPPAAAAPGMPPSAGLSTEQIQKYLDENKQLILAILENQNLGKLAECAQYQAQLQKNLLY
LAAIADTQPQTSVSRPQMAPPAASPGAGHYMSQVPMFPPRTPLTPQQMQEQQLQQQQAQMLPFAGQ
MVARPGAVNGIPQAPQVEQPAYAAGGASSEPSGTESHRSTGADNDGGSGLADQS

**SEQ ID NO: 111, *Lactuca serriola* SYT1 cDNA DW110765**
ATGAAGCAGCCGATGATGCCGAATCCAATGATGTCTTCTTCGTTTCCTCCTACAAACATCACCACC
GATCAGATCCAAAAGTTCCTTGATGAAAACAAGCAACTAATTATAGCAATAATGAGCAACCTAAAT
CTTGGAAAGCTTGCTGAATGTGCCCAGTACCAAGCTCTACTCCAAAAAAATTTGATGTATCTAGCA
GCCATTGCAGATGCTCAACCACCTACACCTACACCAACACTAAATATCTCTTATNAGATGGGCCCG
GTTCCACATCCAGGGATGCCACAGCAAGGTGGATTTTACATGGCGCAGCAGCACCCTCAGGCGGCT
GTAATGACGGCTCAGCCACCTTCTGGTTTTCCACAACCGATGCCTGGTATGCAATTTAACAGCCCA
CAGGCTATTCAAGGGCAGATGGGCGGGAGGTCCGGTGGGCCGCCAAGCTCAGCCGCTAGTGATGTC
TGGAGAGGAAGCATGCAAGATGGTGGTGGTGGTGCTGCTGCTGATGGTGGTAAGGATGGTCATGCT
GGCGGTGGACCTGAGGAAGCAAAGTAA

**SEQ ID NO: 112, *Lactuca serriola* SYT1 polypeptide**
MKQPMMPNPMMSSSFPPTNITTDQIQKFLDENKQLIIAIMSNLNLGKLAECAQYQALLQKNLMYLA
AIADAQPPTPTPTLNISYXMGPVPHPGMPQQGGFYMAQQHPQAAVMTAQPPSGFPQPMPGMQFNSP
QAIQGQMGGRSGGPPSSAASDVWRGSMQDGGGGAAADGGKDGHAGGGPEEAK

**SEQ ID NO: 113, *Lycopersicon esculentum* SYT1 cDNA AW934450.1 BP893155.1**
ATGCAGCAGCACCTGATGCAGATGCAGCCCATGATGGCAGCTTACTATCCAACGAACGTCACTACT
GACCATATTCAACAGTATTTGGATGAAAACAAATCACTCATTCTGAAGATTGTTGAGAGCCAGAAC
TCTGGGAAACTCAGTGAATGTGCGGAGAACCAAGCTAGGCTTCAGAGGAATCTGATGTACCTTGCT

**FIGURE 6 (continued)**

GCGATTGCTGATTCACAACCTCAACCTTCTAGCATGCATTCTCAGTTCTCTTCTGGTGGGATGATG
CAGCCAGGGACACACAGTTACTTGCAGCAGCAGCAGCAGCAACAACAAGCGCAACAAATGGCAACA
CAACAACTCATGGCTGCAAGATCCTCGTCGATGCTCTATGGACAACAGCAGCAGCAATCTCAGTTA
TCGCAATATCAACAAGGCTTGCATAGTAGCCAACTCGGCATGAGTTCTGGCAGTGGCGGAAGCACT
GGACTTCATCACATGCTTCAAAGTGAATCATCACCTCATGGTGGTGGTTTCTCTCATGACTTCGGC
CGCGCAAATAAGCAAGACATTGGGAGTAGTATGTCTGCTGAAGGGCGCGGCGGAAGTTCAGGTGGT
GAGAATCTTTATCTGAAAGCTTCTGAGGATTGA

**SEQ ID NO: 114, *Lycopersicon esculentum* SYT1 polypeptide**
MQQHLMQMQPMMAAYYPTNVTTDHIQQYLDENKSLILKIVESQNSGKLSECAENQARLQRNLMYLA
AIADSQPQPSSMHSQFSSGGMMQPGTHSYLQQQQQQQAQQMATQQLMAARSSSMLYGQQQQQSQL
SQYQQGLHSSQLGMSSGSGGSTGLHHMLQSESSPHGGGFSHDFGRANKQDIGSSMSAEGRGGSSGG
ENLYLKASED

**SEQ ID NO: 115, *Malus domestica* SYT2 cDNA CV084230 DR997566**
ATGCAGCAGCCACCACAAATGATCCCCGTCATGCCTTCATTTCCTCCCACCAACATCACCACCGAA
CAAATTCAGAAGTACCTTGATGACAACAAAAAGTTGATTCTGGCAATATTGGATAATCAAAATCTT
GGAAAACTTGCTGAGTGTGCTCAGTACCAGGCTCTGCTTCAAAAGAATCTGATGTATTTAGCAGCA
ATTGCCGATGCGCAACCACAGGCACCAGCTGCCCCTCCCCAGATGGCCCCACATCCTGCTATGCAA
CAGGCAGGATATTACATGCAACATCCTCAGGCAGCAGCAATGGCTCAGCAACAGGGTATTTTCTCC
CCAAAGATGCCGATGCAATTCAATAACATGCATCAAATGCACGATCCACAGCAGCACCAACAAGCC
ATGCAAGGGCAAATGGGAATGAGACCTGGAGGGCCTAACGGCATGCCTTCCATGCTTCATACTGAG
GCCACACATGGTGGTGGTAGTGGCGGCCCAAATTCAGCTGGAGACCCAAATGATGGGCGTGGAGGA
AGCAAGCAAGACGCCTCTGAGTCTGGGGCAGGTGGTGATGGCCAGGGGACCTCAGCCGGCGGGCGT
GGAACTGGTGATGGAGAGGACGGCAAGTGA

**SEQ ID NO: 116, *Malus domestica* SYT2 polypeptide**
MQQPPQMIPVMPSFPPTNITTEQIQKYLDDNKKLILAILDNQNLGKLAECAQYQALLQKNLMYLAA
IADAQPQAPAAPPQMAPHPAMQQAGYYMQHPQAAAMAQQQGIFSPKMPMQFNNMHQMHDPQQHQQA
MQGQMGMRPGGPNGMPSMLHTEATHGGGSGGPNSAGDPNDGRGGSKQDASESGAGGDGQGTSAGGR
GTGDGEDGK

**SEQ ID NO: 117, *Medicago truncculata* SYT2 cDNA CA858743 BI310799.1 AL382135.1**
ATGCAGCAGACACCTCAAATGATTCCTATGATGCCTTCATTCCCACAACAAACAAACATAACCACT
GAGCAGATTCAAAAATATCTTGATGAGAACAAGAAGCTGATCCTGGCAATATTGGACAATCAAAAT
CTTGGAAAACTTGCAGAATGTGCCCAGTACCAAGCTCAGCTTCAGAAGAATTTGATGTATTTAGCT
GCAATTGCTGACGCGCAGCCACAAACACCGGCCTTGCCTCCACAGATGGCCCCGCACCCTGCGATG
CAACAAGGATTCTATATGCAACATCCTCAGGCTGCAGCAATGGCTCAGCAACAAGGAATGTTCCCC
CAAAAAATGCCAATGCAGTTCGGTAATCCGCATCAAATGCAGGATCAGCAGCATCAGCAGCAACAA
CAGCAGCTACATCAGCAAGCTATGCAAGGTCAAATGGGACTTAGACCTGGAGGGATAAATAACGGC
ATGCATCCAATGCACAACGAGGCTGCTCTCGGAGGTAGCGGCAGTGGTGGTCAAATGACGGGCGTG
GTGGTGGAGCAAGCAAGATGCTTCGGAGCTGGGACAGCCGGCGGTGATGGTCAAGGAACCTCTGCC
GCAGCTGCGCACAACAGTGGAGATGCTTCAGAAGAAGGAAAGTAA

**SEQ ID NO: 118, *Medicago truncatula* SYT2 polypeptide**
MQQTPQMIPMMPSFPQQTNITTEQIQKYLDENKKLILAILDNQNLGKLAECAQYQAQLQKNLMYLA
AIADAQPQTPALPPQMAPHPAMQQGFYMQHPQAAAMAQQQGMFPQKMPMQFGNPHQMQDQQHQQQQ
QQLHQQAMQGQMGLRPGGINNGMHPMHNEAALGGSGSGGPNDGRGGGSKQDASEAGTAGGDGQGTS
AAAAHNSGDASEEGK

**FIGURE 6 (continued)**

**SEQ ID NO: 119, *Panicum virgatum* SYT3 cDNA DN152517**
ATGCAGCAGCAGATGCCCATGCAGTCGGCGCCCCCGGCGACCGGCATCACCACCGAGCAGATCCAA
AAGTATTTGGATGAAAATAAGCAGCTTATTTGGCCATCCTGGAAAATCAGAACTTAGGAAAGTTG
GCTGAATGTGCTCAGTATCAAGCTCAGCTTCAAAAGAATCTCTTGTACCTGGCTGCGATTGCAGAT
GCCCAACCCCAACCACCACAGAACCCTGCAAGTCGCCCACAGATGATGCAACCTGGCATGGTACCA
GGTGCAGGGCATTACATGTCCCAAGTACCAATGTTCCCGCCAAGAACACCATTAACCCCGCAACAG
ATGCAAGAACAGCAGCAGCAGCAGCAGCAGCTTCAACAGCAGCAAGCACAGGCTCTTGCTTTCCCG
GGACAGATGGTCATGAGACCTACCATTAATGGCATGCAGCCTATGCAAGCCGACCCTGCTGCCGCC
GCCGCCAGCCTACAGCAGTCAGCACCTGGCCCTACTGATGGGCGAGGAGGCAAGCAAGATGCAACT
GCTGGGGTGAGCACAGAGCCTTCTGGCACCGAGAGCCACAAGAGCACAACCGCAGCAGATCACGAT
GTGGGCACTGATGTCGCGGAGAAATCCTAA

**SEQ ID NO: 120, *Panicum virgatum* SYT3 polypeptide**
MQQQMPMQSAPPATGITTEQIQKYLDENKQLILAILENQNLGKLAECAQYQAQLQKNLLYLAAIAD
AQPQPPQNPASRPQMMQPGMVPGAGHYMSQVPMFPPRTPLTPQQMQEQQQQQQLQQQQAQALAFP
GQMVMRPTINGMQPMQADPAAAAASLQQSAPGPTDGRGGKQDATAGVSTEPSGTESHKSTTAADHD
VGTDVAEKS

**SEQ ID NO: 121, *Picea sitchensis* SYT1 cDNA DR484100 DR478464.1**
ATGCAGCAGCATCTCATGCAAATGCAGCCCATGATGGCGGCATACGCCTCCAACAACATCACCACT
GATCACATCCAGAAGTACCTGGATGAGAACAAGCAGTTGATTCTGGCAATTCTGGACAACCAAAAT
CTTGGAAAGCTCAATGAGTGTGCTCAGTACCAAGCAAAACTTCAGCAGAATTTGATGTATCTGGCT
GCGATTGCTGATTCTCAACCACAAGCACAAACTGCACATGCTCAGATTCCTCCTAATGCAGTGATG
CAGTCTGGTGGGCATTACATGCAGCACCAGCAGGCACAGCAACAAGTGACTCCTCAGTCTCTGATG
GCAGCTAGATCTTCCATGCTGTATTCTCAGCAGCCGATGGCTGCTTTGCATCAAGCTCAGCAACAA
CAGCAGCAGCAGCATCAGCAGCAACAACAATCTCTTCACAGCCAGCTTGGCATAAATTCTGGAGGA
AGCAGTGGATTGCATATGTTGCATGGTGAGACAAACATGGGATGTAATGGGCCTCTCTCATCTGGG
GGCTTCCCTGAATTTGGGCGTGGGTCTGCTACCTCTGCTGAAGGTATGCAGGCCAACAGGGGCTTC
ACTATAGATCGTGGTTCAAATAAGCAGGATGGAGTAGGATCAGAGAATGCCCATCCAGGTGCTGGT
GATGGAAGAGGGAGTTCAACTGGAGGGCAGAATGCAGATGAGTCAGAACCATCATACCTGAAAGCC
TCCGAAGAAGAAGGAAACTAG

**SEQ ID NO: 122, *Picea sitchensis* SYT1 polypeptide**
MQQHLMQMQPMMAAYASNNITTDHIQKYLDENKQLILAILDNQNLGKLNECAQYQAKLQQNLMYLA
AIADSQPQAQTAHAQIPPNAVMQSGGHYMQHQQAQQQVTPQSLMAARSSMLYSQQPMAALHQAQQQ
QQQQHQQQQQSLHSQLGINSGGSSGLHMLHGETNMGCNGPLSSGGFPEFGRGSATSAEGMQANRGF
TIDRGSNKQDGVGSENAHPGAGDGRGSSTGGQNADESEPSYLKASEEEGN

**SEQ ID NO: 123, *Pinus taeda* SYT1 cDNA DT625916**
ATGCAGCAGCACCTCATGCAAATGCAGCCCATGATGGCGGCCTACGCCTCCAACAATATCACCACT
GATCACATCCAGAAGTACCTGGATGAGAACAAGCAGTTGATTCTGGCAATTTTGGACAACCAAAAT
CTCGGAAAGCTCAATGAGTGTGCTCAATACCAAGCAAAACTTCAGCAGAATTTGATGTATCTGGCT
GCTATTGCTGATTCTCAACCTCAAGCACAAACTGCACATGCTCAGATTCCTCCAAATGCGGTGATG
CAGTCTGGTGGGCATTACATGCAGCATCAACAGGCACAGCAACAAGTTACTCCTCAGTCTCTGATG
GCAGCTAGATCTTCCATACTGTATGCTCAGCAACAACAGCAGCAGCAGCATCAGCAGCATCAGCAG
CAACAGCAGCAACAACAGTCTCTTCACAGCCAGCTTGGCATAAATTCTGGAGGAAGCAGCGGTTTG
CATATGTTGCATGGTGAGACAAACATGGGATGTAATGGGCCTCTGTCATCTGGGGGATTCCCTGAA

## FIGURE 6 (continued)

TTTGGGCGTGGGTCTGCTACCTCTGCTGATGGTATGCAGGTGAACAGGGGCTTTGCTATAGATCGT
GGTTCAAACAAGCAGGATGGAGTTGGATCAGAGAATGCCCATGCTGGTGCTGGTGATGGAAGAGGG
AGTTCAACTGGAGGGCAGAATGCAGATGAGTCAGAACCATCATACCTGAAGGCCTCCGAGGAAGAA
GGAAACTAG

**SEQ ID NO: 124, *Pinus taeda* SYT1 polypeptide**
MQQHLMQMQPMMAAYASNNITTDHIQKYLDENKQLILAILDNQNLGKLNECAQYQAKLQQNLMYLA
AIADSQPQAQTAHAQIPPNAVMQSGGHYMQHQQAQQQVTPQSLMAARSSILYAQQQQQQHQQHQQ
QQQQQQSLHSQLGINSGGSSGLHMLHGETNMGCNGPLSSGGFPEFGRGSATSADGMQVNRGFAIDR
GSNKQDGVGSENAHAGAGDGRGSSTGGQNADESEPSYLKASEEEGN

**SEQ ID NO: 125, *Populus tremula* SYT1 cDNA DT476906**
ATGCAACAGCACCTGATGCAGATGCAGCCCATGATGGCAGCCTATTACCCCAGCAACGTCACTACT
GATCATATTCAACAGTATCTGGACGAAAACAAGTCATTGATTTTGAAGATTGTTGAGAGCCAGAAT
TCAGGGAAACTCAGTGAGTGTGCAGAGAACCAAGCAAGACTGCAACAAAATCTCATGTACTTGGCT
GCAATTGCTGATTGTCAGCCCCAACCACCTACCATGCATGCCCAGTTCCCTTCCAGCGGCATTATG
CAGCCAGGAGCACATTACATGCAGCATCAACAAGCTCAACAGATGACACCACAAGCCCTTATGGCT
GCACGCTCTTCTATGCTGCAGTATGCTCAACAGCCATTCTCAGCGCTTCAACAACAGCAAGCCTTA
CACAGCCAGCTCGGCATGAGCTCTGGTGGAAGCGCAGGACTTCATATGATGCAAAGCGAGGCTAAC
ACTGCAGGAGGCAGTGGAGCTCTTGGTGCTGGACGATTTCCTGATTTTGGCATGGATGCCTCCAGT
AGAGGAATCGCAAGTGGGAGCAAGCAAGATATTCGGAGTGCAGGGTCTAGTGAAGGGCGAGGAGGA
AGCTCTGGAGGCCAGGGTGGTGATGGAGGTGAAACCCTTTACTTGAAATCTGCTGATGATGGGAAC
TGA

**SEQ ID NO: 126, *Populus tremula* polypeptide**
MQQHLMQMQPMMAAYYPSNVTTDHIQQYLDENKSLILKIVESQNSGKLSECAENQARLQQNLMYLA
AIADCQPQPPTMHAQFPSSGIMQPGAHYMQHQQAQQMTPQALMAARSSMLQYAQQPFSALQQQQAL
HSQLGMSSGGSAGLHMMQSEANTAGGSGALGAGRFPDFGMDASSRGIASGSKQDIRSAGSSEGRGG
SSGGQGGDGGETLYLKSADDGN

**SEQ ID NO: 127, *Saccharum officinarum* SYT1 cDNA CA078249.1 CA078630 CA082679 CA234526 CA239244 CA083312**
ATGCAGCAGCAACACCTGATGCAGATGAACCAGAACATGATTGGGGGCTACACCTCTCCTGCCGCT
GTGACAACCGATCTCATCCAGCAGTACCTGGATGAGAACAAGCAGCTGATCCTGGCCATCCTCGAC
AACCAGAACAATGGCAAGGTGGAGGAGTGCGAACGGCACCAAGCTAAGCTCCAGCACAACCTCATG
TACCTGGCCGCCATCGCCGACAGCCAGCCACCACAGACTGCACCACTATCACAATACCCGTCCAAC
CTGATGATGCAGCCGGGCCCTCGGTACATGCCACCGCAGTCCGGGCAGATGATGAGCCCGCAGTCG
CTAATGGCGGCGCGGTCCTCCATGATGTACGCGCACCCGTCCATGTCACCACTCCAGCAGCAGCAG
GCAGCGCACGGGCAGCTGGGCATGGCTTCAGGGGGCGGCGGTGGCACGACCAGTGGGTTCAACATC
CTCCATGGCGAGGCCAGTATGGGCGGTGCTGGTGGCGCTTGTGCCGGCAACAACATGATGAACGCC
GGCATGTTCTCAGGCTTTGGCCGCAGCGGCAGTGGCGCCAAGGAGGGATCGACCTCGCTGTCGGTT
GACGTCCGTGGTGGCACCAGCTCCGGCGCGCAAAGCGGGGACGGCGAGTACCTGAAAGCAGGCACC
GAGGAAGAAGGCAGTTAA

**SEQ ID NO: 128, *Saccharum officinarum* SYT1 polypeptide**
MQQQHLMQMNQNMIGGYTSPAAVTTDLIQQYLDENKQLILAILDNQNNGKVEECERHQAKLQHNLM
YLAAIADSQPPQTAPLSQYPSNLMMQPGPRYMPPQSGQMMSPQSLMAARSSMMYAHPSMSPLQQQQ
AAHGQLGMASGGGGGTTSGFNILHGEASMGGAGGACAGNNMMNAGMFSGFGRSGSGAKEGSTSLSV
DVRGGTSSGAQSGDGEYLKAGTEEEGS

**FIGURE 6 (continued)**

**SEQ ID NO: 129, *Saccharum officinarum* SYT2 cDNA CA110367**
ATGCAGCAGCCGATGCCCATGCAGCCGCAGGCGCCGGAGATGACCCCGGCCGCCGGAATCACCACG
GAGCAGATCCAAAAGTATCTGGATGAGAATAAGCAGCTTATTTTGGCTATTTTGGAAAATCAGAAC
CTAGGAAAATTGGCAGAATGTGCTCAGTATCAATCACAACTTCAGAAGAACCTCTTGTATCTCGCT
GCAATCGCAGATGCCCAACCACAGACTGCTGTAAGCCGCCCTCAGATGGCGCCGCCTGGTGCATTG
CCTGGAGTAGGGCAGTACATGTCACAGGTGCCTATGTTCCCACCGAGGACACCTCTAACACCCCAG
CAGATGCAGGAGCAGCAACTTCAGCAGCAGCAGGCTCAGCTGCTAAATTTCAGTGGCCTAATGGTT
GCTAGACCTGGCATGGTCAACGGCATGCCTCAGTCCATTCAAGTTCAGCAAGCTCAGCCACCACCA
GCAGGGAACAAACAGGATGCTGGTGGGGTCGCCTCGGAGCCCTCGGGCATTGAGAACCACAGGAGC
ACTGGTGGTGATAATGATGGTGGAAGCGACTAG

**SEQ ID NO: 130, *Saccharum officinarum* SYT2 polypeptide**
MQQPMPMQPQAPEMTPAAGITTEQIQKYLDENKQLILAILENQNLGKLAECAQYQSQLQKNLLYLA
AIADAQPQTAVSRPQMAPPGALPGVGQYMSQVPMFPPRTPLTPQQMQEQQLQQQQAQLLNFSGLMV
ARPGMVNGMPQSIQVQQAQPPPAGNKQDAGGVASEPSGIENHRSTGGDNDGGSD

**SEQ ID NO: 131, *Saccharum officinarum* SYT3 cDNA CA161933.1 CA265085**
ATGCAGCAGCAGATGCCCATGCCGCCGGCGCCCGCTGCGGCGGCGGCGCCCCCGGCGGCCGGCATC
ACCACCGAGCAGATCCAAAAGTATTTGGACGAAAATAAGCAACTTATTTTGGCCATCCTGGAAAAT
CAGAACTTAGGAAAGTTGGCTGAATGTGCTCAGTATCAAGCTCAACTTCAAAAGAACCTCTTGTAC
CTGGCTGCGATTGCTGATGCCCAACCCCAGCCACCACAAAACCCTGCAGGTCGCCCTCAGATGATG
CAACCTGGTATAGTGCCAGGTGCGGGGCATTACATGTCACAAGTACCAATGTTCCCTCCAAGAACT
CCATTAACCCCACAGCAGATGCAAGAGCAGCAGCAGCAACAGCTTCAGCAGCAGCAAGCGCAGGCT
CTTACATTCCCTGGACAGATGGTCATGAGACCAGCTACCATCAACGGCATACAGCAGCCTATGCAA
GCTGACCCTGCCCGGGCAGCGGAGCTGCAACAACCACCACCTATCCCAGCTGACGGGCGAGTAAGC
AAGCAGCAGGACACAACGGCTGGCGTGAGCTCAGAGCCTTCTGCCAATGAGAGCCACAAGACCACA
ACTGGAGCAGATAGTGAGGCAGGTGGTGACGTGGCGGAGAAATCCTAA

**SEQ ID NO: 132, *Saccharum officinarum* SYT3 polypeptide**
MQQQMPMPPAPAAAAAPPAAGITTEQIQKYLDENKQLILAILENQNLGKLAECAQYQAQLQKNLLY
LAAIADAQPQPPQNPAGRPQMMQPGIVPGAGHYMSQVPMFPPRTPLTPQQMQEQQQQLQQQQAQA
LTFPGQMVMRPATINGIQQPMQADPARAAELQQPPPIPADGRVSKQQDTTAGVSSEPSANESHKTT
TGADSEAGGDVAEKS

**SEQ ID NO: 133, *Solanum tuberosum* SYT1 cDNA CK265597**
ATGCAGCAGCACCTGATGCAGATGCAGCCCATGATGGCAGCTTACTATCCAACGAACGTCACTACT
GACCATATTCAACAGTATTTGGATGAGAACAAATCACTCATTCTGAAAATTGTTGAGAGCCAAAAC
TCGGGAAAACTCAGTGAATGTGCAGAGAACCAAGCTAGGCTTCAGAGGAATCTGATGTACCTTGCT
GCTATTGCTGATTCACAACCTCAGCCTTCTAGCATGCATTCTCAGTTCTCTTCTGGTGGGATGATG
CAGCCAGGGACACACACAGTTACCTGCAGCAGCAGCAGCAGCAACAACAAGCGCAACAAATGGCAACA
CAACAACTCATGGCTGCAAGATCCTCATCAATGCTCTATGGACAACAACAGCAGCAGCAGCAGCAG
TCTCAGTTATCACAATTTCAACAAGGCTTGCATAGTAGCCAACTTGGCATGAGTTCTGGCAGTGGT
GGAAGCACTGGACTTCATCACATGCTTCAAAGTGAATCATCACCTCATGGTGGTGGTTTCTCTCAT
GACTTCGGCCGTGCAAATAAGCAAGACATTGGGAGTAGTATGTCTGCTGAAGGGCGCGGCGGAAGC
TCAGGTGGTGATGGTGGTGAGAATCTTTATCTGAAAGCTTCTGAGGATTGA

## FIGURE 6 (continued)

**SEQ ID NO: 134, *Solanum tuberosum* SYT1 polypeptide**
MQQHLMQMQPMMAAYYPTNVTTDHIQQYLDENKSLILKIVESQNSGKLSECAENQARLQRNLMYLA
AIADSQPQPSSMHSQFSSGGMMQPGTHSYLQQQQQQQAQQMATQQLMAARSSSMLYGQQQQQQQ
SQLSQFQQGLHSSQLGMSSGSGGSTGLHHMLQSESSPHGGGFSHDFGRANKQDIGSSMSAEGRGGS
SGGDGGENLYLKASED

**SEQ ID NO: 135, *Sorghum bicolor* SYT3 cDNA CX611128**
ATGCAGCAGCAGATGCCCATGCCGCCGGCGCCCGCTGCGGCGGCGGCGACGGCGCCCCCGGCGGCC
GGCATCACCACCGAGCAGATCCAGAAGTATTTGGACGAAAATAAGCAACTTATTTTGGCCATCCTA
GAAAATCAGAACTTAGGAAAGTTGGCTGAATGTGCTCAGTATCAAGCTCAACTTCAAAAGAACCTC
TTGTACCTGGCTGCGATTGCTGATGCCCAACCCCGACCACCGCAAAACCCTGCAGGTCGCCCTCAG
ATGATGCAACCTGGTATAGTGCCAGGTGCAGGGCATTACATGTCACAAGTACCAATGTTCCCTCCA
AGAACTCCATTAACCCCACAGCAAATGCAAGAGCAGCAGCAGCAACAGCTTCAGCAGCAGCAAGCG
CAGGCTCTTGCATTCCCTGGGCAGATGGTCATGAGACCAGCTACCATCAACGGCATGCAGCAGCCT
ATGCAGGCTGACCCTGCCCGGGCAGCGGAGCTGCAACAGCCAGCATCTGTCCCAGCCGACGGGCGA
GTAAGCAAGCAGGACACAGCGGCTGGGGTGAGCTCAGAGCCTTCTGCCAATGAGAGCCACAAGACC
ACAACCGGAGCAGATAGTGAGGCAGGTGGAGACGTGGCGGAGAAATCCTAA

**SEQ ID NO: 136, *Sorghum bicolor* SYT3 polypeptide**
MQQQMPMPPAPAAAAATAPPAAGITTEQIQKYLDENKQLILAILENQNLGKLAECAQYQAQLQKNL
LYLAAIADAQPRPPQNPAGRPQMMQPGIVPGAGHYMSQVPMFPPRTPLTPQQMQEQQQQLQQQQA
QALAFPGQMVMRPATINGMQQPMQADPARAAELQQPASVPADGRVSKQDTAAGVSSEPSANESHKT
TTGADSEAGGDVAEKS

**SEQ ID NO: 137, *Triticum aestivum* SYT2 cDNA CD901951**
ATGCAGCAAGCGATGCCCATGCCGCCGGCGGCGGCGGCGCCGGGGATGCCTCCGTCTGCTGGCCTC
AGCACCGAGCAGATCCAAAAGTACCTGGATGAAAATAAGCAACTAATTTTGGCTATCTTGGAAAAT
CAGAACCTGGGAAAGTTGGCGGAATGTGCTCAGTATCAAGCTCAGCTTCAGAAGAATCTTTTGTAT
TTGGCTGCAATCGCTGATACTCAGCCACAGACCACTGTAAGCCGTCCTCAGATGGCACCACCTAGT
GCATCCCCAGGGGCAGGGCATTACATGTCACAGGTGCCAATGTTCCCTCCGAGGACCCCTCTAACG
CCTCAGCAGATGCAGGAGCAGCAACTACAGCAGCAACAGGCTCAGATGCTTCCGTTTGCTGGTCAA
ATGGTTGCGAGACCTGGGGCTGTCAATGGCATGCCTCAGGCCCCTCAAGTTGAACCAGCCTATGCA
GCAGGTGGGGCCAGTTCTGAGCCTTCTGGCACTGAGAGCCACAGGAGCACTGGTGCCGATAATGAC
GGGGGGAGCGGCTGGGCTGATCAGTCCTAA

**SEQ ID NO: 138, *Triticum aestivum* SYT2 polypeptide**
MQQAMPMPPAAAAPGMPPSAGLSTEQIQKYLDENKQLILAILENQNLGKLAECAQYQAQLQKNLLY
LAAIADTQPQTTVSRPQMAPPSASPGAGHYMSQVPMFPPRTPLTPQQMQEQQLQQQQAQMLPFAGQ
MVARPGAVNGMPQAPQVEPAYAAGGASSEPSGTESHRSTGADNDGGSGWADQS

**SEQ ID NO: 139, *Triticum aestivum* SYT3 cDNA BJ246754 BJ252709**
ATGCAGCAGGCGATGTCCTTGCCCCCGGGAGCGGTCGGCGCGGTGTCCTCGCCGGCCGGCATCACC
ACCGAGCAGATCCAAAAGTATTTGGATGAAAATAAGCAACTTATTTTGGCCATCCTTGAAAATCAG
AACCTAGGAAAGTTGGCTGAATGTGCTCAGTATCAAGCTCAACTCCAAAAGAATCTCTTGTATCTA
GCTGCTATCGCGGATGCCCAACCACCACAGAACCCTACAAGTCACCCTCAGATGGTGCAGCCTGGT
AGTATGCAAGGTGCAGGGCATTACATGTCACAAGTACCAATGTTCCCTCCAAGAACGCCTTTAACC
CCACAGCAGATGCAAGAGCAGCAGCACCAGCAGCTTCAGCAGCAGCAAGCCCAGGCCCTTTCTTTC
CCCGCCCAGGTGGTCATGAGACCAGGCACCGTCAACGGCATGCAGCAGCCTATGCAAGCAGCCGGC

## FIGURE 6 (continued)

GACCTCCAGCCAGCAGCAGCACCTGGAGGGAGCAAGCAGGACGCCGCAGTGGCTGGGGCCAGCTCG
GAACCATCTGGCACCAAGAGCCACAAGAACGCGGGAGCAGAGGAGGTGGGCGCTGATGTAGCAGAA
CAATCCTAA

**SEQ ID NO: 140, *Triticum aestivum* SYT3 polypeptide**
MQQAMSLPPGAVGAVSSPAGITTEQIQKYLDENKQLILAILENQNLGKLAECAQYQAQLQKNLLYL
AAIADAQPPQNPTSHPQMVQPGSMQGAGHYMSQVPMFPPRTPLTPQQMQEQQHQQLQQQQAQALSF
PAQVVMRPGTVNGMQQPMQAAGDLQPAAAPGGSKQDAAVAGASSEPSGTKSHKNAGAEEVGADVAE
QS

**SEQ ID NO: 141, *Vitis vinifera* SYT1 cDNA DV219834**
ATGCAGCAGCACCTGATGCAGATGCAGCCCATGATGGCAGCCTATTACCCCAGCAACGTCACCACT
GATCACATTCAGCAGTATCTTGATGAAAACAAGTCATTGATTCTGAAGATTGTTGAGAGCCAGAAT
TCAGGAAAATTGACTGAATGTGCAGAGAACCAGGCAAGACTACAGAGAAACCTCATGTACCTGGCT
GCAATTGCTGATTCTCAACCCCAACCACCCACCATGCATGCTCAGTTCCCTCCTAGTGGCATTGTT
CAGCCAGGAGCTCACTACATGCAACACCAACAAGCTCAACAAATGACACCACAGTCGCTCCTGGCT
GCACGCTCCTCCATGCTGTACACCCAACAACCATTTTCGGCCCTGCAACAACAACAAGCCATCCAT
AGCCAGCTTGGCATGGGCTCTGGTGGAAGTGCAGGACTTCACATGCTGCAAAGCGAGGGGAGTAAT
CCAGGAGGCAATGGAACACTGGGGACTGGTGGGTTTCCTGATTTCAGCCGTGGAACTTCTGGAGAA
GGCCTGCAGGCTGCAGGCAGGGGAATGGCTGGTGGGAGCAAGCAAGATATGGGAAATGCAGAAGGG
CGAGGAGGGAACTCAGGAGGTCAGGGTGGGGATGGAGGTGAGACTCTTTACTTGAAAGCTGCTGAA
GATGGGAATTGA

**SEQ ID NO: 142, *Vitis vinifera* SYT1 polypeptide**
MQQHLMQMQPMMAAYYPSNVTTDHIQQYLDENKSLILKIVESQNSGKLTECAENQARLQRNLMYLA
AIADSQPQPPTMHAQFPPSGIVQPGAHYMQHQQAQQMTPQSLLAARSSMLYTQQPFSALQQQQAIH
SQLGMGSGGSAGLHMLQSEGSNPGGNGTLGTGGFPDFSRGTSGEGLQAAGRGMAGGSKQDMGNAEG
RGGNSGGQGGDGGETLYLKAAEDGN

**SEQ ID NO: 143, *Zea mays* SYT3 cDNA CO468901**
ATGCAGCAGCAGATGCCCATGCCGCCGGCGCCCGCTGCCGCCGCGGCGGCGGCGCCCCCGGCGGCA
GGCATCACTACCGAGCAGATCCAGAAGTATTTGGACGAAAATAAGCAACTTATTTTGGCCATCCTG
GAAAATCAGAACTTAGGGAAGTTGGCTGAATGTGCTCAGTATCAAGCTCAACTTCAAAAGAACCTC
TTGTACCTGGCTGCGATTGCTGATGCCCAACCCCAGCCTCCGCAAAACCCTGCAGGTCGCCCTCAG
ATGATGCAGCCTGGTATAGTGCCAGGTGCGGGGCATTACATGTCACAAGTACCAATGTTCCCTCCA
AGAACCCCATTAACCCCACAGCAGATGCAGGAGCAGCAGCAACAACAACAGTTTCAGCAGCAGCAG
CAGCAAGTGCAGGCTCTTACATTTCCTGGACAGATGGTCATGAGACCAGGCACCATCAACGGCATG
CAGCAGCAGCAGCCTATGCAGGCTGACCCTGCCCGGGCAGCAGCGGAGCTGCAGCAGGCAGCACCT
ATCCCAGCTGACGGGCGAGGAAGCAAGCAGGACACCGCGGGTGGGGCGAGCTCAGAGCCTTCTGCC
AATGAGAGCCACAAGAGCGCCACCGGAGCAGATACCGAGGCAGGTGGCGACGTGGCCGAGAAATCC
TAA

**SEQ ID NO: 144, *Zea mays* SYT3 polypeptide**
MQQQMPMPPAPAAAAAAAPPAAGITTEQIQKYLDENKQLILAILENQNLGKLAECAQYQAQLQKNL
LYLAAIADAQPQPPQNPAGRPQMMQPGIVPGAGHYMSQVPMFPPRTPLTPQQMQEQQQQQFQQQQ
QQVQALTFPGQMVMRPGTINGMQQQQPMQADPARAAAELQQAAPIPADGRGSKQDTAGGASSEPSA
NESHKSATGADTEAGGDVAEKS

## FIGURE 6 (continued)

**SEQ ID NO: 145, *Oryza sativa* GOS2 promoter PRO0129**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTCCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTTCGATCCATATCTTCCGGTCGAGTTCTTGG
TCGATCTCTTCCCTCCTCCACCTCCTCCTCACAGGGTATGTGCCCTTCGGTTGTTCTTGGATTTAT
TGTTCTAGGTTGTGTAGTACGGGCGTTGATGTTAGGAAAGGGGATCTGTATCTGTGATGATTCCTG
TTCTTGGATTTGGGATAGAGGGGTTCTTGATGTTGCATGTTATCGGTTCGGTTTGATTAGTAGTAT
GGTTTTCAATCGTCTGGAGAGCTCTATGGAAATGAAATGGTTTAGGGTACGGAATCTTGCGATTTT
GTGAGTACCTTTTGTTTGAGGTAAAATCAGAGCACCGGTGATTTTGCTTGGTGTAATAAAAGTACG
GTTGTTTGGTCCTCGATTCTGGTAGTGATGCTTCTCGATTTGACGAAGCTATCCTTTGTTTATTCC
CTATTGAACAAAAATAATCCAACTTTGAAGACGGTCCCGTTGATGAGATTGAATGATTGATTCTTA
AGCCTGTCCAAAATTTCGCAGCTGGCTTGTTTAGATACAGTAGTCCCCATCACGAAATTCATGGAA
ACAGTTATAATCCTCAGGAACAGGGGATTCCCTGTTCTTCCGATTTGCTTTAGTCCCAGAATTTTT
TTTCCCAAATATCTTAAAAAGTCACTTTCTGGTTCAGTTCAATGAATTGATTGCTACAAATAATGC
TTTTATAGCGTTATCCTAGCTGTAGTTCAGTTAATAGGTAATACCCCTATAGTTTAGTCAGGAGAA
GAACTTATCCGATTTCTGATCTCCATTTTTAATTATATGAAATGAACTGTAGCATAAGCAGTATTC
ATTTGGATTATTTTTTTTATTAGCTCTCACCCCTTCATTATTCTGAGCTGAAAGTCTGGCATGAAC
TGTCCTCAATTTTGTTTTCAAATTCACATCGATTATCTATGCATTATCCTCTTGTATCTACCTGTA
GAAGTTTCTTTTTGGTTATTCCTTGACTGCTTGATTACAGAAAGAAATTTATGAAGCTGTAATCGG
GATAGTTATACTGCTTGTTCTTATGATTCATTTCCTTTGTGCAGTTCTTGGTGTAGCTTGCCACTT
TCACCAGCAAAGTTC

**SEQ ID NO: 146, Box I**
IQ(Q/K)(Y/M/F/H)L(D/E)(E/D)N(K/N)XLI

Where X is any amino acid

**SEQ ID NO: 147, Box II**
NL(M/L/V)YLA(A/T)IAD

**SEQ ID NO: 148, prm06681**
GGGGACAAGTTTGTACAAAAAAGCAGGCTTAAACAATGCAACAGCACCTGATG

**SEQ ID NO: 149, Prm06682**
GGGGACCACTTTGTACAAGAAAGCTGGGTCATCATTAAGATTCCTTGTGC

## FIGURE 6 (continued)

**SEQ ID NO: 150, *Brassica napus* SYT cDNA**
ATGCAACAGCACCTGATGCAGATGCAGCCCATGATGGCTGGTTACTACCCCAGCAATGTCACCTCT
GATCATATTCAGCAGTACTTGGACGAGAACAAATCGTTGATTCTGAAGATAGTTGAATCTCAAAAC
TCGGGAAAGCTCAGCGAGTGTGCCGAGAACCAGGCAAGGCTTCAACGCAACTTAATGTACTTAGCT
GCAATTGCAGATTCTCAGCCTCAACCTCCAAGCATGCATAGCCAGTATGGAACTGCTGGTGGTGGT
GGGTTGATGCAGGGAGAAGGAGGGTCACACTATTTGCAACAGCAACAGGCAATTCAACAGCAGCAG
AGTCAGCAGTCTCTAATGGCGGCTCGATCTTCAATGTTGTATGCTCAGCAGCAGCAACAGCCTTAT
GCAACGCTTCAGCAGCAGCAATTGCACCATAGCCAGCTTGGGATGAGCTCAAGCAGCGGAGGAGGA
AGCAGCGGTCTCCATATGCTACAGGGAGAGGCTGGTGGGTTTCATGATTTTGGCCGTGAGAAGTTG
GAAATGGGAAGTGGTGAAGGCAGAGGAGGAAGCTCAGGGGATGGTGGAGAAACCCTTTACTTGAAG
TCATCAGATGATGGGAACTGA

**SEQ ID NO: 151, *Brassica napus* SYT polypeptide**
MQQHLMQMQPMMAGYYPSNVTSDHIQQYLDENKSLILKIVESQNSGKLSECAENQARLQRNLMYLA
AIADSQPQPPSMHSQYGTAGGGGLMQGEGGSHYLQQQQAIQQQQSQQSLMAARSSMLYAQQQQQPY
ATLQQQQLHHSQLGMSSSSGGGSSGLHMLQGEAGGFHDFGREKLEMGSGEGRGGSSGDGGETLYLK
SSDDGN

**SEQ ID NO: 152, *Glycine max* SYT cDNA**
ATGCAGCAGCACCTGATGCAGATGCAGCCCATGATGGCTGCCTACTACCCCAACAACGTCACCACT
GATCACATTCAACAGTACCTGGATGAGAACAAGTCCTTGATTCTGAAGATTGTTGAAAGCCAGAAT
TCTGGCAAGCTGAGCGAGTGTGCCGAGAACCAATCAAGGCTGCAGAGAAATCTCATGTACCTAGCT
GCAATAGCTGATTCTCAACCACAACCATCTCCATTGGCTGGTCAGTATCCTTCTAGTGGACTTGTG
CAGCAGGGAGCACACTACATGCAGGCTCAACAGGCTCAGCAGATGTCACAACAACAGCTAATGGCT
TCGCGCTCCTCGCTCCTGTACTCCCAACAGCCTTTCTCAGTGCTTCAACAGCAGCAAGGCATGCAC
AGCCAACTTGGCATGAGCTCCAGTGGAAGTCAAGGCCTCCACATGCTGCAAAGTGAAGCCACTAAT
GTTGGAGGCAATGCAACCATAGGAACCGGAGGAGGGTTTCCGGACTTTGTACGCATTGGTAGTGGC
AAGCAAGATATTGGAATCTCTGGTGAAGGCAGAGGAGGAAACTCTAGTGGCCACTCTGGTGATGGT
GGTGAGACACTTAATTACCTGAAAGCTGCTGGTGATGGAAACTGA

**SEQ ID NO: 153, *Glycine max* SYT polypeptide**
MQQHLMQMQPMMAAYYPNNVTTDHIQQYLDENKSLILKIVESQNSGKLSECAENQSRLQRNLMYLA
AIADSQPQPSPLAGQYPSSGLVQQGAHYMQAQQAQQMSQQQLMASRSSLLYSQQPFSVLQQQQGMH
SQLGMSSSGSQGLHMLQSEATNVGGNATIGTGGGFPDFVRIGSGKQDIGISGEGRGGNSSGHSGDG
GETLNYLKAAGDGN

## FIGURE 6 (continued)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004350553 A
- WO 2006079655 A
- US 5811238 A
- US 6395547 B
- WO 2004070039 A
- WO 2004065596 A
- US 4962028 A
- WO 0114572 A
- WO 9514098 A
- WO 9412015 A
- EP 99106056 A
- US 5565350 A, Kmiec
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- WO 0015815 A
- EP 1198985 A1
- US 5352605 A
- WO 8402913 A
- EP 388186 A
- EP 335528 A
- WO 9706268 A
- WO 9519443 A
- WO 9321334 A
- US 5187267 A
- WO 9612814 A
- EP 0375091 A
- US 5608152 A
- WO 9845461 A
- US 5504200 A
- WO 9113980 A
- WO 0026388 A
- WO 9515389 A
- WO 9523230 A
- WO 9916890 A
- US 5677474 A
- US 5530149 A
- EP 571741 A
- JP 6062870 A
- WO 9808962 A
- US 5689040 A
- EP 781849 A
- DE 19644478 A
- EP 0249676 A
- US 57673666 B
- US 6225105 B
- US 5164310 A


### Non-patent literature cited in the description


- **WANG et al.** *Planta,* 2003, vol. 218, 1-14
- **BOYER.** *Science,* 1982, vol. 218, 443-448
- **MCKERSIE ; LESHEM.** Stress and stress coping in cultivated plants. Kluwer Academic Publishers, 1994
- **FRINK et al.** *Proc Natl Acad Sci USA,* 1999, vol. 96 (4), 1175-1180
- **TITTONELL et al.** *Agric Ecosys & Environ,* 2005, vol. 105, 213
- **FASOULA ; TOLLENAAR.** *Maydica,* 2005, vol. 50, 39
- **STEEGE et al.** *Plant Physiology,* 2005, vol. 139, 1078
- **HITTALMANI et al.** *Theoretical Applied Genetics,* 2003, vol. 107, 679
- **KIM HJ ; KENDE H.** *Proc Nat Acad Sc,* 2004, vol. 101, 13374-9
- **HORIGUCHI et al.** *Plant J,* 2005, vol. 43, 68-78
- **VAN DER KNAAP E et al.** *Plant Phys,* 2000, vol. 122, 695-704
- **NÄÄR AM et al.** *Annu Rev Biochem,* 2001, vol. 70, 475-501
- **THAETE et al.** *Hum Molec Genet,* 1999, vol. 8, 585-591
- **CREIGHTON.** Proteins. 1984
- **TERPE.** *Appl. Microbiol. Biotechnol.,* 2003, vol. 60, 523-533
- **MEINKOTH ; WAHL.** *Anal. Biochem.,* 1984, vol. 138, 267-284

- **SAMBROOK et al.** Molecular Cloning: a laboratory manual. Cold Spring Harbor Laboratory Press, 2001
- Current Protocols in Molecular Biology. John Wiley & Sons, 1989
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46

- **DEROSE et al.** *Plant Mol Biol,* 1996, vol. 32, 1029-35
- **LEAH et al.** *Plant J.,* 1994, vol. 4, 579-89
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318

- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **GASTEIGER E et al.** ExPASy: the proteomics server for in-depth protein knowledge and analysis. *Nucleic Acids Res,* 2003, vol. 31, 3784-3788
- **FIELD et al.** *Nature,* 1989, vol. 340 (6230), 245-246
- **JIAN-KANG ZHU.** *TRENDS in Plant Science,* February 2001, vol. 6 (2
- **RABBANI et al.** *Plant Physiology,* December 2003, vol. 133, 1755-1767
- **BENFEY et al.** *EMBO J.,* 1989, vol. 8, 2195-2202
- **FRANCK et al.** *Cell,* 1980, vol. 21, 285-294
- **WARD et al.** *Plant. Mol. Biol.,* 1993, 22
- **LEISNER.** *Proc Natl Acad Sci USA,* 1988, vol. 85 (5), 2553-2557
- **COMAI.** *Plant Mol Biol,* 1990, vol. 15 (3), 373-381
- **SCHENK.** *Plant Mol Biol,* 1999, vol. 39 (6), 1221-1230
- **JAIN et al.** *Crop Science,* 1999, vol. 39 (6), 1696-1701
- **GATZ et al.** *Plant J.,* 1992, vol. 2, 397-404
- **WARD et al.** *Plant. Mol. Biol.,* 1993, vol. 22, 361-366
- **BAEUMLEIN et al.** *Mol Gen Genet,* 1991, vol. 225 (3), 459-67
- **BAEUMLEIN et al.** *Plant Journal,* 1992, vol. 2 (2), 233-9
- **STOCKHAUS et al.** *EMBO J.,* 1989, vol. 8, 2445
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BEMATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet,* 1997, vol. 7, 22-28
- **DEAR ; COOK.** *Nucleic Acid Res.,* 1989, vol. 17, 6795-6807
- **SAMBROOK.** Molecular Cloning: a laboratory manual. Cold Spring Harbor Laboratory Press, 2001
- **AUSUBEL et al.** Current Protocols in Molecular Biology. Current Protocols, 1994, vol. 1, 2
- **R.D.D. CROY.** Plant Molecular Biology Labfax. BIOS Scientific Publications Ltd (UK) and Blackwell Scientific Publications (UK), 1993
- Agrobacterium Protocols. **AN, G.** Methods in Molecular Biology. Humana Press, vol. 44, 47-62
- **BEVAN.** *Nucleic Acid Research,* 1984, vol. 12, 8711-8721
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659